# EUROPEAN PATENT APPLICATION

(11) **EP 4 768 595 A1**
(43) Date of publication of application: **01.07.2026**
(21) Application number: 24223174.4
(22) Date of filing: 24.12.2024
(51) Int. Cl.: C12Q 1/66, G01N 33/564, G01N 33/74

(54) **ANTIGENS AND RELATED ASSAY TO DETECT AND MEASURE AUTO-ANTIBODIES IN A SUBJECT DEVELOPING TYPE 1 DIABETES (T1D)**

(71) Applicant: Fondazione Centro San Raffaele, 20132 Milano (IT); Ospedale San Raffaele S.r.l., 20132 Milano (IT); Università Vita-Salute San Raffaele, 20132 Milan (IT)
(72) Inventor: Lampasona, Vito, 20132 Milano (IT); Piemonti, Lorenzo, 20132 Milano (IT); Brigatti, Cristina, 20132 Milano (IT); Marzinotto, Ilaria, 20132 Milano (IT)
(74) Representative: Delbarba, Andrea

(57) **Abstract**

The present invention relates to a test kit for performing an assay to detect and measure at least one autoantibody molecule in a subject developing type 1 diabetes (T1D). The invention more specifically relates to a recombinant antigen molecule fused to a bioluminescent reporter protein to detect an autoantibody molecule against a self-antigen. Said self-antigen is preferably expressed by pancreatic islet beta cells and include insulin, proinsulin, Glutamate decarboxylase 65, islet antigen 2, and Zinc transporter 8. More specifically, the present invention relates to an assay comprising a set of at least three recombinant antigen molecules in a single composition, the antigen molecules being selected from the list comprising recombinant human Glutamate decarboxylase 65, recombinant human islet antigen 2, recombinant human Zinc transporter 8, recombinant human insulin, and recombinant human proinsulin. The invention further relates to methods for determining the presence and/or level of at least one autoantibody molecule in a sample from a human subject developing T1D using said recombinant antigen molecules. According to the invention, the presence, absence, or amount of one or more autoantibody molecules against said self-antigens is determined with a luciferase immune precipitation system (LIPS) assay or a modified version of a LIPS assay, i.e., a solid phase capture LIPS (scLIPS) assay.

## Description

### FIELD OF THE INVENTION

The present invention relates to a test kit for performing an assay to detect and measure at least one autoantibody molecule in a subject developing type 1 diabetes (T1D). The invention more specifically relates to a recombinant antigen molecule fused to a bioluminescent reporter protein to detect an autoantibody molecule against a self-antigen. Said self-antigen is preferably expressed by pancreatic islet beta cells and include insulin, proinsulin, Glutamate decarboxylase 65, islet antigen 2, and Zinc transporter 8. More specifically, the present invention relates to an assay comprising a set of at least three recombinant antigen molecules in a single composition, the antigen molecules being selected from the list comprising recombinant human Glutamate decarboxylase 65, recombinant human islet antigen 2, recombinant human Zinc transporter 8, recombinant human insulin, and recombinant human proinsulin. The invention further relates to methods for determining the presence and/or level of at least one autoantibody molecule in a sample from a human subject developing T1D using said recombinant antigen molecules. According to the invention, the presence, absence, or amount of one or more autoantibody molecules against said self-antigens is determined with a luciferase immune precipitation system (LIPS) assay or a modified version of a LIPS assay, i.e., a solid phase capture LIPS (scLIPS) assay.

### BACKGROUND

In type 1 diabetes (T1D), a dysfunctional immune system mounts a response against self-antigens expressed by pancreatic islet beta cells. Beta cells are the only cell type in the human body responsible for the expression and regulated secretion into the bloodstream of insulin, a key hormone regulating glucose metabolism. Eventually, the progressive destruction of beta cells by the immune system leads to a state of chronic insulin insufficiency and hyperglycemia that can be corrected only by the administration of exogenous insulin.

Because clinical symptoms of T1D usually present acutely, the disease onset is often associated with a variety of complications for patients (especially for children) with a heavy burden on patients, families, and the healthcare system. Importantly, despite the availability of insulin therapy for the last 100 years, T1D onset can still result in dramatic and even fatal consequences for the patients.

Moreover, insulin therapy is not a cure but a replacement therapy that must be sustained throughout life. Therefore, the battle against T1D will only be won when either the development of autoimmunity against the endocrine pancreas will be prevented or the attack of the immune system against insulin producing beta cells will at least be stopped or contained. Even partial success in this area would prove of tremendous benefit for both patients and society at large. In fact, the rising incidence of the disease over the past decades, together with the earlier age at diagnosis observed in numerous patients, implies that ever increasing numbers of subjects will develop T1D¹.

Four self-proteins expressed in pancreatic islet beta cells have been identified as the major targets of islets autoimmunity. These self-proteins are insulin, proinsulin, glutamic acid decarboxylase 65 kDa isoform, Islet antigen 2 (also known as ICA512), and the zinc transporter 8. Accordingly, the corresponding islet autoantibodies are referred to as IAA (Insulin Auto Antibodies), GADA (Glutamic Acid Decarboxylase Antibodies), IA-2A (Islet Antigen 2 Antibodies) and ZnT8A (Zinc Transporter 8 Antibodies), respectively. Islet autoantibodies are key markers of autoimmunity associated with T1D and are used in a variety of clinical and research settings that include the diagnostics of T1D², the prediction of future T1D development³, the use as prognostic markers in the context of pancreas⁴ or islet transplantation⁵, and as surrogate markers of efficacy in the context of experimental therapies aimed at T1D prevention⁶.

Several pilot studies are ongoing in which screening for islet autoantibodies is conducted in children or adults from the general population⁷. Frida is a such prototypical study in which over 165000 children were screened to diagnose pre-symptomatic T1D. After the detection of islet autoantibodies, educational and monitoring programs of children and their families are implemented, so that patients can begin insulin therapy early enough to prevent serious side effects at the onset of clinical disease. The study has already demonstrated that children diagnosed with pre-symptomatic T1D have milder diabetes at clinical onset⁸.

While the use of genetic risk screening might improve the efficiency of screening^{9,10}, all current screening (Fr1DA, ASK) or surveillance (TEDDY, GGPAD, DIPP, BABY DIAB, DAISY) programs require the conduction of hundreds of thousands if not millions of islet autoantibody (IA) tests. For example, the anti-CD3 trial depended on a very large and expensive network, where close relatives of current diabetes patients that have an increased risk of developing the disease are recruited. However, >90% of future patients do not have a close relative and tapping this much larger source of prediabetic individuals is key for the timely offer of therapy and the further conduction of additional prevention trials. Identifying persons with pre-diabetes among the general population is therefore necessary. Nevertheless, only a fraction of individuals tested positive for single islet autoantibodies with current assays will progress to overt disease. Hence, there is a requirement for measuring several different islet autoantibodies since this can identify individuals at high risk (>50%) of progression more efficiently and in a relatively short time frame. Multiplex formats of ELISA, ECL, and ADAP assays in the context of T1D have been described^{11,12,13}.

Currently, the strategy adopted from the German Frida study⁸, the largest ongoing scale population screening program, is based on a two-stage autoantibody screening strategy for pre-type 1 diabetes. This strategy consists in initial stage (3-screen stage) using a three-screen test that checks for the presence of autoantibodies commonly associated with T1D. These autoantibodies include Glutamic Acid Decarboxylase Autoantibodies (GADA), insulinoma antigen 2 autoantibodies (IA-2A) and Zinc Transporter 8 autoantibodies (ZnT8A). The test is designed to be highly sensitive and individuals who test positive are selected for the second stage of the screening. In the second stage, individuals who tested positive in the first stage undergo a more detailed analysis using individual autoantibody assays. This step is crucial for determining the antigen specificity and titers of the autoantibodies. The goal is to identify subjects who are positive for multiple autoantibodies, as this significantly increases the risk of progression to T1D. This two-stage approach allows for efficient use of resources by initially screening a broad population with a less expensive test, followed by more costly and detailed testing for those at apparent risk.

Compared to common humoral responses, like for example those against pathogens or vaccines, the measurement of islet autoantibodies has proved often problematic. The consensus is that islet autoantibodies are often very low in titer and mostly directed against conformational epitopes. Therefore, islet autoantibody measurement requires very sensitive assays based on formats able to preserve the overall structure of the target antigens. These reasons explain the emergence of Radio Binding Assays (RBAs) as the de facto gold standard assay for islet autoantibody measurement¹⁴.

In RBA, recombinant radiolabeled antigens are used as ligands in liquid phase to immunoprecipitate serum islet autoantibodies. However, the use of radio-chemicals has become a liability, which links in a downward spiral the need to comply with strict regulations, the logistic difficulties of their use and disposal, increasing costs, and the likely problematic availability of these reagents in the future. For these reasons, RBAs are ill suited to the very large-scale screening of the general population currently under consideration or for new longitudinal studies of islet autoimmunity.

Bridge-ELISA assays^{15,16} have shown a robust performance in detecting islet autoantibodies in serum samples, with one notable exception: insulin autoantibodies (IAA). IAA measurement might be important for screening programs, as they often are the first detectable biomarker of islet autoimmunity to appear. Following early studies which showed that IAA measured by RBA were more closely associated with T1D than those measured by simple ELISA¹⁷, IAA are still measured by RBA in the vast majority of laboratories. Currently, no reliable bridge-ELISAs for IAA have been brought to the market.

Other more recent non-radioisotopic assays for islet autoantibody detection have also shown a competitive performance, based either on the Electro Chemi Luminescent (ECL)^{18,19,20} or the

Antibody Dependent Agglutination PCR (ADAP)²¹ formats. Among the known limitations of these alternative non-radioisotopic assays is their requirement for serum volumes several times larger than those of RBAs or the need of dedicated platforms. This comparatively large consumption of serum, relatively innocuous in a conventional diagnostic setting, constitutes a severe drawback to screening studies where serum (especially from young children) is usually collected by capillary sampling at home. Use of large volume assays for islet autoantibody measurement also squanders precious samples collected for cohort studies that could otherwise be employed for answering a wide range of scientific questions. Therefore, practical considerations and the need to use samples judiciously, mandates that islet autoantibody tests consume the lowest possible amount of serum. Furthermore, both the ECL and ADAP formats are not implementable on platforms that find common application in current high throughput diagnostic settings. In summary, better assays for islet autoantibodies are therefore required to address the issues of high assay costs, high serum consumption, and inadequate performance.

Luciferase immune precipitation system (LIPS) is an assay format that, while similar to the gold standard RBA in its basic principles, dispenses with the need for radioactive tracers. LIPS offers reduced serum consumption, faster execution time and has been successfully applied to the measurement of islet autoantibodies. Classical luciferase immune precipitation system (LIPS) assays using Firefly and Renilla luciferases have been used to test for islet autoantibodies to the self-proteins glutamic acid decarboxylase 65 (GAD65) and insulinoma-associated protein (IA)-2β^{22,23}.

More recently, a commercially available small but highly processive NanoLuc^{™24} nanoluciferase reporter protein replacing the originally described Firefly and Renilla luciferases (cf. Ref. 19) have been expressed in a variety of recombinant T1D-associated antigen constructs. For example, such nanoluciferase reporter protein has been used in LIPS assay to detect islet autoantibodies to zinc transporter 8²⁵, GADA65²⁶, or insulin²⁷.

However, the ability of assays to discriminate between health and disease still varies widely even amongst established assays. For large screening studies in the general population, where the individual risk of T1D is low, it is particularly important that assays achieve high specificity, to avoid unnecessary anxiety and inconvenience to those found positive for islet autoantibodies. Furthermore, to achieve the greatest benefit, screening should be performed in young children, who would be selected for follow-up even if found positive for a single islet autoantibody.

In summary, there is currently a need for robust, sensitive, specific, and cheap high-throughput assays for the detection of islet autoantibodies.

### SUMMARY OF THE INVENTION

The present invention addresses the above-mentioned needs and provides a test kit for performing an assay, the assay comprising a set of at least three recombinant antigen molecules in a single composition. In a preferred aspect, the antigen molecules are selected from the group comprising recombinant antigen molecules selected from the group comprising recombinant human Glutamate decarboxylase 65, recombinant human islet antigen 2, recombinant human Zinc transporter 8, recombinant human insulin, and recombinant human proinsulin. In another preferred aspect, the antigen molecules are selected from the group consisting of recombinant antigen molecules selected from the group comprising recombinant human Glutamate decarboxylase 65, recombinant human islet antigen 2, recombinant human Zinc transporter 8, recombinant human insulin, and recombinant human proinsulin. In one further aspect, the recombinant antigen molecules are fused to a bioluminescent reporter protein. According to the invention, the test kit also comprises a substrate for the bioluminescent reporter protein.

The recombinant antigen molecules according to the invention can be used for the diagnosis of type 1 diabetes in human patients. It is expected that said use of the recombinant antigen molecules will improve the rapidity and the accuracy of detection of islet autoantibodies due to the assay multiplexing, their specificity, and sensitivity.

Thus, the recombinant antigen molecules according to the invention are particularly suitable for binding to an autoantibody molecule, specifically an islet autoantibody molecule, more specifically an autoantibody with specificity to human insulin, human proinsulin, human Glutamate decarboxylase 65, human islet antigen 2, or human Zinc transporter 8, further optionally wherein the antibody molecule is at least one islet autoantibody selected from the group comprising islet autoantibodies IAA (Insulin Auto Antibodies), GADA (Glutamic Acid Decarboxylase Antibodies), IA-2A (Islet Antigen 2 Antibodies), and ZnT8A (Zinc Transporter 8 Antibodies). Preferably, the binding of the autoantibody molecule to at least one recombinant antigen molecule according to the invention is binding in a liquid phase detection assay. This is particularly advantageous compared to other detection assays, e.g., such as ELISA, as the structure of the recombinant antigen, i.e., the tertiary structure of the recombinant antigen, is preserved.

The recombinant antigen molecules according to the invention can be used in a luciferase immune precipitation system (LIPS) assay or a modified version of the LIPS assay, herein referred to as a solid phase capture LIPS (scLIPS) assay.

The present invention further provides a set of nucleic acids encoding the recombinant antigen molecules of the test kit. Preferably, each recombinant antigen molecule is encoded by a separate nucleic acid.

The present invention also relates a vector encoding for:
a) a recombinant human insulin antigen molecule comprising a nucleotide sequence at least 85% identical to a nucleotide sequence selected from the group consisting of SEQ ID NOs: 1, 2, 10, 11, and 22, at least 90% identical to a nucleotide sequence selected from the group consisting of SEQ ID NOs: 1, 2, 10, 11, and 22, at least 95% identical to a nucleotide sequence selected from the group consisting of SEQ ID NOs: 1, 2, 10, 11, and 22, at least 98% identical to a nucleotide sequence selected from the group consisting of SEQ ID NOs: 1, 2, 10, 11, and 22, at least 99% identical to a nucleotide sequence selected from the group consisting of SEQ ID NOs: 1, 2, 10, 11, and 22, identical to a nucleotide sequence selected from the group consisting of SEQ ID NOs: 1, 2, 10, 11, and 22, optionally wherein the recombinant human insulin antigen molecule comprises a nucleotide sequence at least 85% identical to a nucleotide sequence selected from SEQ ID NOs: 1 or 2, at least 90% identical to a nucleotide sequence selected from SEQ ID NOs: 1 or 2, at least 95% identical to a nucleotide sequence selected from SEQ ID NOs: 1 or 2, at least 98% identical to a nucleotide sequence selected from SEQ ID NOs: 1 or 2, at least 99% identical to a nucleotide sequence selected from SEQ ID NOs: 1 or 2, identical to a nucleotide sequence selected from SEQ ID NOs: 1 or 2;
b) a recombinant human proinsulin antigen molecule comprises a nucleotide sequence at least 85% identical to a nucleotide sequence selected from the group consisting of SEQ ID NOs: 8 and 9, at least 90% identical to a nucleotide sequence selected from the group consisting of SEQ ID NOs: 8 and 9, at least 95% identical to a nucleotide sequence selected from the group consisting of SEQ ID NOs: 8 and 9, at least 98% identical to a nucleotide sequence selected from the group consisting of SEQ ID NOs: 8 and 9, at least 99% identical to a nucleotide sequence selected from the group consisting of SEQ ID NOs: 8 and 9, identical to a nucleotide sequence selected from the group consisting of SEQ ID NOs: 8 and 9;
c) a recombinant human Glutamate decarboxylase 65 antigen molecule comprising a nucleotide sequence at least 85% identical to a nucleotide sequence selected from the group consisting of SEQ ID NOs: 3, 7, and 16 to 18, at least 90% identical to a nucleotide sequence selected from the group consisting of SEQ ID NOs: 3, 7, and 16 to 18, at least 95% identical to a nucleotide sequence selected from the group consisting of SEQ ID NOs: 3, 7, and 16 to 18, at least 98% identical to a nucleotide sequence selected from the group consisting of SEQ ID NOs: 3, 7, and 16 to 18, at least 99% identical to a nucleotide sequence selected from the group consisting of SEQ ID NOs: 3, 7, and 16 to 18, identical to a nucleotide sequence selected from the group consisting of SEQ ID NOs: 3, 7, and 16 to 18, optionally wherein the recombinant human Glutamate decarboxylase 65 antigen molecule comprises a nucleotide sequence at least 85% identical to the nucleotide sequence encoded by SEQ ID NO: 3, at least 90% identical to the nucleotide sequence encoded by SEQ ID NO: 3, at least 95% identical to the nucleotide sequence encoded by SEQ ID NO: 3, at least 98% identical to the nucleotide sequence encoded by SEQ ID NO: 3, at least 99% identical to the nucleotide sequence encoded by SEQ ID NO: 3, identical to the nucleotide sequence encoded by SEQ ID NO: 3;
d) a recombinant human islet antigen 2 antigen molecule comprising a nucleotide sequence at least 85% identical to a nucleotide sequence selected from the group consisting of SEQ ID NOs: 4, 14, 15, and 19, at least 90% identical to a nucleotide sequence selected from the group consisting of SEQ ID NOs: 4, 14, 15, and 19, at least 95% identical to a nucleotide sequence selected from the group consisting of SEQ ID NOs: 4, 14, 15, and 19, at least 98% identical to a nucleotide sequence selected from the group consisting of SEQ ID NOs: 4, 14, 15, and 19, at least 99% identical to a nucleotide sequence selected from the group consisting of SEQ ID NOs: 4, 14, 15, and 19, identical to a nucleotide sequence selected from the group consisting of SEQ ID NOs: 4, 14, 15, and 19, optionally wherein the recombinant human islet antigen 2 antigen molecule comprises a nucleotide sequence at least 85% identical to the nucleotide sequence encoded by SEQ ID NO: 4, at least 90% identical to the nucleotide sequence encoded by SEQ ID NO: 4, at least 95% identical to the nucleotide sequence encoded by SEQ ID NO: 4, at least 98% identical to the nucleotide sequence encoded by SEQ ID NO: 4, at least 99% identical to the nucleotide sequence encoded by SEQ ID NO: 4, identical to the nucleotide sequence encoded by SEQ ID NO: 4; or
e) a recombinant human Zinc transporter 8 antigen molecule comprising a nucleotide sequence at least 85% identical to a nucleotide sequence selected from the group consisting of SEQ ID NOs: 5, 6, 12, 13, and 20, at least 90% identical to a nucleotide sequence selected from the group consisting of SEQ ID NOs: 5, 6, 12, 13, and 20, at least 95% identical to a nucleotide sequence selected from the group consisting of SEQ ID NOs: 5, 6, 12, 13, and 20, at least 98% identical to a nucleotide sequence selected from the group consisting of SEQ ID NOs: 5, 6, 12, 13, and 20, at least 99% identical to a nucleotide sequence selected from the group consisting of SEQ ID NOs: 5, 6, 12, 13, and 20, identical to a nucleotide sequence selected from the group consisting of SEQ ID NOs: 5, 6, 12, 13, and 20, optionally wherein the recombinant human Zinc transporter 8 antigen molecule comprises a nucleotide sequence at least 85% identical to the nucleotide sequence encoded by SEQ ID NOs: 5 or 6, at least 90% identical to the nucleotide sequence selected from SEQ ID NOs: 5 or 6, at least 95% identical to the nucleotide sequence selected from SEQ ID NOs: 5 or 6, at least 98% identical to the nucleotide sequence selected from SEQ ID NOs: 5 or 6, at least 99% identical to the nucleotide sequence selected from SEQ ID NOs: 5 or 6, identical to the nucleotide selected from SEQ ID NOs: 5 or 6.

In addition, the present invention provides a cell comprising the set of nucleic acids, or a nucleic acid, or a vector, and expressing the recombinant antigen molecules. In one embodiment, the recombinant antigen coding sequence is inserted into a lentiviral vector. Preferably, the cell is a stably expressing transfectant cell line, such as an Expi-293F cell line.

Additionally, the present invention also provides a method for producing the recombinant antigen molecules according to the present invention, the method comprising expressing said nucleic acids or said vector in a cell according to the present invention, and harvesting said recombinant antigen molecules.

In a further aspect, the present invention provides an *in vitro* method for determining the presence and/or level of an antibody molecule in a sample. In one aspect, the sample is a blood sample, a serum sample, or a plasma sample obtained from a human subject.

The *in vitro* method comprises the steps of:
a) contacting the sample with the set of at least three recombinant antigen molecules, wherein a complex between the recombinant antigen molecule and the autoantibody molecule (referred to as "antigen molecule-autoantibody complex") is formed;
b) removing the unbound recombinant antigen molecules;
c) contacting the recombinant antigen molecule-autoantibody complex with a protein;
d) contacting the bioluminescence reporter protein covalently linked to the recombinant antigen molecule with the substrate; and
e) determining the presence, absence, and/or amount of one or more autoantibody molecules in the sample that binds to one or more recombinant antigen molecules.

The presence, absence, or amount of one or more autoantibody molecules in the sample is determined by measuring the bioluminescence activity of the bioluminescent reporter molecule.

The at least one autoantibody molecule is against a self-antigen. Preferably, the at least one autoantibody molecule is an islet autoantibody molecule. More preferably, the islet autoantibody molecule has specificity to human insulin, human proinsulin, human Glutamate decarboxylase 65, human islet antigen 2, or human Zinc transporter 8. Most preferably, the islet autoantibody molecule is selected from the group comprising or consisting of islet autoantibodies IAA (Insulin Auto Antibodies), GADA (Glutamic Acid Decarboxylase Antibodies), IA-2A (Islet Antigen 2 Antibodies), and ZnT8A (Zinc Transporter 8 Antibodies).

The invention is illustrated by means of non-limiting examples referring to the following figures:

### BRIEF DESCRIPTION OF THE FIGURES

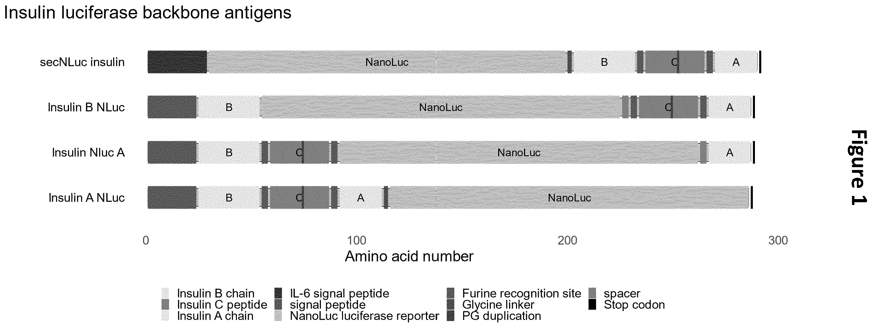

**Figure 1****:** Schematics of insulin luciferase backbone antigens.

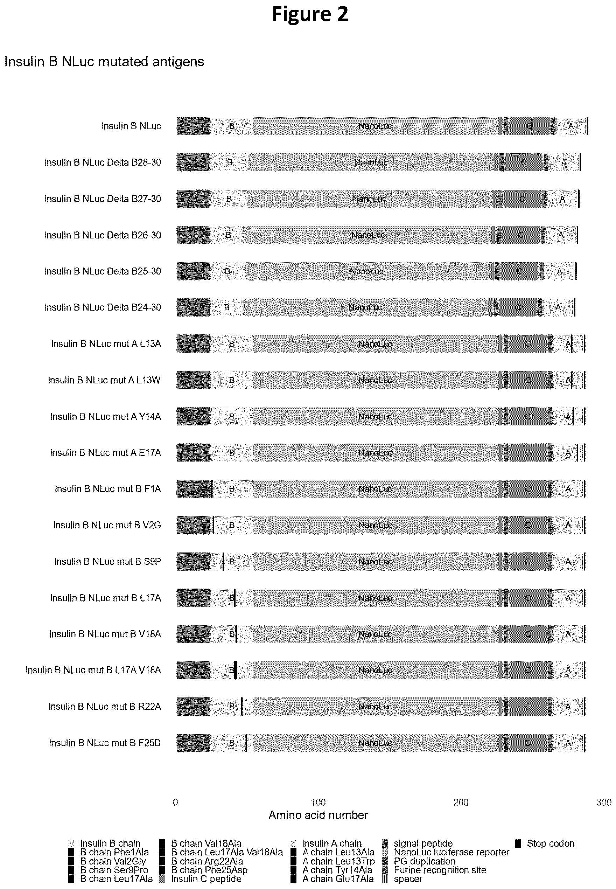

**Figure 2****:** Schematics of insulin B NLuc mutated antigens.

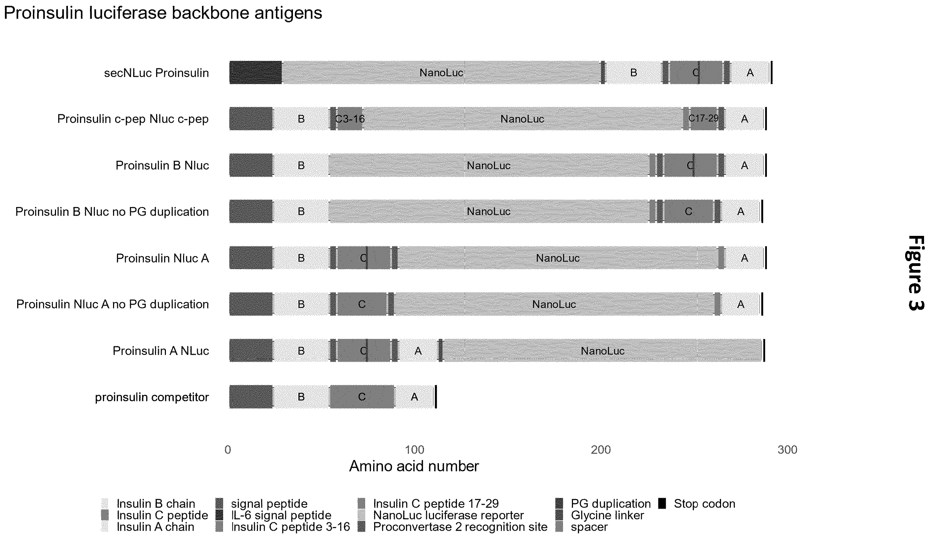

**Figure 3****:** Schematics of proinsulin luciferase backbone antigens.

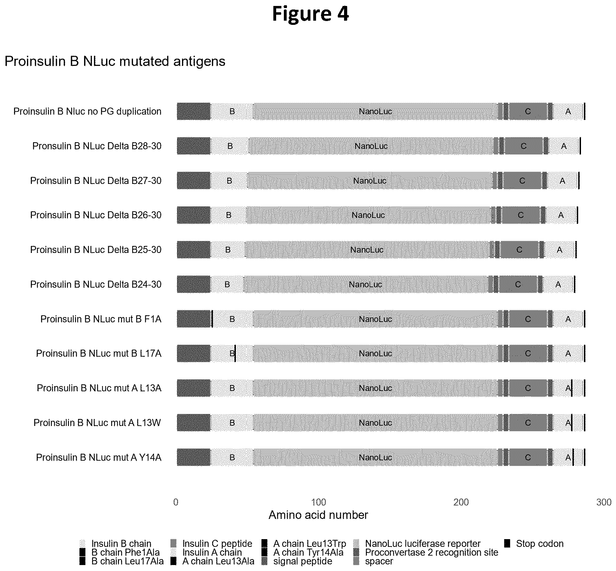

**Figure 4****:** Schematics of proinsulin B NLuc mutated antigens.

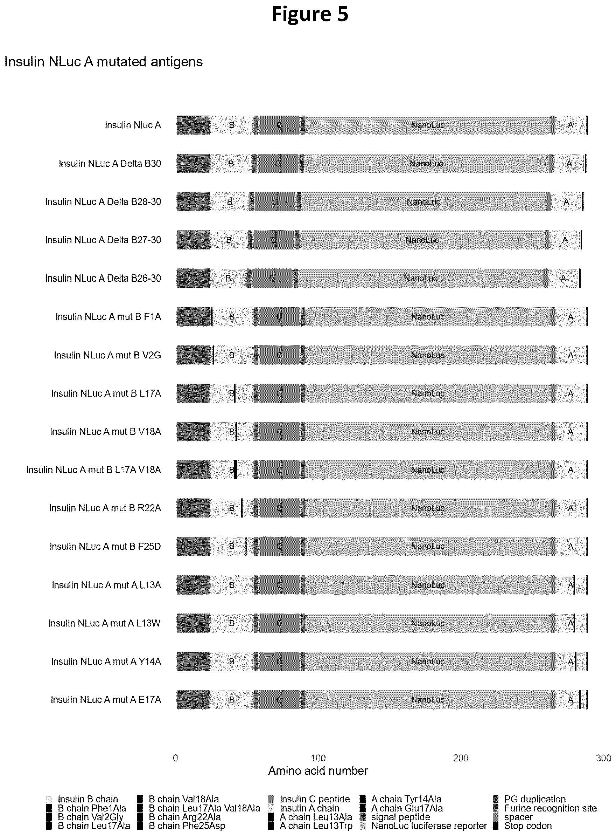

**Figure 5****:** Schematics of insulin NLuc A mutated antigens.

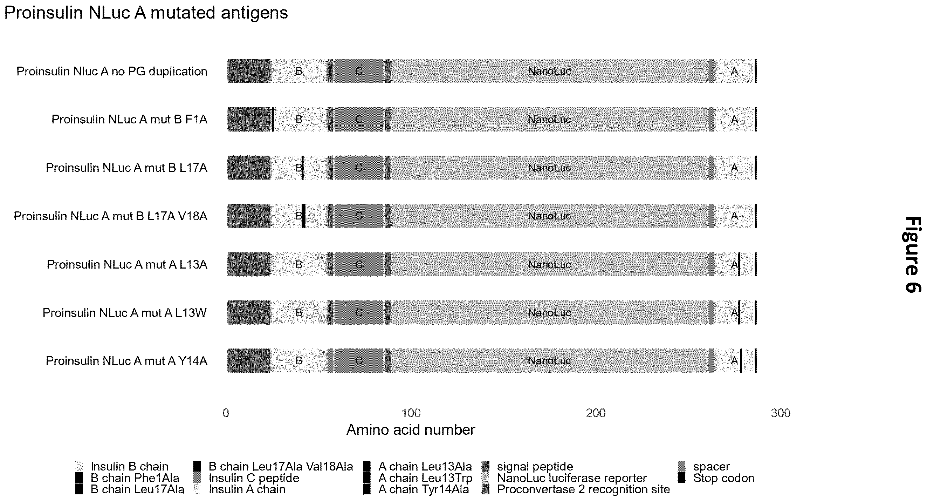

**Figure 6****:** Schematics of proinsulin NLuc A mutated antigens.

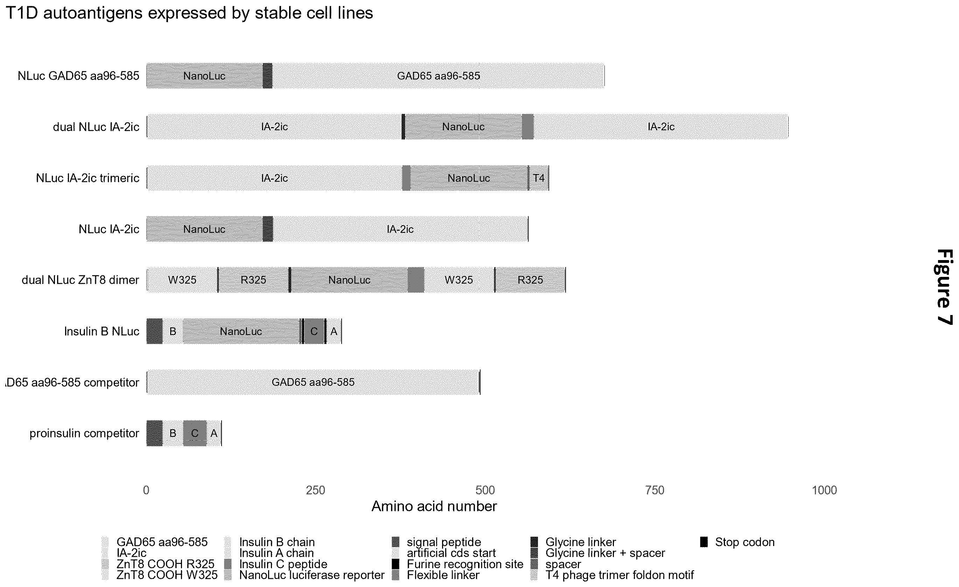

**Figure 7****:** Schematics of NanoLuc tagged T1D autoantigens expressed by stable cell lines.

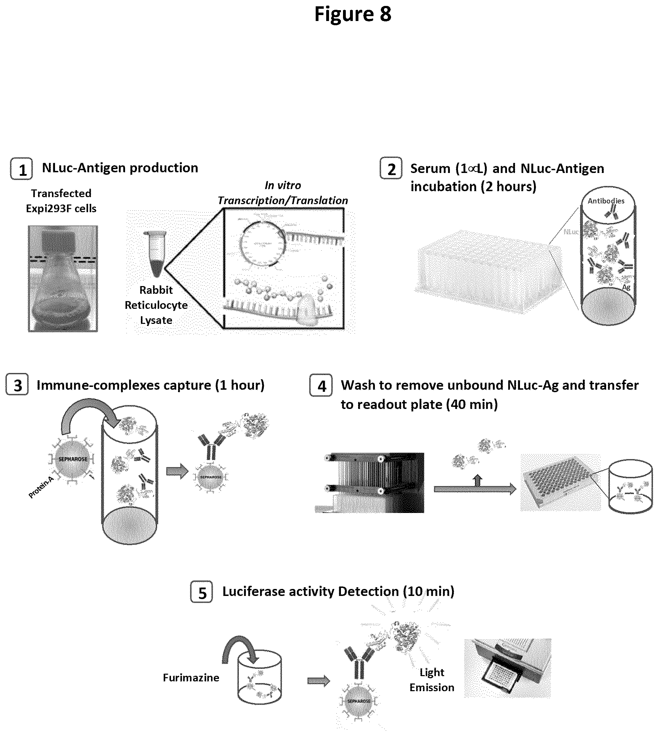

**Figure 8****:** Schematics of the luciferase immune precipitation system (LIPS).

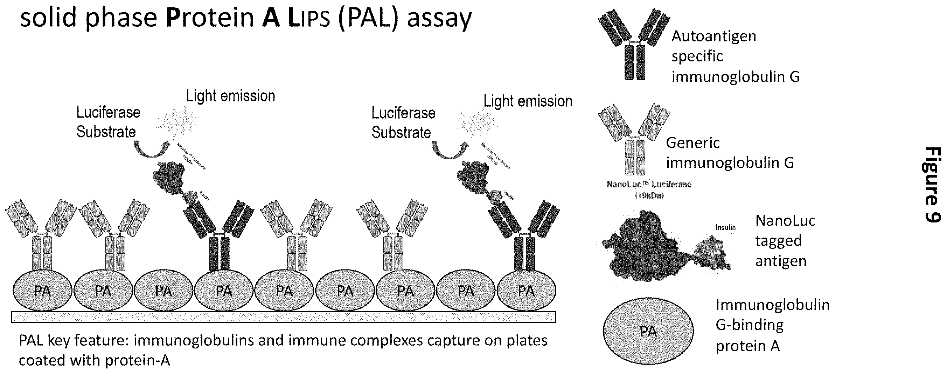

**Figure 9****:** Schematics of the solid phase capture LIPS (scLIPS) assay, also referred to as solid phase Protein A LIPS (PAL). LIPS refers to luciferase immune precipitation system (LIPS). The key features of the scLIPS assay comprise immunoglobulins and immune complexes are captured in plates coated with protein-A. Luciferase-tagged recombinant antigen molecules bound to islets autoantigens are detected after the addition of luciferase substate and recording of the ensuing light emission.

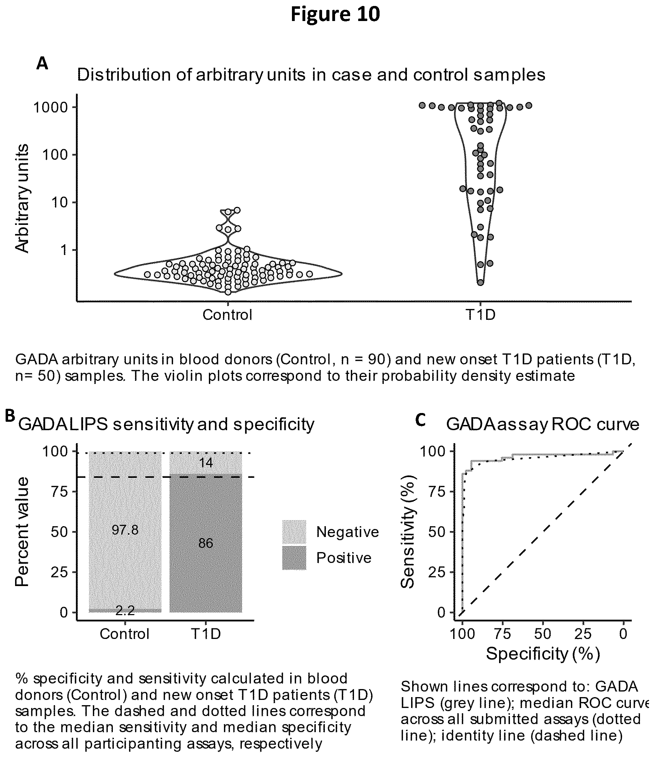

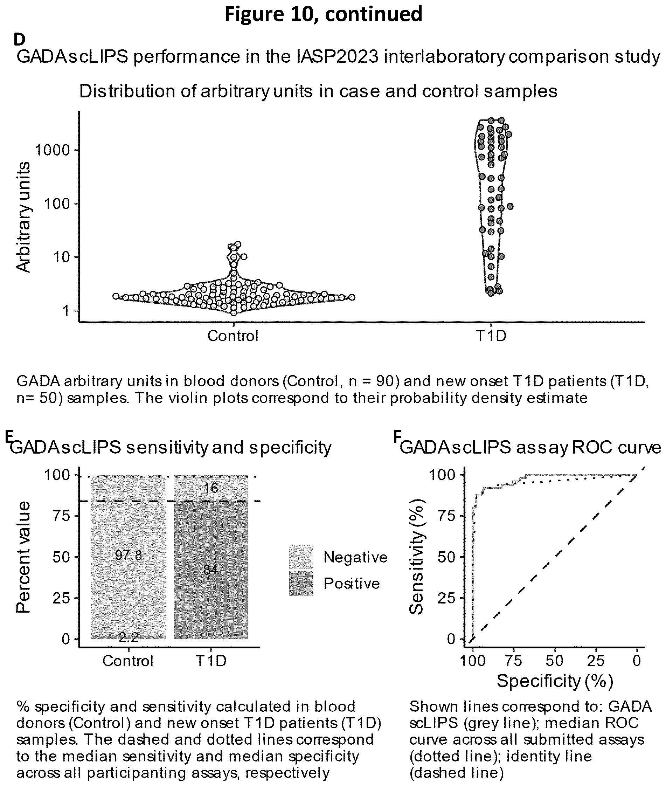

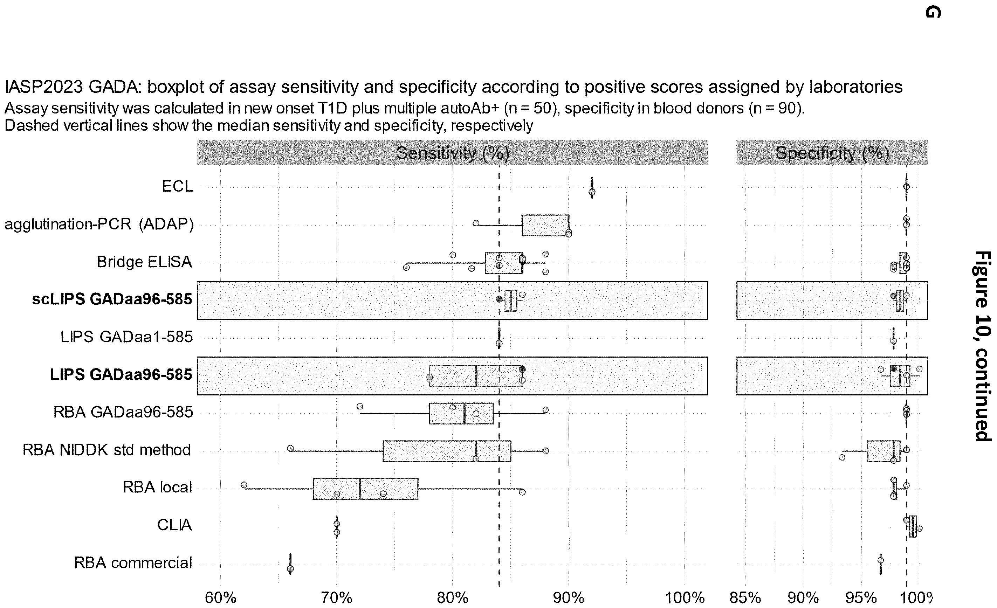

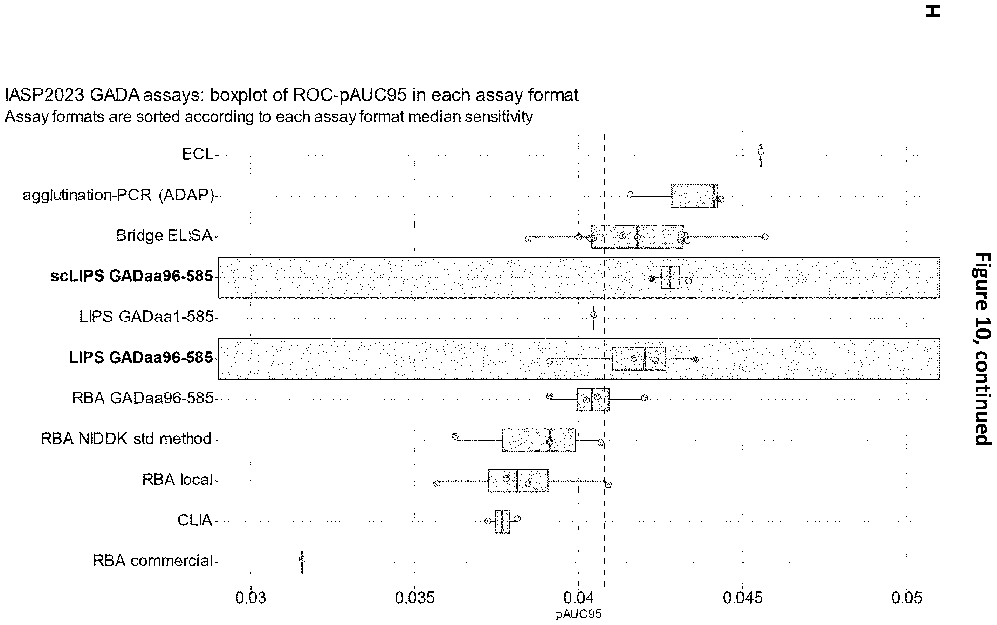

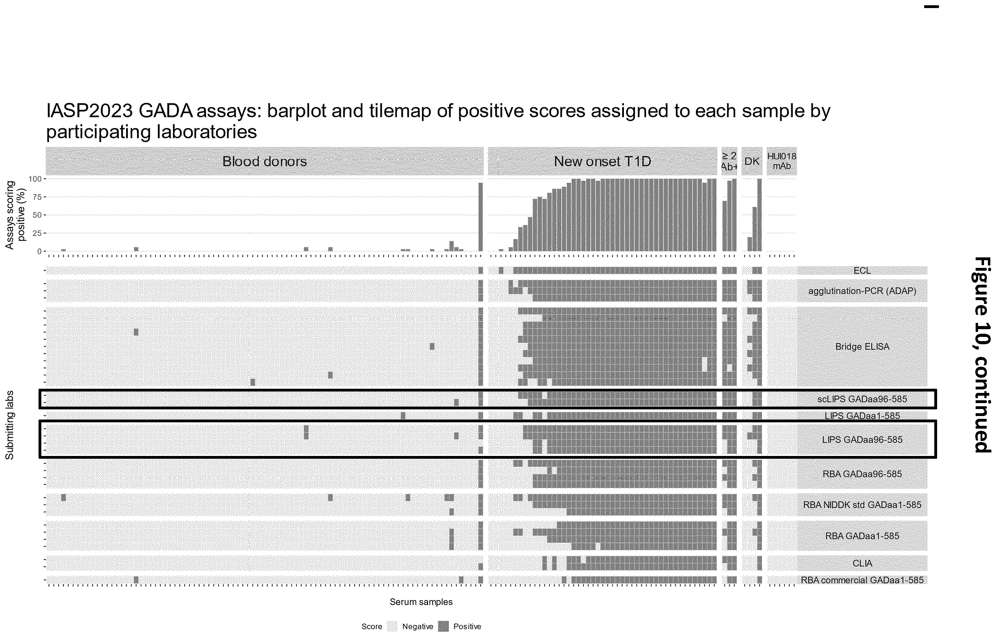

**Figure 10****: GADA LIPS and scLIPS performance in the IASP2023 interlaboratory comparison study. (A) Distribution of arbitrary units in case and control samples in GADA LIPS.** GADA arbitrary units in blood donors (control, n = 90) and new onset T1D patients (T1D, n= 50) samples. The violin plots correspond to their probability density estimate. **(B) GADA LIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(C) GADA assay ROC curve in LIPS.** Shown lines correspond to: GADA LIPS (grey lines); median ROC curve across all submitted assays (dotted line); identity line (dashed line). **(D) Distribution of arbitrary units in case and control samples in GADA scLIPS.** GADA arbitrary units in blood donors (control, n = 90) and new onset T1D patients (T1D, n= 50) samples. The violin plots correspond to their probability density estimate. **(E) GADA scLIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(F) GADA assay ROC curve in scLIPS.** Shown lines correspond to: GADA scLIPS (grey lines); median ROC curve across all submitted assays (dotted line); identity line (dashed line). (G) Boxplot of assay sensitivity and specificity according to positive scores assigned by laboratories. (H) Boxplot of ROC-pAUC95 across all submitted assays. **(I)** Boxplot and tilemap of positive scores assigned to each sample across all submitted assays.

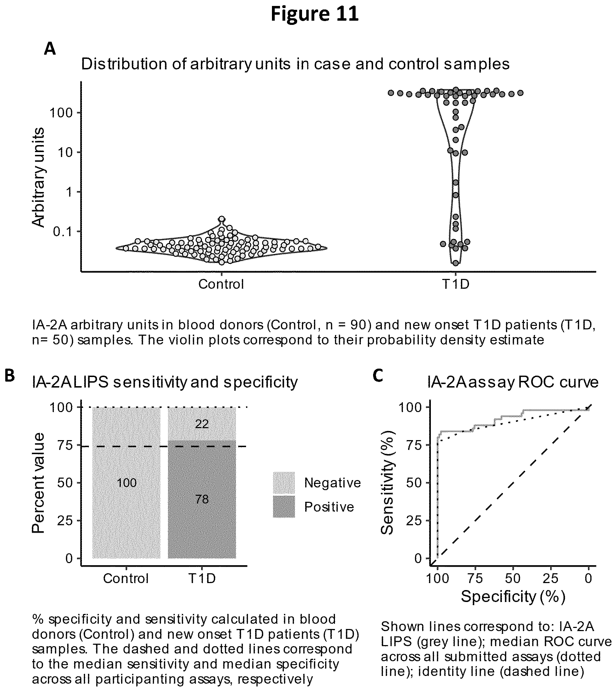

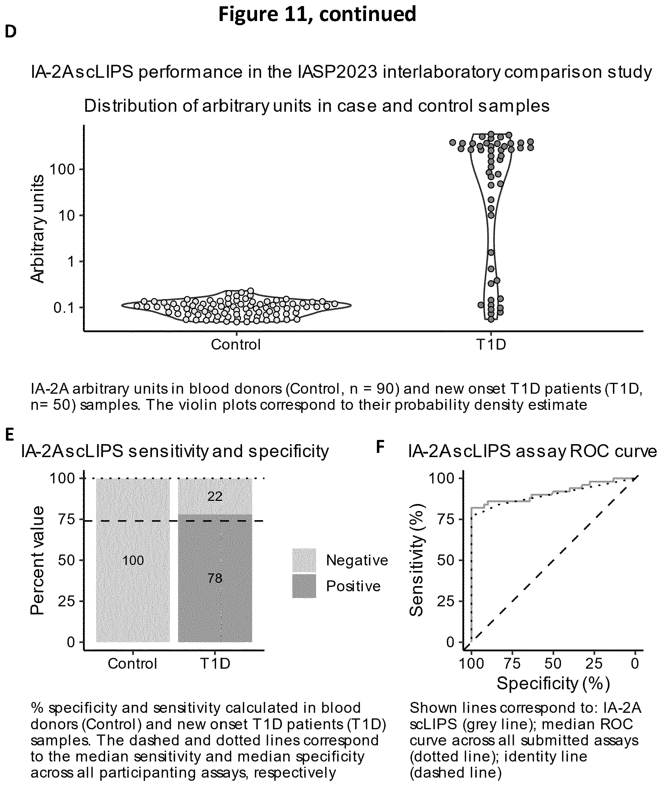

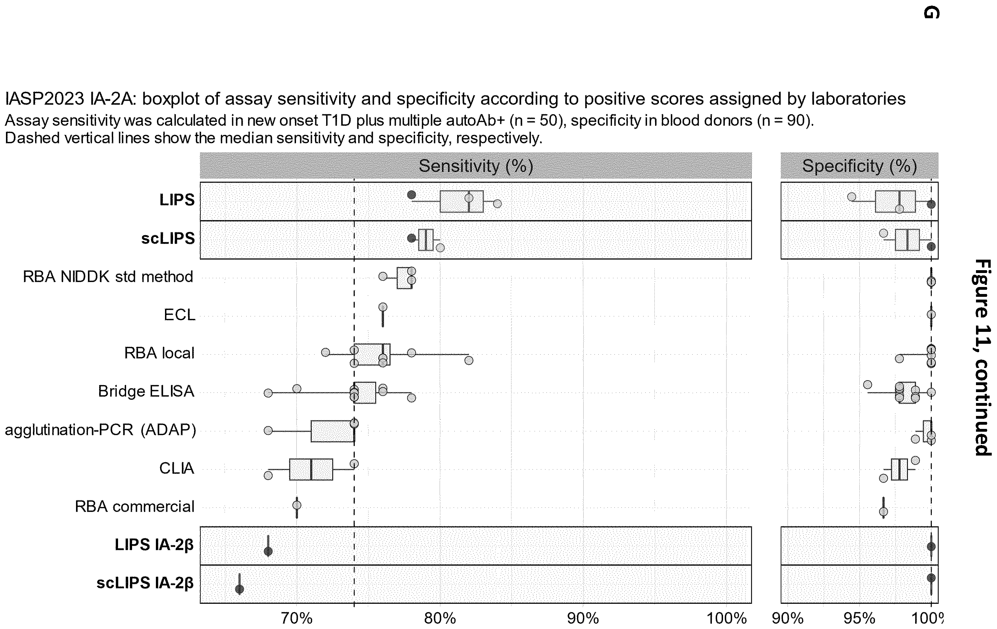

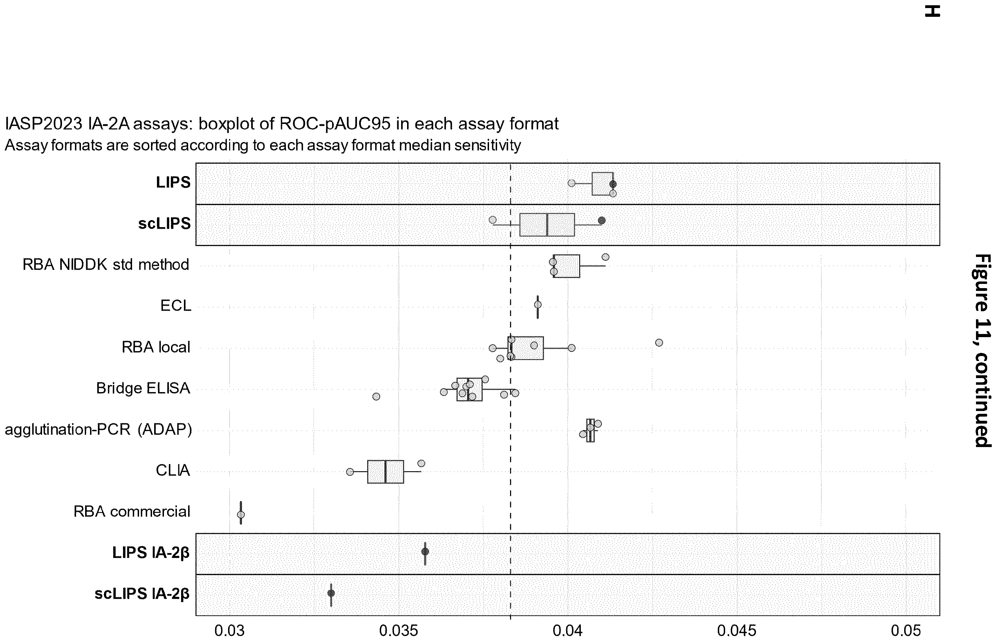

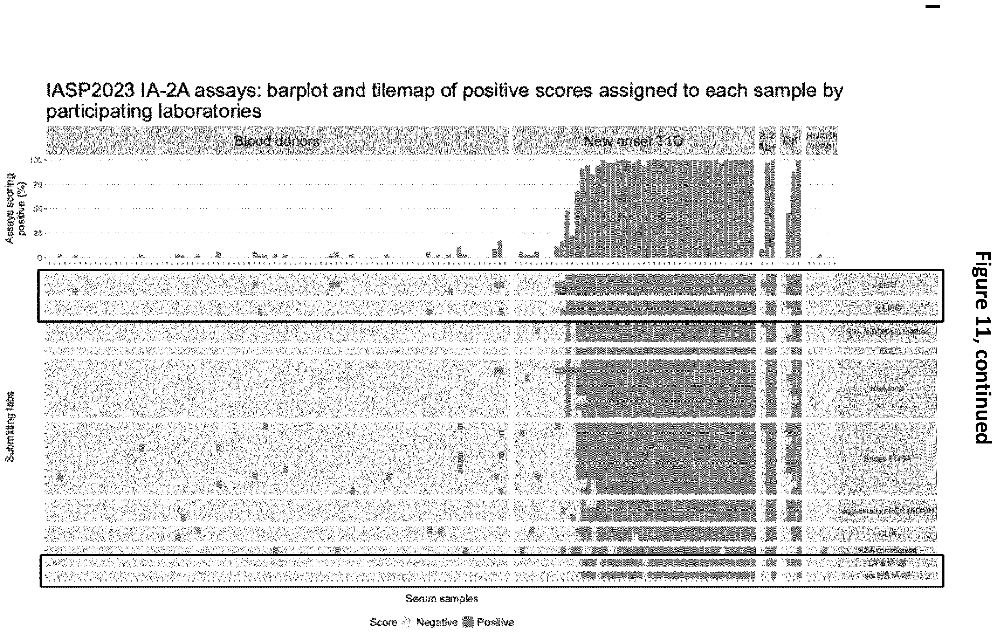

**Figure 11****: IA-2A LIPS and scLIPS performances in the IASP2023 interlaboratory comparison study. (A) Distribution of arbitrary units in case and control samples in IA-2A LIPS.** IA-2A arbitrary units in blood donors (control, n = 90) and new onset T1D patients (T1D, n= 50) samples. The violin plots correspond to their probability density estimate. **(B) IA-2A LIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(C) IA-2A assay ROC curve in LIPS.** Shown lines correspond to: IA-2A LIPS (grey lines); median ROC curve across all submitted assays (dotted line); identity line (dashed line). **(D) Distribution of arbitrary units in case and control samples in IA-2A scLIPS.** IA-2A arbitrary units in blood donors (control, n = 90) and new onset T1D patients (T1D, n= 50) samples. The violin plots correspond to their probability density estimate. **(E) IA-2A scLIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(F) IA-2A assay ROC curve in scLIPS.** Shown lines correspond to: IA-2A scLIPS (grey lines); median ROC curve across all submitted assays (dotted line); identity line (dashed line). **(G)** Boxplot of assay sensitivity and specificity according to positive scores assigned by laboratories. **(H)** Boxplot of ROC-pAUC95 across all submitted assays. **(I)** Boxplot and tilemap of positive scores assigned to each sample across all submitted assays.

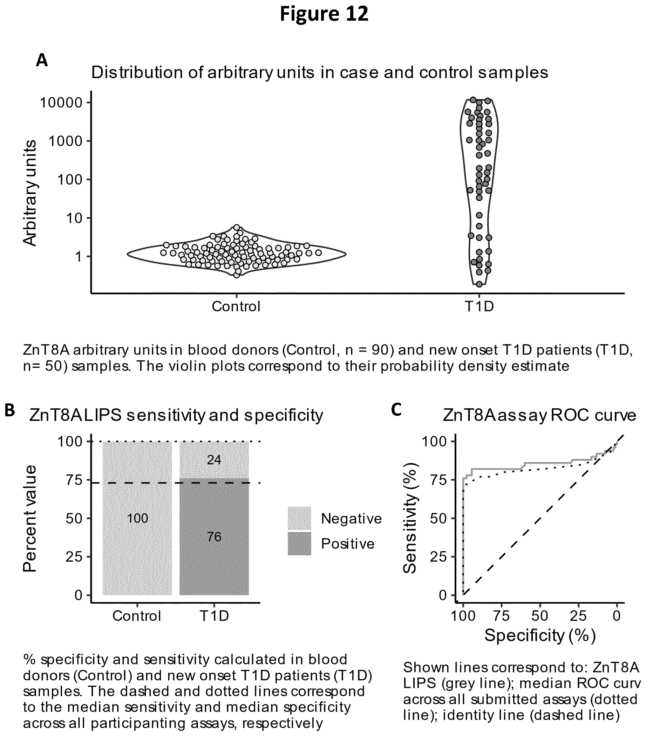

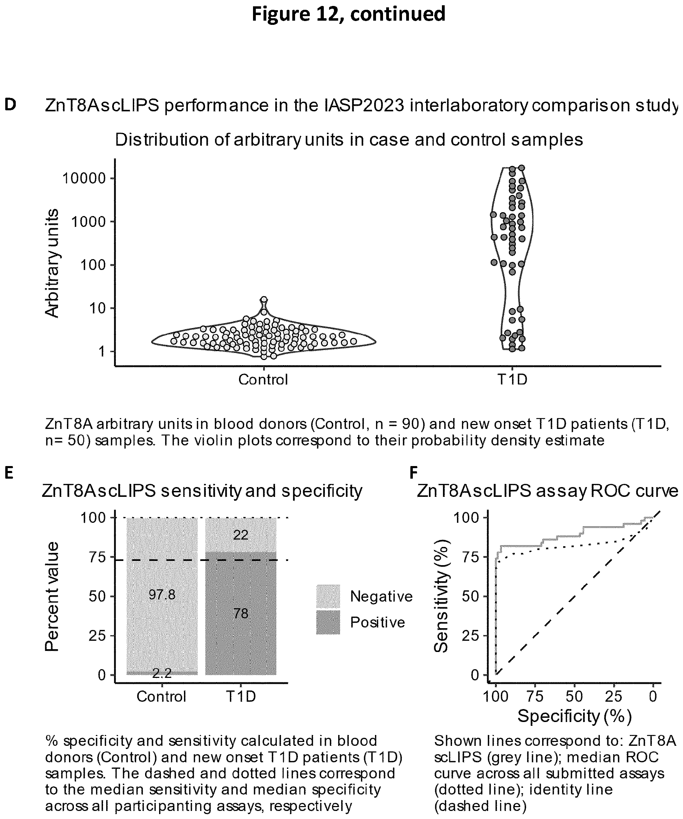

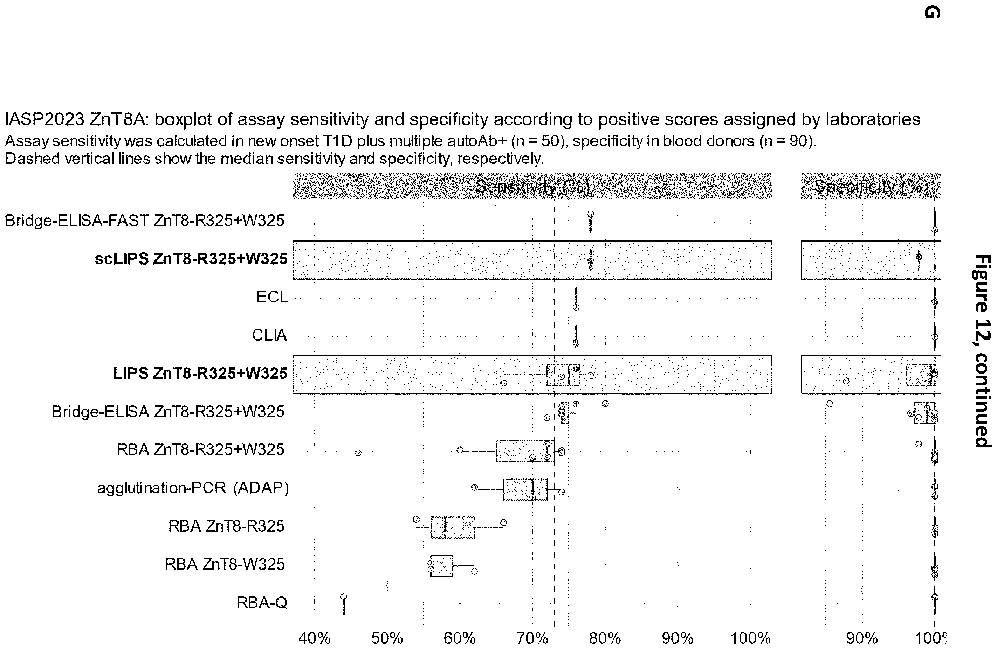

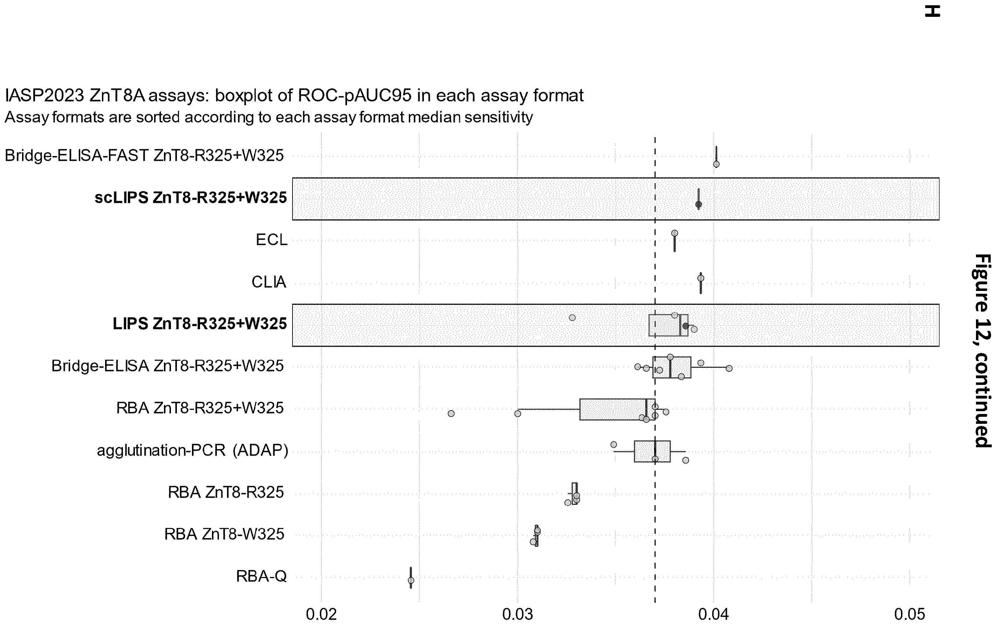

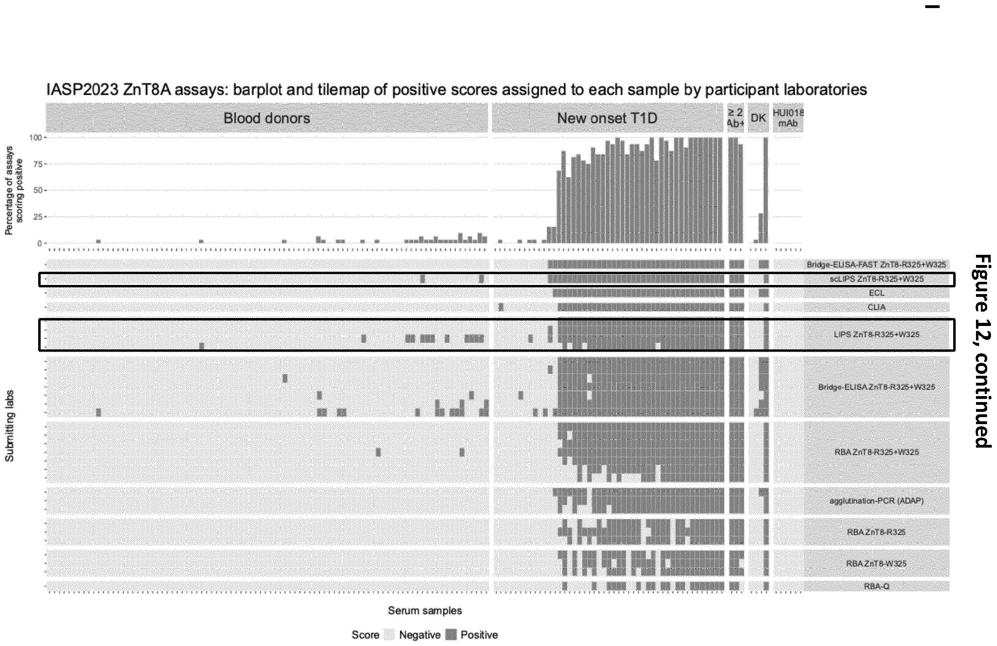

**Figure 12****: ZnT8A LIPS and scLIPS performances in the IASP2023 interlaboratory comparison study. (A) Distribution of arbitrary units in case and control samples.** ZnT8A arbitrary units in blood donors (control, n = 90) and new onset T1D patients (T1D, n= 50) samples. The violin plots correspond to their probability density estimate. **(B) ZnT8A LIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(C) ZnT8A assay ROC curve.** Shown lines correspond to: ZnT8A LIPS (grey lines); median ROC curve across all submitted assays (dotted line); identity line (dashed line). **(D) Distribution of arbitrary units in case and control samples.** ZnT8A arbitrary units in blood donors (control, n = 90) and new onset T1D patients (T1D, n= 50) samples. The violin plots correspond to their probability density estimate. **(E) ZnT8A scLIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(F) ZnT8A assay ROC curve.** Shown lines correspond to: ZnT8A scLIPS (grey lines); median ROC curve across all submitted assays (dotted line); identity line (dashed line). **(G)** Boxplot of assay sensitivity and specificity across the submitted assay formats. **(H)** Boxplot of ROC-pAUC95 across all submitted assays. **(I)** Boxplot and tilemap of positive scores assigned to each sample across all submitted assays.

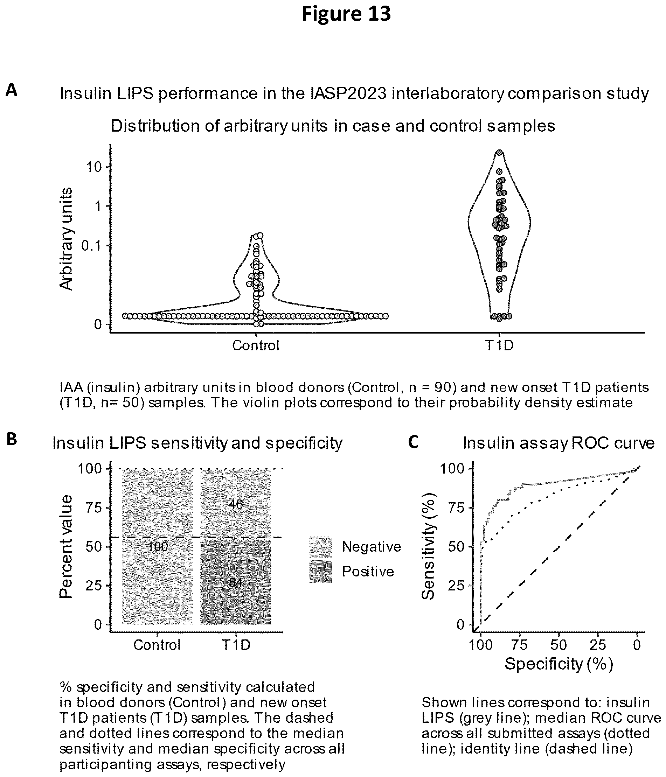

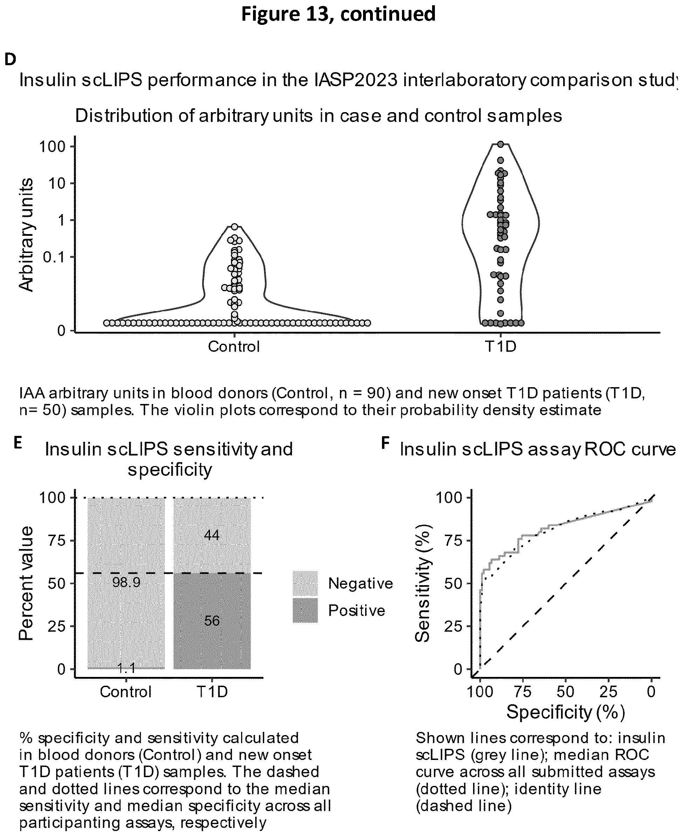

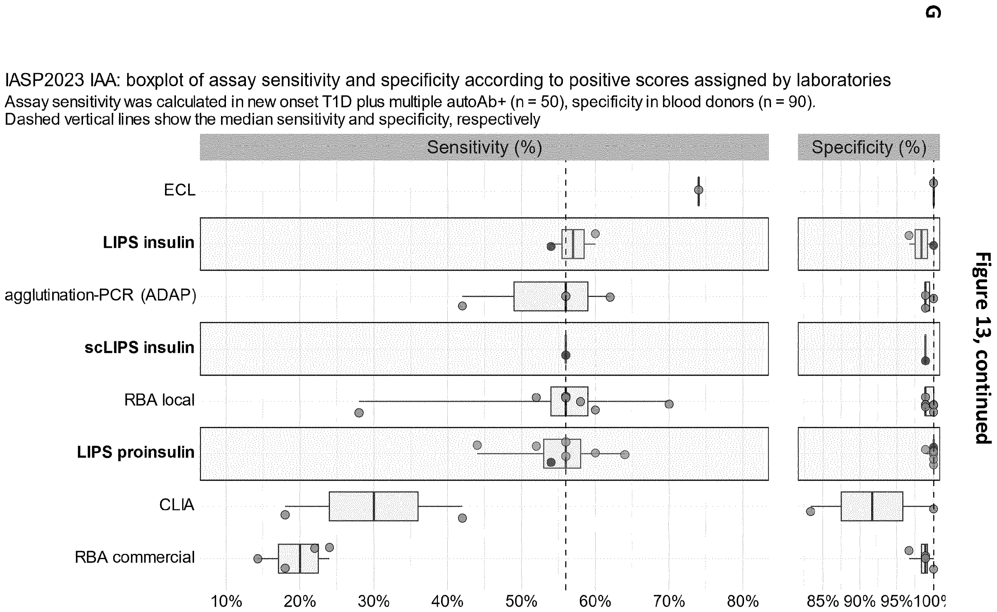

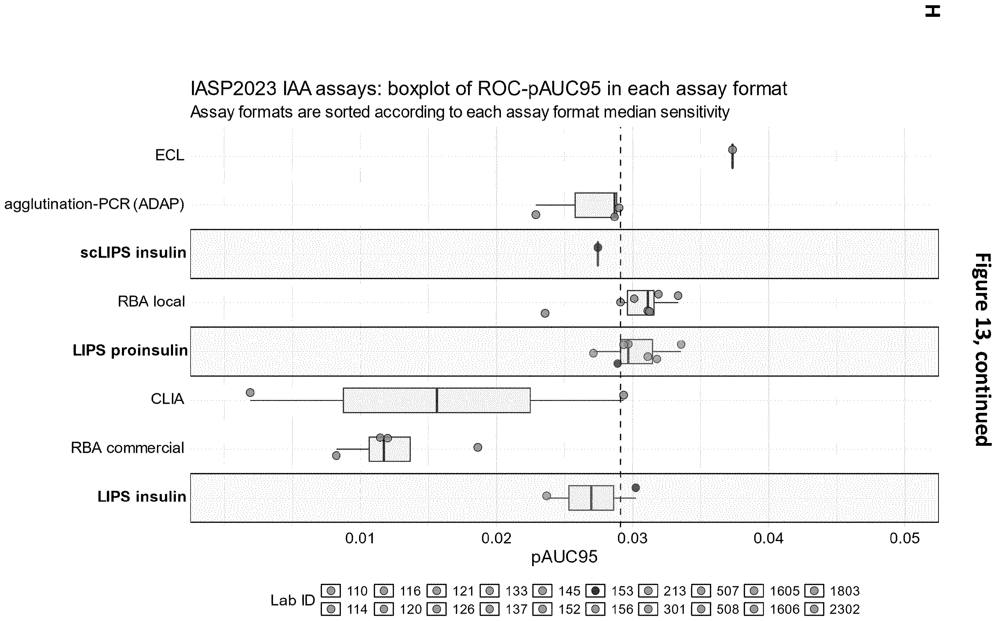

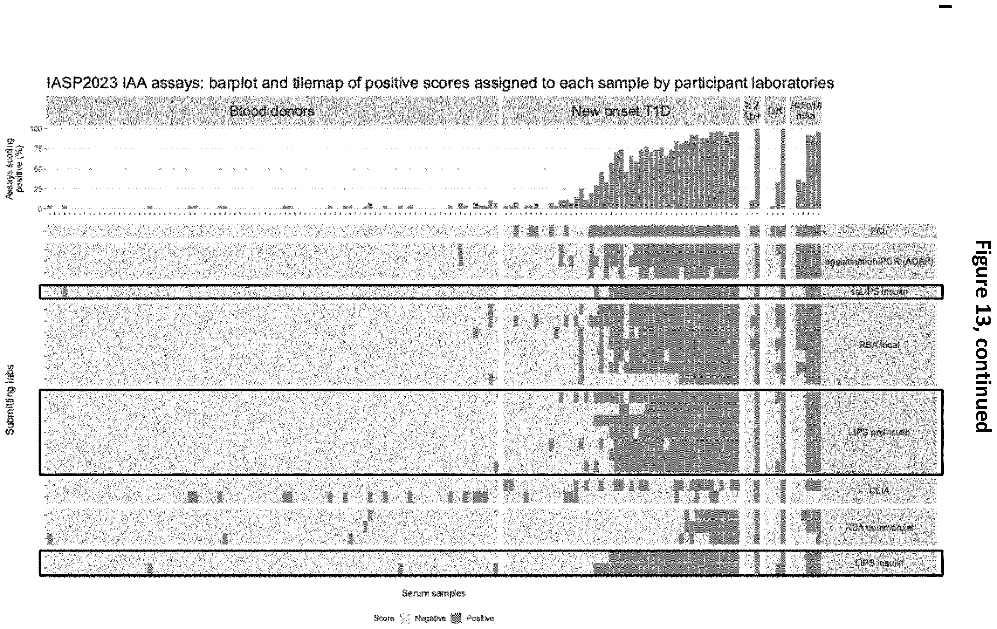

**Figure 13****: IAA LIPS and scLIPS performances in the IASP2023 interlaboratory comparison study. (A) Distribution of arbitrary units in case and control samples.** IAA arbitrary units in blood donors (control, n = 90) and new onset T1D patients (T1D, n= 50) samples. The violin plots correspond to their probability density estimate. **(B) Insulin LIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(C) Insulin assay ROC curve.** Shown lines correspond to: Insulin LIPS (grey lines); median ROC curve across all submitted assays (dotted line); identity line (dashed line). **(D) Distribution of arbitrary units in case and control samples.** IAA arbitrary units in blood donors (control, n = 90) and new onset T1D patients (T1D, n= 50) samples. The violin plots correspond to their probability density estimate. **(E) Insulin scLIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(F) Insulin assay ROC curve.** Shown lines correspond to: Insulin scLIPS (grey lines); median ROC curve across all submitted assays (dotted line); identity line (dashed line). **(G)** Boxplot of assay sensitivity and specificity across the submitted assay formats. **(H)** Boxplot of ROC-pAUC95 across all submitted assays. **(I)** Boxplot and tilemap of positive scores assigned to each sample across all submitted assays.

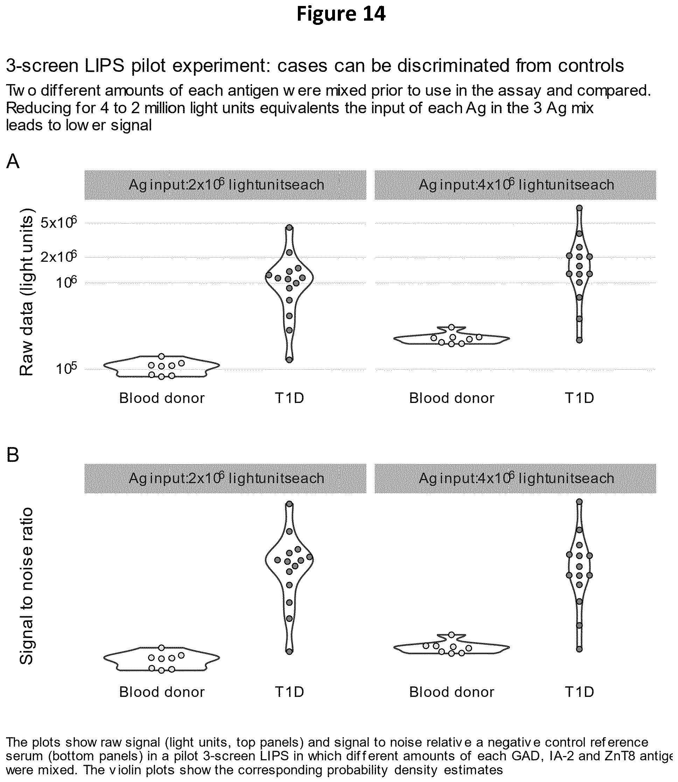

**Figure 14****: Multiplexed LIPS performance pilot experiment optimization of antigen input in the assay. (A)** The plots show raw signal (light units) detected after pilot multiplexed LIPS assays in blood donors (n = 8) and new onset T1D patients (n= 14) samples. The assays differed by the amount of each GAD, IA-2 and ZnT8 antigens that were multiplexed: 4 million light units equivalents of each antigen i.e., like in single LIPS assays and 2 million light units equivalents of each antigen, respectively. The violin plots show the corresponding probability density estimates. **(B)** Distribution of signal to noise ratio in case and control samples. The plots show the signal to noise relative to a negative control reference serum detected after pilot multiplexed LIPS assays in blood donors (n = 8) and new onset T1D patients (n= 14) samples. The assays differed by the amount of each GAD, IA-2 and ZnT8 antigens that were multiplexed: 4 million light units equivalents of each antigen i.e., like in single LIPS assays and 2 million light units equivalents of each antigen, respectively. The violin plots show the corresponding probability density estimates.

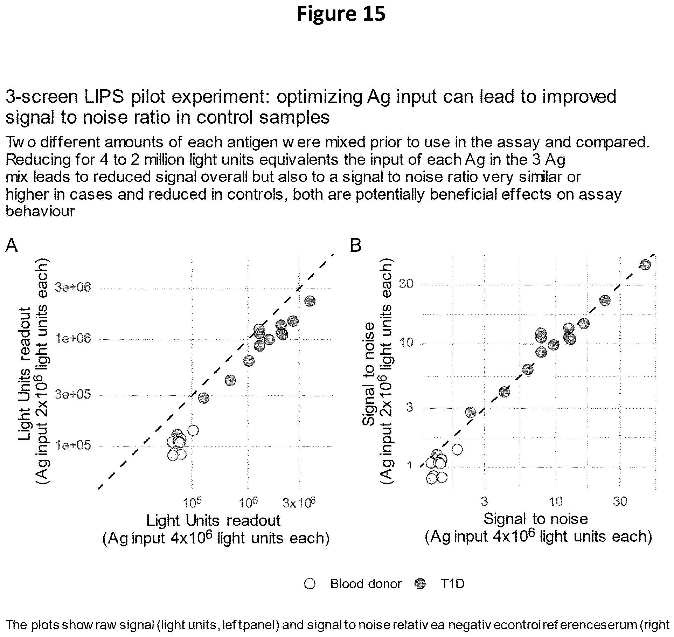

**Figure 15****: Optimization of antigen input in multiplexed LIPS leads to improved signal to noise ratio in control samples. (A)** The plots show raw signal (light units, left panel) and signal to noise relative a negative control reference serum (right panel) in a pilot 3-screen LIPS in which different amounts of each GAD, IA-2 and ZnT8 antigens were mixed. The violin plots show the corresponding probability density estimates.

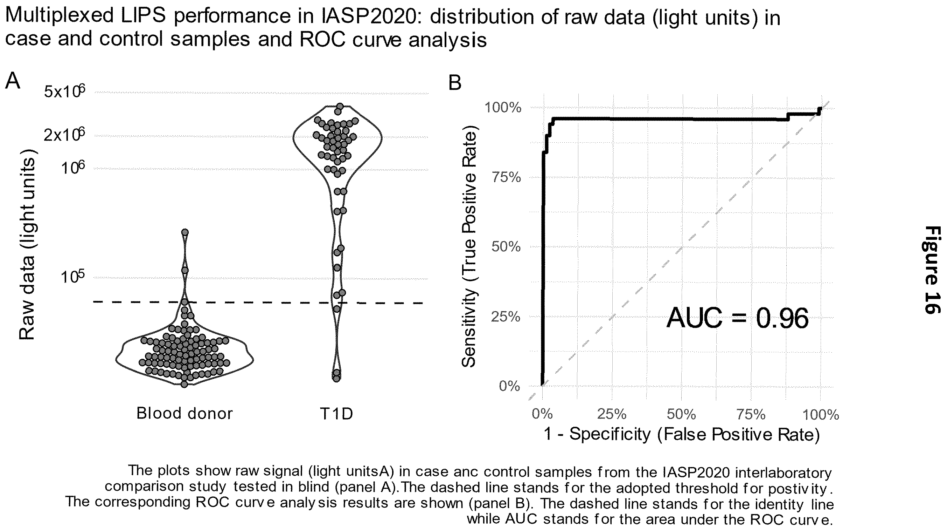

**Figure 16****: Multiplexed LIPS performance in discriminating case from control samples from the IASP2020 interlaboratory comparison study. (A)** Distribution of arbitrary units in case and control samples. Multiplexed arbitrary units in blood donors (control, n = 90) and new onset T1D patients (T1D, n= 50) samples. The violin plots correspond to their probability density estimate. **(B)** Multiplexed assay ROC curve. Shown lines correspond to: Multiplexed LIPS (black line); identity line (dashed line).

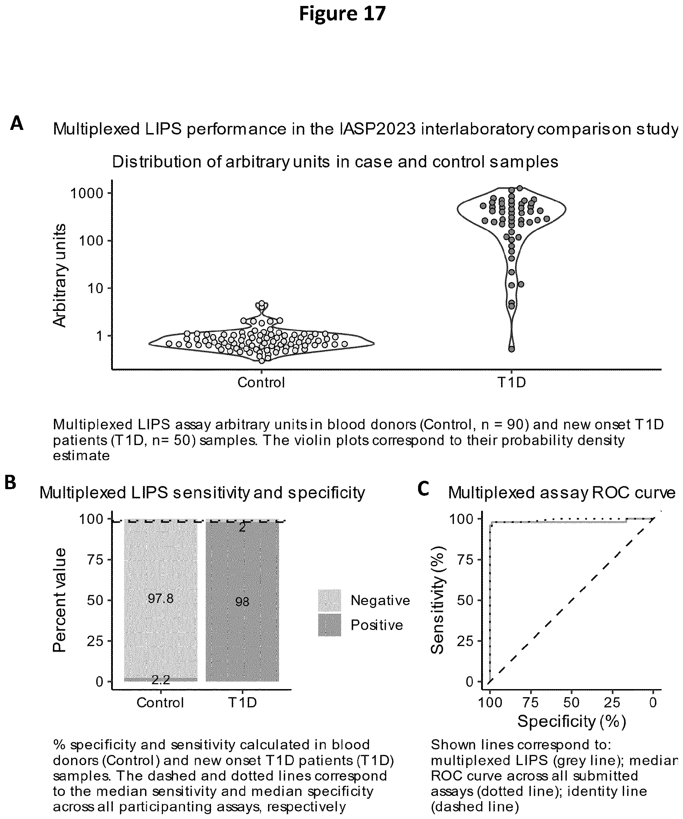

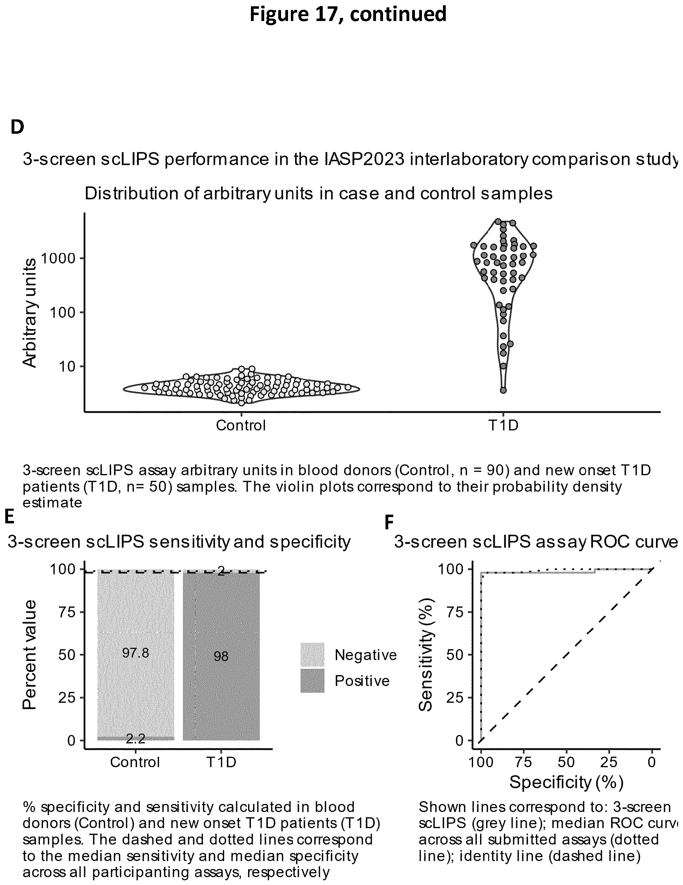

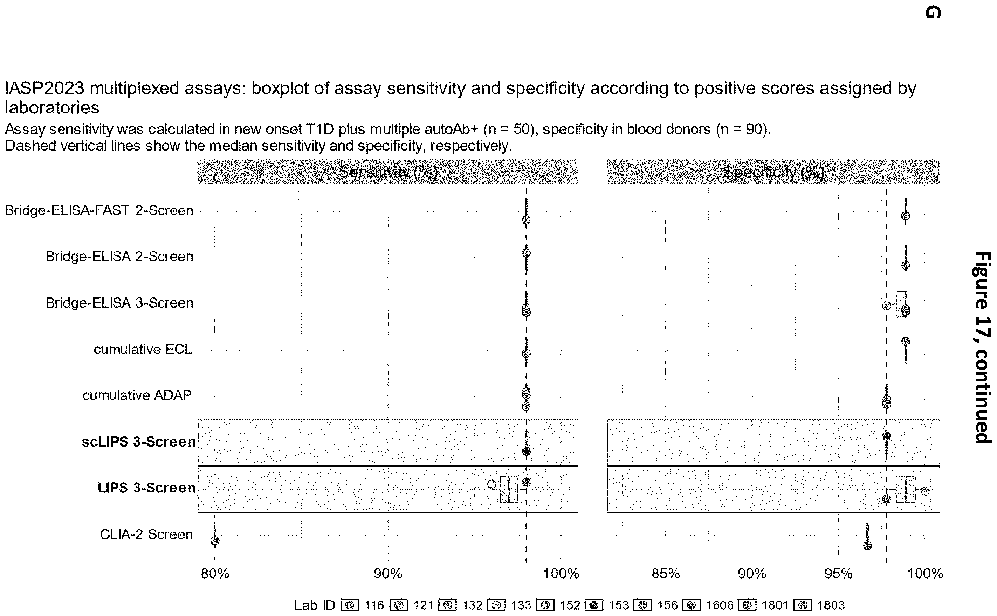

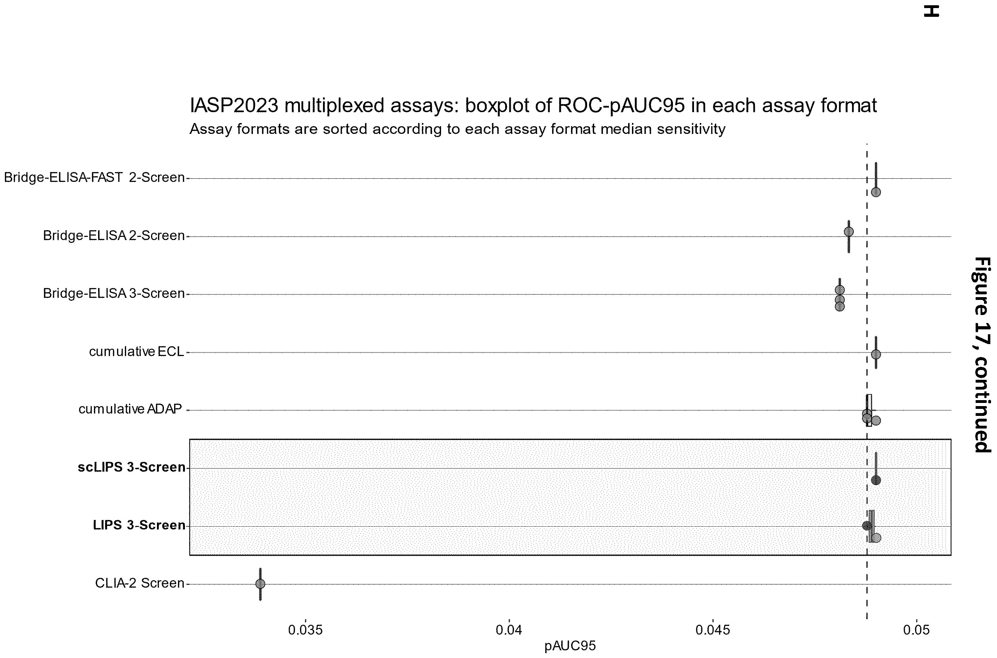

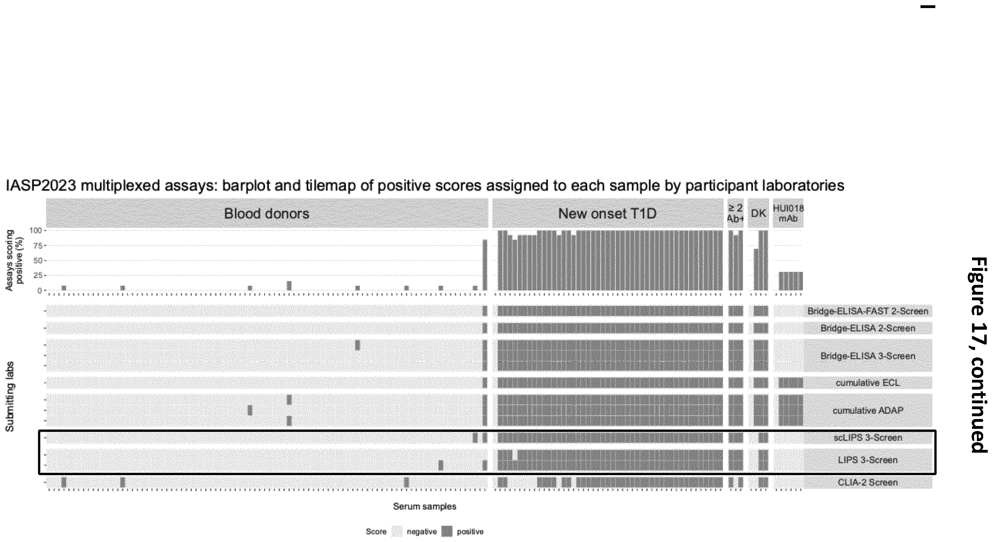

**Figure 17****: Multiplexed LIPS and scLIPS performances in discriminating case from control samples tested in blind in the IASP2023 interlaboratory comparison study. (A) Distribution of arbitrary units in case and control samples.** Multiplexed arbitrary units in blood donors (control, n = 90) and new onset T1D patients (T1D, n= 50) samples. The violin plots correspond to their probability density estimate. **(B) Multiplexed LIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(C) Multiplexed assay ROC curve.** Shown lines correspond to: Multiplexed LIPS (grey lines); median ROC curve across all submitted assays (dotted line); identity line (dashed line). **(D) Distribution of arbitrary units in case and control samples.** Multiplexed arbitrary units in blood donors (control, n = 90) and new onset T1D patients (T1D, n= 50) samples. The violin plots correspond to their probability density estimate. **(E) Multiplexed scLIPS sensitivity and specificity.** Percent specificity and sensitivity calculated in blood donors (control) and new onset T1D patients (T1D) samples. The dashed and dotted lines correspond to the median sensitivity and median specificity across all participating assay, respectively. **(F) Multiplexed assay ROC curve.** Shown lines correspond to: Multiplexed scLIPS (grey lines); median ROC curve across all submitted assays (dotted line); identity line (dashed line). **(G)** Boxplot of assay sensitivity and specificity across the submitted assay formats. (H) Boxplot of ROC-pAUC95 across all submitted assays. **(I)** Boxplot and tilemap of positive scores assigned to each sample across all submitted assays.

### DETAILED DESCRIPTION OF THE INVENTION

### Definitions

All terms as used herein, unless otherwise stated, shall be understood in their ordinary meaning as known in the art. Other more specific definitions for certain terms as used in the present application are as set forth below and are intended to apply uniformly throughout the description and claims unless an otherwise expressly set out definition provides a broader definition.

In the context of the invention, the term **"recombinant antigen molecule"** will be understood as a recombinant antigen molecule derived from a self-antigen molecule. A self-antigen may be found in all cell types or be highly specific for a specific cell type in one organ of the body. It may comprise, but is not limited to, proteins, nucleic acids, carbohydrates, lipids, or various combinations of these. In the context of the invention, the recombinant antigen molecules are derived from an antigen molecule expressed by pancreatic beta cells and includes, but is not limited to human insulin, proinsulin, human Glutamate decarboxylase 65, human islet antigen 2, and human Zinc transporter 8. The recombinant antigen molecule according to the invention can be modified by site-directed mutagenesis of coding sequences to change specific amino acid residues or to introduce restriction sites at desired location in the coding sequence or to produce truncated forms. According to the invention, the recombinant antigen molecules comprise the regulatory region of a target gene, which is preferably fused with the DNA coding sequence of the reporter protein.

All proteins in accordance with the invention, including the recombinant antigen molecules according to the invention, can be obtained by methods known in the art. Such methods include methods for the production of recombinant antigen molecules, e.g., of recombinant proteins. The recombinant antigen molecules of the invention can be expressed in recombinant host cells according to the invention, such as mammalian cells, e.g., Expi293F^{™} cells.

In the context of the invention, the term **"antibody"** or **"antibody molecule"** as used herein refers to any functional antibody that is capable of specific binding to the antigen of interest, as generally outlined in chapter 7 of Paul, W.E. (Ed.).: Fundamental Immunology 2nd Ed. Raven Press, Ltd., New York 1989, which is incorporated herein by reference. The skilled person understands the term "specific binding" in the context of antigen-antibody of the present invention. The specific binding of an antibody to its antigen(s) excludes non-specific binding, such as for instance non-specific antibody binding to endogenous Fc receptors (FcRs) or non-specific binding due to ionic and/or hydrophobic interactions. The skilled person is able to detect and measure autoantibodies specifically binding to the recombinant antigen molecules of the present invention.

An antibody or antibody molecule that react with self-molecules, i.e., self-antigens, is referred to as natural antibody or **"autoantibody"** or **"autoantibody molecule".** Autoantibodies can be used as indicators of disease, e.g., their detection can be used to detect a loss of tolerance and inflammation in a subject with an autoimmune disorder. **"Islet autoantibody molecules"** are well-known in the field and generally understood as autoantibody molecules with specificity to e.g., human insulin, human proinsulin, human Glutamate decarboxylase 65, human islet antigen 2, and human Zinc transporter 8. The islet autoantibodies detected by the recombinant antigen molecules according to the invention include but are not limited to human IAA (Insulin Auto Antibodies), human GADA (Glutamic Acid Decarboxylase Antibodies), human IA-2A (Islet Antigen 2 Antibodies), and human ZnT8A (Zinc Transporter 8 Antibodies).

In the context of the invention, when the reporter molecule is a reporter protein such as luciferase or a derivate thereof, then the reporter protein will be fused to the recombinant antigen molecule. The term **"fused"** refers to the genetic or chemical combination of a reporter protein with another protein to form a single, continuous polypeptide chain. This fusion enables the reporter protein, which may provide detectable or measurable properties (such as fluorescence, luminescence, or enzymatic activity), to be expressed and analyzed in conjunction with the target protein, i.e., the antigen molecule. The resulting fused recombinant antigen molecule retains the functional characteristics of both the reporter and the antigen molecule.

The term **"bioluminescent reporter protein"** is well known in the art and will be understood by the skilled person in the art. A reporter protein referred herein is well known in the art and include, for instance, a luciferase reporter protein, a nanoluciferase reporter protein, and a NanoLuc^{™} (NLuc) luciferase reporter protein. According to the invention, a substrate of the reporter protein is added to the assay and the reporter protein catalyzes a reaction that produces light. The amount of light produced can be detected and provides a quantitative measure of the level of the reporter protein in the sample; the level of the reporter protein in a sample may be used to determine the presence and/or level of an antibody molecule in a sample.

In the context of the method of the present invention, the term **"contacting the sample with the recombinant antigen molecules of the test kit"** means contacting the recombinant antigen with the sample from a human subject, under the conditions and for a period of time which is sufficient for the determination of the presence of autoantibodies in the sample from a human subject. As used herein, the term "contact" or "contacting" means bringing together, either directly or indirectly, the recombinant antigen molecules with the sample from a human subject. Contacting may occur, for example, in any number of buffers, salts, solutions, or in cell culture medium.

In accordance with the invention, the term **"single composition"** refers to a formulation containing at least one type of molecule. Specifically, a single composition may include at least one, two, or three recombinant antigen molecules.

In the context of the invention, a **"positive sample"** refers to a biological sample obtained from a human subject that, when analysed using the method described herein, exhibits characteristics indicative of an increased risk for developing type 1 diabetes. The sample contains one or more autoantibody molecules associated with type 1 diabetes. It will be understood that the amount of the detected autoantibody molecule(s) in the sample exceeds a predetermined threshold value, as established by comparison with a standard curve for known amounts of said autoantibody molecules. The presence and/or quantity of the autoantibody molecule(s) in the sample, when evaluated in the context of the screening method, suggests an elevated likelihood of the subject developing type 1 diabetes. A sample may be classified as "positive" based on either the mere presence of specific autoantibody molecules or when the quantity of such molecules surpasses a defined concentration threshold, as determined by the standard curve comparison. The classification of a sample as "positive" serves as a key indicator in the overall screening process for assessing an individual's risk of developing type 1 diabetes, and may inform subsequent diagnostic or preventive measures. Detection and measurement of islet autoantibodies may be assessed by comparing the level of autoantibodies in a sample from a subject at risk of developing T1D, to the level of autoantibodies in a control sample, i.e., a sample from a subject that is not at risk of developing T1D, wherein an increase level of autoantibodies indicates an increased probability of developing T1D. Preferably, the level is statistically significant as assessed by appropriate statistical tests which are known in the art.

Throughout the description and claims the term **"comprise"** and variations of the term (e.g., "comprising", "having", "including", "containing"), are not intended to exclude other technical features, additives, components, or steps. Furthermore, the term "comprise" and variations of the term encompasses the case of "consisting of". Additional objects, advantages and features of the invention will become apparent to those skilled in the art upon examination of the description or may be learned by practice of the invention.

In this specification and claims, the use of the terms "a" and "an" and "the" and similar referents in the context of describing the invention (especially in the context of the following claims) are to be construed to cover both the singular and the plural, unless otherwise indicated herein or clearly contradicted by context.

Unless otherwise indicated, the term **"at least"** preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The use of any and all examples, or exemplary language (e.g., **"such as")** provided herein, is intended merely to better illuminate the invention and does not pose a limitation on the scope of the invention unless otherwise claimed. No language in the specification should be construed as indicating any non-claimed element as essential to the practice of the invention.

If the term **"about"** as used in connection with a numerical value throughout the specification and the claims denotes an interval of accuracy, familiar and acceptable to a person skilled in the art. For instance, the term "about" means the indicated value ± 1% of its value, or the term "about" means the indicated value ± 2% of its value, or the term "about" means the indicated value ± 5% of its value, the term "about" means the indicated value ± 10% of its value, or the term "about" means the indicated value ± 20% of its value, or the term "about" means the indicated value ± 30% of its value; preferably the term "about" means exactly the indicated value (± 0%).

Unless otherwise defined, all technical and scientific terms used herein have the same meaning as commonly understood by one of ordinary skilled in the art to which this invention belongs. Units, prefixes, and symbols are denoted in their "Systeme International de Unites" (SI) accepted form.

Numeric ranges are inclusive of the numbers defining the range.

Unless otherwise defined below, the terms used in the present invention shall be understood in accordance with their common meaning known to the person skilled in the art. All publications, patents and patent applications cited herein are hereby incorporated by reference in their entirety for all purposes. Publications referred to herein may be cited by specifying the full literature reference in the text, or by naming the author and the publication year (e.g., "Liberati et al. (2018)") or a reference number (e.g., "1") and by specifying the corresponding full literature reference in the "References" section.

The headings provided herein are not limitations of the various aspects or embodiments of the disclosure. Furthermore, the present invention covers all possible combinations of particular aspects and embodiments described herein.

The figures and the experimental part/examples are only given to further illustrate the invention and should not be interpreted or construed as limiting the scope of the invention and/or of the appended claims in any way, unless explicitly indicated otherwise herein.

The present invention also provides the following embodiments which may be combined with any aspect or embodiment described throughout the entirety of the present invention. The embodiments illustrated and discussed in this specification are intended only to teach those skilled in the art the best way known to the inventors to make and use the invention. Modifications and variation of the above-described embodiments of the invention are possible without departing from the invention, as appreciated by those skilled in the art in light of the above teachings. It is therefore understood that, within the scope of the claims and their equivalents, the invention may be practiced otherwise than as specifically described.

### Sequences

Preferred amino acid sequences referred to in the present application can be independently selected from the following sequences. The sequences are represented in an N-terminal to C-terminal order; and they are represented in the one-letter amino acid code.

The following non-limiting exemplary amino acid sequences were used in the experimental examples of the present application and/or are disclosed herein as preferred amino acid sequences in accordance with the invention:

### Legend of Table 1:

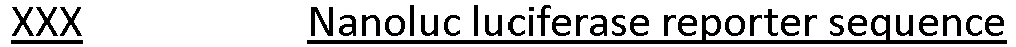

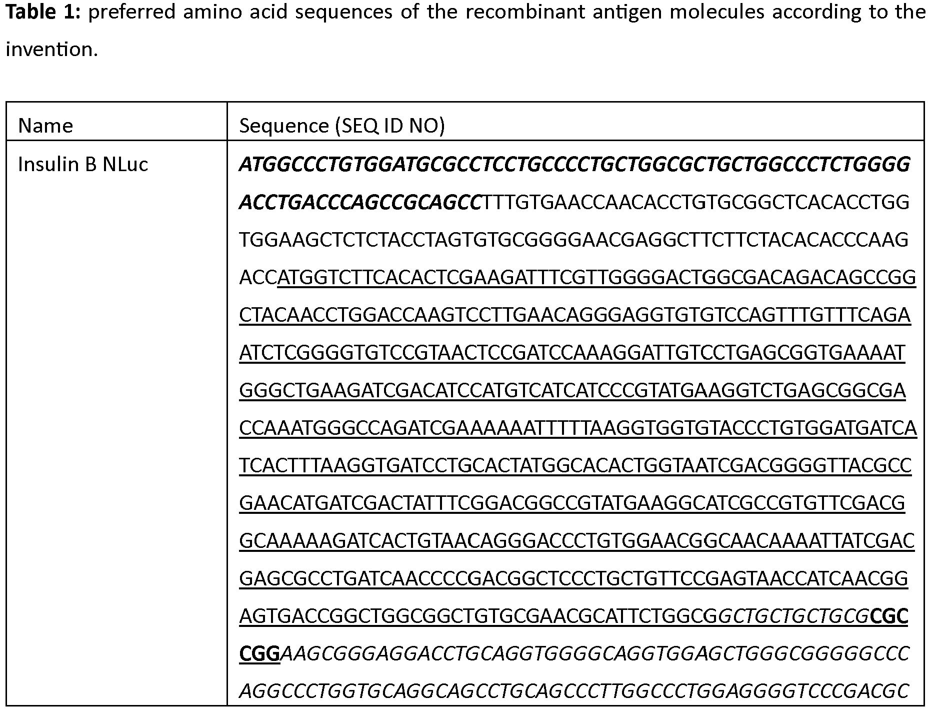

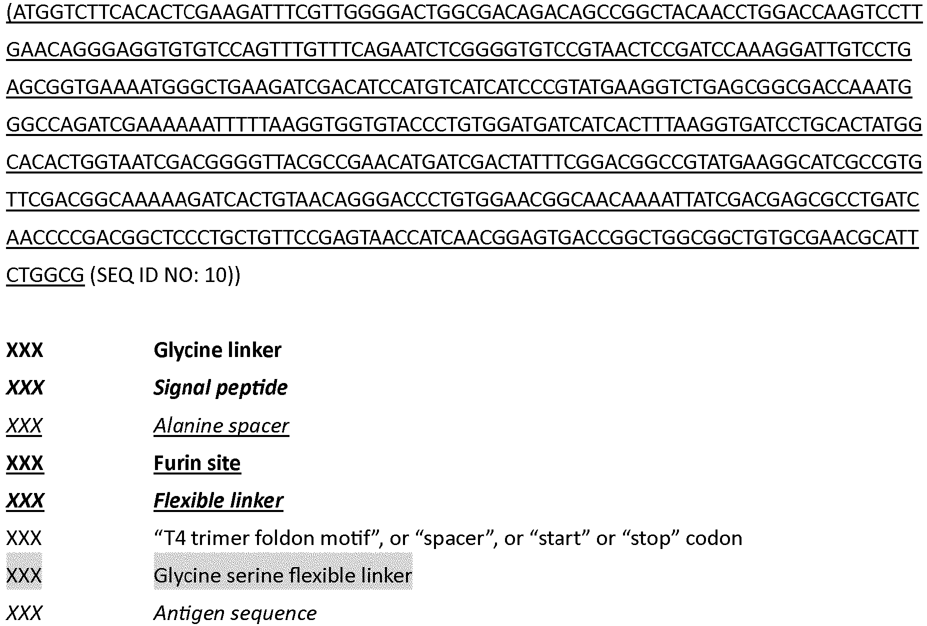

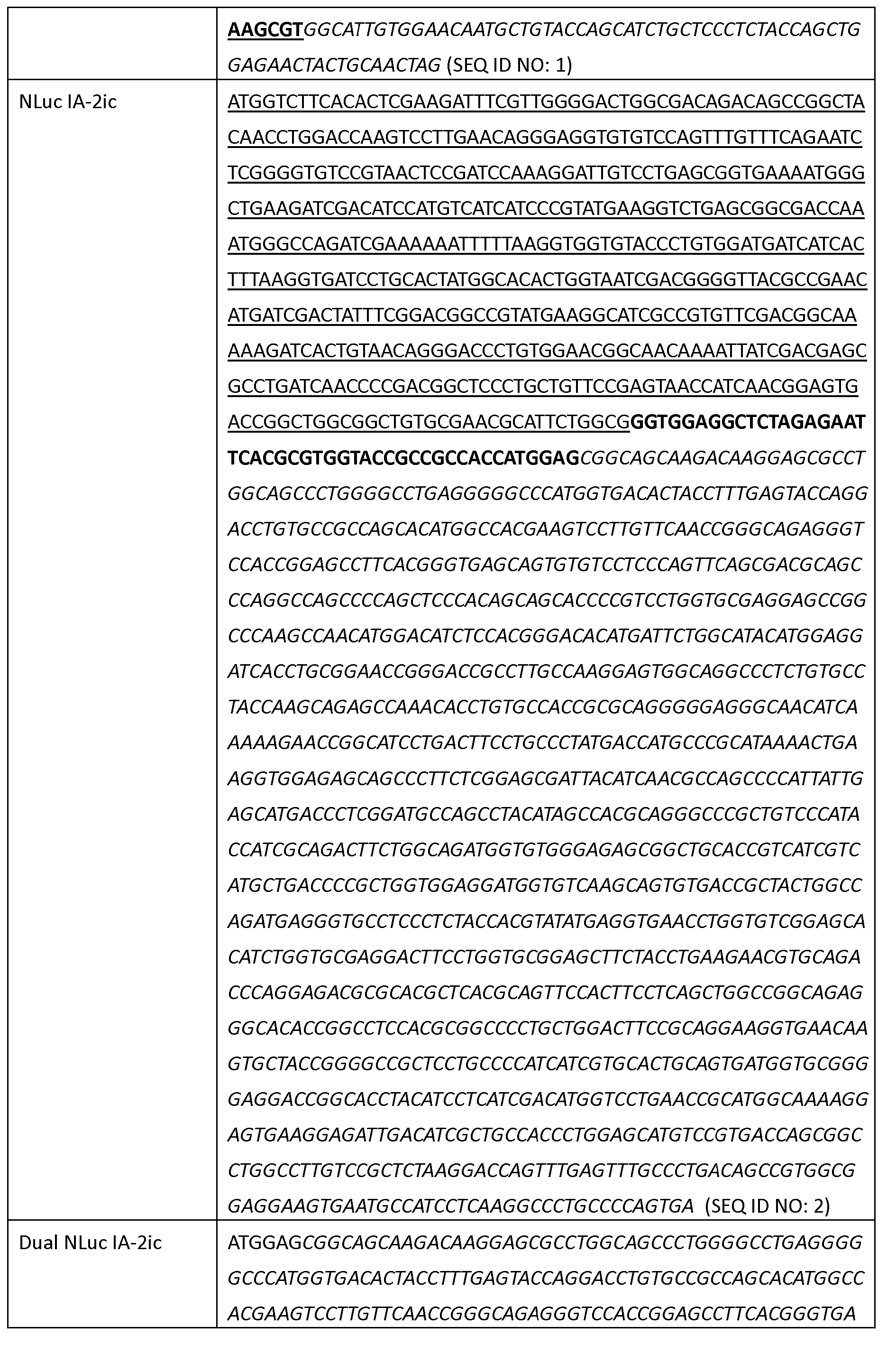

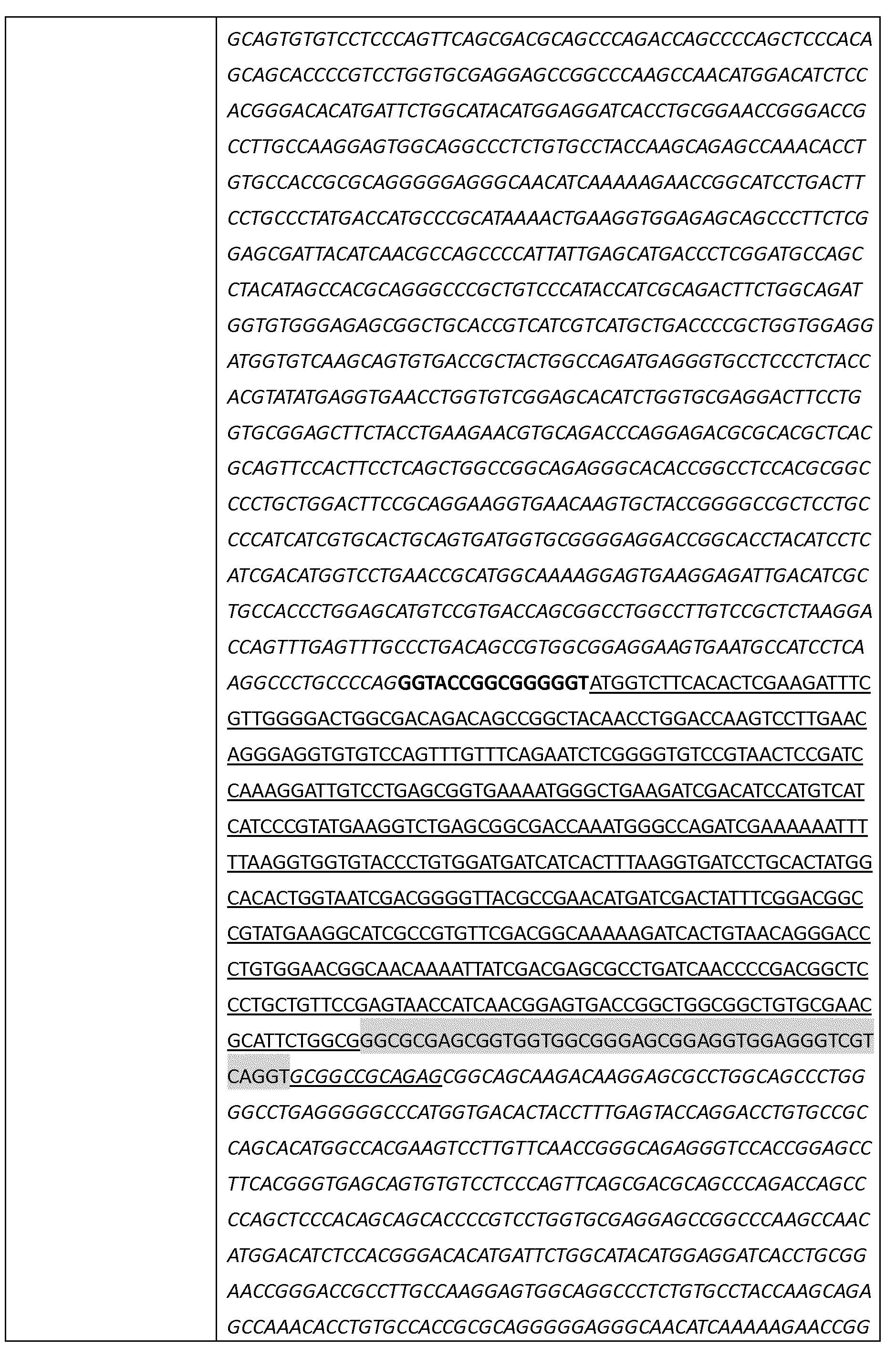

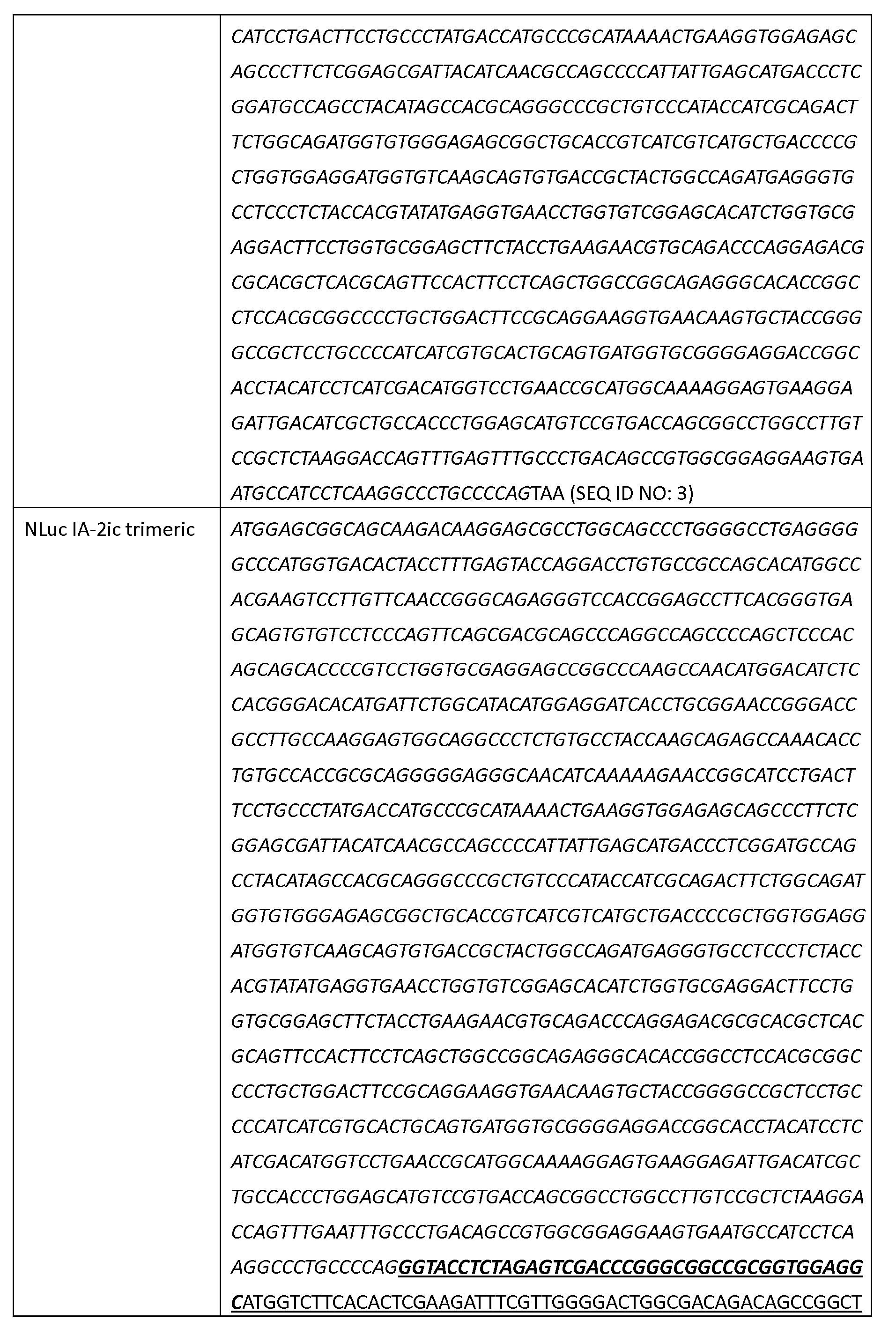

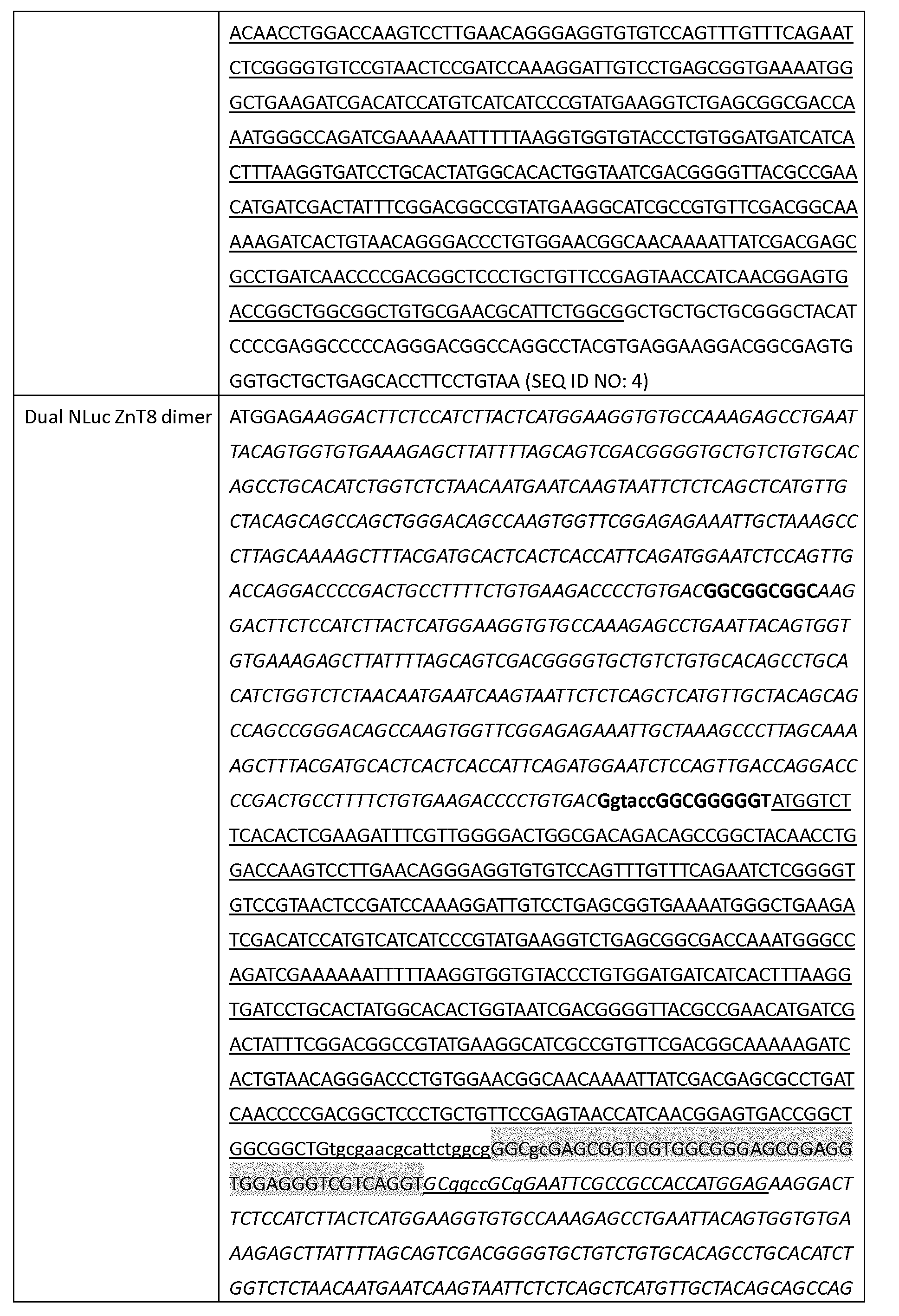

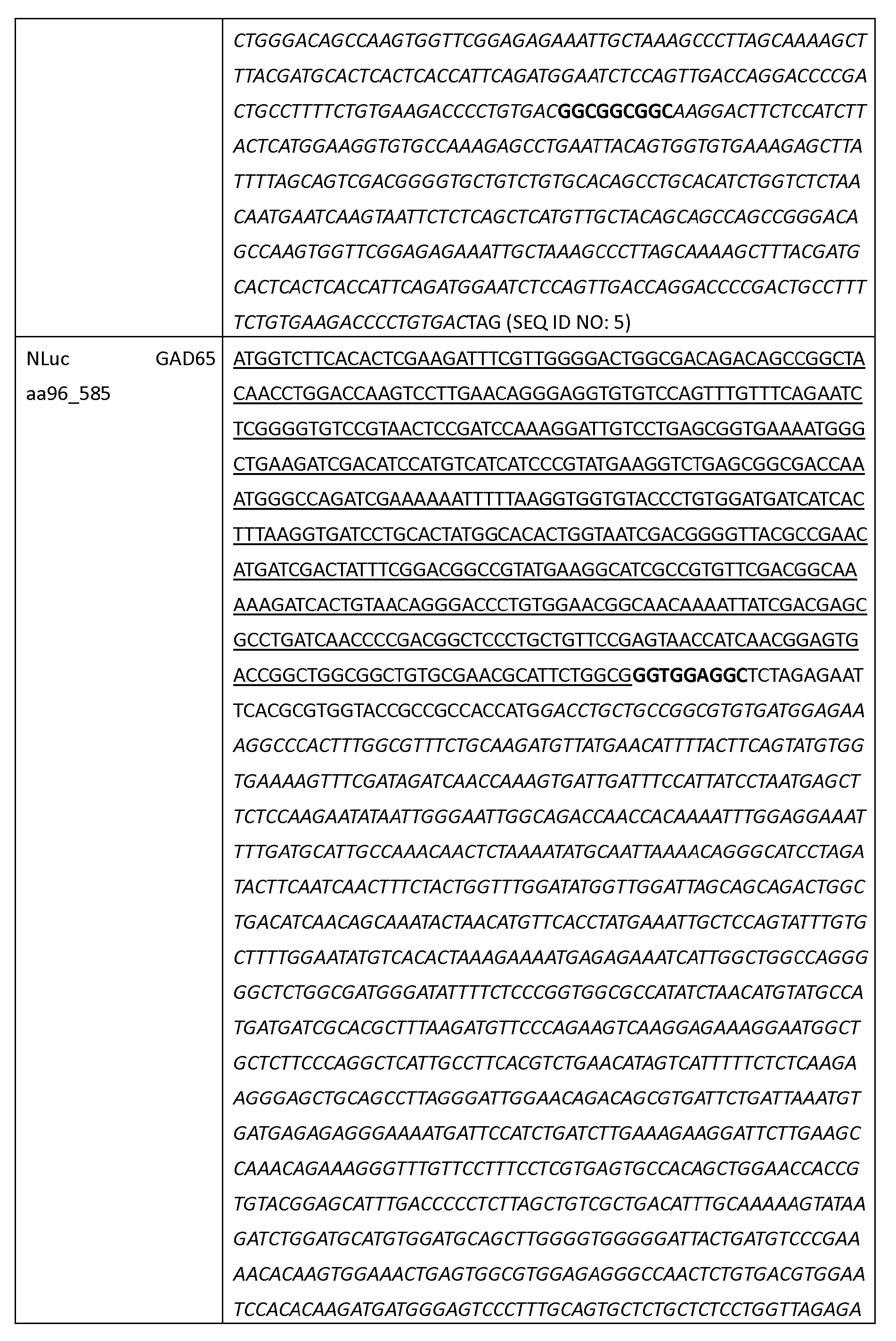

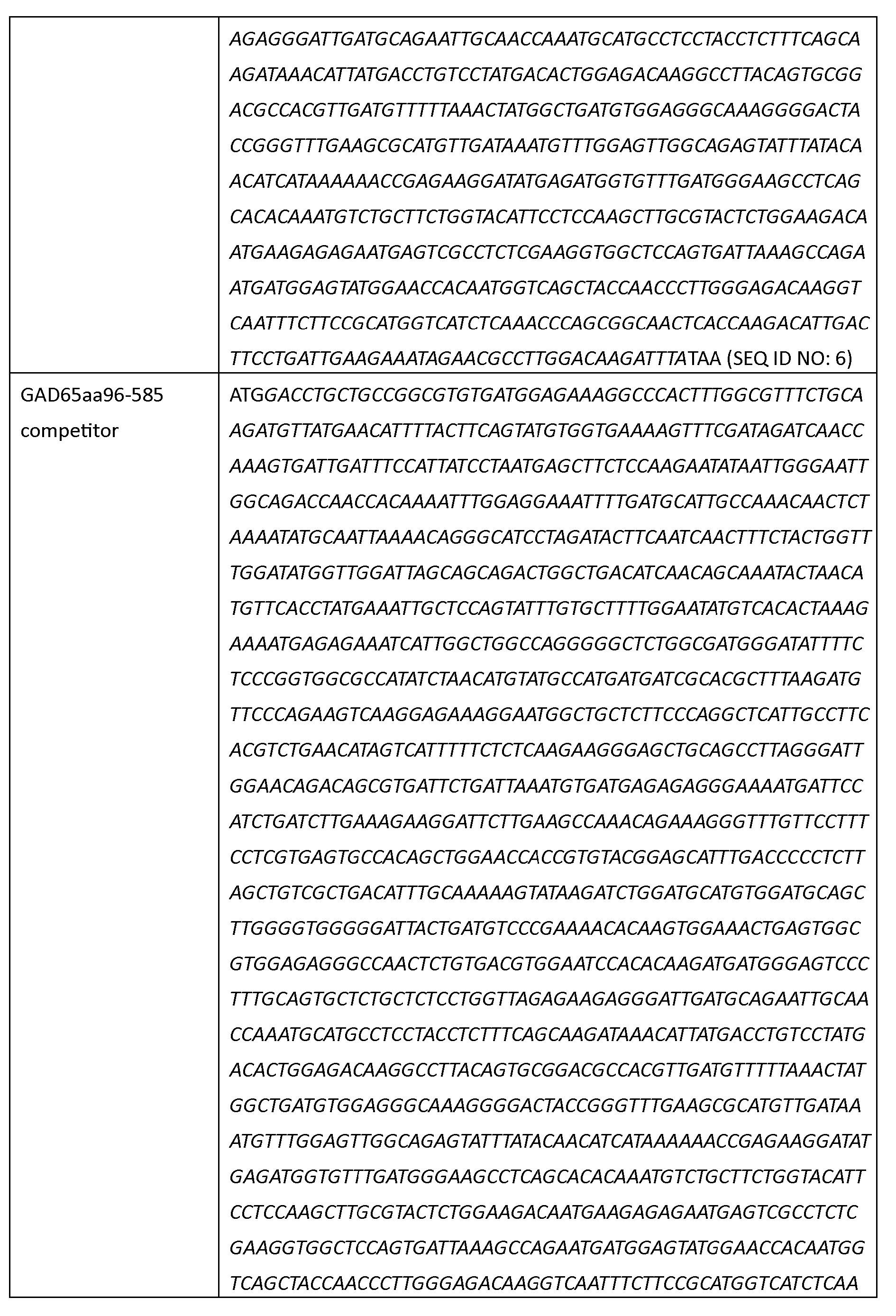

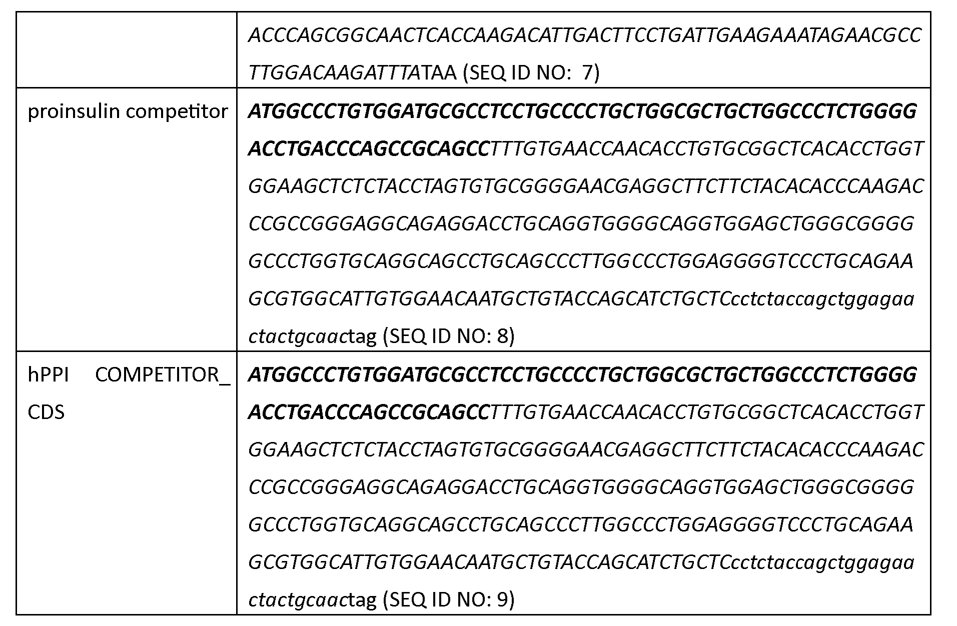

Further sequences of recombinant antigen molecules according to the invention:

**Table 2: exemplary amino acid sequences of mutated insulin B NLuc recombinant antigen molecules.**

| Name | Sequence (SEQ ID NO) |
|---|---|
| Insulin B NLuc Delta B24-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCATGGTCTTCACACTCGAAGATTTCGTTGGGGACTG GCGACAGACAGCCGGCTACAACCTGGACCAAGTCCTTGAACAGGGAGGTGTGTCCAGT TTGTTTCAGAATCTCGGGGTGTCCGTAACTCCGATCCAAAGGATTGTCCTGAGCGGTGA AAATGGGCTGAAGATCGACATCCATGTCATCATCCCGTATGAAGGTCTGAGCGGCGACC AAATGGGCCAGATCGAAAAAATTTTTAAGGTGGTGTACCCTGTGGATGATCATCACTTTA AGGTGATCCTGCACTATGGCACACTGGTAATCGACGGGGTTACGCCGAACATGATCGAC TATTTCGGACGGCCGTATGAAGGCATCGCCGTGTTCGACGGCAAAAAGATCACTGTAAC AGGGACCCTGTGGAACGGCAACAAAATTATCGACGAGCGCCTGATCAACCCCGACGGC TCCCTGCTGTTCCGAGTAACCATCAACGGAGTGACCGGCTGGCGGCTGTGCGAACGCAT TCTGGCGGCTGCTGCTGCGCGCCGGAAGCGGGAGGACCTGCAGGTGGGGCAGGTGG AGCTGGGCGGGGGCCCAGGCGCAGGCAGCCTGCAGCCCTTGGCCCTGGAGGGGTCCC GACGCAAGCGTGGCATTGTGGAACAATGCTGTACCAGCATCTGCTCCCTCTACCAGCTG GAGAACTACTGCAACTAG (SEQ ID NO: 11) |
| Insulin B NLuc Delta B25-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCATGGTCTTCACACTCGAAGATTTCGTTGGGGAC TGGCGACAGACAGCCGGCTACAACCTGGACCAAGTCCTTGAACAGGGAGGTGTGTCCA GTTTGTTTCAGAATCTCGGGGTGTCCGTAACTCCGATCCAAAGGATTGTCCTGAGCGGT GAAAATGGGCTGAAGATCGACATCCATGTCATCATCCCGTATGAAGGTCTGAGCGGCGA CCAAATGGGCCAGATCGAAAAAATTTTTAAGGTGGTGTACCCTGTGGATGATCATCACTT TAAGGTGATCCTGCACTATGGCACACTGGTAATCGACGGGGTTACGCCGAACATGATCG ACTATTTCGGACGGCCGTATGAAGGCATCGCCGTGTTCGACGGCAAAAAGATCACTGTA ACAGGGACCCTGTGGAACGGCAACAAAATTATCGACGAGCGCCTGATCAACCCCGACG GCTCCCTGCTGTTCCGAGTAACCATCAACGGAGTGACCGGCTGGCGGCTGTGCGAACG CATTCTGGCGGCTGCTGCTGCGCGCCGGAAGCGGGAGGACCTGCAGGTGGGGCAGGT GGAGCTGGGCGGGGGCCCAGGCGCAGGCAGCCTGCAGCCCTTGGCCCTGGAGGGGT CCCGACGCAAGCGTGGCATTGTGGAACAATGCTGTACCAGCATCTGCTCCCTCTACCAG CTGGAGAACTACTGCAACTAG (SEQ ID NO: 12) |
| Insulin B NLuc Delta B26-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCATGGTCTTCACACTCGAAGATTTCGTTGGG GACTGGCGACAGACAGCCGGCTACAACCTGGACCAAGTCCTTGAACAGGGAGGTGTG TCCAGTTTGTTTCAGAATCTCGGGGTGTCCGTAACTCCGATCCAAAGGATTGTCCTGAGC GGTGAAAATGGGCTGAAGATCGACATCCATGTCATCATCCCGTATGAAGGTCTGAGCGG CGACCAAATGGGCCAGATCGAAAAAATTTTTAAGGTGGTGTACCCTGTGGATGATCATC ACTTTAAGGTGATCCTGCACTATGGCACACTGGTAATCGACGGGGTTACGCCGAACATG ATCGACTATTTCGGACGGCCGTATGAAGGCATCGCCGTGTTCGACGGCAAAAAGATCAC TGTAACAGGGACCCTGTGGAACGGCAACAAAATTATCGACGAGCGCCTGATCAACCCCG ACGGCTCCCTGCTGTTCCGAGTAACCATCAACGGAGTGACCGGCTGGCGGCTGTGCGA ACGCATTCTGGCGGCTGCTGCTGCGCGCCGGAAGCGGGAGGACCTGCAGGTGGGGCA GGTGGAGCTGGGCGGGGGCCCAGGCGCAGGCAGCCTGCAGCCCTTGGCCCTGGAGG GGTCCCGACGCAAGCGTGGCATTGTGGAACAATGCTGTACCAGCATCTGCTCCCTCTACC AGCTGGAGAACTACTGCAACTAG (SEQ ID NO: 13) |
| Insulin B NLuc Delta B27-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACATGGTCTTCACACTCGAAGATTTCGTT GGGGACTGGCGACAGACAGCCGGCTACAACCTGGACCAAGTCCTTGAACAGGGAGGT GTGTCCAGTTTGTTTCAGAATCTCGGGGTGTCCGTAACTCCGATCCAAAGGATTGTCCTG AGCGGTGAAAATGGGCTGAAGATCGACATCCATGTCATCATCCCGTATGAAGGTCTGAG CGGCGACCAAATGGGCCAGATCGAAAAAATTTTTAAGGTGGTGTACCCTGTGGATGATC |
| | ATCACTTTAAGGTGATCCTGCACTATGGCACACTGGTAATCGACGGGGTTACGCCGAACA TGATCGACTATTTCGGACGGCCGTATGAAGGCATCGCCGTGTTCGACGGCAAAAAGATC ACTGTAACAGGGACCCTGTGGAACGGCAACAAAATTATCGACGAGCGCCTGATCAACCC CGACGGCTCCCTGCTGTTCCGAGTAACCATCAACGGAGTGACCGGCTGGCGGCTGTGC GAACGCATTCTGGCGGCTGCTGCTGCGCGCCGGAAGCGGGAGGACCTGCAGGTGGGG CAGGTGGAGCTGGGCGGGGGCCCAGGCGCAGGCAGCCTGCAGCCCTTGGCCCTGGA GGGGTCCCGACGCAAGCGTGGCATTGTGGAACAATGCTGTACCAGCATCTGCTCCCTCT ACCAGCTGGAGAACTACTGCAACTAG (SEQ ID NO: 14) |
| Insulin B NLuc Delta B28-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACAATGGTCTTCACACTCGAAGATTTC GTTGGGGACTGGCGACAGACAGCCGGCTACAACCTGGACCAAGTCCTTGAACAGGGA GGTGTGTCCAGTTTGTTTCAGAATCTCGGGGTGTCCGTAACTCCGATCCAAAGGATTGTC CTGAGCGGTGAAAATGGGCTGAAGATCGACATCCATGTCATCATCCCGTATGAAGGTCT GAGCGGCGACCAAATGGGCCAGATCGAAAAAATTTTTAAGGTGGTGTACCCTGTGGAT GATCATCACTTTAAGGTGATCCTGCACTATGGCACACTGGTAATCGACGGGGTTACGCCG AACATGATCGACTATTTCGGACGGCCGTATGAAGGCATCGCCGTGTTCGACGGCAAAAA GATCACTGTAACAGGGACCCTGTGGAACGGCAACAAAATTATCGACGAGCGCCTGATCA ACCCCGACGGCTCCCTGCTGTTCCGAGTAACCATCAACGGAGTGACCGGCTGGCGGCT GTGCGAACGCATTCTGGCGGCTGCTGCTGCGCGCCGGAAGCGGGAGGACCTGCAGGT GGGGCAGGTGGAGCTGGGCGGGGGCCCAGGCGCAGGCAGCCTGCAGCCCTTGGCCC TGGAGGGGTCCCGACGCAAGCGTGGCATTGTGGAACAATGCTGTACCAGCATCTGCTCC CTCTACCAGCTGGAGAACTACTGCAACTAG (SEQ ID NO: 15) |
| Insulin B NLuc mut A E17A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT |
| | GCAGCCCTTGGCCCTGGAGGGGTCCCGACGCAAGCGTGGCATTGTGGAACAATGCTGT ACCAGCATCTGCTCCCTCTACCAGCTGGCGAACTACTGCAACTAG (SEQ ID NO: 16) |
| Insulin B NLuc mut A L13A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCcGCctaccagctggagaactactgcaactag (SEQ ID NO: 17) |
| Insulin B NLuc mut A L13W | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCTGGTACCAGCTGGAGAACTACTGCAACTAG (SEQ ID NO: 18) |
| Insulin B NLuc mut A Y14A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA |
| | AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCGCCCAGCTGGAGAACTACTGCAACTAG (SEQ ID NO: 19) |
| Insulin B NLuc mut B F1A | ATGGCCCTGTGGATGCGCCTCCTGCCCCTGCTGGCGCTGCTGGCCCTCTGGGGACCTGA CCCAGCCGCAGCCGCTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 20) |
| Insulin B NLuc mut B F25D | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCGACTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC |
| | CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 21) |
| Insulin B NLuc mut B L17A V18A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACGCAGCGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 22) |
| Insulin B NLuc mut B L17A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACGCAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 23) |
| Insulin B NLuc mut B R22A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAAGCAGGCTTCTTCTACACACCCAAGACCATGGTCTTCACACTC GAAGATTTCGTTGGGGACTGGCGACAGACAGCCGGCTACAACCTGGACCAAGTCCTTG |
| | AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 24) |
| Insulin B NLuc mut B S9P | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCCCCCACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 25) |
| Insulin B NLuc mut B V18A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGCGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC |
| | TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 26) |
| Insulin B NLuc mut B V2G | ATGGCCCTGTGGATGCGCCTCCTGCCCCTGCTGGCGCTGCTGGCCCTCTGGGGACCTGA CCCAGCCGCAGCCTTTGGGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 27) |

**Table 3: exemplary amino acid sequences of mutated Insulin NLuc A recombinant antigen molecules.**

| Name | Sequence (SEQ ID NO) |
|---|---|
| Insulin NLuc A Delta B25-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCCGCCGGAAGCGGGAGGACCTGCAGGTGGGG CAGGTGGAGCTGGGCGGGGGCCCAGGCGCAGGCAGCCTGCAGCCCTTGGCCCTGGA GGGGTCCCGACGCAAGCGTATGGTCTTCACACTCGAAGATTTCGTTGGGGACTGGCGA CAGACAGCCGGCTACAACCTGGACCAAGTCCTTGAACAGGGAGGTGTGTCCAGTTTGT TTCAGAATCTCGGGGTGTCCGTAACTCCGATCCAAAGGATTGTCCTGAGCGGTGAAAAT GGGCTGAAGATCGACATCCATGTCATCATCCCGTATGAAGGTCTGAGCGGCGACCAAAT GGGCCAGATCGAAAAAATTTTTAAGGTGGTGTACCCTGTGGATGATCATCACTTTAAGGT GATCCTGCACTATGGCACACTGGTAATCGACGGGGTTACGCCGAACATGATCGACTATTT CGGACGGCCGTATGAAGGCATCGCCGTGTTCGACGGCAAAAAGATCACTGTAACAGGG ACCCTGTGGAACGGCAACAAAATTATCGACGAGCGCCTGATCAACCCCGACGGCTCCCT GCTGTTCCGAGTAACCATCAACGGAGTGACCGGCTGGCGGCTGTGCGAACGCATTCTG GCGGCTGCTGCTGCGGGCATTGTGGAACAATGCTGTACCAGCATCTGCTCCCTCTACCA GCTGGAGAACTACTGCAACTAG (SEQ ID NO: 28) |
| Insulin NLuc A Delta B26-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCCGCCGGAAGCGGGAGGACCTGCAGGTGG GGCAGGTGGAGCTGGGCGGGGGCCCAGGCCCTGGTGCAGGCAGCCTGCAGCCCTTG GCCCTGGAGGGGTCCCGACGCAAGCGTATGGTCTTCACACTCGAAGATTTCGTTGGGG ACTGGCGACAGACAGCCGGCTACAACCTGGACCAAGTCCTTGAACAGGGAGGTGTGTC CAGTTTGTTTCAGAATCTCGGGGTGTCCGTAACTCCGATCCAAAGGATTGTCCTGAGCG GTGAAAATGGGCTGAAGATCGACATCCATGTCATCATCCCGTATGAAGGTCTGAGCGGC GACCAAATGGGCCAGATCGAAAAAATTTTTAAGGTGGTGTACCCTGTGGATGATCATCA CTTTAAGGTGATCCTGCACTATGGCACACTGGTAATCGACGGGGTTACGCCGAACATGAT CGACTATTTCGGACGGCCGTATGAAGGCATCGCCGTGTTCGACGGCAAAAAGATCACTG TAACAGGGACCCTGTGGAACGGCAACAAAATTATCGACGAGCGCCTGATCAACCCCGA CGGCTCCCTGCTGTTCCGAGTAACCATCAACGGAGTGACCGGCTGGCGGCTGTGCGAA CGCATTCTGGCGGCTGCTGCTGCGGGCATTGTGGAACAATGCTGTACCAGCATCTGCTC CCTCTACCAGCTGGAGAACTACTGCAACTAG (SEQ ID NO: 29) |
| Insulin NLuc A Delta B27-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACCGCCGGAAGCGGGAGGACCTGCAGG TGGGGCAGGTGGAGCTGGGCGGGGGCCCAGGCCCTGGTGCAGGCAGCCTGCAGCCC TTGGCCCTGGAGGGGTCCCGACGCAAGCGTATGGTCTTCACACTCGAAGATTTCGTTGG GGACTGGCGACAGACAGCCGGCTACAACCTGGACCAAGTCCTTGAACAGGGAGGTGT GTCCAGTTTGTTTCAGAATCTCGGGGTGTCCGTAACTCCGATCCAAAGGATTGTCCTGAG CGGTGAAAATGGGCTGAAGATCGACATCCATGTCATCATCCCGTATGAAGGTCTGAGCG GCGACCAAATGGGCCAGATCGAAAAAATTTTTAAGGTGGTGTACCCTGTGGATGATCAT CACTTTAAGGTGATCCTGCACTATGGCACACTGGTAATCGACGGGGTTACGCCGAACAT GATCGACTATTTCGGACGGCCGTATGAAGGCATCGCCGTGTTCGACGGCAAAAAGATCA CTGTAACAGGGACCCTGTGGAACGGCAACAAAATTATCGACGAGCGCCTGATCAACCCC GACGGCTCCCTGCTGTTCCGAGTAACCATCAACGGAGTGACCGGCTGGCGGCTGTGCG AACGCATTCTGGCGGCTGCTGCTGCGGGCATTGTGGAACAATGCTGTACCAGCATCTGC TCCCTCTACCAGCTGGAGAACTACTGCAACTAG (SEQ ID NO: 30) |
| Insulin NLuc A Delta B28-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACGCCGGAAGCGGGAGGACCTGCA GGTGGGGCAGGTGGAGCTGGGCGGGGGCCCAGGCCCTGGTGCAGGCAGCCTGCAGC CCTTGGCCCTGGAGGGGTCCCGACGCAAGCGTATGGTCTTCACACTCGAAGATTTCGTT GGGGACTGGCGACAGACAGCCGGCTACAACCTGGACCAAGTCCTTGAACAGGGAGGT GTGTCCAGTTTGTTTCAGAATCTCGGGGTGTCCGTAACTCCGATCCAAAGGATTGTCCTG |
| | AGCGGTGAAAATGGGCTGAAGATCGACATCCATGTCATCATCCCGTATGAAGGTCTGAG CGGCGACCAAATGGGCCAGATCGAAAAAATTTTTAAGGTGGTGTACCCTGTGGATGATC ATCACTTTAAGGTGATCCTGCACTATGGCACACTGGTAATCGACGGGGTTACGCCGAACA TGATCGACTATTTCGGACGGCCGTATGAAGGCATCGCCGTGTTCGACGGCAAAAAGATC ACTGTAACAGGGACCCTGTGGAACGGCAACAAAATTATCGACGAGCGCCTGATCAACCC CGACGGCTCCCTGCTGTTCCGAGTAACCATCAACGGAGTGACCGGCTGGCGGCTGTGC GAACGCATTCTGGCGGCTGCTGCTGCGGGCATTGTGGAACAATGCTGTACCAGCATCTG CTCCCTCTACCAGCTGGAGAACTACTGCAACTAG (SEQ ID NO: 31) |
| Insulin NLuc A Delta B30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGCGCCGGAAGCGGGAGG ACCTGCAGGTGGGGCAGGTGGAGCTGGGCGGGGGCCCAGGCCCTGGTGCAGGCAGC CTGCAGCCCTTGGCCCTGGAGGGGTCCCGACGCAAGCGTATGGTCTTCACACTCGAAG ATTTCGTTGGGGACTGGCGACAGACAGCCGGCTACAACCTGGACCAAGTCCTTGAACA GGGAGGTGTGTCCAGTTTGTTTCAGAATCTCGGGGTGTCCGTAACTCCGATCCAAAGGA TTGTCCTGAGCGGTGAAAATGGGCTGAAGATCGACATCCATGTCATCATCCCGTATGAAG GTCTGAGCGGCGACCAAATGGGCCAGATCGAAAAAATTTTTAAGGTGGTGTACCCTGTG GATGATCATCACTTTAAGGTGATCCTGCACTATGGCACACTGGTAATCGACGGGGTTACG CCGAACATGATCGACTATTTCGGACGGCCGTATGAAGGCATCGCCGTGTTCGACGGCAA AAAGATCACTGTAACAGGGACCCTGTGGAACGGCAACAAAATTATCGACGAGCGCCTG ATCAACCCCGACGGCTCCCTGCTGTTCCGAGTAACCATCAACGGAGTGACCGGCTGGCG GCTGTGCGAACGCATTCTGGCGGCTGCTGCTGCGGGCATTGTGGAACAATGCTGTACCA GCATCTGCTCCCTCTACCAGCTGGAGAACTACTGCAACTAG (SEQ ID NO: 32) |
| Insulin NLuc A mut A L13A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG |
| | GCGGCTGTGCGAACGCATTCTGGCGGCTGCTGCTGCGGGCATTGTGGAACAATGCTGT ACCAGCATCTGCTCCGCCTACCAGCTGGAGAACTACTGCAACTAG (SEQ ID NO: 33) |
| Insulin NLuc A mut A L13W | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG GCGGCTGTGCGAACGCATTCTGGCGGCTGCTGCTGCGGGCATTGTGGAACAATGCTGT ACCAGCATCTGCTCCTGGTACCAGCTGGAGAACTACTGCAACTAG (SEQ ID NO: 34) |
| Insulin NLuc A mut B F1A | ATGGCCCTGTGGATGCGCCTCCTGCCCCTGCTGGCGCTGCTGGCCCTCTGGGGACCTGA CCCAGCCGCAGCCGCTGTGAACCAACACCTGTGCGGCTCACACCTGGTGGAAGCTCTCT ACCTAGTGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG GCGGCTGTGCGAACGCATTCTGGCGGCTGCTGCTGCGGGCATTGTGGAACAATGCTGT ACCAGCATCTGCTCCCTCTACCAGCTGGAGAACTACTGCAACTAG (SEQ ID NO: 35) |
| Insulin NLuc A mut B F25D | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCGACTACACACCCAAGACCCGCCGGAAGCGGG AGGACCTGCAGGTGGGGCAGGTGGAGCTGGGCGGGGGCCCAGGCCCTGGTGCAGGC AGCCTGCAGCCCTTGGCCCTGGAGGGGTCCCGACGCAAGCGTATGGTCTTCACACTCG |
| | AAGATTTCGTTGGGGACTGGCGACAGACAGCCGGCTACAACCTGGACCAAGTCCTTGA ACAGGGAGGTGTGTCCAGTTTGTTTCAGAATCTCGGGGTGTCCGTAACTCCGATCCAAA GGATTGTCCTGAGCGGTGAAAATGGGCTGAAGATCGACATCCATGTCATCATCCCGTATG AAGGTCTGAGCGGCGACCAAATGGGCCAGATCGAAAAAATTTTTAAGGTGGTGTACCC TGTGGATGATCATCACTTTAAGGTGATCCTGCACTATGGCACACTGGTAATCGACGGGGT TACGCCGAACATGATCGACTATTTCGGACGGCCGTATGAAGGCATCGCCGTGTTCGACG GCAAAAAGATCACTGTAACAGGGACCCTGTGGAACGGCAACAAAATTATCGACGAGCG CCTGATCAACCCCGACGGCTCCCTGCTGTTCCGAGTAACCATCAACGGAGTGACCGGCT GGCGGCTGTGCGAACGCATTCTGGCGGCTGCTGCTGCGGGCATTGTGGAACAATGCTG TACCAGCATCTGCTCCCTCTACCAGCTGGAGAACTACTGCAACTAG (SEQ ID NO: 36) |
| Insulin NLuc A mut B L17A V18A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACGCAGCGTGCGGGGAACGAGGCTTCTTCTACACACCCAAGACCCGCCGGAAGCGGG AGGACCTGCAGGTGGGGCAGGTGGAGCTGGGCGGGGGCCCAGGCCCTGGTGCAGGC AGCCTGCAGCCCTTGGCCCTGGAGGGGTCCCGACGCAAGCGTATGGTCTTCACACTCG AAGATTTCGTTGGGGACTGGCGACAGACAGCCGGCTACAACCTGGACCAAGTCCTTGA ACAGGGAGGTGTGTCCAGTTTGTTTCAGAATCTCGGGGTGTCCGTAACTCCGATCCAAA GGATTGTCCTGAGCGGTGAAAATGGGCTGAAGATCGACATCCATGTCATCATCCCGTATG AAGGTCTGAGCGGCGACCAAATGGGCCAGATCGAAAAAATTTTTAAGGTGGTGTACCC TGTGGATGATCATCACTTTAAGGTGATCCTGCACTATGGCACACTGGTAATCGACGGGGT TACGCCGAACATGATCGACTATTTCGGACGGCCGTATGAAGGCATCGCCGTGTTCGACG GCAAAAAGATCACTGTAACAGGGACCCTGTGGAACGGCAACAAAATTATCGACGAGCG CCTGATCAACCCCGACGGCTCCCTGCTGTTCCGAGTAACCATCAACGGAGTGACCGGCT GGCGGCTGTGCGAACGCATTCTGGCGGCTGCTGCGGGCATTGTGGAACAATGCTG TACCAGCATCTGCTCCCTCTACCAGCTGGAGAACTACTGCAACTAG (SEQ ID NO: 37) |
| Insulin NLuc A mut B L17A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACGCAGTGTGCGGGGAACGAGGCTTCTTCTACACACCCAAGACCCGCCGGAAGCGGG AGGACCTGCAGGTGGGGCAGGTGGAGCTGGGCGGGGGCCCAGGCCCTGGTGCAGGC AGCCTGCAGCCCTTGGCCCTGGAGGGGTCCCGACGCAAGCGTATGGTCTTCACACTCG AAGATTTCGTTGGGGACTGGCGACAGACAGCCGGCTACAACCTGGACCAAGTCCTTGA ACAGGGAGGTGTGTCCAGTTTGTTTCAGAATCTCGGGGTGTCCGTAACTCCGATCCAAA GGATTGTCCTGAGCGGTGAAAATGGGCTGAAGATCGACATCCATGTCATCATCCCGTATG AAGGTCTGAGCGGCGACCAAATGGGCCAGATCGAAAAAATTTTTAAGGTGGTGTACCC TGTGGATGATCATCACTTTAAGGTGATCCTGCACTATGGCACACTGGTAATCGACGGGGT TACGCCGAACATGATCGACTATTTCGGACGGCCGTATGAAGGCATCGCCGTGTTCGACG GCAAAAAGATCACTGTAACAGGGACCCTGTGGAACGGCAACAAAATTATCGACGAGCG |
| | CCTGATCAACCCCGACGGCTCCCTGCTGTTCCGAGTAACCATCAACGGAGTGACCGGCT GGCGGCTGTGCGAACGCATTCTGGCGGCTGCTGCTGCGGGCATTGTGGAACAATGCTG TACCAGCATCTGCTCCCTCTACCAGCTGGAGAACTACTGCAACTAG (SEQ ID NO: 38) |
| Insulin NLuc A mut B R22A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG GCGGCTGTGCGAACGCATTCTGGCGGCTGCTGCTGCGGGCATTGTGGAACAATGCTGT ACCAGCATCTGCTCCCTCTACCAGCTGGAGAACTACTGCAACTAG (SEQ ID NO: 39) |
| Insulin NLuc A mut B V18A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGCGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG GCGGCTGTGCGAACGCATTCTGGCGGCTGCTGCTGCGGGCATTGTGGAACAATGCTGT ACCAGCATCTGCTCCCTCTACCAGCTGGAGAACTACTGCAACTAG (SEQ ID NO: 40) |
| Insulin NLuc A mut B V2G | ATGGCCCTGTGGATGCGCCTCCTGCCCCTGCTGGCGCTGCTGGCCCTCTGGGGACCTGA CCCAGCCGCAGCCTTTGGGAACCAACACCTGTGCGGCTCACACCTGGTGGAAGCTCTCT ACCTAGTGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA |
| | GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG GCGGCTGTGCGAACGCATTCTGGCGGCTGCTGCTGCGGGCATTGTGGAACAATGCTGT ACCAGCATCTGCTCCCTCTACCAGCTGGAGAACTACTGCAACTAG (SEQ ID NO: 41) |
| Insulin NLuc A mut BR22A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAAGCAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG GCGGCTGTGCGAACGCATTCTGGCGGCTGCTGCTGCGGGCATTGTGGAACAATGCTGT ACCAGCATCTGCTCCCTCTACCAGCTGGAGAACTACTGCAACTAG (SEQ ID NO: 42) |

**Table 4: exemplary amino acid sequences of recombinant insulin luciferase backbone antigen molecules.**

| Name | Sequence (SEQ ID NO) |
|---|---|
| Insulin A NLuc | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTGGCATTGTGGAACAATG CTGTACCAGCATCTGCTCcctctaccagctggagaactactgcaacGGTGGAGGCATGGTCTTCA CACTCGAAGATTTCGTTGGGGACTGGCGACAGACAGCCGGCTACAACCTGGACCAAGT |
| | CCTTGAACAGGGAGGTGTGTCCAGTTTGTTTCAGAATCTCGGGGTGTCCGTAACTCCGA TCCAAAGGATTGTCCTGAGCGGTGAAAATGGGCTGAAGATCGACATCCATGTCATCATC CCGTATGAAGGTCTGAGCGGCGACCAAATGGGCCAGATCGAAAAAATTTTTAAGGTGG TGTACCCTGTGGATGATCATCACTTTAAGGTGATCCTGCACTATGGCACACTGGTAATCG ACGGGGTTACGCCGAACATGATCGACTATTTCGGACGGCCGTATGAAGGCATCGCCGTG TTCGACGGCAAAAAGATCACTGTAACAGGGACCCTGTGGAACGGCAACAAAATTATCG ACGAGCGCCTGATCAACCCCGACGGCTCCCTGCTGTTCCGAGTAACCATCAACGGAGTG ACCGGCTGGCGGCTGTGCGAACGCATTCTGGCGtag (SEQ ID NO: 43) |
| Insulin B NLuc | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGAAGCGGGAGGAC CTGCAGGTGGGGCAGGTGGAGCTGGGCGGGGGCCCAGGCCCTGGTGCAGGCAGCCT GCAGCCCTTGGCCCTGGAGGGGTCCCGACGCAAGCGTGGCATTGTGGAACAATGCTGT ACCAGCATCTGCTCCCTCTACCAGCTGGAGAACTACTGCAACTAG (SEQ ID NO: 44) |
| Insulin NLuc A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGAAGCGGGA GGACCTGCAGGTGGGGCAGGTGGAGCTGGGCGGGGGCCCAGGCCCTGGTGCAGGCA GCCTGCAGCCCTTGGCCCTGGAGGGGTCCCGACGCAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG |
| | GCGGCTGTGCGAACGCATTCTGGCGGCTGCTGCTGCGGGCATTGTGGAACAATGCTGT ACCAGCATCTGCTCCCTCTACCAGCTGGAGAACTACTGCAACTAG (SEQ ID NO: 45) |
| secNLuc insulin | ATGAACTCCTTCTCCACAAGCGCCTTCGGTCCAGTTGCCTTCTCCCTGGGCCTGCTCCTG GTGTTGCCTGCTGCCTTCCCTGCCCCAGTCTTCACACTCGAAGATTTCGTTGGGGACTG GCGACAGACAGCCGGCTACAACCTGGACCAAGTCCTTGAACAGGGAGGTGTGTCCAGT TTGTTTCAGAATCTCGGGGTGTCCGTAACTCCGATCCAAAGGATTGTCCTGAGCGGTGA AAATGGGCTGAAGATCGACATCCATGTCATCATCCCGTATGAAGGTCTGAGCGGCGACC AAATGGGCCAGATCGAAAAAATTTTTAAGGTGGTGTACCCTGTGGATGATCATCACTTTA AGGTGATCCTGCACTATGGCACACTGGTAATCGACGGGGTTACGCCGAACATGATCGAC TATTTCGGACGGCCGTATGAAGGCATCGCCGTGTTCGACGGCAAAAAGATCACTGTAAC AGGGACCCTGTGGAACGGCAACAAAATTATCGACGAGCGCCTGATCAACCCCGACGGC TCCCTGCTGTTCCGAGTAACCATCAACGGAGTGACCGGCTGGCGGCTGTGCGAACGCAT TCTGGCGGGTGGAGGCTTTGTGAACCAACACCTGTGCGGCTCACACCTGGTGGAAGCT CTCTACCTAGTGTGCGGGGAACGAGGCTTCTTCTACACACCCAAGACCCGCCGGAAGC GGGAGGACCTGCAGGTGGGGCAGGTGGAGCTGGGCGGGGGCCCAGGCCCTGGTGCA GGCAGCCTGCAGCCCTTGGCCCTGGAGGGGTCCCGACGCAAGCGTGGCATTGTGGAA CAATGCTGTACCAGCATCTGCTCCCTCTACCAGCTGGAGAACTACTGCAACTAG (SEQ ID NO: 46) |

**Table 5: exemplary amino acid sequences of recombinant insulin antigen molecules in a pCMVTnT vector.**

| Name | Sequence (SEQ ID NO) |
|---|---|
| pCMVTnT INS Bchaindel24-30Nluc | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC |
| | TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACtcgaGaattCATGGCCCTGTGGATGCGCCTCCTGCCCCTGCTG GCGCTGCTGGCCCTCTGGGGACCTGACCCAGCCGCAGCCTTTGTGAACCAACACCTGT GCGGCTCACACCTGGTGGAAGCTCTCTACCTAGTGTGCGGGGAACGAGGCATGGTCTT CACACTCGAAGATTTCGTTGGGGACTGGCGACAGACAGCCGGCTACAACCTGGACCAA GTCCTTGAACAGGGAGGTGTGTCCAGTTTGTTTCAGAATCTCGGGGTGTCCGTAACTCC GATCCAAAGGATTGTCCTGAGCGGTGAAAATGGGCTGAAGATCGACATCCATGTCATCA TCCCGTATGAAGGTCTGAGCGGCGACCAAATGGGCCAGATCGAAAAAATTTTTAAGGT GGTGTACCCTGTGGATGATCATCACTTTAAGGTGATCCTGCACTATGGCACACTGGTAAT CGACGGGGTTACGCCGAACATGATCGACTATTTCGGACGGCCGTATGAAGGCATCGCCG TGTTCGACGGCAAAAAGATCACTGTAACAGGGACCCTGTGGAACGGCAACAAAATTAT CGACGAGCGCCTGATCAACCCCGACGGCTCCCTGCTGTTCCGAGTAACCATCAACGGAG TGACCGGCTGGCGGCTGTGCGAACGCATTCTGGCGgctgctgctgcGCGCCGGAAGcggG AGGACCTGCAGGTGGGGCAGGTGGAGCTGGGCGGGggccCaggcGCAGGCAGCCTGC AGCCCTTGGCCCTGGAGGGGTCCcgacgcAAGCGTGGCATTGTGGAACAATGCTGTACC AGCATCTGCTCcctctaccagctggagaactactgcaactagGaattCACGCGTGgtacCTCTAGAGT CGACCCGGGCGGCCGCTTCGAGCAGACATGATAAGATACATTGATGAGTTTGGACAAA CCACAACTAGAATGCAGTGAAAAAAATGCTTTATTTGTGAAATTTGTGATGCTATTGCTT TATTTGTAACCATTATAAGCTGCAATAAACAAGTTAACAACAACAATTGCATTCATTTTATG TTTCAGGTTCAGGGGGAGATGTGGGAGGTTTTTTAAAGCAAGTAAAACCTCTACAAAT GTGGTAAAATCGATAAGGATCCGGGCTGGCGTAATAGCGAAGAGGCCCGCACCGATCG CCCTTCCCAACAGTTGCGCAGCCTGAATGGCGAATGGACGCGCCCTGTAGCGGCGCATT AAGCGCGGCGGGTGTGGTGGTTACGCGCAGCGTGACCGCTACACTTGCCAGCGCCCTA GCGCCCGCTCCTTTCGCTTTCTTCCCTTCCTTTCTCGCCACGTTCGCCGGCTTTCCCCGTC AAGCTCTAAATCGGGGGCTCCCTTTAGGGTTCCGATTTAGTGCTTTACGGCACCTCGACC CCAAAAAACTTGATTAGGGTGATGGTTCACGTAGTGGGCCATCGCCCTGATAGACGGTT TTTCGCCCTTTGACGTTGGAGTCCACGTTCTTTAATAGTGGACTCTTGTTCCAAACTGGA ACAACACTCAACCCTATCTCGGTCTATTCTTTTGATTTATAAGGGATTTTGCCGATTTCGG CCTATTGGTTAAAAAATGAGCTGATTTAACAAAAATTTAACGCGAATTTTAACAAAATATT AACGCTTACAATTTCCTGATGCGGTATTTTCTCCTTACGCATCTGTGCGGTATTTCACACC GCATATGGTGCACTCTCAGTACAATCTGCTCTGATGCCGCATAGTTAAGCCAGCCCCGAC ACCCGCCAACACCCGCTGACGCGCCCTGACGGGCTTGTCTGCTCCCGGCATCCGCTTAC AGACAAGCTGTGACCGTCTCCGGGAGCTGCATGTGTCAGAGGTTTTCACCGTCATCACC GAAACGCGCGAGACGAAAGGGCCTCGTGATACGCCTATTTTTATAGGTTAATGTCATGAT AATAATGGTTTCTTAGACGTCAGGTGGCACTTTTCGGGGAAATGTGCGCGGAACCCCTA |
| | TTTGTTTATTTTTCTAAATACATTCAAATATGTATCCGCTCATGAGACAATAACCCTGATAA ATGCTTCAATAATATTGAAAAAGGAAGAGTATGAGTATTCAACATTTCCGTGTCGCCCTTA TTCCCTTTTTTGCGGCATTTTGCCTTCCTGTTTTTGCTCACCCAGAAACGCTGGTGAAAG TAAAAGATGCTGAAGATCAGTTGGGTGCACGAGTGGGTTACATCGAACTGGATCTCAAC AGCGGTAAGATCCTTGAGAGTTTTCGCCCCGAAGAACGTTTTCCAATGATGAGCACTTT TAAAGTTCTGCTATGTGGCGCGGTATTATCCCGTATTGACGCCGGGCAAGAGCAACTCG GTCGCCGCATACACTATTCTCAGAATGACTTGGTTGAGTACTCACCAGTCACAGAAAAGC ATCTTACGGATGGCATGACAGTAAGAGAATTATGCAGTGCTGCCATAACCATGAGTGATA ACACTGCGGCCAACTTACTTCTGACAACGATCGGAGGACCGAAGGAGCTAACCGCTTT TTTGCACAACATGGGGGATCATGTAACTCGCCTTGATCGTTGGGAACCGGAGCTGAATG AAGCCATACCAAACGACGAGCGTGACACCACGATGCCTGTAGCAATGGCAACAACGTT GCGCAAACTATTAACTGGCGAACTACTTACTCTAGCTTCCCGGCAACAATTAATAGACTG GATGGAGGCGGATAAAGTTGCAGGACCACTTCTGCGCTCGGCCCTTCCGGCTGGCTGG TTTATTGCTGATAAATCTGGAGCCGGTGAGCGTGGGTCTCGCGGTATCATTGCAGCACTG GGGCCAGATGGTAAGCCCTCCCGTATCGTAGTTATCTACACGACGGGGAGTCAGGCAAC TATGGATGAACGAAATAGACAGATCGCTGAGATAGGTGCCTCACTGATTAAGCATTGGTA ACTGTCAGACCAAGTTTACTCATATATACTTTAGATTGATTTAAAACTTCATTTTTAATTTA AAAGGATCTAGGTGAAGATCCTTTTTGATAATCTCATGACCAAAATCCCTTAACGTGAGT TTTCGTTCCACTGAGCGTCAGACCCCGTAGAAAAGATCAAAGGATCTTCTTGAGATCCTT TTTTTCTGCGCGTAATCTGCTGCTTGCAAACAAAAAAACCACCGCTACCAGCGGTGGTT TGTTTGCCGGATCAAGAGCTACCAACTCTTTTTCCGAAGGTAACTGGCTTCAGCAGAGC GCAGATACCAAATACTGTTCTTCTAGTGTAGCCGTAGTTAGGCCACCACTTCAAGAACTC TGTAGCACCGCCTACATACCTCGCTCTGCTAATCCTGTTACCAGTGGCTGCTGCCAGTGG CGATAAGTCGTGTCTTACCGGGTTGGACTCAAGACGATAGTTACCGGATAAGGCGCAGC GGTCGGGCTGAACGGGGGGTTCGTGCACACAGCCCAGCTTGGAGCGAACGACCTACA CCGAACTGAGATACCTACAGCGTGAGCTATGAGAAAGCGCCACGCTTCCCGAAGGGAG AAAGGCGGACAGGTATCCGGTAAGCGGCAGGGTCGGAACAGGAGAGCGCACGAGGG AGCTTCCAGGGGGAAACGCCTGGTATCTTTATAGTCCTGTCGGGTTTCGCCACCTCTGA CTTGAGCGTCGATTTTTGTGATGCTCGTCAGGGGGGCGGAGCCTATGGAAAAACGCCA GCAACGCGGCCTTTTTACGGTTCCTGGCCTTTTGCTGGCCTTTTGCTCACATGGCTCGAC AGATCT (SEQ ID NO: 47) |
| pCMVTnT IN Bchaindel25-30Nluc | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG |
| | CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACtcgaGaattCATGGCCCTGTGGATGCGCCTCCTGCCCCTGCTG GCGCTGCTGGCCCTCTGGGGACCTGACCCAGCCGCAGCCTTTGTGAACCAACACCTGT GCGGCTCACACCTGGTGGAAGCTCTCTACCTAGTGTGCGGGGAACGAGGCTTCATGGT CTTCACACTCGAAGATTTCGTTGGGGACTGGCGACAGACAGCCGGCTACAACCTGGAC CAAGTCCTTGAACAGGGAGGTGTGTCCAGTTTGTTTCAGAATCTCGGGGTGTCCGTAAC TCCGATCCAAAGGATTGTCCTGAGCGGTGAAAATGGGCTGAAGATCGACATCCATGTCA TCATCCCGTATGAAGGTCTGAGCGGCGACCAAATGGGCCAGATCGAAAAAATTTTTAAG GTGGTGTACCCTGTGGATGATCATCACTTTAAGGTGATCCTGCACTATGGCACACTGGTA ATCGACGGGGTTACGCCGAACATGATCGACTATTTCGGACGGCCGTATGAAGGCATCGC CGTGTTCGACGGCAAAAAGATCACTGTAACAGGGACCCTGTGGAACGGCAACAAAATT ATCGACGAGCGCCTGATCAACCCCGACGGCTCCCTGCTGTTCCGAGTAACCATCAACGG AGTGACCGGCTGGCGGCTGTGCGAACGCATTCTGGCGgctgctgctgcGCGCCGGAAGcgg GAGGACCTGCAGGTGGGGCAGGTGGAGCTGGGCGGGggccCaggcGCAGGCAGCCTG CAGCCCTTGGCCCTGGAGGGGTCCcgacgcAAGCGTGGCATTGTGGAACAATGCTGTAC CAGCATCTGCTCcctctaccagctggagaactactgcaactagGaattCACGCGTGgtacCTCTAGAG TCGACCCGGGCGGCCGCTTCGAGCAGACATGATAAGATACATTGATGAGTTTGGACAAA CCACAACTAGAATGCAGTGAAAAAAATGCTTTATTTGTGAAATTTGTGATGCTATTGCTT TATTTGTAACCATTATAAGCTGCAATAAACAAGTTAACAACAACAATTGCATTCATTTTATG TTTCAGGTTCAGGGGGAGATGTGGGAGGTTTTTTAAAGCAAGTAAAACCTCTACAAAT GTGGTAAAATCGATAAGGATCCGGGCTGGCGTAATAGCGAAGAGGCCCGCACCGATCG CCCTTCCCAACAGTTGCGCAGCCTGAATGGCGAATGGACGCGCCCTGTAGCGGCGCATT AAGCGCGGCGGGTGTGGTGGTTACGCGCAGCGTGACCGCTACACTTGCCAGCGCCCTA GCGCCCGCTCCTTTCGCTTTCTTCCCTTCCTTTCTCGCCACGTTCGCCGGCTTTCCCCGTC AAGCTCTAAATCGGGGGCTCCCTTTAGGGTTCCGATTTAGTGCTTTACGGCACCTCGACC CCAAAAAACTTGATTAGGGTGATGGTTCACGTAGTGGGCCATCGCCCTGATAGACGGTT |
| | TTTCGCCCTTTGACGTTGGAGTCCACGTTCTTTAATAGTGGACTCTTGTTCCAAACTGGA ACAACACTCAACCCTATCTCGGTCTATTCTTTTGATTTATAAGGGATTTTGCCGATTTCGG CCTATTGGTTAAAAAATGAGCTGATTTAACAAAAATTTAACGCGAATTTTAACAAAATATT AACGCTTACAATTTCCTGATGCGGTATTTTCTCCTTACGCATCTGTGCGGTATTTCACACC GCATATGGTGCACTCTCAGTACAATCTGCTCTGATGCCGCATAGTTAAGCCAGCCCCGAC ACCCGCCAACACCCGCTGACGCGCCCTGACGGGCTTGTCTGCTCCCGGCATCCGCTTAC AGACAAGCTGTGACCGTCTCCGGGAGCTGCATGTGTCAGAGGTTTTCACCGTCATCACC GAAACGCGCGAGACGAAAGGGCCTCGTGATACGCCTATTTTTATAGGTTAATGTCATGAT AATAATGGTTTCTTAGACGTCAGGTGGCACTTTTCGGGGAAATGTGCGCGGAACCCCTA TTTGTTTATTTTTCTAAATACATTCAAATATGTATCCGCTCATGAGACAATAACCCTGATAA ATGCTTCAATAATATTGAAAAAGGAAGAGTATGAGTATTCAACATTTCCGTGTCGCCCTTA TTCCCTTTTTTGCGGCATTTTGCCTTCCTGTTTTTGCTCACCCAGAAACGCTGGTGAAAG TAAAAGATGCTGAAGATCAGTTGGGTGCACGAGTGGGTTACATCGAACTGGATCTCAAC AGCGGTAAGATCCTTGAGAGTTTTCGCCCCGAAGAACGTTTTCCAATGATGAGCACTTT TAAAGTTCTGCTATGTGGCGCGGTATTATCCCGTATTGACGCCGGGCAAGAGCAACTCG GTCGCCGCATACACTATTCTCAGAATGACTTGGTTGAGTACTCACCAGTCACAGAAAAGC ATCTTACGGATGGCATGACAGTAAGAGAATTATGCAGTGCTGCCATAACCATGAGTGATA ACACTGCGGCCAACTTACTTCTGACAACGATCGGAGGACCGAAGGAGCTAACCGCTTT TTTGCACAACATGGGGGATCATGTAACTCGCCTTGATCGTTGGGAACCGGAGCTGAATG AAGCCATACCAAACGACGAGCGTGACACCACGATGCCTGTAGCAATGGCAACAACGTT GCGCAAACTATTAACTGGCGAACTACTTACTCTAGCTTCCCGGCAACAATTAATAGACTG GATGGAGGCGGATAAAGTTGCAGGACCACTTCTGCGCTCGGCCCTTCCGGCTGGCTGG TTTATTGCTGATAAATCTGGAGCCGGTGAGCGTGGGTCTCGCGGTATCATTGCAGCACTG GGGCCAGATGGTAAGCCCTCCCGTATCGTAGTTATCTACACGACGGGGAGTCAGGCAAC TATGGATGAACGAAATAGACAGATCGCTGAGATAGGTGCCTCACTGATTAAGCATTGGTA ACTGTCAGACCAAGTTTACTCATATATACTTTAGATTGATTTAAAACTTCATTTTTAATTTA AAAGGATCTAGGTGAAGATCCTTTTTGATAATCTCATGACCAAAATCCCTTAACGTGAGT TTTCGTTCCACTGAGCGTCAGACCCCGTAGAAAAGATCAAAGGATCTTCTTGAGATCCTT TTTTTCTGCGCGTAATCTGCTGCTTGCAAACAAAAAAACCACCGCTACCAGCGGTGGTT TGTTTGCCGGATCAAGAGCTACCAACTCTTTTTCCGAAGGTAACTGGCTTCAGCAGAGC GCAGATACCAAATACTGTTCTTCTAGTGTAGCCGTAGTTAGGCCACCACTTCAAGAACTC TGTAGCACCGCCTACATACCTCGCTCTGCTAATCCTGTTACCAGTGGCTGCTGCCAGTGG CGATAAGTCGTGTCTTACCGGGTTGGACTCAAGACGATAGTTACCGGATAAGGCGCAGC GGTCGGGCTGAACGGGGGGTTCGTGCACACAGCCCAGCTTGGAGCGAACGACCTACA CCGAACTGAGATACCTACAGCGTGAGCTATGAGAAAGCGCCACGCTTCCCGAAGGGAG AAAGGCGGACAGGTATCCGGTAAGCGGCAGGGTCGGAACAGGAGAGCGCACGAGGG AGCTTCCAGGGGGAAACGCCTGGTATCTTTATAGTCCTGTCGGGTTTCGCCACCTCTGA |
| | CTTGAGCGTCGATTTTTGTGATGCTCGTCAGGGGGGCGGAGCCTATGGAAAAACGCCA GCAACGCGGCCTTTTTACGGTTCCTGGCCTTTTGCTGGCCTTTTGCTCACATGGCTCGAC AGATCT (SEQ ID NO: 48) |
| pCMVTnT IN Bchaindel26-30Nluc | S TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACtcgaGaattCATGGCCCTGTGGATGCGCCTCCTGCCCCTGCTG GCGCTGCTGGCCCTCTGGGGACCTGACCCAGCCGCAGCCTTTGTGAACCAACACCTGT GCGGCTCACACCTGGTGGAAGCTCTCTACCTAGTGTGCGGGGAACGAGGCTTCTTCATG GTCTTCACACTCGAAGATTTCGTTGGGGACTGGCGACAGACAGCCGGCTACAACCTGG ACCAAGTCCTTGAACAGGGAGGTGTGTCCAGTTTGTTTCAGAATCTCGGGGTGTCCGTA ACTCCGATCCAAAGGATTGTCCTGAGCGGTGAAAATGGGCTGAAGATCGACATCCATGT CATCATCCCGTATGAAGGTCTGAGCGGCGACCAAATGGGCCAGATCGAAAAAATTTTTA AGGTGGTGTACCCTGTGGATGATCATCACTTTAAGGTGATCCTGCACTATGGCACACTGG TAATCGACGGGGTTACGCCGAACATGATCGACTATTTCGGACGGCCGTATGAAGGCATC GCCGTGTTCGACGGCAAAAAGATCACTGTAACAGGGACCCTGTGGAACGGCAACAAAA TTATCGACGAGCGCCTGATCAACCCCGACGGCTCCCTGCTGTTCCGAGTAACCATCAAC GGAGTGACCGGCTGGCGGCTGTGCGAACGCATTCTGGCGgctgctgctgcGCGCCGGAAG cggGAGGACCTGCAGGTGGGGCAGGTGGAGCTGGGCGGGggccCaggcGCAGGCAGCC TGCAGCCCTTGGCCCTGGAGGGGTCCcgacgcAAGCGTGGCATTGTGGAACAATGCTGT ACCAGCATCTGCTCcctctaccagctggagaactactgcaactagGaattCACGCGTGgtacCTCTAG AGTCGACCCGGGCGGCCGCTTCGAGCAGACATGATAAGATACATTGATGAGTTTGGAC |
| | AAACCACAACTAGAATGCAGTGAAAAAAATGCTTTATTTGTGAAATTTGTGATGCTATTG CTTTATTTGTAACCATTATAAGCTGCAATAAACAAGTTAACAACAACAATTGCATTCATTTT ATGTTTCAGGTTCAGGGGGAGATGTGGGAGGTTTTTTAAAGCAAGTAAAACCTCTACA AATGTGGTAAAATCGATAAGGATCCGGGCTGGCGTAATAGCGAAGAGGCCCGCACCGA TCGCCCTTCCCAACAGTTGCGCAGCCTGAATGGCGAATGGACGCGCCCTGTAGCGGCG CATTAAGCGCGGCGGGTGTGGTGGTTACGCGCAGCGTGACCGCTACACTTGCCAGCGC CCTAGCGCCCGCTCCTTTCGCTTTCTTCCCTTCCTTTCTCGCCACGTTCGCCGGCTTTCCC CGTCAAGCTCTAAATCGGGGGCTCCCTTTAGGGTTCCGATTTAGTGCTTTACGGCACCTC GACCCCAAAAAACTTGATTAGGGTGATGGTTCACGTAGTGGGCCATCGCCCTGATAGAC GGTTTTTCGCCCTTTGACGTTGGAGTCCACGTTCTTTAATAGTGGACTCTTGTTCCAAAC TGGAACAACACTCAACCCTATCTCGGTCTATTCTTTTGATTTATAAGGGATTTTGCCGATT TCGGCCTATTGGTTAAAAAATGAGCTGATTTAACAAAAATTTAACGCGAATTTTAACAAA ATATTAACGCTTACAATTTCCTGATGCGGTATTTTCTCCTTACGCATCTGTGCGGTATTTCA CACCGCATATGGTGCACTCTCAGTACAATCTGCTCTGATGCCGCATAGTTAAGCCAGCCC CGACACCCGCCAACACCCGCTGACGCGCCCTGACGGGCTTGTCTGCTCCCGGCATCCGC TTACAGACAAGCTGTGACCGTCTCCGGGAGCTGCATGTGTCAGAGGTTTTCACCGTCAT CACCGAAACGCGCGAGACGAAAGGGCCTCGTGATACGCCTATTTTTATAGGTTAATGTC ATGATAATAATGGTTTCTTAGACGTCAGGTGGCACTTTTCGGGGAAATGTGCGCGGAAC CCCTATTTGTTTATTTTTCTAAATACATTCAAATATGTATCCGCTCATGAGACAATAACCCTG ATAAATGCTTCAATAATATTGAAAAAGGAAGAGTATGAGTATTCAACATTTCCGTGTCGCC CTTATTCCCTTTTTTGCGGCATTTTGCCTTCCTGTTTTTGCTCACCCAGAAACGCTGGTGA AAGTAAAAGATGCTGAAGATCAGTTGGGTGCACGAGTGGGTTACATCGAACTGGATCTC AACAGCGGTAAGATCCTTGAGAGTTTTCGCCCCGAAGAACGTTTTCCAATGATGAGCAC TTTTAAAGTTCTGCTATGTGGCGCGGTATTATCCCGTATTGACGCCGGGCAAGAGCAACT CGGTCGCCGCATACACTATTCTCAGAATGACTTGGTTGAGTACTCACCAGTCACAGAAAA GCATCTTACGGATGGCATGACAGTAAGAGAATTATGCAGTGCTGCCATAACCATGAGTG ATAACACTGCGGCCAACTTACTTCTGACAACGATCGGAGGACCGAAGGAGCTAACCGC TTTTTTGCACAACATGGGGGATCATGTAACTCGCCTTGATCGTTGGGAACCGGAGCTGA ATGAAGCCATACCAAACGACGAGCGTGACACCACGATGCCTGTAGCAATGGCAACAAC GTTGCGCAAACTATTAACTGGCGAACTACTTACTCTAGCTTCCCGGCAACAATTAATAGA CTGGATGGAGGCGGATAAAGTTGCAGGACCACTTCTGCGCTCGGCCCTTCCGGCTGGC TGGTTTATTGCTGATAAATCTGGAGCCGGTGAGCGTGGGTCTCGCGGTATCATTGCAGC ACTGGGGCCAGATGGTAAGCCCTCCCGTATCGTAGTTATCTACACGACGGGGAGTCAGG CAACTATGGATGAACGAAATAGACAGATCGCTGAGATAGGTGCCTCACTGATTAAGCATT GGTAACTGTCAGACCAAGTTTACTCATATATACTTTAGATTGATTTAAAACTTCATTTTTAA TTTAAAAGGATCTAGGTGAAGATCCTTTTTGATAATCTCATGACCAAAATCCCTTAACGTG AGTTTTCGTTCCACTGAGCGTCAGACCCCGTAGAAAAGATCAAAGGATCTTCTTGAGAT |
| | CCTTTTTTTCTGCGCGTAATCTGCTGCTTGCAAACAAAAAAACCACCGCTACCAGCGGT GGTTTGTTTGCCGGATCAAGAGCTACCAACTCTTTTTCCGAAGGTAACTGGCTTCAGCA GAGCGCAGATACCAAATACTGTTCTTCTAGTGTAGCCGTAGTTAGGCCACCACTTCAAGA ACTCTGTAGCACCGCCTACATACCTCGCTCTGCTAATCCTGTTACCAGTGGCTGCTGCCA GTGGCGATAAGTCGTGTCTTACCGGGTTGGACTCAAGACGATAGTTACCGGATAAGGCG CAGCGGTCGGGCTGAACGGGGGGTTCGTGCACACAGCCCAGCTTGGAGCGAACGACC TACACCGAACTGAGATACCTACAGCGTGAGCTATGAGAAAGCGCCACGCTTCCCGAAG GGAGAAAGGCGGACAGGTATCCGGTAAGCGGCAGGGTCGGAACAGGAGAGCGCACG AGGGAGCTTCCAGGGGGAAACGCCTGGTATCTTTATAGTCCTGTCGGGTTTCGCCACCT CTGACTTGAGCGTCGATTTTTGTGATGCTCGTCAGGGGGGCGGAGCCTATGGAAAAAC GCCAGCAACGCGGCCTTTTTACGGTTCCTGGCCTTTTGCTGGCCTTTTGCTCACATGGCT CGACAGATCT (SEQ ID NO: 49) |
| pCMVTnT INS Bchaindel27-30Nluc | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACtcgaGaattCATGGCCCTGTGGATGCGCCTCCTGCCCCTGCTG GCGCTGCTGGCCCTCTGGGGACCTGACCCAGCCGCAGCCTTTGTGAACCAACACCTGT GCGGCTCACACCTGGTGGAAGCTCTCTACCTAGTGTGCGGGGAACGAGGCTTCTTCTAC ATGGTCTTCACACTCGAAGATTTCGTTGGGGACTGGCGACAGACAGCCGGCTACAACCT GGACCAAGTCCTTGAACAGGGAGGTGTGTCCAGTTTGTTTCAGAATCTCGGGGTGTCC GTAACTCCGATCCAAAGGATTGTCCTGAGCGGTGAAAATGGGCTGAAGATCGACATCCA TGTCATCATCCCGTATGAAGGTCTGAGCGGCGACCAAATGGGCCAGATCGAAAAAATTT |
| | TTAAGGTGGTGTACCCTGTGGATGATCATCACTTTAAGGTGATCCTGCACTATGGCACAC TGGTAATCGACGGGGTTACGCCGAACATGATCGACTATTTCGGACGGCCGTATGAAGGC ATCGCCGTGTTCGACGGCAAAAAGATCACTGTAACAGGGACCCTGTGGAACGGCAACA AAATTATCGACGAGCGCCTGATCAACCCCGACGGCTCCCTGCTGTTCCGAGTAACCATCA ACGGAGTGACCGGCTGGCGGCTGTGCGAACGCATTCTGGCGgctgctgctgcGCGCCGGA AGcggGAGGACCTGCAGGTGGGGCAGGTGGAGCTGGGCGGGggccCaggcGCAGGCAG CCTGCAGCCCTTGGCCCTGGAGGGGTCCcgacgcAAGCGTGGCATTGTGGAACAATGCT GTACCAGCATCTGCTCcctctaccagctggagaactactgcaactagGaattCACGCGTGgtacCTCT AGAGTCGACCCGGGCGGCCGCTTCGAGCAGACATGATAAGATACATTGATGAGTTTGG ACAAACCACAACTAGAATGCAGTGAAAAAAATGCTTTATTTGTGAAATTTGTGATGCTAT TGCTTTATTTGTAACCATTATAAGCTGCAATAAACAAGTTAACAACAACAATTGCATTCAT TTTATGTTTCAGGTTCAGGGGGAGATGTGGGAGGTTTTTTAAAGCAAGTAAAACCTCTA CAAATGTGGTAAAATCGATAAGGATCCGGGCTGGCGTAATAGCGAAGAGGCCCGCACC GATCGCCCTTCCCAACAGTTGCGCAGCCTGAATGGCGAATGGACGCGCCCTGTAGCGG CGCATTAAGCGCGGCGGGTGTGGTGGTTACGCGCAGCGTGACCGCTACACTTGCCAGC GCCCTAGCGCCCGCTCCTTTCGCTTTCTTCCCTTCCTTTCTCGCCACGTTCGCCGGCTTTC CCCGTCAAGCTCTAAATCGGGGGCTCCCTTTAGGGTTCCGATTTAGTGCTTTACGGCACC TCGACCCCAAAAAACTTGATTAGGGTGATGGTTCACGTAGTGGGCCATCGCCCTGATAG ACGGTTTTTCGCCCTTTGACGTTGGAGTCCACGTTCTTTAATAGTGGACTCTTGTTCCAA ACTGGAACAACACTCAACCCTATCTCGGTCTATTCTTTTGATTTATAAGGGATTTTGCCGA TTTCGGCCTATTGGTTAAAAAATGAGCTGATTTAACAAAAATTTAACGCGAATTTTAACA AAATATTAACGCTTACAATTTCCTGATGCGGTATTTTCTCCTTACGCATCTGTGCGGTATTT CACACCGCATATGGTGCACTCTCAGTACAATCTGCTCTGATGCCGCATAGTTAAGCCAGC CCCGACACCCGCCAACACCCGCTGACGCGCCCTGACGGGCTTGTCTGCTCCCGGCATCC GCTTACAGACAAGCTGTGACCGTCTCCGGGAGCTGCATGTGTCAGAGGTTTTCACCGTC ATCACCGAAACGCGCGAGACGAAAGGGCCTCGTGATACGCCTATTTTTATAGGTTAATGT CATGATAATAATGGTTTCTTAGACGTCAGGTGGCACTTTTCGGGGAAATGTGCGCGGAA CCCCTATTTGTTTATTTTTCTAAATACATTCAAATATGTATCCGCTCATGAGACAATAACCCT GATAAATGCTTCAATAATATTGAAAAAGGAAGAGTATGAGTATTCAACATTTCCGTGTCG CCCTTATTCCCTTTTTTGCGGCATTTTGCCTTCCTGTTTTTGCTCACCCAGAAACGCTGGT GAAAGTAAAAGATGCTGAAGATCAGTTGGGTGCACGAGTGGGTTACATCGAACTGGAT CTCAACAGCGGTAAGATCCTTGAGAGTTTTCGCCCCGAAGAACGTTTTCCAATGATGAG CACTTTTAAAGTTCTGCTATGTGGCGCGGTATTATCCCGTATTGACGCCGGGCAAGAGCA ACTCGGTCGCCGCATACACTATTCTCAGAATGACTTGGTTGAGTACTCACCAGTCACAGA AAAGCATCTTACGGATGGCATGACAGTAAGAGAATTATGCAGTGCTGCCATAACCATGA GTGATAACACTGCGGCCAACTTACTTCTGACAACGATCGGAGGACCGAAGGAGCTAAC CGCTTTTTTGCACAACATGGGGGATCATGTAACTCGCCTTGATCGTTGGGAACCGGAGC |
| | TGAATGAAGCCATACCAAACGACGAGCGTGACACCACGATGCCTGTAGCAATGGCAAC AACGTTGCGCAAACTATTAACTGGCGAACTACTTACTCTAGCTTCCCGGCAACAATTAAT AGACTGGATGGAGGCGGATAAAGTTGCAGGACCACTTCTGCGCTCGGCCCTTCCGGCT GGCTGGTTTATTGCTGATAAATCTGGAGCCGGTGAGCGTGGGTCTCGCGGTATCATTGC AGCACTGGGGCCAGATGGTAAGCCCTCCCGTATCGTAGTTATCTACACGACGGGGAGTC AGGCAACTATGGATGAACGAAATAGACAGATCGCTGAGATAGGTGCCTCACTGATTAAG CATTGGTAACTGTCAGACCAAGTTTACTCATATATACTTTAGATTGATTTAAAACTTCATTT TTAATTTAAAAGGATCTAGGTGAAGATCCTTTTTGATAATCTCATGACCAAAATCCCTTAA CGTGAGTTTTCGTTCCACTGAGCGTCAGACCCCGTAGAAAAGATCAAAGGATCTTCTTG AGATCCTTTTTTTCTGCGCGTAATCTGCTGCTTGCAAACAAAAAAACCACCGCTACCAGC GGTGGTTTGTTTGCCGGATCAAGAGCTACCAACTCTTTTTCCGAAGGTAACTGGCTTCA GCAGAGCGCAGATACCAAATACTGTTCTTCTAGTGTAGCCGTAGTTAGGCCACCACTTCA AGAACTCTGTAGCACCGCCTACATACCTCGCTCTGCTAATCCTGTTACCAGTGGCTGCTG CCAGTGGCGATAAGTCGTGTCTTACCGGGTTGGACTCAAGACGATAGTTACCGGATAAG GCGCAGCGGTCGGGCTGAACGGGGGGTTCGTGCACACAGCCCAGCTTGGAGCGAAC GACCTACACCGAACTGAGATACCTACAGCGTGAGCTATGAGAAAGCGCCACGCTTCCCG AAGGGAGAAAGGCGGACAGGTATCCGGTAAGCGGCAGGGTCGGAACAGGAGAGCGC ACGAGGGAGCTTCCAGGGGGAAACGCCTGGTATCTTTATAGTCCTGTCGGGTTTCGCCA CCTCTGACTTGAGCGTCGATTTTTGTGATGCTCGTCAGGGGGGCGGAGCCTATGGAAAA ACGCCAGCAACGCGGCCTTTTTACGGTTCCTGGCCTTTTGCTGGCCTTTTGCTCACATG GCTCGACAGATCT (SEQ ID NO: 50) |
| pCMVTnT INS Bchaindel28-30Nluc | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT |
| | GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACtcgaGaattCATGGCCCTGTGGATGCGCCTCCTGCCCCTGCTG GCGCTGCTGGCCCTCTGGGGACCTGACCCAGCCGCAGCCTTTGTGAACCAACACCTGT GCGGCTCACACCTGGTGGAAGCTCTCTACCTAGTGTGCGGGGAACGAGGCTTCTTCTAC ACAATGGTCTTCACACTCGAAGATTTCGTTGGGGACTGGCGACAGACAGCCGGCTACA ACCTGGACCAAGTCCTTGAACAGGGAGGTGTGTCCAGTTTGTTTCAGAATCTCGGGGT GTCCGTAACTCCGATCCAAAGGATTGTCCTGAGCGGTGAAAATGGGCTGAAGATCGAC ATCCATGTCATCATCCCGTATGAAGGTCTGAGCGGCGACCAAATGGGCCAGATCGAAAA AATTTTTAAGGTGGTGTACCCTGTGGATGATCATCACTTTAAGGTGATCCTGCACTATGG CACACTGGTAATCGACGGGGTTACGCCGAACATGATCGACTATTTCGGACGGCCGTATG AAGGCATCGCCGTGTTCGACGGCAAAAAGATCACTGTAACAGGGACCCTGTGGAACGG CAACAAAATTATCGACGAGCGCCTGATCAACCCCGACGGCTCCCTGCTGTTCCGAGTAA CCATCAACGGAGTGACCGGCTGGCGGCTGTGCGAACGCATTCTGGCGgctgctgctgcGC GCCGGAAGcggGAGGACCTGCAGGTGGGGCAGGTGGAGCTGGGCGGGggccCaggcGC AGGCAGCCTGCAGCCCTTGGCCCTGGAGGGGTCCcgacgcAAGCGTGGCATTGTGGAAC AATGCTGTACCAGCATCTGCTCcctctaccagctggagaactactgcaactagGaattCACGCGTGgt acCTCTAGAGTCGACCCGGGCGGCCGCTTCGAGCAGACATGATAAGATACATTGATGAG TTTGGACAAACCACAACTAGAATGCAGTGAAAAAAATGCTTTATTTGTGAAATTTGTGAT GCTATTGCTTTATTTGTAACCATTATAAGCTGCAATAAACAAGTTAACAACAACAATTGCA TTCATTTTATGTTTCAGGTTCAGGGGGAGATGTGGGAGGTTTTTTAAAGCAAGTAAAAC CTCTACAAATGTGGTAAAATCGATAAGGATCCGGGCTGGCGTAATAGCGAAGAGGCCCG CACCGATCGCCCTTCCCAACAGTTGCGCAGCCTGAATGGCGAATGGACGCGCCCTGTAG CGGCGCATTAAGCGCGGCGGGTGTGGTGGTTACGCGCAGCGTGACCGCTACACTTGCC AGCGCCCTAGCGCCCGCTCCTTTCGCTTTCTTCCCTTCCTTTCTCGCCACGTTCGCCGGC TTTCCCCGTCAAGCTCTAAATCGGGGGCTCCCTTTAGGGTTCCGATTTAGTGCTTTACGG CACCTCGACCCCAAAAAACTTGATTAGGGTGATGGTTCACGTAGTGGGCCATCGCCCTG ATAGACGGTTTTTCGCCCTTTGACGTTGGAGTCCACGTTCTTTAATAGTGGACTCTTGTT CCAAACTGGAACAACACTCAACCCTATCTCGGTCTATTCTTTTGATTTATAAGGGATTTTG CCGATTTCGGCCTATTGGTTAAAAAATGAGCTGATTTAACAAAAATTTAACGCGAATTTT AACAAAATATTAACGCTTACAATTTCCTGATGCGGTATTTTCTCCTTACGCATCTGTGCGG TATTTCACACCGCATATGGTGCACTCTCAGTACAATCTGCTCTGATGCCGCATAGTTAAGC CAGCCCCGACACCCGCCAACACCCGCTGACGCGCCCTGACGGGCTTGTCTGCTCCCGG CATCCGCTTACAGACAAGCTGTGACCGTCTCCGGGAGCTGCATGTGTCAGAGGTTTTCA CCGTCATCACCGAAACGCGCGAGACGAAAGGGCCTCGTGATACGCCTATTTTTATAGGT TAATGTCATGATAATAATGGTTTCTTAGACGTCAGGTGGCACTTTTCGGGGAAATGTGCG CGGAACCCCTATTTGTTTATTTTTCTAAATACATTCAAATATGTATCCGCTCATGAGACAAT |
| | AACCCTGATAAATGCTTCAATAATATTGAAAAAGGAAGAGTATGAGTATTCAACATTTCC GTGTCGCCCTTATTCCCTTTTTTGCGGCATTTTGCCTTCCTGTTTTTGCTCACCCAGAAAC GCTGGTGAAAGTAAAAGATGCTGAAGATCAGTTGGGTGCACGAGTGGGTTACATCGAA CTGGATCTCAACAGCGGTAAGATCCTTGAGAGTTTTCGCCCCGAAGAACGTTTTCCAAT GATGAGCACTTTTAAAGTTCTGCTATGTGGCGCGGTATTATCCCGTATTGACGCCGGGCA AGAGCAACTCGGTCGCCGCATACACTATTCTCAGAATGACTTGGTTGAGTACTCACCAGT CACAGAAAAGCATCTTACGGATGGCATGACAGTAAGAGAATTATGCAGTGCTGCCATAA CCATGAGTGATAACACTGCGGCCAACTTACTTCTGACAACGATCGGAGGACCGAAGGA GCTAACCGCTTTTTTGCACAACATGGGGGATCATGTAACTCGCCTTGATCGTTGGGAAC CGGAGCTGAATGAAGCCATACCAAACGACGAGCGTGACACCACGATGCCTGTAGCAAT GGCAACAACGTTGCGCAAACTATTAACTGGCGAACTACTTACTCTAGCTTCCCGGCAAC AATTAATAGACTGGATGGAGGCGGATAAAGTTGCAGGACCACTTCTGCGCTCGGCCCTT CCGGCTGGCTGGTTTATTGCTGATAAATCTGGAGCCGGTGAGCGTGGGTCTCGCGGTAT CATTGCAGCACTGGGGCCAGATGGTAAGCCCTCCCGTATCGTAGTTATCTACACGACGG GGAGTCAGGCAACTATGGATGAACGAAATAGACAGATCGCTGAGATAGGTGCCTCACT GATTAAGCATTGGTAACTGTCAGACCAAGTTTACTCATATATACTTTAGATTGATTTAAAA CTTCATTTTTAATTTAAAAGGATCTAGGTGAAGATCCTTTTTGATAATCTCATGACCAAAA TCCCTTAACGTGAGTTTTCGTTCCACTGAGCGTCAGACCCCGTAGAAAAGATCAAAGGA TCTTCTTGAGATCCTTTTTTTCTGCGCGTAATCTGCTGCTTGCAAACAAAAAAACCACCG CTACCAGCGGTGGTTTGTTTGCCGGATCAAGAGCTACCAACTCTTTTTCCGAAGGTAAC TGGCTTCAGCAGAGCGCAGATACCAAATACTGTTCTTCTAGTGTAGCCGTAGTTAGGCCA CCACTTCAAGAACTCTGTAGCACCGCCTACATACCTCGCTCTGCTAATCCTGTTACCAGTG GCTGCTGCCAGTGGCGATAAGTCGTGTCTTACCGGGTTGGACTCAAGACGATAGTTACC GGATAAGGCGCAGCGGTCGGGCTGAACGGGGGGTTCGTGCACACAGCCCAGCTTGGA GCGAACGACCTACACCGAACTGAGATACCTACAGCGTGAGCTATGAGAAAGCGCCACG CTTCCCGAAGGGAGAAAGGCGGACAGGTATCCGGTAAGCGGCAGGGTCGGAACAGG AGAGCGCACGAGGGAGCTTCCAGGGGGAAACGCCTGGTATCTTTATAGTCCTGTCGGG TTTCGCCACCTCTGACTTGAGCGTCGATTTTTGTGATGCTCGTCAGGGGGGCGGAGCCT ATGGAAAAACGCCAGCAACGCGGCCTTTTTACGGTTCCTGGCCTTTTGCTGGCCTTTTG CTCACATGGCTCGACAGATCT (SEQ ID NO: 51) |
| pCMVTnT insulin Nluc-Achain A L13A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG |
| | GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCCGCCGGAAGcggGAGGACCTGCAGGTGGGGCAGGTGGAGCTGGG CGGGggccCaggccCTGGTGCAGGCAGCCTGCAGCCCTTGGCCCTGGAGGGGTCCcgacg cAAGCGTATGGTCTTCACACTCGAAGATTTCGTTGGGGACTGGCGACAGACAGCCGGC TACAACCTGGACCAAGTCCTTGAACAGGGAGGTGTGTCCAGTTTGTTTCAGAATCTCGG GGTGTCCGTAACTCCGATCCAAAGGATTGTCCTGAGCGGTGAAAATGGGCTGAAGATC GACATCCATGTCATCATCCCGTATGAAGGTCTGAGCGGCGACCAAATGGGCCAGATCGA AAAAATTTTTAAGGTGGTGTACCCTGTGGATGATCATCACTTTAAGGTGATCCTGCACTAT GGCACACTGGTAATCGACGGGGTTACGCCGAACATGATCGACTATTTCGGACGGCCGTA TGAAGGCATCGCCGTGTTCGACGGCAAAAAGATCACTGTAACAGGGACCCTGTGGAAC GGCAACAAAATTATCGACGAGCGCCTGATCAACCCCGACGGCTCCCTGCTGTTCCGAGT AACCATCAACGGAGTGACCGGCTGGCGGCTGTGCGAACGCATTCTGGCGgctgctgctgc GGGCATTGTGGAACAATGCTGTACCAGCATCTGCTCcGCctaccagctggagaactactgcaact agGaattCACGCGTGGTACCTCTAGAGTCGACCCGGGCGGCCGCTTCGAGCAGACATGAT AAGATACATTGATGAGTTTGGACAAACCACAACTAGAATGCAGTGAAAAAAATGCTTTA TTTGTGAAATTTGTGATGCTATTGCTTTATTTGTAACCATTATAAGCTGCAATAAACAAGTT AACAACAACAATTGCATTCATTTTATGTTTCAGGTTCAGGGGGAGATGTGGGAGGTTTT TTAAAGCAAGTAAAACCTCTACAAATGTGGTAAAATCGATAAGGATCCGGGCTGGCGTA ATAGCGAAGAGGCCCGCACCGATCGCCCTTCCCAACAGTTGCGCAGCCTGAATGGCGA ATGGACGCGCCCTGTAGCGGCGCATTAAGCGCGGCGGGTGTGGTGGTTACGCGCAGCG TGACCGCTACACTTGCCAGCGCCCTAGCGCCCGCTCCTTTCGCTTTCTTCCCTTCCTTTCT CGCCACGTTCGCCGGCTTTCCCCGTCAAGCTCTAAATCGGGGGCTCCCTTTAGGGTTCC GATTTAGTGCTTTACGGCACCTCGACCCCAAAAAACTTGATTAGGGTGATGGTTCACGTA GTGGGCCATCGCCCTGATAGACGGTTTTTCGCCCTTTGACGTTGGAGTCCACGTTCTTTA |
| | ATAGTGGACTCTTGTTCCAAACTGGAACAACACTCAACCCTATCTCGGTCTATTCTTTTGA TTTATAAGGGATTTTGCCGATTTCGGCCTATTGGTTAAAAAATGAGCTGATTTAACAAAA ATTTAACGCGAATTTTAACAAAATATTAACGCTTACAATTTCCTGATGCGGTATTTTCTCCT TACGCATCTGTGCGGTATTTCACACCGCATATGGTGCACTCTCAGTACAATCTGCTCTGAT GCCGCATAGTTAAGCCAGCCCCGACACCCGCCAACACCCGCTGACGCGCCCTGACGGG CTTGTCTGCTCCCGGCATCCGCTTACAGACAAGCTGTGACCGTCTCCGGGAGCTGCATG TGTCAGAGGTTTTCACCGTCATCACCGAAACGCGCGAGACGAAAGGGCCTCGTGATAC GCCTATTTTTATAGGTTAATGTCATGATAATAATGGTTTCTTAGACGTCAGGTGGCACTTT TCGGGGAAATGTGCGCGGAACCCCTATTTGTTTATTTTTCTAAATACATTCAAATATGTAT CCGCTCATGAGACAATAACCCTGATAAATGCTTCAATAATATTGAAAAAGGAAGAGTATG AGTATTCAACATTTCCGTGTCGCCCTTATTCCCTTTTTTGCGGCATTTTGCCTTCCTGTTTT TGCTCACCCAGAAACGCTGGTGAAAGTAAAAGATGCTGAAGATCAGTTGGGTGCACGA GTGGGTTACATCGAACTGGATCTCAACAGCGGTAAGATCCTTGAGAGTTTTCGCCCCGA AGAACGTTTTCCAATGATGAGCACTTTTAAAGTTCTGCTATGTGGCGCGGTATTATCCCG TATTGACGCCGGGCAAGAGCAACTCGGTCGCCGCATACACTATTCTCAGAATGACTTGG TTGAGTACTCACCAGTCACAGAAAAGCATCTTACGGATGGCATGACAGTAAGAGAATTA TGCAGTGCTGCCATAACCATGAGTGATAACACTGCGGCCAACTTACTTCTGACAACGATC GGAGGACCGAAGGAGCTAACCGCTTTTTTGCACAACATGGGGGATCATGTAACTCGCC TTGATCGTTGGGAACCGGAGCTGAATGAAGCCATACCAAACGACGAGCGTGACACCAC GATGCCTGTAGCAATGGCAACAACGTTGCGCAAACTATTAACTGGCGAACTACTTACTCT AGCTTCCCGGCAACAATTAATAGACTGGATGGAGGCGGATAAAGTTGCAGGACCACTTC TGCGCTCGGCCCTTCCGGCTGGCTGGTTTATTGCTGATAAATCTGGAGCCGGTGAGCGT GGGTCTCGCGGTATCATTGCAGCACTGGGGCCAGATGGTAAGCCCTCCCGTATCGTAGT TATCTACACGACGGGGAGTCAGGCAACTATGGATGAACGAAATAGACAGATCGCTGAG ATAGGTGCCTCACTGATTAAGCATTGGTAACTGTCAGACCAAGTTTACTCATATATACTTT AGATTGATTTAAAACTTCATTTTTAATTTAAAAGGATCTAGGTGAAGATCCTTTTTGATAA TCTCATGACCAAAATCCCTTAACGTGAGTTTTCGTTCCACTGAGCGTCAGACCCCGTAGA AAAGATCAAAGGATCTTCTTGAGATCCTTTTTTTCTGCGCGTAATCTGCTGCTTGCAAAC AAAAAAACCACCGCTACCAGCGGTGGTTTGTTTGCCGGATCAAGAGCTACCAACTCTTT TTCCGAAGGTAACTGGCTTCAGCAGAGCGCAGATACCAAATACTGTTCTTCTAGTGTAGC CGTAGTTAGGCCACCACTTCAAGAACTCTGTAGCACCGCCTACATACCTCGCTCTGCTAA TCCTGTTACCAGTGGCTGCTGCCAGTGGCGATAAGTCGTGTCTTACCGGGTTGGACTCA AGACGATAGTTACCGGATAAGGCGCAGCGGTCGGGCTGAACGGGGGGTTCGTGCACA CAGCCCAGCTTGGAGCGAACGACCTACACCGAACTGAGATACCTACAGCGTGAGCTATG AGAAAGCGCCACGCTTCCCGAAGGGAGAAAGGCGGACAGGTATCCGGTAAGCGGCAG GGTCGGAACAGGAGAGCGCACGAGGGAGCTTCCAGGGGGAAACGCCTGGTATCTTTA TAGTCCTGTCGGGTTTCGCCACCTCTGACTTGAGCGTCGATTTTTGTGATGCTCGTCAGG |
| | GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 52) |
| pCMVTnT insulin Nluc-Achain chain F1A | c- B TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCGCTGTGAACCAACACCTG TGCGGCTCACACCTGGTGGAAGCTCTCTACCTAGTGTGCGGGGAACGAGGCTTCTTCTA CACACCCAAGACCCGCCGGAAGcggGAGGACCTGCAGGTGGGGCAGGTGGAGCTGGG CGGGggccCaggccCTGGTGCAGGCAGCCTGCAGCCCTTGGCCCTGGAGGGGTCCcgacg cAAGCGTATGGTCTTCACACTCGAAGATTTCGTTGGGGACTGGCGACAGACAGCCGGC TACAACCTGGACCAAGTCCTTGAACAGGGAGGTGTGTCCAGTTTGTTTCAGAATCTCGG GGTGTCCGTAACTCCGATCCAAAGGATTGTCCTGAGCGGTGAAAATGGGCTGAAGATC GACATCCATGTCATCATCCCGTATGAAGGTCTGAGCGGCGACCAAATGGGCCAGATCGA AAAAATTTTTAAGGTGGTGTACCCTGTGGATGATCATCACTTTAAGGTGATCCTGCACTAT GGCACACTGGTAATCGACGGGGTTACGCCGAACATGATCGACTATTTCGGACGGCCGTA TGAAGGCATCGCCGTGTTCGACGGCAAAAAGATCACTGTAACAGGGACCCTGTGGAAC GGCAACAAAATTATCGACGAGCGCCTGATCAACCCCGACGGCTCCCTGCTGTTCCGAGT AACCATCAACGGAGTGACCGGCTGGCGGCTGTGCGAACGCATTCTGGCGgctgctgctgc GGGCATTGTGGAACAATGCTGTACCAGCATCTGCTCcctctaccagctggagaactactgcaacta gGaattCACGCGTGGTACCTCTAGAGTCGACCCGGGCGGCCGCTTCGAGCAGACATGATA AGATACATTGATGAGTTTGGACAAACCACAACTAGAATGCAGTGAAAAAAATGCTTTATT |
| | TGTGAAATTTGTGATGCTATTGCTTTATTTGTAACCATTATAAGCTGCAATAAACAAGTTA ACAACAACAATTGCATTCATTTTATGTTTCAGGTTCAGGGGGAGATGTGGGAGGTTTTT TAAAGCAAGTAAAACCTCTACAAATGTGGTAAAATCGATAAGGATCCGGGCTGGCGTAA TAGCGAAGAGGCCCGCACCGATCGCCCTTCCCAACAGTTGCGCAGCCTGAATGGCGAA TGGACGCGCCCTGTAGCGGCGCATTAAGCGCGGCGGGTGTGGTGGTTACGCGCAGCGT GACCGCTACACTTGCCAGCGCCCTAGCGCCCGCTCCTTTCGCTTTCTTCCCTTCCTTTCTC GCCACGTTCGCCGGCTTTCCCCGTCAAGCTCTAAATCGGGGGCTCCCTTTAGGGTTCCG ATTTAGTGCTTTACGGCACCTCGACCCCAAAAAACTTGATTAGGGTGATGGTTCACGTAG TGGGCCATCGCCCTGATAGACGGTTTTTCGCCCTTTGACGTTGGAGTCCACGTTCTTTAA TAGTGGACTCTTGTTCCAAACTGGAACAACACTCAACCCTATCTCGGTCTATTCTTTTGAT TTATAAGGGATTTTGCCGATTTCGGCCTATTGGTTAAAAAATGAGCTGATTTAACAAAAA TTTAACGCGAATTTTAACAAAATATTAACGCTTACAATTTCCTGATGCGGTATTTTCTCCTT ACGCATCTGTGCGGTATTTCACACCGCATATGGTGCACTCTCAGTACAATCTGCTCTGATG CCGCATAGTTAAGCCAGCCCCGACACCCGCCAACACCCGCTGACGCGCCCTGACGGGC TTGTCTGCTCCCGGCATCCGCTTACAGACAAGCTGTGACCGTCTCCGGGAGCTGCATGT GTCAGAGGTTTTCACCGTCATCACCGAAACGCGCGAGACGAAAGGGCCTCGTGATACG CCTATTTTTATAGGTTAATGTCATGATAATAATGGTTTCTTAGACGTCAGGTGGCACTTTTC GGGGAAATGTGCGCGGAACCCCTATTTGTTTATTTTTCTAAATACATTCAAATATGTATCC GCTCATGAGACAATAACCCTGATAAATGCTTCAATAATATTGAAAAAGGAAGAGTATGAG TATTCAACATTTCCGTGTCGCCCTTATTCCCTTTTTTGCGGCATTTTGCCTTCCTGTTTTTG CTCACCCAGAAACGCTGGTGAAAGTAAAAGATGCTGAAGATCAGTTGGGTGCACGAGT GGGTTACATCGAACTGGATCTCAACAGCGGTAAGATCCTTGAGAGTTTTCGCCCCGAAG AACGTTTTCCAATGATGAGCACTTTTAAAGTTCTGCTATGTGGCGCGGTATTATCCCGTAT TGACGCCGGGCAAGAGCAACTCGGTCGCCGCATACACTATTCTCAGAATGACTTGGTTG AGTACTCACCAGTCACAGAAAAGCATCTTACGGATGGCATGACAGTAAGAGAATTATGC AGTGCTGCCATAACCATGAGTGATAACACTGCGGCCAACTTACTTCTGACAACGATCGG AGGACCGAAGGAGCTAACCGCTTTTTTGCACAACATGGGGGATCATGTAACTCGCCTTG ATCGTTGGGAACCGGAGCTGAATGAAGCCATACCAAACGACGAGCGTGACACCACGAT GCCTGTAGCAATGGCAACAACGTTGCGCAAACTATTAACTGGCGAACTACTTACTCTAGC TTCCCGGCAACAATTAATAGACTGGATGGAGGCGGATAAAGTTGCAGGACCACTTCTGC GCTCGGCCCTTCCGGCTGGCTGGTTTATTGCTGATAAATCTGGAGCCGGTGAGCGTGGG TCTCGCGGTATCATTGCAGCACTGGGGCCAGATGGTAAGCCCTCCCGTATCGTAGTTATC TACACGACGGGGAGTCAGGCAACTATGGATGAACGAAATAGACAGATCGCTGAGATAG GTGCCTCACTGATTAAGCATTGGTAACTGTCAGACCAAGTTTACTCATATATACTTTAGAT TGATTTAAAACTTCATTTTTAATTTAAAAGGATCTAGGTGAAGATCCTTTTTGATAATCTC ATGACCAAAATCCCTTAACGTGAGTTTTCGTTCCACTGAGCGTCAGACCCCGTAGAAAA GATCAAAGGATCTTCTTGAGATCCTTTTTTTCTGCGCGTAATCTGCTGCTTGCAAACAAA |
| | AAAACCACCGCTACCAGCGGTGGTTTGTTTGCCGGATCAAGAGCTACCAACTCTTTTTC CGAAGGTAACTGGCTTCAGCAGAGCGCAGATACCAAATACTGTTCTTCTAGTGTAGCCG TAGTTAGGCCACCACTTCAAGAACTCTGTAGCACCGCCTACATACCTCGCTCTGCTAATCC TGTTACCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCACACAG CCCAGCTTGGAGCGAACGACCTACACCGAACTGAGATACCTACAGCGTGAGCTATGAG AAAGCGCCACGCTTCCCGAAGGGAGAAAGGCGGACAGGTATCCGGTAAGCGGCAGG GTCGGAACAGGAGAGCGCACGAGGGAGCTTCCAGGGGGAAACGCCTGGTATCTTTAT AGTCCTGTCGGGTTTCGCCACCTCTGACTTGAGCGTCGATTTTTGTGATGCTCGTCAGG GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 53) |
| pCMVTnT insulin Nluc-Achain B L17A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACGCAGTGTGCGGGGAACGAGGCTTCTTCT ACACACCCAAGACCCGCCGGAAGcggGAGGACCTGCAGGTGGGGCAGGTGGAGCTGG GCGGGggccCaggccCTGGTGCAGGCAGCCTGCAGCCCTTGGCCCTGGAGGGGTCCcgac gcAAGCGTATGGTCTTCACACTCGAAGATTTCGTTGGGGACTGGCGACAGACAGCCGGC TACAACCTGGACCAAGTCCTTGAACAGGGAGGTGTGTCCAGTTTGTTTCAGAATCTCGG GGTGTCCGTAACTCCGATCCAAAGGATTGTCCTGAGCGGTGAAAATGGGCTGAAGATC |
| | GACATCCATGTCATCATCCCGTATGAAGGTCTGAGCGGCGACCAAATGGGCCAGATCGA AAAAATTTTTAAGGTGGTGTACCCTGTGGATGATCATCACTTTAAGGTGATCCTGCACTAT GGCACACTGGTAATCGACGGGGTTACGCCGAACATGATCGACTATTTCGGACGGCCGTA TGAAGGCATCGCCGTGTTCGACGGCAAAAAGATCACTGTAACAGGGACCCTGTGGAAC GGCAACAAAATTATCGACGAGCGCCTGATCAACCCCGACGGCTCCCTGCTGTTCCGAGT AACCATCAACGGAGTGACCGGCTGGCGGCTGTGCGAACGCATTCTGGCGgctgctgctgc GGGCATTGTGGAACAATGCTGTACCAGCATCTGCTCcctctaccagctggagaactactgcaacta gGaattCACGCGTGGTACCTCTAGAGTCGACCCGGGCGGCCGCTTCGAGCAGACATGATA AGATACATTGATGAGTTTGGACAAACCACAACTAGAATGCAGTGAAAAAAATGCTTTATT TGTGAAATTTGTGATGCTATTGCTTTATTTGTAACCATTATAAGCTGCAATAAACAAGTTA ACAACAACAATTGCATTCATTTTATGTTTCAGGTTCAGGGGGAGATGTGGGAGGTTTTT TAAAGCAAGTAAAACCTCTACAAATGTGGTAAAATCGATAAGGATCCGGGCTGGCGTAA TAGCGAAGAGGCCCGCACCGATCGCCCTTCCCAACAGTTGCGCAGCCTGAATGGCGAA TGGACGCGCCCTGTAGCGGCGCATTAAGCGCGGCGGGTGTGGTGGTTACGCGCAGCGT GACCGCTACACTTGCCAGCGCCCTAGCGCCCGCTCCTTTCGCTTTCTTCCCTTCCTTTCTC GCCACGTTCGCCGGCTTTCCCCGTCAAGCTCTAAATCGGGGGCTCCCTTTAGGGTTCCG ATTTAGTGCTTTACGGCACCTCGACCCCAAAAAACTTGATTAGGGTGATGGTTCACGTAG TGGGCCATCGCCCTGATAGACGGTTTTTCGCCCTTTGACGTTGGAGTCCACGTTCTTTAA TAGTGGACTCTTGTTCCAAACTGGAACAACACTCAACCCTATCTCGGTCTATTCTTTTGAT TTATAAGGGATTTTGCCGATTTCGGCCTATTGGTTAAAAAATGAGCTGATTTAACAAAAA TTTAACGCGAATTTTAACAAAATATTAACGCTTACAATTTCCTGATGCGGTATTTTCTCCTT ACGCATCTGTGCGGTATTTCACACCGCATATGGTGCACTCTCAGTACAATCTGCTCTGATG CCGCATAGTTAAGCCAGCCCCGACACCCGCCAACACCCGCTGACGCGCCCTGACGGGC TTGTCTGCTCCCGGCATCCGCTTACAGACAAGCTGTGACCGTCTCCGGGAGCTGCATGT GTCAGAGGTTTTCACCGTCATCACCGAAACGCGCGAGACGAAAGGGCCTCGTGATACG CCTATTTTTATAGGTTAATGTCATGATAATAATGGTTTCTTAGACGTCAGGTGGCACTTTTC GGGGAAATGTGCGCGGAACCCCTATTTGTTTATTTTTCTAAATACATTCAAATATGTATCC GCTCATGAGACAATAACCCTGATAAATGCTTCAATAATATTGAAAAAGGAAGAGTATGAG TATTCAACATTTCCGTGTCGCCCTTATTCCCTTTTTTGCGGCATTTTGCCTTCCTGTTTTTG CTCACCCAGAAACGCTGGTGAAAGTAAAAGATGCTGAAGATCAGTTGGGTGCACGAGT GGGTTACATCGAACTGGATCTCAACAGCGGTAAGATCCTTGAGAGTTTTCGCCCCGAAG AACGTTTTCCAATGATGAGCACTTTTAAAGTTCTGCTATGTGGCGCGGTATTATCCCGTAT TGACGCCGGGCAAGAGCAACTCGGTCGCCGCATACACTATTCTCAGAATGACTTGGTTG AGTACTCACCAGTCACAGAAAAGCATCTTACGGATGGCATGACAGTAAGAGAATTATGC AGTGCTGCCATAACCATGAGTGATAACACTGCGGCCAACTTACTTCTGACAACGATCGG AGGACCGAAGGAGCTAACCGCTTTTTTGCACAACATGGGGGATCATGTAACTCGCCTTG ATCGTTGGGAACCGGAGCTGAATGAAGCCATACCAAACGACGAGCGTGACACCACGAT |
| | GCCTGTAGCAATGGCAACAACGTTGCGCAAACTATTAACTGGCGAACTACTTACTCTAGC TTCCCGGCAACAATTAATAGACTGGATGGAGGCGGATAAAGTTGCAGGACCACTTCTGC GCTCGGCCCTTCCGGCTGGCTGGTTTATTGCTGATAAATCTGGAGCCGGTGAGCGTGGG TCTCGCGGTATCATTGCAGCACTGGGGCCAGATGGTAAGCCCTCCCGTATCGTAGTTATC TACACGACGGGGAGTCAGGCAACTATGGATGAACGAAATAGACAGATCGCTGAGATAG GTGCCTCACTGATTAAGCATTGGTAACTGTCAGACCAAGTTTACTCATATATACTTTAGAT TGATTTAAAACTTCATTTTTAATTTAAAAGGATCTAGGTGAAGATCCTTTTTGATAATCTC ATGACCAAAATCCCTTAACGTGAGTTTTCGTTCCACTGAGCGTCAGACCCCGTAGAAAA GATCAAAGGATCTTCTTGAGATCCTTTTTTTCTGCGCGTAATCTGCTGCTTGCAAACAAA AAAACCACCGCTACCAGCGGTGGTTTGTTTGCCGGATCAAGAGCTACCAACTCTTTTTC CGAAGGTAACTGGCTTCAGCAGAGCGCAGATACCAAATACTGTTCTTCTAGTGTAGCCG TAGTTAGGCCACCACTTCAAGAACTCTGTAGCACCGCCTACATACCTCGCTCTGCTAATCC TGTTACCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCACACAG CCCAGCTTGGAGCGAACGACCTACACCGAACTGAGATACCTACAGCGTGAGCTATGAG AAAGCGCCACGCTTCCCGAAGGGAGAAAGGCGGACAGGTATCCGGTAAGCGGCAGG GTCGGAACAGGAGAGCGCACGAGGGAGCTTCCAGGGGGAAACGCCTGGTATCTTTAT AGTCCTGTCGGGTTTCGCCACCTCTGACTTGAGCGTCGATTTTTGTGATGCTCGTCAGG GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 54) |
| pCMVTnT insulin Nluc-Achain B R22A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG |
| | GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCCGCCGGAAGcggGAGGACCTGCAGGTGGGGCAGGTGGAGCTGGG CGGGggccCaggccCTGGTGCAGGCAGCCTGCAGCCCTTGGCCCTGGAGGGGTCCcgacg cAAGCGTATGGTCTTCACACTCGAAGATTTCGTTGGGGACTGGCGACAGACAGCCGGC TACAACCTGGACCAAGTCCTTGAACAGGGAGGTGTGTCCAGTTTGTTTCAGAATCTCGG GGTGTCCGTAACTCCGATCCAAAGGATTGTCCTGAGCGGTGAAAATGGGCTGAAGATC GACATCCATGTCATCATCCCGTATGAAGGTCTGAGCGGCGACCAAATGGGCCAGATCGA AAAAATTTTTAAGGTGGTGTACCCTGTGGATGATCATCACTTTAAGGTGATCCTGCACTAT GGCACACTGGTAATCGACGGGGTTACGCCGAACATGATCGACTATTTCGGACGGCCGTA TGAAGGCATCGCCGTGTTCGACGGCAAAAAGATCACTGTAACAGGGACCCTGTGGAAC GGCAACAAAATTATCGACGAGCGCCTGATCAACCCCGACGGCTCCCTGCTGTTCCGAGT AACCATCAACGGAGTGACCGGCTGGCGGCTGTGCGAACGCATTCTGGCGgctgctgctgc GGGCATTGTGGAACAATGCTGTACCAGCATCTGCTCcctctaccagctggagaactactgcaacta gGaattCACGCGTGGTACCTCTAGAGTCGACCCGGGCGGCCGCTTCGAGCAGACATGATA AGATACATTGATGAGTTTGGACAAACCACAACTAGAATGCAGTGAAAAAAATGCTTTATT TGTGAAATTTGTGATGCTATTGCTTTATTTGTAACCATTATAAGCTGCAATAAACAAGTTA ACAACAACAATTGCATTCATTTTATGTTTCAGGTTCAGGGGGAGATGTGGGAGGTTTTT TAAAGCAAGTAAAACCTCTACAAATGTGGTAAAATCGATAAGGATCCGGGCTGGCGTAA TAGCGAAGAGGCCCGCACCGATCGCCCTTCCCAACAGTTGCGCAGCCTGAATGGCGAA TGGACGCGCCCTGTAGCGGCGCATTAAGCGCGGCGGGTGTGGTGGTTACGCGCAGCGT GACCGCTACACTTGCCAGCGCCCTAGCGCCCGCTCCTTTCGCTTTCTTCCCTTCCTTTCTC GCCACGTTCGCCGGCTTTCCCCGTCAAGCTCTAAATCGGGGGCTCCCTTTAGGGTTCCG ATTTAGTGCTTTACGGCACCTCGACCCCAAAAAACTTGATTAGGGTGATGGTTCACGTAG TGGGCCATCGCCCTGATAGACGGTTTTTCGCCCTTTGACGTTGGAGTCCACGTTCTTTAA TAGTGGACTCTTGTTCCAAACTGGAACAACACTCAACCCTATCTCGGTCTATTCTTTTGAT TTATAAGGGATTTTGCCGATTTCGGCCTATTGGTTAAAAAATGAGCTGATTTAACAAAAA TTTAACGCGAATTTTAACAAAATATTAACGCTTACAATTTCCTGATGCGGTATTTTCTCCTT ACGCATCTGTGCGGTATTTCACACCGCATATGGTGCACTCTCAGTACAATCTGCTCTGATG CCGCATAGTTAAGCCAGCCCCGACACCCGCCAACACCCGCTGACGCGCCCTGACGGGC TTGTCTGCTCCCGGCATCCGCTTACAGACAAGCTGTGACCGTCTCCGGGAGCTGCATGT GTCAGAGGTTTTCACCGTCATCACCGAAACGCGCGAGACGAAAGGGCCTCGTGATACG CCTATTTTTATAGGTTAATGTCATGATAATAATGGTTTCTTAGACGTCAGGTGGCACTTTTC GGGGAAATGTGCGCGGAACCCCTATTTGTTTATTTTTCTAAATACATTCAAATATGTATCC GCTCATGAGACAATAACCCTGATAAATGCTTCAATAATATTGAAAAAGGAAGAGTATGAG |
| | TATTCAACATTTCCGTGTCGCCCTTATTCCCTTTTTTGCGGCATTTTGCCTTCCTGTTTTTG CTCACCCAGAAACGCTGGTGAAAGTAAAAGATGCTGAAGATCAGTTGGGTGCACGAGT GGGTTACATCGAACTGGATCTCAACAGCGGTAAGATCCTTGAGAGTTTTCGCCCCGAAG AACGTTTTCCAATGATGAGCACTTTTAAAGTTCTGCTATGTGGCGCGGTATTATCCCGTAT TGACGCCGGGCAAGAGCAACTCGGTCGCCGCATACACTATTCTCAGAATGACTTGGTTG AGTACTCACCAGTCACAGAAAAGCATCTTACGGATGGCATGACAGTAAGAGAATTATGC AGTGCTGCCATAACCATGAGTGATAACACTGCGGCCAACTTACTTCTGACAACGATCGG AGGACCGAAGGAGCTAACCGCTTTTTTGCACAACATGGGGGATCATGTAACTCGCCTTG ATCGTTGGGAACCGGAGCTGAATGAAGCCATACCAAACGACGAGCGTGACACCACGAT GCCTGTAGCAATGGCAACAACGTTGCGCAAACTATTAACTGGCGAACTACTTACTCTAGC TTCCCGGCAACAATTAATAGACTGGATGGAGGCGGATAAAGTTGCAGGACCACTTCTGC GCTCGGCCCTTCCGGCTGGCTGGTTTATTGCTGATAAATCTGGAGCCGGTGAGCGTGGG TCTCGCGGTATCATTGCAGCACTGGGGCCAGATGGTAAGCCCTCCCGTATCGTAGTTATC TACACGACGGGGAGTCAGGCAACTATGGATGAACGAAATAGACAGATCGCTGAGATAG GTGCCTCACTGATTAAGCATTGGTAACTGTCAGACCAAGTTTACTCATATATACTTTAGAT TGATTTAAAACTTCATTTTTAATTTAAAAGGATCTAGGTGAAGATCCTTTTTGATAATCTC ATGACCAAAATCCCTTAACGTGAGTTTTCGTTCCACTGAGCGTCAGACCCCGTAGAAAA GATCAAAGGATCTTCTTGAGATCCTTTTTTTCTGCGCGTAATCTGCTGCTTGCAAACAAA AAAACCACCGCTACCAGCGGTGGTTTGTTTGCCGGATCAAGAGCTACCAACTCTTTTTC CGAAGGTAACTGGCTTCAGCAGAGCGCAGATACCAAATACTGTTCTTCTAGTGTAGCCG TAGTTAGGCCACCACTTCAAGAACTCTGTAGCACCGCCTACATACCTCGCTCTGCTAATCC TGTTACCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCACACAG CCCAGCTTGGAGCGAACGACCTACACCGAACTGAGATACCTACAGCGTGAGCTATGAG AAAGCGCCACGCTTCCCGAAGGGAGAAAGGCGGACAGGTATCCGGTAAGCGGCAGG GTCGGAACAGGAGAGCGCACGAGGGAGCTTCCAGGGGGAAACGCCTGGTATCTTTAT AGTCCTGTCGGGTTTCGCCACCTCTGACTTGAGCGTCGATTTTTGTGATGCTCGTCAGG GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 55) |
| pCMVTnT insulin Nluc-Achain Bchain V2G | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT |
| | CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGGGAACCAACACCTG TGCGGCTCACACCTGGTGGAAGCTCTCTACCTAGTGTGCGGGGAACGAGGCTTCTTCTA CACACCCAAGACCCGCCGGAAGcggGAGGACCTGCAGGTGGGGCAGGTGGAGCTGGG CGGGggccCaggccCTGGTGCAGGCAGCCTGCAGCCCTTGGCCCTGGAGGGGTCCcgacg cAAGCGTATGGTCTTCACACTCGAAGATTTCGTTGGGGACTGGCGACAGACAGCCGGC TACAACCTGGACCAAGTCCTTGAACAGGGAGGTGTGTCCAGTTTGTTTCAGAATCTCGG GGTGTCCGTAACTCCGATCCAAAGGATTGTCCTGAGCGGTGAAAATGGGCTGAAGATC GACATCCATGTCATCATCCCGTATGAAGGTCTGAGCGGCGACCAAATGGGCCAGATCGA AAAAATTTTTAAGGTGGTGTACCCTGTGGATGATCATCACTTTAAGGTGATCCTGCACTAT GGCACACTGGTAATCGACGGGGTTACGCCGAACATGATCGACTATTTCGGACGGCCGTA TGAAGGCATCGCCGTGTTCGACGGCAAAAAGATCACTGTAACAGGGACCCTGTGGAAC GGCAACAAAATTATCGACGAGCGCCTGATCAACCCCGACGGCTCCCTGCTGTTCCGAGT AACCATCAACGGAGTGACCGGCTGGCGGCTGTGCGAACGCATTCTGGCGgctgctgctgc GGGCATTGTGGAACAATGCTGTACCAGCATCTGCTCcctctaccagctggagaactactgcaacta gGaattCACGCGTGGTACCTCTAGAGTCGACCCGGGCGGCCGCTTCGAGCAGACATGATA AGATACATTGATGAGTTTGGACAAACCACAACTAGAATGCAGTGAAAAAAATGCTTTATT TGTGAAATTTGTGATGCTATTGCTTTATTTGTAACCATTATAAGCTGCAATAAACAAGTTA ACAACAACAATTGCATTCATTTTATGTTTCAGGTTCAGGGGGAGATGTGGGAGGTTTTT TAAAGCAAGTAAAACCTCTACAAATGTGGTAAAATCGATAAGGATCCGGGCTGGCGTAA TAGCGAAGAGGCCCGCACCGATCGCCCTTCCCAACAGTTGCGCAGCCTGAATGGCGAA TGGACGCGCCCTGTAGCGGCGCATTAAGCGCGGCGGGTGTGGTGGTTACGCGCAGCGT GACCGCTACACTTGCCAGCGCCCTAGCGCCCGCTCCTTTCGCTTTCTTCCCTTCCTTTCTC GCCACGTTCGCCGGCTTTCCCCGTCAAGCTCTAAATCGGGGGCTCCCTTTAGGGTTCCG ATTTAGTGCTTTACGGCACCTCGACCCCAAAAAACTTGATTAGGGTGATGGTTCACGTAG TGGGCCATCGCCCTGATAGACGGTTTTTCGCCCTTTGACGTTGGAGTCCACGTTCTTTAA TAGTGGACTCTTGTTCCAAACTGGAACAACACTCAACCCTATCTCGGTCTATTCTTTTGAT |
| | TTATAAGGGATTTTGCCGATTTCGGCCTATTGGTTAAAAAATGAGCTGATTTAACAAAAA TTTAACGCGAATTTTAACAAAATATTAACGCTTACAATTTCCTGATGCGGTATTTTCTCCTT ACGCATCTGTGCGGTATTTCACACCGCATATGGTGCACTCTCAGTACAATCTGCTCTGATG CCGCATAGTTAAGCCAGCCCCGACACCCGCCAACACCCGCTGACGCGCCCTGACGGGC TTGTCTGCTCCCGGCATCCGCTTACAGACAAGCTGTGACCGTCTCCGGGAGCTGCATGT GTCAGAGGTTTTCACCGTCATCACCGAAACGCGCGAGACGAAAGGGCCTCGTGATACG CCTATTTTTATAGGTTAATGTCATGATAATAATGGTTTCTTAGACGTCAGGTGGCACTTTTC GGGGAAATGTGCGCGGAACCCCTATTTGTTTATTTTTCTAAATACATTCAAATATGTATCC GCTCATGAGACAATAACCCTGATAAATGCTTCAATAATATTGAAAAAGGAAGAGTATGAG TATTCAACATTTCCGTGTCGCCCTTATTCCCTTTTTTGCGGCATTTTGCCTTCCTGTTTTTG CTCACCCAGAAACGCTGGTGAAAGTAAAAGATGCTGAAGATCAGTTGGGTGCACGAGT GGGTTACATCGAACTGGATCTCAACAGCGGTAAGATCCTTGAGAGTTTTCGCCCCGAAG AACGTTTTCCAATGATGAGCACTTTTAAAGTTCTGCTATGTGGCGCGGTATTATCCCGTAT TGACGCCGGGCAAGAGCAACTCGGTCGCCGCATACACTATTCTCAGAATGACTTGGTTG AGTACTCACCAGTCACAGAAAAGCATCTTACGGATGGCATGACAGTAAGAGAATTATGC AGTGCTGCCATAACCATGAGTGATAACACTGCGGCCAACTTACTTCTGACAACGATCGG AGGACCGAAGGAGCTAACCGCTTTTTTGCACAACATGGGGGATCATGTAACTCGCCTTG ATCGTTGGGAACCGGAGCTGAATGAAGCCATACCAAACGACGAGCGTGACACCACGAT GCCTGTAGCAATGGCAACAACGTTGCGCAAACTATTAACTGGCGAACTACTTACTCTAGC TTCCCGGCAACAATTAATAGACTGGATGGAGGCGGATAAAGTTGCAGGACCACTTCTGC GCTCGGCCCTTCCGGCTGGCTGGTTTATTGCTGATAAATCTGGAGCCGGTGAGCGTGGG TCTCGCGGTATCATTGCAGCACTGGGGCCAGATGGTAAGCCCTCCCGTATCGTAGTTATC TACACGACGGGGAGTCAGGCAACTATGGATGAACGAAATAGACAGATCGCTGAGATAG GTGCCTCACTGATTAAGCATTGGTAACTGTCAGACCAAGTTTACTCATATATACTTTAGAT TGATTTAAAACTTCATTTTTAATTTAAAAGGATCTAGGTGAAGATCCTTTTTGATAATCTC ATGACCAAAATCCCTTAACGTGAGTTTTCGTTCCACTGAGCGTCAGACCCCGTAGAAAA GATCAAAGGATCTTCTTGAGATCCTTTTTTTCTGCGCGTAATCTGCTGCTTGCAAACAAA AAAACCACCGCTACCAGCGGTGGTTTGTTTGCCGGATCAAGAGCTACCAACTCTTTTTC CGAAGGTAACTGGCTTCAGCAGAGCGCAGATACCAAATACTGTTCTTCTAGTGTAGCCG TAGTTAGGCCACCACTTCAAGAACTCTGTAGCACCGCCTACATACCTCGCTCTGCTAATCC TGTTACCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCACACAG CCCAGCTTGGAGCGAACGACCTACACCGAACTGAGATACCTACAGCGTGAGCTATGAG AAAGCGCCACGCTTCCCGAAGGGAGAAAGGCGGACAGGTATCCGGTAAGCGGCAGG GTCGGAACAGGAGAGCGCACGAGGGAGCTTCCAGGGGGAAACGCCTGGTATCTTTAT AGTCCTGTCGGGTTTCGCCACCTCTGACTTGAGCGTCGATTTTTGTGATGCTCGTCAGG |
| | GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 56) |
| pCMVTnT insulin Nluc. Achain de B26-30 | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCC GCCGGAAGcggGAGGACCTGCAGGTGGGGCAGGTGGAGCTGGGCGGGggccCaggccC TGGTGCAGGCAGCCTGCAGCCCTTGGCCCTGGAGGGGTCCcgacgcAAGCGTATGGTCT TCACACTCGAAGATTTCGTTGGGGACTGGCGACAGACAGCCGGCTACAACCTGGACCA AGTCCTTGAACAGGGAGGTGTGTCCAGTTTGTTTCAGAATCTCGGGGTGTCCGTAACTC CGATCCAAAGGATTGTCCTGAGCGGTGAAAATGGGCTGAAGATCGACATCCATGTCATC ATCCCGTATGAAGGTCTGAGCGGCGACCAAATGGGCCAGATCGAAAAAATTTTTAAGGT GGTGTACCCTGTGGATGATCATCACTTTAAGGTGATCCTGCACTATGGCACACTGGTAAT CGACGGGGTTACGCCGAACATGATCGACTATTTCGGACGGCCGTATGAAGGCATCGCCG TGTTCGACGGCAAAAAGATCACTGTAACAGGGACCCTGTGGAACGGCAACAAAATTAT CGACGAGCGCCTGATCAACCCCGACGGCTCCCTGCTGTTCCGAGTAACCATCAACGGAG TGACCGGCTGGCGGCTGTGCGAACGCATTCTGGCGgctgctgctgcGGGCATTGTGGAAC AATGCTGTACCAGCATCTGCTCcctctaccagctggagaactactgcaactagGaattCACGCGTGG TACCTCTAGAGTCGACCCGGGCGGCCGCTTCGAGCAGACATGATAAGATACATTGATGA GTTTGGACAAACCACAACTAGAATGCAGTGAAAAAAATGCTTTATTTGTGAAATTTGTG |
| | ATGCTATTGCTTTATTTGTAACCATTATAAGCTGCAATAAACAAGTTAACAACAACAATTG CATTCATTTTATGTTTCAGGTTCAGGGGGAGATGTGGGAGGTTTTTTAAAGCAAGTAAA ACCTCTACAAATGTGGTAAAATCGATAAGGATCCGGGCTGGCGTAATAGCGAAGAGGCC CGCACCGATCGCCCTTCCCAACAGTTGCGCAGCCTGAATGGCGAATGGACGCGCCCTGT AGCGGCGCATTAAGCGCGGCGGGTGTGGTGGTTACGCGCAGCGTGACCGCTACACTTG CCAGCGCCCTAGCGCCCGCTCCTTTCGCTTTCTTCCCTTCCTTTCTCGCCACGTTCGCCG GCTTTCCCCGTCAAGCTCTAAATCGGGGGCTCCCTTTAGGGTTCCGATTTAGTGCTTTAC GGCACCTCGACCCCAAAAAACTTGATTAGGGTGATGGTTCACGTAGTGGGCCATCGCCC TGATAGACGGTTTTTCGCCCTTTGACGTTGGAGTCCACGTTCTTTAATAGTGGACTCTTG TTCCAAACTGGAACAACACTCAACCCTATCTCGGTCTATTCTTTTGATTTATAAGGGATTT TGCCGATTTCGGCCTATTGGTTAAAAAATGAGCTGATTTAACAAAAATTTAACGCGAATT TTAACAAAATATTAACGCTTACAATTTCCTGATGCGGTATTTTCTCCTTACGCATCTGTGCG GTATTTCACACCGCATATGGTGCACTCTCAGTACAATCTGCTCTGATGCCGCATAGTTAAG CCAGCCCCGACACCCGCCAACACCCGCTGACGCGCCCTGACGGGCTTGTCTGCTCCCG GCATCCGCTTACAGACAAGCTGTGACCGTCTCCGGGAGCTGCATGTGTCAGAGGTTTTC ACCGTCATCACCGAAACGCGCGAGACGAAAGGGCCTCGTGATACGCCTATTTTTATAGG TTAATGTCATGATAATAATGGTTTCTTAGACGTCAGGTGGCACTTTTCGGGGAAATGTGC GCGGAACCCCTATTTGTTTATTTTTCTAAATACATTCAAATATGTATCCGCTCATGAGACAA TAACCCTGATAAATGCTTCAATAATATTGAAAAAGGAAGAGTATGAGTATTCAACATTTCC GTGTCGCCCTTATTCCCTTTTTTGCGGCATTTTGCCTTCCTGTTTTTGCTCACCCAGAAAC GCTGGTGAAAGTAAAAGATGCTGAAGATCAGTTGGGTGCACGAGTGGGTTACATCGAA CTGGATCTCAACAGCGGTAAGATCCTTGAGAGTTTTCGCCCCGAAGAACGTTTTCCAAT GATGAGCACTTTTAAAGTTCTGCTATGTGGCGCGGTATTATCCCGTATTGACGCCGGGCA AGAGCAACTCGGTCGCCGCATACACTATTCTCAGAATGACTTGGTTGAGTACTCACCAGT CACAGAAAAGCATCTTACGGATGGCATGACAGTAAGAGAATTATGCAGTGCTGCCATAA CCATGAGTGATAACACTGCGGCCAACTTACTTCTGACAACGATCGGAGGACCGAAGGA GCTAACCGCTTTTTTGCACAACATGGGGGATCATGTAACTCGCCTTGATCGTTGGGAAC CGGAGCTGAATGAAGCCATACCAAACGACGAGCGTGACACCACGATGCCTGTAGCAAT GGCAACAACGTTGCGCAAACTATTAACTGGCGAACTACTTACTCTAGCTTCCCGGCAAC AATTAATAGACTGGATGGAGGCGGATAAAGTTGCAGGACCACTTCTGCGCTCGGCCCTT CCGGCTGGCTGGTTTATTGCTGATAAATCTGGAGCCGGTGAGCGTGGGTCTCGCGGTAT CATTGCAGCACTGGGGCCAGATGGTAAGCCCTCCCGTATCGTAGTTATCTACACGACGG GGAGTCAGGCAACTATGGATGAACGAAATAGACAGATCGCTGAGATAGGTGCCTCACT GATTAAGCATTGGTAACTGTCAGACCAAGTTTACTCATATATACTTTAGATTGATTTAAAA CTTCATTTTTAATTTAAAAGGATCTAGGTGAAGATCCTTTTTGATAATCTCATGACCAAAA TCCCTTAACGTGAGTTTTCGTTCCACTGAGCGTCAGACCCCGTAGAAAAGATCAAAGGA TCTTCTTGAGATCCTTTTTTTCTGCGCGTAATCTGCTGCTTGCAAACAAAAAAACCACCG |
| | CTACCAGCGGTGGTTTGTTTGCCGGATCAAGAGCTACCAACTCTTTTTCCGAAGGTAAC TGGCTTCAGCAGAGCGCAGATACCAAATACTGTTCTTCTAGTGTAGCCGTAGTTAGGCCA CCACTTCAAGAACTCTGTAGCACCGCCTACATACCTCGCTCTGCTAATCCTGTTACCAGTG GCTGCTGCCAGTGGCGATAAGTCGTGTCTTACCGGGTTGGACTCAAGACGATAGTTACC GGATAAGGCGCAGCGGTCGGGCTGAACGGGGGGTTCGTGCACACAGCCCAGCTTGGA GCGAACGACCTACACCGAACTGAGATACCTACAGCGTGAGCTATGAGAAAGCGCCACG CTTCCCGAAGGGAGAAAGGCGGACAGGTATCCGGTAAGCGGCAGGGTCGGAACAGG AGAGCGCACGAGGGAGCTTCCAGGGGGAAACGCCTGGTATCTTTATAGTCCTGTCGGG TTTCGCCACCTCTGACTTGAGCGTCGATTTTTGTGATGCTCGTCAGGGGGGCGGAGCCT ATGGAAAAACGCCAGCAACGCGGCCTTTTTACGGTTCCTGGCCTTTTGCTGGCCTTTTG CTCACATGGCTCGACAGATCT (SEQ ID NO: 57) |
| pCMVTnT insulin Nluc-Achain del B27-30 | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CCGCCGGAAGcggGAGGACCTGCAGGTGGGGCAGGTGGAGCTGGGCGGGggccCaggc cCTGGTGCAGGCAGCCTGCAGCCCTTGGCCCTGGAGGGGTCCcgacgcAAGCGTATGGT CTTCACACTCGAAGATTTCGTTGGGGACTGGCGACAGACAGCCGGCTACAACCTGGAC CAAGTCCTTGAACAGGGAGGTGTGTCCAGTTTGTTTCAGAATCTCGGGGTGTCCGTAAC TCCGATCCAAAGGATTGTCCTGAGCGGTGAAAATGGGCTGAAGATCGACATCCATGTCA |
| | TCATCCCGTATGAAGGTCTGAGCGGCGACCAAATGGGCCAGATCGAAAAAATTTTTAAG GTGGTGTACCCTGTGGATGATCATCACTTTAAGGTGATCCTGCACTATGGCACACTGGTA ATCGACGGGGTTACGCCGAACATGATCGACTATTTCGGACGGCCGTATGAAGGCATCGC CGTGTTCGACGGCAAAAAGATCACTGTAACAGGGACCCTGTGGAACGGCAACAAAATT ATCGACGAGCGCCTGATCAACCCCGACGGCTCCCTGCTGTTCCGAGTAACCATCAACGG AGTGACCGGCTGGCGGCTGTGCGAACGCATTCTGGCGgctgctgctgcGGGCATTGTGGA ACAATGCTGTACCAGCATCTGCTCcctctaccagctggagaactactgcaactagGaattCACGCGT GGTACCTCTAGAGTCGACCCGGGCGGCCGCTTCGAGCAGACATGATAAGATACATTGAT GAGTTTGGACAAACCACAACTAGAATGCAGTGAAAAAAATGCTTTATTTGTGAAATTTG TGATGCTATTGCTTTATTTGTAACCATTATAAGCTGCAATAAACAAGTTAACAACAACAAT TGCATTCATTTTATGTTTCAGGTTCAGGGGGAGATGTGGGAGGTTTTTTAAAGCAAGTA AAACCTCTACAAATGTGGTAAAATCGATAAGGATCCGGGCTGGCGTAATAGCGAAGAGG CCCGCACCGATCGCCCTTCCCAACAGTTGCGCAGCCTGAATGGCGAATGGACGCGCCCT GTAGCGGCGCATTAAGCGCGGCGGGTGTGGTGGTTACGCGCAGCGTGACCGCTACACT TGCCAGCGCCCTAGCGCCCGCTCCTTTCGCTTTCTTCCCTTCCTTTCTCGCCACGTTCGCC GGCTTTCCCCGTCAAGCTCTAAATCGGGGGCTCCCTTTAGGGTTCCGATTTAGTGCTTTA CGGCACCTCGACCCCAAAAAACTTGATTAGGGTGATGGTTCACGTAGTGGGCCATCGCC CTGATAGACGGTTTTTCGCCCTTTGACGTTGGAGTCCACGTTCTTTAATAGTGGACTCTT GTTCCAAACTGGAACAACACTCAACCCTATCTCGGTCTATTCTTTTGATTTATAAGGGATT TTGCCGATTTCGGCCTATTGGTTAAAAAATGAGCTGATTTAACAAAAATTTAACGCGAAT TTTAACAAAATATTAACGCTTACAATTTCCTGATGCGGTATTTTCTCCTTACGCATCTGTGC GGTATTTCACACCGCATATGGTGCACTCTCAGTACAATCTGCTCTGATGCCGCATAGTTAA GCCAGCCCCGACACCCGCCAACACCCGCTGACGCGCCCTGACGGGCTTGTCTGCTCCC GGCATCCGCTTACAGACAAGCTGTGACCGTCTCCGGGAGCTGCATGTGTCAGAGGTTTT CACCGTCATCACCGAAACGCGCGAGACGAAAGGGCCTCGTGATACGCCTATTTTTATAG GTTAATGTCATGATAATAATGGTTTCTTAGACGTCAGGTGGCACTTTTCGGGGAAATGTG CGCGGAACCCCTATTTGTTTATTTTTCTAAATACATTCAAATATGTATCCGCTCATGAGACA ATAACCCTGATAAATGCTTCAATAATATTGAAAAAGGAAGAGTATGAGTATTCAACATTTC CGTGTCGCCCTTATTCCCTTTTTTGCGGCATTTTGCCTTCCTGTTTTTGCTCACCCAGAAA CGCTGGTGAAAGTAAAAGATGCTGAAGATCAGTTGGGTGCACGAGTGGGTTACATCGA ACTGGATCTCAACAGCGGTAAGATCCTTGAGAGTTTTCGCCCCGAAGAACGTTTTCCAA TGATGAGCACTTTTAAAGTTCTGCTATGTGGCGCGGTATTATCCCGTATTGACGCCGGGC AAGAGCAACTCGGTCGCCGCATACACTATTCTCAGAATGACTTGGTTGAGTACTCACCA GTCACAGAAAAGCATCTTACGGATGGCATGACAGTAAGAGAATTATGCAGTGCTGCCAT AACCATGAGTGATAACACTGCGGCCAACTTACTTCTGACAACGATCGGAGGACCGAAG GAGCTAACCGCTTTTTTGCACAACATGGGGGATCATGTAACTCGCCTTGATCGTTGGGA ACCGGAGCTGAATGAAGCCATACCAAACGACGAGCGTGACACCACGATGCCTGTAGCA |
| | ATGGCAACAACGTTGCGCAAACTATTAACTGGCGAACTACTTACTCTAGCTTCCCGGCAA CAATTAATAGACTGGATGGAGGCGGATAAAGTTGCAGGACCACTTCTGCGCTCGGCCCT TCCGGCTGGCTGGTTTATTGCTGATAAATCTGGAGCCGGTGAGCGTGGGTCTCGCGGTA TCATTGCAGCACTGGGGCCAGATGGTAAGCCCTCCCGTATCGTAGTTATCTACACGACGG GGAGTCAGGCAACTATGGATGAACGAAATAGACAGATCGCTGAGATAGGTGCCTCACT GATTAAGCATTGGTAACTGTCAGACCAAGTTTACTCATATATACTTTAGATTGATTTAAAA CTTCATTTTTAATTTAAAAGGATCTAGGTGAAGATCCTTTTTGATAATCTCATGACCAAAA TCCCTTAACGTGAGTTTTCGTTCCACTGAGCGTCAGACCCCGTAGAAAAGATCAAAGGA TCTTCTTGAGATCCTTTTTTTCTGCGCGTAATCTGCTGCTTGCAAACAAAAAAACCACCG CTACCAGCGGTGGTTTGTTTGCCGGATCAAGAGCTACCAACTCTTTTTCCGAAGGTAAC TGGCTTCAGCAGAGCGCAGATACCAAATACTGTTCTTCTAGTGTAGCCGTAGTTAGGCCA CCACTTCAAGAACTCTGTAGCACCGCCTACATACCTCGCTCTGCTAATCCTGTTACCAGTG GCTGCTGCCAGTGGCGATAAGTCGTGTCTTACCGGGTTGGACTCAAGACGATAGTTACC GGATAAGGCGCAGCGGTCGGGCTGAACGGGGGGTTCGTGCACACAGCCCAGCTTGGA GCGAACGACCTACACCGAACTGAGATACCTACAGCGTGAGCTATGAGAAAGCGCCACG CTTCCCGAAGGGAGAAAGGCGGACAGGTATCCGGTAAGCGGCAGGGTCGGAACAGG AGAGCGCACGAGGGAGCTTCCAGGGGGAAACGCCTGGTATCTTTATAGTCCTGTCGGG TTTCGCCACCTCTGACTTGAGCGTCGATTTTTGTGATGCTCGTCAGGGGGGCGGAGCCT ATGGAAAAACGCCAGCAACGCGGCCTTTTTACGGTTCCTGGCCTTTTGCTGGCCTTTTG CTCACATGGCTCGACAGATCT (SEQ ID NO: 58) |
| pCMVTnT insulin Nluc-Achain del B28-30 | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG |
| | GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACGCCGGAAGcggGAGGACCTGCAGGTGGGGCAGGTGGAGCTGGGCGGGggccC aggccCTGGTGCAGGCAGCCTGCAGCCCTTGGCCCTGGAGGGGTCCcgacgcAAGCGTAT GGTCTTCACACTCGAAGATTTCGTTGGGGACTGGCGACAGACAGCCGGCTACAACCTG GACCAAGTCCTTGAACAGGGAGGTGTGTCCAGTTTGTTTCAGAATCTCGGGGTGTCCG TAACTCCGATCCAAAGGATTGTCCTGAGCGGTGAAAATGGGCTGAAGATCGACATCCAT GTCATCATCCCGTATGAAGGTCTGAGCGGCGACCAAATGGGCCAGATCGAAAAAATTTT TAAGGTGGTGTACCCTGTGGATGATCATCACTTTAAGGTGATCCTGCACTATGGCACACT GGTAATCGACGGGGTTACGCCGAACATGATCGACTATTTCGGACGGCCGTATGAAGGCA TCGCCGTGTTCGACGGCAAAAAGATCACTGTAACAGGGACCCTGTGGAACGGCAACAA AATTATCGACGAGCGCCTGATCAACCCCGACGGCTCCCTGCTGTTCCGAGTAACCATCAA CGGAGTGACCGGCTGGCGGCTGTGCGAACGCATTCTGGCGgctgctgctgcGGGCATTGT GGAACAATGCTGTACCAGCATCTGCTCcctctaccagctggagaactactgcaactagGaattCACG CGTGGTACCTCTAGAGTCGACCCGGGCGGCCGCTTCGAGCAGACATGATAAGATACATT GATGAGTTTGGACAAACCACAACTAGAATGCAGTGAAAAAAATGCTTTATTTGTGAAAT TTGTGATGCTATTGCTTTATTTGTAACCATTATAAGCTGCAATAAACAAGTTAACAACAAC AATTGCATTCATTTTATGTTTCAGGTTCAGGGGGAGATGTGGGAGGTTTTTTAAAGCAA GTAAAACCTCTACAAATGTGGTAAAATCGATAAGGATCCGGGCTGGCGTAATAGCGAAG AGGCCCGCACCGATCGCCCTTCCCAACAGTTGCGCAGCCTGAATGGCGAATGGACGCG CCCTGTAGCGGCGCATTAAGCGCGGCGGGTGTGGTGGTTACGCGCAGCGTGACCGCTA CACTTGCCAGCGCCCTAGCGCCCGCTCCTTTCGCTTTCTTCCCTTCCTTTCTCGCCACGTT CGCCGGCTTTCCCCGTCAAGCTCTAAATCGGGGGCTCCCTTTAGGGTTCCGATTTAGTG CTTTACGGCACCTCGACCCCAAAAAACTTGATTAGGGTGATGGTTCACGTAGTGGGCCA TCGCCCTGATAGACGGTTTTTCGCCCTTTGACGTTGGAGTCCACGTTCTTTAATAGTGGA CTCTTGTTCCAAACTGGAACAACACTCAACCCTATCTCGGTCTATTCTTTTGATTTATAAG GGATTTTGCCGATTTCGGCCTATTGGTTAAAAAATGAGCTGATTTAACAAAAATTTAACG CGAATTTTAACAAAATATTAACGCTTACAATTTCCTGATGCGGTATTTTCTCCTTACGCATC TGTGCGGTATTTCACACCGCATATGGTGCACTCTCAGTACAATCTGCTCTGATGCCGCATA GTTAAGCCAGCCCCGACACCCGCCAACACCCGCTGACGCGCCCTGACGGGCTTGTCTG CTCCCGGCATCCGCTTACAGACAAGCTGTGACCGTCTCCGGGAGCTGCATGTGTCAGAG GTTTTCACCGTCATCACCGAAACGCGCGAGACGAAAGGGCCTCGTGATACGCCTATTTT TATAGGTTAATGTCATGATAATAATGGTTTCTTAGACGTCAGGTGGCACTTTTCGGGGAA ATGTGCGCGGAACCCCTATTTGTTTATTTTTCTAAATACATTCAAATATGTATCCGCTCATG AGACAATAACCCTGATAAATGCTTCAATAATATTGAAAAAGGAAGAGTATGAGTATTCAA |
| | CATTTCCGTGTCGCCCTTATTCCCTTTTTTGCGGCATTTTGCCTTCCTGTTTTTGCTCACCC AGAAACGCTGGTGAAAGTAAAAGATGCTGAAGATCAGTTGGGTGCACGAGTGGGTTAC ATCGAACTGGATCTCAACAGCGGTAAGATCCTTGAGAGTTTTCGCCCCGAAGAACGTTT TCCAATGATGAGCACTTTTAAAGTTCTGCTATGTGGCGCGGTATTATCCCGTATTGACGCC GGGCAAGAGCAACTCGGTCGCCGCATACACTATTCTCAGAATGACTTGGTTGAGTACTC ACCAGTCACAGAAAAGCATCTTACGGATGGCATGACAGTAAGAGAATTATGCAGTGCTG CCATAACCATGAGTGATAACACTGCGGCCAACTTACTTCTGACAACGATCGGAGGACCG AAGGAGCTAACCGCTTTTTTGCACAACATGGGGGATCATGTAACTCGCCTTGATCGTTG GGAACCGGAGCTGAATGAAGCCATACCAAACGACGAGCGTGACACCACGATGCCTGTA GCAATGGCAACAACGTTGCGCAAACTATTAACTGGCGAACTACTTACTCTAGCTTCCCGG CAACAATTAATAGACTGGATGGAGGCGGATAAAGTTGCAGGACCACTTCTGCGCTCGGC CCTTCCGGCTGGCTGGTTTATTGCTGATAAATCTGGAGCCGGTGAGCGTGGGTCTCGCG GTATCATTGCAGCACTGGGGCCAGATGGTAAGCCCTCCCGTATCGTAGTTATCTACACGA CGGGGAGTCAGGCAACTATGGATGAACGAAATAGACAGATCGCTGAGATAGGTGCCTC ACTGATTAAGCATTGGTAACTGTCAGACCAAGTTTACTCATATATACTTTAGATTGATTTAA AACTTCATTTTTAATTTAAAAGGATCTAGGTGAAGATCCTTTTTGATAATCTCATGACCAA AATCCCTTAACGTGAGTTTTCGTTCCACTGAGCGTCAGACCCCGTAGAAAAGATCAAAG GATCTTCTTGAGATCCTTTTTTTCTGCGCGTAATCTGCTGCTTGCAAACAAAAAAACCAC CGCTACCAGCGGTGGTTTGTTTGCCGGATCAAGAGCTACCAACTCTTTTTCCGAAGGTA ACTGGCTTCAGCAGAGCGCAGATACCAAATACTGTTCTTCTAGTGTAGCCGTAGTTAGGC CACCACTTCAAGAACTCTGTAGCACCGCCTACATACCTCGCTCTGCTAATCCTGTTACCAG TGGCTGCTGCCAGTGGCGATAAGTCGTGTCTTACCGGGTTGGACTCAAGACGATAGTTA CCGGATAAGGCGCAGCGGTCGGGCTGAACGGGGGGTTCGTGCACACAGCCCAGCTTG GAGCGAACGACCTACACCGAACTGAGATACCTACAGCGTGAGCTATGAGAAAGCGCCA CGCTTCCCGAAGGGAGAAAGGCGGACAGGTATCCGGTAAGCGGCAGGGTCGGAACA GGAGAGCGCACGAGGGAGCTTCCAGGGGGAAACGCCTGGTATCTTTATAGTCCTGTCG GGTTTCGCCACCTCTGACTTGAGCGTCGATTTTTGTGATGCTCGTCAGGGGGGCGGAG CCTATGGAAAAACGCCAGCAACGCGGCCTTTTTACGGTTCCTGGCCTTTTGCTGGCCTT TTGCTCACATGGCTCGACAGATCT (SEQ ID NO: 59) |
| pCMVTnT insulin Nluc-Achain del B30 | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT |
| | CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGCGCCGGAAGcggGAGGACCTGCAGGTGGGGCAGGTGGAGCTGGGCG GGggccCaggccCTGGTGCAGGCAGCCTGCAGCCCTTGGCCCTGGAGGGGTCCcgacgcA AGCGTATGGTCTTCACACTCGAAGATTTCGTTGGGGACTGGCGACAGACAGCCGGCTA CAACCTGGACCAAGTCCTTGAACAGGGAGGTGTGTCCAGTTTGTTTCAGAATCTCGGG GTGTCCGTAACTCCGATCCAAAGGATTGTCCTGAGCGGTGAAAATGGGCTGAAGATCGA CATCCATGTCATCATCCCGTATGAAGGTCTGAGCGGCGACCAAATGGGCCAGATCGAAA AAATTTTTAAGGTGGTGTACCCTGTGGATGATCATCACTTTAAGGTGATCCTGCACTATG GCACACTGGTAATCGACGGGGTTACGCCGAACATGATCGACTATTTCGGACGGCCGTAT GAAGGCATCGCCGTGTTCGACGGCAAAAAGATCACTGTAACAGGGACCCTGTGGAACG GCAACAAAATTATCGACGAGCGCCTGATCAACCCCGACGGCTCCCTGCTGTTCCGAGTA ACCATCAACGGAGTGACCGGCTGGCGGCTGTGCGAACGCATTCTGGCGgctgctgctgcG GGCATTGTGGAACAATGCTGTACCAGCATCTGCTCcctctaccagctggagaactactgcaactag GaattCACGCGTGGTACCTCTAGAGTCGACCCGGGCGGCCGCTTCGAGCAGACATGATA AGATACATTGATGAGTTTGGACAAACCACAACTAGAATGCAGTGAAAAAAATGCTTTATT TGTGAAATTTGTGATGCTATTGCTTTATTTGTAACCATTATAAGCTGCAATAAACAAGTTA ACAACAACAATTGCATTCATTTTATGTTTCAGGTTCAGGGGGAGATGTGGGAGGTTTTT TAAAGCAAGTAAAACCTCTACAAATGTGGTAAAATCGATAAGGATCCGGGCTGGCGTAA TAGCGAAGAGGCCCGCACCGATCGCCCTTCCCAACAGTTGCGCAGCCTGAATGGCGAA TGGACGCGCCCTGTAGCGGCGCATTAAGCGCGGCGGGTGTGGTGGTTACGCGCAGCGT GACCGCTACACTTGCCAGCGCCCTAGCGCCCGCTCCTTTCGCTTTCTTCCCTTCCTTTCTC GCCACGTTCGCCGGCTTTCCCCGTCAAGCTCTAAATCGGGGGCTCCCTTTAGGGTTCCG ATTTAGTGCTTTACGGCACCTCGACCCCAAAAAACTTGATTAGGGTGATGGTTCACGTAG TGGGCCATCGCCCTGATAGACGGTTTTTCGCCCTTTGACGTTGGAGTCCACGTTCTTTAA TAGTGGACTCTTGTTCCAAACTGGAACAACACTCAACCCTATCTCGGTCTATTCTTTTGAT |
| | TTATAAGGGATTTTGCCGATTTCGGCCTATTGGTTAAAAAATGAGCTGATTTAACAAAAA TTTAACGCGAATTTTAACAAAATATTAACGCTTACAATTTCCTGATGCGGTATTTTCTCCTT ACGCATCTGTGCGGTATTTCACACCGCATATGGTGCACTCTCAGTACAATCTGCTCTGATG CCGCATAGTTAAGCCAGCCCCGACACCCGCCAACACCCGCTGACGCGCCCTGACGGGC TTGTCTGCTCCCGGCATCCGCTTACAGACAAGCTGTGACCGTCTCCGGGAGCTGCATGT GTCAGAGGTTTTCACCGTCATCACCGAAACGCGCGAGACGAAAGGGCCTCGTGATACG CCTATTTTTATAGGTTAATGTCATGATAATAATGGTTTCTTAGACGTCAGGTGGCACTTTTC GGGGAAATGTGCGCGGAACCCCTATTTGTTTATTTTTCTAAATACATTCAAATATGTATCC GCTCATGAGACAATAACCCTGATAAATGCTTCAATAATATTGAAAAAGGAAGAGTATGAG TATTCAACATTTCCGTGTCGCCCTTATTCCCTTTTTTGCGGCATTTTGCCTTCCTGTTTTTG CTCACCCAGAAACGCTGGTGAAAGTAAAAGATGCTGAAGATCAGTTGGGTGCACGAGT GGGTTACATCGAACTGGATCTCAACAGCGGTAAGATCCTTGAGAGTTTTCGCCCCGAAG AACGTTTTCCAATGATGAGCACTTTTAAAGTTCTGCTATGTGGCGCGGTATTATCCCGTAT TGACGCCGGGCAAGAGCAACTCGGTCGCCGCATACACTATTCTCAGAATGACTTGGTTG AGTACTCACCAGTCACAGAAAAGCATCTTACGGATGGCATGACAGTAAGAGAATTATGC AGTGCTGCCATAACCATGAGTGATAACACTGCGGCCAACTTACTTCTGACAACGATCGG AGGACCGAAGGAGCTAACCGCTTTTTTGCACAACATGGGGGATCATGTAACTCGCCTTG ATCGTTGGGAACCGGAGCTGAATGAAGCCATACCAAACGACGAGCGTGACACCACGAT GCCTGTAGCAATGGCAACAACGTTGCGCAAACTATTAACTGGCGAACTACTTACTCTAGC TTCCCGGCAACAATTAATAGACTGGATGGAGGCGGATAAAGTTGCAGGACCACTTCTGC GCTCGGCCCTTCCGGCTGGCTGGTTTATTGCTGATAAATCTGGAGCCGGTGAGCGTGGG TCTCGCGGTATCATTGCAGCACTGGGGCCAGATGGTAAGCCCTCCCGTATCGTAGTTATC TACACGACGGGGAGTCAGGCAACTATGGATGAACGAAATAGACAGATCGCTGAGATAG GTGCCTCACTGATTAAGCATTGGTAACTGTCAGACCAAGTTTACTCATATATACTTTAGAT TGATTTAAAACTTCATTTTTAATTTAAAAGGATCTAGGTGAAGATCCTTTTTGATAATCTC ATGACCAAAATCCCTTAACGTGAGTTTTCGTTCCACTGAGCGTCAGACCCCGTAGAAAA GATCAAAGGATCTTCTTGAGATCCTTTTTTTCTGCGCGTAATCTGCTGCTTGCAAACAAA AAAACCACCGCTACCAGCGGTGGTTTGTTTGCCGGATCAAGAGCTACCAACTCTTTTTC CGAAGGTAACTGGCTTCAGCAGAGCGCAGATACCAAATACTGTTCTTCTAGTGTAGCCG TAGTTAGGCCACCACTTCAAGAACTCTGTAGCACCGCCTACATACCTCGCTCTGCTAATCC TGTTACCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCACACAG CCCAGCTTGGAGCGAACGACCTACACCGAACTGAGATACCTACAGCGTGAGCTATGAG AAAGCGCCACGCTTCCCGAAGGGAGAAAGGCGGACAGGTATCCGGTAAGCGGCAGG GTCGGAACAGGAGAGCGCACGAGGGAGCTTCCAGGGGGAAACGCCTGGTATCTTTAT AGTCCTGTCGGGTTTCGCCACCTCTGACTTGAGCGTCGATTTTTGTGATGCTCGTCAGG |
| | GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 60) |
| pCMVTnT_hF PI furin Nlucstop | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCCGCCGGAAGcggGAGGACCTGCAGGTGGGGCAGGTGGAGCTGGG CGGGggccCaggccCTGGTGCAGGCAGCCTGCAGCCCTTGGCCCTGGAGGGGTCCcgacg cAAGCGTGGCATTGTGGAACAATGCTGTACCAGCATCTGCTCcctctaccagctggagaactact gcaacGGTGGAGGCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGtag GaattCACGCGTGGTACCTCTAGAGTCGACCCGGGCGGCCGCTTCGAGCAGACATGATA AGATACATTGATGAGTTTGGACAAACCACAACTAGAATGCAGTGAAAAAAATGCTTTATT |
| | TGTGAAATTTGTGATGCTATTGCTTTATTTGTAACCATTATAAGCTGCAATAAACAAGTTA ACAACAACAATTGCATTCATTTTATGTTTCAGGTTCAGGGGGAGATGTGGGAGGTTTTT TAAAGCAAGTAAAACCTCTACAAATGTGGTAAAATCGATAAGGATCCGGGCTGGCGTAA TAGCGAAGAGGCCCGCACCGATCGCCCTTCCCAACAGTTGCGCAGCCTGAATGGCGAA TGGACGCGCCCTGTAGCGGCGCATTAAGCGCGGCGGGTGTGGTGGTTACGCGCAGCGT GACCGCTACACTTGCCAGCGCCCTAGCGCCCGCTCCTTTCGCTTTCTTCCCTTCCTTTCTC GCCACGTTCGCCGGCTTTCCCCGTCAAGCTCTAAATCGGGGGCTCCCTTTAGGGTTCCG ATTTAGTGCTTTACGGCACCTCGACCCCAAAAAACTTGATTAGGGTGATGGTTCACGTAG TGGGCCATCGCCCTGATAGACGGTTTTTCGCCCTTTGACGTTGGAGTCCACGTTCTTTAA TAGTGGACTCTTGTTCCAAACTGGAACAACACTCAACCCTATCTCGGTCTATTCTTTTGAT TTATAAGGGATTTTGCCGATTTCGGCCTATTGGTTAAAAAATGAGCTGATTTAACAAAAA TTTAACGCGAATTTTAACAAAATATTAACGCTTACAATTTCCTGATGCGGTATTTTCTCCTT ACGCATCTGTGCGGTATTTCACACCGCATATGGTGCACTCTCAGTACAATCTGCTCTGATG CCGCATAGTTAAGCCAGCCCCGACACCCGCCAACACCCGCTGACGCGCCCTGACGGGC TTGTCTGCTCCCGGCATCCGCTTACAGACAAGCTGTGACCGTCTCCGGGAGCTGCATGT GTCAGAGGTTTTCACCGTCATCACCGAAACGCGCGAGACGAAAGGGCCTCGTGATACG CCTATTTTTATAGGTTAATGTCATGATAATAATGGTTTCTTAGACGTCAGGTGGCACTTTTC GGGGAAATGTGCGCGGAACCCCTATTTGTTTATTTTTCTAAATACATTCAAATATGTATCC GCTCATGAGACAATAACCCTGATAAATGCTTCAATAATATTGAAAAAGGAAGAGTATGAG TATTCAACATTTCCGTGTCGCCCTTATTCCCTTTTTTGCGGCATTTTGCCTTCCTGTTTTTG CTCACCCAGAAACGCTGGTGAAAGTAAAAGATGCTGAAGATCAGTTGGGTGCACGAGT GGGTTACATCGAACTGGATCTCAACAGCGGTAAGATCCTTGAGAGTTTTCGCCCCGAAG AACGTTTTCCAATGATGAGCACTTTTAAAGTTCTGCTATGTGGCGCGGTATTATCCCGTAT TGACGCCGGGCAAGAGCAACTCGGTCGCCGCATACACTATTCTCAGAATGACTTGGTTG AGTACTCACCAGTCACAGAAAAGCATCTTACGGATGGCATGACAGTAAGAGAATTATGC AGTGCTGCCATAACCATGAGTGATAACACTGCGGCCAACTTACTTCTGACAACGATCGG AGGACCGAAGGAGCTAACCGCTTTTTTGCACAACATGGGGGATCATGTAACTCGCCTTG ATCGTTGGGAACCGGAGCTGAATGAAGCCATACCAAACGACGAGCGTGACACCACGAT GCCTGTAGCAATGGCAACAACGTTGCGCAAACTATTAACTGGCGAACTACTTACTCTAGC TTCCCGGCAACAATTAATAGACTGGATGGAGGCGGATAAAGTTGCAGGACCACTTCTGC GCTCGGCCCTTCCGGCTGGCTGGTTTATTGCTGATAAATCTGGAGCCGGTGAGCGTGGG TCTCGCGGTATCATTGCAGCACTGGGGCCAGATGGTAAGCCCTCCCGTATCGTAGTTATC TACACGACGGGGAGTCAGGCAACTATGGATGAACGAAATAGACAGATCGCTGAGATAG GTGCCTCACTGATTAAGCATTGGTAACTGTCAGACCAAGTTTACTCATATATACTTTAGAT TGATTTAAAACTTCATTTTTAATTTAAAAGGATCTAGGTGAAGATCCTTTTTGATAATCTC ATGACCAAAATCCCTTAACGTGAGTTTTCGTTCCACTGAGCGTCAGACCCCGTAGAAAA GATCAAAGGATCTTCTTGAGATCCTTTTTTTCTGCGCGTAATCTGCTGCTTGCAAACAAA |
| | AAAACCACCGCTACCAGCGGTGGTTTGTTTGCCGGATCAAGAGCTACCAACTCTTTTTC CGAAGGTAACTGGCTTCAGCAGAGCGCAGATACCAAATACTGTTCTTCTAGTGTAGCCG TAGTTAGGCCACCACTTCAAGAACTCTGTAGCACCGCCTACATACCTCGCTCTGCTAATCC TGTTACCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCACACAG CCCAGCTTGGAGCGAACGACCTACACCGAACTGAGATACCTACAGCGTGAGCTATGAG AAAGCGCCACGCTTCCCGAAGGGAGAAAGGCGGACAGGTATCCGGTAAGCGGCAGG GTCGGAACAGGAGAGCGCACGAGGGAGCTTCCAGGGGGAAACGCCTGGTATCTTTAT AGTCCTGTCGGGTTTCGCCACCTCTGACTTGAGCGTCGATTTTTGTGATGCTCGTCAGG GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 61) |
| pCMVTnT_hP reProlns B chain Nluc furin | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT |
| | GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGAAGcggGAGGACCTGCAGGTGGGGCAGGTGGAGCTGGGCGGGg gccCaggccCTGGTGCAGGCAGCCTGCAGCCCTTGGCCCTGGAGGGGTCCcgacgcAAGC GTGGCATTGTGGAACAATGCTGTACCAGCATCTGCTCcctctaccagctggagaactactgcaact agGaattCACGCGTGGTACCTCTAGAGTCGACCCGGGCGGCCGCTTCGAGCAGACATGAT AAGATACATTGATGAGTTTGGACAAACCACAACTAGAATGCAGTGAAAAAAATGCTTTA TTTGTGAAATTTGTGATGCTATTGCTTTATTTGTAACCATTATAAGCTGCAATAAACAAGTT AACAACAACAATTGCATTCATTTTATGTTTCAGGTTCAGGGGGAGATGTGGGAGGTTTT TTAAAGCAAGTAAAACCTCTACAAATGTGGTAAAATCGATAAGGATCCGGGCTGGCGTA ATAGCGAAGAGGCCCGCACCGATCGCCCTTCCCAACAGTTGCGCAGCCTGAATGGCGA ATGGACGCGCCCTGTAGCGGCGCATTAAGCGCGGCGGGTGTGGTGGTTACGCGCAGCG TGACCGCTACACTTGCCAGCGCCCTAGCGCCCGCTCCTTTCGCTTTCTTCCCTTCCTTTCT CGCCACGTTCGCCGGCTTTCCCCGTCAAGCTCTAAATCGGGGGCTCCCTTTAGGGTTCC GATTTAGTGCTTTACGGCACCTCGACCCCAAAAAACTTGATTAGGGTGATGGTTCACGTA GTGGGCCATCGCCCTGATAGACGGTTTTTCGCCCTTTGACGTTGGAGTCCACGTTCTTTA ATAGTGGACTCTTGTTCCAAACTGGAACAACACTCAACCCTATCTCGGTCTATTCTTTTGA TTTATAAGGGATTTTGCCGATTTCGGCCTATTGGTTAAAAAATGAGCTGATTTAACAAAA ATTTAACGCGAATTTTAACAAAATATTAACGCTTACAATTTCCTGATGCGGTATTTTCTCCT TACGCATCTGTGCGGTATTTCACACCGCATATGGTGCACTCTCAGTACAATCTGCTCTGAT GCCGCATAGTTAAGCCAGCCCCGACACCCGCCAACACCCGCTGACGCGCCCTGACGGG CTTGTCTGCTCCCGGCATCCGCTTACAGACAAGCTGTGACCGTCTCCGGGAGCTGCATG TGTCAGAGGTTTTCACCGTCATCACCGAAACGCGCGAGACGAAAGGGCCTCGTGATAC GCCTATTTTTATAGGTTAATGTCATGATAATAATGGTTTCTTAGACGTCAGGTGGCACTTT TCGGGGAAATGTGCGCGGAACCCCTATTTGTTTATTTTTCTAAATACATTCAAATATGTAT CCGCTCATGAGACAATAACCCTGATAAATGCTTCAATAATATTGAAAAAGGAAGAGTATG AGTATTCAACATTTCCGTGTCGCCCTTATTCCCTTTTTTGCGGCATTTTGCCTTCCTGTTTT TGCTCACCCAGAAACGCTGGTGAAAGTAAAAGATGCTGAAGATCAGTTGGGTGCACGA GTGGGTTACATCGAACTGGATCTCAACAGCGGTAAGATCCTTGAGAGTTTTCGCCCCGA AGAACGTTTTCCAATGATGAGCACTTTTAAAGTTCTGCTATGTGGCGCGGTATTATCCCG TATTGACGCCGGGCAAGAGCAACTCGGTCGCCGCATACACTATTCTCAGAATGACTTGG TTGAGTACTCACCAGTCACAGAAAAGCATCTTACGGATGGCATGACAGTAAGAGAATTA TGCAGTGCTGCCATAACCATGAGTGATAACACTGCGGCCAACTTACTTCTGACAACGATC GGAGGACCGAAGGAGCTAACCGCTTTTTTGCACAACATGGGGGATCATGTAACTCGCC TTGATCGTTGGGAACCGGAGCTGAATGAAGCCATACCAAACGACGAGCGTGACACCAC |
| | GATGCCTGTAGCAATGGCAACAACGTTGCGCAAACTATTAACTGGCGAACTACTTACTCT AGCTTCCCGGCAACAATTAATAGACTGGATGGAGGCGGATAAAGTTGCAGGACCACTTC TGCGCTCGGCCCTTCCGGCTGGCTGGTTTATTGCTGATAAATCTGGAGCCGGTGAGCGT GGGTCTCGCGGTATCATTGCAGCACTGGGGCCAGATGGTAAGCCCTCCCGTATCGTAGT TATCTACACGACGGGGAGTCAGGCAACTATGGATGAACGAAATAGACAGATCGCTGAG ATAGGTGCCTCACTGATTAAGCATTGGTAACTGTCAGACCAAGTTTACTCATATATACTTT AGATTGATTTAAAACTTCATTTTTAATTTAAAAGGATCTAGGTGAAGATCCTTTTTGATAA TCTCATGACCAAAATCCCTTAACGTGAGTTTTCGTTCCACTGAGCGTCAGACCCCGTAGA AAAGATCAAAGGATCTTCTTGAGATCCTTTTTTTCTGCGCGTAATCTGCTGCTTGCAAAC AAAAAAACCACCGCTACCAGCGGTGGTTTGTTTGCCGGATCAAGAGCTACCAACTCTTT TTCCGAAGGTAACTGGCTTCAGCAGAGCGCAGATACCAAATACTGTTCTTCTAGTGTAGC CGTAGTTAGGCCACCACTTCAAGAACTCTGTAGCACCGCCTACATACCTCGCTCTGCTAA TCCTGTTACCAGTGGCTGCTGCCAGTGGCGATAAGTCGTGTCTTACCGGGTTGGACTCA AGACGATAGTTACCGGATAAGGCGCAGCGGTCGGGCTGAACGGGGGGTTCGTGCACA CAGCCCAGCTTGGAGCGAACGACCTACACCGAACTGAGATACCTACAGCGTGAGCTATG AGAAAGCGCCACGCTTCCCGAAGGGAGAAAGGCGGACAGGTATCCGGTAAGCGGCAG GGTCGGAACAGGAGAGCGCACGAGGGAGCTTCCAGGGGGAAACGCCTGGTATCTTTA TAGTCCTGTCGGGTTTCGCCACCTCTGACTTGAGCGTCGATTTTTGTGATGCTCGTCAGG GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 62) |
| pCMVTnT_hP reProlns furin Nluc-Achain | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG |
| | GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCCGCCGGAAGcggGAGGACCTGCAGGTGGGGCAGGTGGAGCTGGG CGGGggccCaggccCTGGTGCAGGCAGCCTGCAGCCCTTGGCCCTGGAGGGGTCCcgacg cAAGCGTATGGTCTTCACACTCGAAGATTTCGTTGGGGACTGGCGACAGACAGCCGGC TACAACCTGGACCAAGTCCTTGAACAGGGAGGTGTGTCCAGTTTGTTTCAGAATCTCGG GGTGTCCGTAACTCCGATCCAAAGGATTGTCCTGAGCGGTGAAAATGGGCTGAAGATC GACATCCATGTCATCATCCCGTATGAAGGTCTGAGCGGCGACCAAATGGGCCAGATCGA AAAAATTTTTAAGGTGGTGTACCCTGTGGATGATCATCACTTTAAGGTGATCCTGCACTAT GGCACACTGGTAATCGACGGGGTTACGCCGAACATGATCGACTATTTCGGACGGCCGTA TGAAGGCATCGCCGTGTTCGACGGCAAAAAGATCACTGTAACAGGGACCCTGTGGAAC GGCAACAAAATTATCGACGAGCGCCTGATCAACCCCGACGGCTCCCTGCTGTTCCGAGT AACCATCAACGGAGTGACCGGCTGGCGGCTGTGCGAACGCATTCTGGCGgctgctgctgc GGGCATTGTGGAACAATGCTGTACCAGCATCTGCTCcctctaccagctggagaactactgcaacta gGaattCACGCGTGGTACCTCTAGAGTCGACCCGGGCGGCCGCTTCGAGCAGACATGATA AGATACATTGATGAGTTTGGACAAACCACAACTAGAATGCAGTGAAAAAAATGCTTTATT TGTGAAATTTGTGATGCTATTGCTTTATTTGTAACCATTATAAGCTGCAATAAACAAGTTA ACAACAACAATTGCATTCATTTTATGTTTCAGGTTCAGGGGGAGATGTGGGAGGTTTTT TAAAGCAAGTAAAACCTCTACAAATGTGGTAAAATCGATAAGGATCCGGGCTGGCGTAA TAGCGAAGAGGCCCGCACCGATCGCCCTTCCCAACAGTTGCGCAGCCTGAATGGCGAA TGGACGCGCCCTGTAGCGGCGCATTAAGCGCGGCGGGTGTGGTGGTTACGCGCAGCGT GACCGCTACACTTGCCAGCGCCCTAGCGCCCGCTCCTTTCGCTTTCTTCCCTTCCTTTCTC GCCACGTTCGCCGGCTTTCCCCGTCAAGCTCTAAATCGGGGGCTCCCTTTAGGGTTCCG ATTTAGTGCTTTACGGCACCTCGACCCCAAAAAACTTGATTAGGGTGATGGTTCACGTAG TGGGCCATCGCCCTGATAGACGGTTTTTCGCCCTTTGACGTTGGAGTCCACGTTCTTTAA TAGTGGACTCTTGTTCCAAACTGGAACAACACTCAACCCTATCTCGGTCTATTCTTTTGAT TTATAAGGGATTTTGCCGATTTCGGCCTATTGGTTAAAAAATGAGCTGATTTAACAAAAA TTTAACGCGAATTTTAACAAAATATTAACGCTTACAATTTCCTGATGCGGTATTTTCTCCTT ACGCATCTGTGCGGTATTTCACACCGCATATGGTGCACTCTCAGTACAATCTGCTCTGATG CCGCATAGTTAAGCCAGCCCCGACACCCGCCAACACCCGCTGACGCGCCCTGACGGGC TTGTCTGCTCCCGGCATCCGCTTACAGACAAGCTGTGACCGTCTCCGGGAGCTGCATGT GTCAGAGGTTTTCACCGTCATCACCGAAACGCGCGAGACGAAAGGGCCTCGTGATACG CCTATTTTTATAGGTTAATGTCATGATAATAATGGTTTCTTAGACGTCAGGTGGCACTTTTC GGGGAAATGTGCGCGGAACCCCTATTTGTTTATTTTTCTAAATACATTCAAATATGTATCC GCTCATGAGACAATAACCCTGATAAATGCTTCAATAATATTGAAAAAGGAAGAGTATGAG |
| | TATTCAACATTTCCGTGTCGCCCTTATTCCCTTTTTTGCGGCATTTTGCCTTCCTGTTTTTG CTCACCCAGAAACGCTGGTGAAAGTAAAAGATGCTGAAGATCAGTTGGGTGCACGAGT GGGTTACATCGAACTGGATCTCAACAGCGGTAAGATCCTTGAGAGTTTTCGCCCCGAAG AACGTTTTCCAATGATGAGCACTTTTAAAGTTCTGCTATGTGGCGCGGTATTATCCCGTAT TGACGCCGGGCAAGAGCAACTCGGTCGCCGCATACACTATTCTCAGAATGACTTGGTTG AGTACTCACCAGTCACAGAAAAGCATCTTACGGATGGCATGACAGTAAGAGAATTATGC AGTGCTGCCATAACCATGAGTGATAACACTGCGGCCAACTTACTTCTGACAACGATCGG AGGACCGAAGGAGCTAACCGCTTTTTTGCACAACATGGGGGATCATGTAACTCGCCTTG ATCGTTGGGAACCGGAGCTGAATGAAGCCATACCAAACGACGAGCGTGACACCACGAT GCCTGTAGCAATGGCAACAACGTTGCGCAAACTATTAACTGGCGAACTACTTACTCTAGC TTCCCGGCAACAATTAATAGACTGGATGGAGGCGGATAAAGTTGCAGGACCACTTCTGC GCTCGGCCCTTCCGGCTGGCTGGTTTATTGCTGATAAATCTGGAGCCGGTGAGCGTGGG TCTCGCGGTATCATTGCAGCACTGGGGCCAGATGGTAAGCCCTCCCGTATCGTAGTTATC TACACGACGGGGAGTCAGGCAACTATGGATGAACGAAATAGACAGATCGCTGAGATAG GTGCCTCACTGATTAAGCATTGGTAACTGTCAGACCAAGTTTACTCATATATACTTTAGAT TGATTTAAAACTTCATTTTTAATTTAAAAGGATCTAGGTGAAGATCCTTTTTGATAATCTC ATGACCAAAATCCCTTAACGTGAGTTTTCGTTCCACTGAGCGTCAGACCCCGTAGAAAA GATCAAAGGATCTTCTTGAGATCCTTTTTTTCTGCGCGTAATCTGCTGCTTGCAAACAAA AAAACCACCGCTACCAGCGGTGGTTTGTTTGCCGGATCAAGAGCTACCAACTCTTTTTC CGAAGGTAACTGGCTTCAGCAGAGCGCAGATACCAAATACTGTTCTTCTAGTGTAGCCG TAGTTAGGCCACCACTTCAAGAACTCTGTAGCACCGCCTACATACCTCGCTCTGCTAATCC TGTTACCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCACACAG CCCAGCTTGGAGCGAACGACCTACACCGAACTGAGATACCTACAGCGTGAGCTATGAG AAAGCGCCACGCTTCCCGAAGGGAGAAAGGCGGACAGGTATCCGGTAAGCGGCAGG GTCGGAACAGGAGAGCGCACGAGGGAGCTTCCAGGGGGAAACGCCTGGTATCTTTAT AGTCCTGTCGGGTTTCGCCACCTCTGACTTGAGCGTCGATTTTTGTGATGCTCGTCAGG GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 63) |
| pCMVTnT_sNI uc_hPPI_furin _c-peptide | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT |
| | CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACtcgaGgaattcGCCGCCACCATGAACTCCTTCTCCACAAGCGCC TTCGGTCCAGTTGCCTTCTCCCTGGGCCTGCTCCTGGTGTTGCCTGCTGCCTTCCCTGCC CCAGTCTTCACACTCGAAGATTTCGTTGGGGACTGGCGACAGACAGCCGGCTACAACC TGGACCAAGTCCTTGAACAGGGAGGTGTGTCCAGTTTGTTTCAGAATCTCGGGGTGTC CGTAACTCCGATCCAAAGGATTGTCCTGAGCGGTGAAAATGGGCTGAAGATCGACATCC ATGTCATCATCCCGTATGAAGGTCTGAGCGGCGACCAAATGGGCCAGATCGAAAAAATT TTTAAGGTGGTGTACCCTGTGGATGATCATCACTTTAAGGTGATCCTGCACTATGGCACA CTGGTAATCGACGGGGTTACGCCGAACATGATCGACTATTTCGGACGGCCGTATGAAGG CATCGCCGTGTTCGACGGCAAAAAGATCACTGTAACAGGGACCCTGTGGAACGGCAAC AAAATTATCGACGAGCGCCTGATCAACCCCGACGGCTCCCTGCTGTTCCGAGTAACCATC AACGGAGTGACCGGCTGGCGGCTGTGCGAACGCATTCTGGCGGGTGGAGGCTTTGTG AACCAACACCTGTGCGGCTCACACCTGGTGGAAGCTCTCTACCTAGTGTGCGGGGAAC GAGGCTTCTTCTACACACCCAAGACCCGCCGGAAGcggGAGGACCTGCAGGTGGGGCA GGTGGAGCTGGGCGGGggccCaggccCTGGTGCAGGCAGCCTGCAGCCCTTGGCCCTGG AGGGGTCCcgacgcAAGCGTGGCATTGTGGAACAATGCTGTACCAGCATCTGCTCcctctac cagctggagaactactgcaactagGAATTCcggtacCTCTAGAGTCGACCCGGGCGGCCGCTTCG AGCAGACATGATAAGATACATTGATGAGTTTGGACAAACCACAACTAGAATGCAGTGAA AAAAATGCTTTATTTGTGAAATTTGTGATGCTATTGCTTTATTTGTAACCATTATAAGCTGC AATAAACAAGTTAACAACAACAATTGCATTCATTTTATGTTTCAGGTTCAGGGGGAGATG TGGGAGGTTTTTTAAAGCAAGTAAAACCTCTACAAATGTGGTAAAATCGATAAGGATCC GGGCTGGCGTAATAGCGAAGAGGCCCGCACCGATCGCCCTTCCCAACAGTTGCGCAGC CTGAATGGCGAATGGACGCGCCCTGTAGCGGCGCATTAAGCGCGGCGGGTGTGGTGGT TACGCGCAGCGTGACCGCTACACTTGCCAGCGCCCTAGCGCCCGCTCCTTTCGCTTTCTT CCCTTCCTTTCTCGCCACGTTCGCCGGCTTTCCCCGTCAAGCTCTAAATCGGGGGCTCCC TTTAGGGTTCCGATTTAGTGCTTTACGGCACCTCGACCCCAAAAAACTTGATTAGGGTG ATGGTTCACGTAGTGGGCCATCGCCCTGATAGACGGTTTTTCGCCCTTTGACGTTGGAG TCCACGTTCTTTAATAGTGGACTCTTGTTCCAAACTGGAACAACACTCAACCCTATCTCG |
| | GTCTATTCTTTTGATTTATAAGGGATTTTGCCGATTTCGGCCTATTGGTTAAAAAATGAGC TGATTTAACAAAAATTTAACGCGAATTTTAACAAAATATTAACGCTTACAATTTCCTGATG CGGTATTTTCTCCTTACGCATCTGTGCGGTATTTCACACCGCATATGGTGCACTCTCAGTA CAATCTGCTCTGATGCCGCATAGTTAAGCCAGCCCCGACACCCGCCAACACCCGCTGAC GCGCCCTGACGGGCTTGTCTGCTCCCGGCATCCGCTTACAGACAAGCTGTGACCGTCTC CGGGAGCTGCATGTGTCAGAGGTTTTCACCGTCATCACCGAAACGCGCGAGACGAAAG GGCCTCGTGATACGCCTATTTTTATAGGTTAATGTCATGATAATAATGGTTTCTTAGACGTC AGGTGGCACTTTTCGGGGAAATGTGCGCGGAACCCCTATTTGTTTATTTTTCTAAATACA TTCAAATATGTATCCGCTCATGAGACAATAACCCTGATAAATGCTTCAATAATATTGAAAA AGGAAGAGTATGAGTATTCAACATTTCCGTGTCGCCCTTATTCCCTTTTTTGCGGCATTTT GCCTTCCTGTTTTTGCTCACCCAGAAACGCTGGTGAAAGTAAAAGATGCTGAAGATCAG TTGGGTGCACGAGTGGGTTACATCGAACTGGATCTCAACAGCGGTAAGATCCTTGAGA GTTTTCGCCCCGAAGAACGTTTTCCAATGATGAGCACTTTTAAAGTTCTGCTATGTGGCG CGGTATTATCCCGTATTGACGCCGGGCAAGAGCAACTCGGTCGCCGCATACACTATTCTC AGAATGACTTGGTTGAGTACTCACCAGTCACAGAAAAGCATCTTACGGATGGCATGACA GTAAGAGAATTATGCAGTGCTGCCATAACCATGAGTGATAACACTGCGGCCAACTTACTT CTGACAACGATCGGAGGACCGAAGGAGCTAACCGCTTTTTTGCACAACATGGGGGATC ATGTAACTCGCCTTGATCGTTGGGAACCGGAGCTGAATGAAGCCATACCAAACGACGAG CGTGACACCACGATGCCTGTAGCAATGGCAACAACGTTGCGCAAACTATTAACTGGCGA ACTACTTACTCTAGCTTCCCGGCAACAATTAATAGACTGGATGGAGGCGGATAAAGTTGC AGGACCACTTCTGCGCTCGGCCCTTCCGGCTGGCTGGTTTATTGCTGATAAATCTGGAG CCGGTGAGCGTGGGTCTCGCGGTATCATTGCAGCACTGGGGCCAGATGGTAAGCCCTC CCGTATCGTAGTTATCTACACGACGGGGAGTCAGGCAACTATGGATGAACGAAATAGAC AGATCGCTGAGATAGGTGCCTCACTGATTAAGCATTGGTAACTGTCAGACCAAGTTTACT CATATATACTTTAGATTGATTTAAAACTTCATTTTTAATTTAAAAGGATCTAGGTGAAGATC CTTTTTGATAATCTCATGACCAAAATCCCTTAACGTGAGTTTTCGTTCCACTGAGCGTCAG ACCCCGTAGAAAAGATCAAAGGATCTTCTTGAGATCCTTTTTTTCTGCGCGTAATCTGCT GCTTGCAAACAAAAAAACCACCGCTACCAGCGGTGGTTTGTTTGCCGGATCAAGAGCT ACCAACTCTTTTTCCGAAGGTAACTGGCTTCAGCAGAGCGCAGATACCAAATACTGTTCT TCTAGTGTAGCCGTAGTTAGGCCACCACTTCAAGAACTCTGTAGCACCGCCTACATACCT CGCTCTGCTAATCCTGTTACCAGTGGCTGCTGCCAGTGGCGATAAGTCGTGTCTTACCGG GTTGGACTCAAGACGATAGTTACCGGATAAGGCGCAGCGGTCGGGCTGAACGGGGGG TTCGTGCACACAGCCCAGCTTGGAGCGAACGACCTACACCGAACTGAGATACCTACAGC GTGAGCTATGAGAAAGCGCCACGCTTCCCGAAGGGAGAAAGGCGGACAGGTATCCGG TAAGCGGCAGGGTCGGAACAGGAGAGCGCACGAGGGAGCTTCCAGGGGGAAACGCC TGGTATCTTTATAGTCCTGTCGGGTTTCGCCACCTCTGACTTGAGCGTCGATTTTTGTGAT |
| | GCTCGTCAGGGGGGCGGAGCCTATGGAAAAACGCCAGCAACGCGGCCTTTTTACGGTT CCTGGCCTTTTGCTGGCCTTTTGCTCACATGGCTCGACAGATCT (SEQ ID NO: 64) |

**Table 6: exemplary amino acid sequences of mutated proinsulin B NLuc recombinant antigen molecules.**

| Name | Sequence (SEQ ID NO) |
|---|---|
| Proinsulin B NLuc Delta B24-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCATGGTCTTCACACTCGAAGATTTCGTTGGGGACTG GCGACAGACAGCCGGCTACAACCTGGACCAAGTCCTTGAACAGGGAGGTGTGTCCAGT TTGTTTCAGAATCTCGGGGTGTCCGTAACTCCGATCCAAAGGATTGTCCTGAGCGGTGA AAATGGGCTGAAGATCGACATCCATGTCATCATCCCGTATGAAGGTCTGAGCGGCGACC AAATGGGCCAGATCGAAAAAATTTTTAAGGTGGTGTACCCTGTGGATGATCATCACTTTA AGGTGATCCTGCACTATGGCACACTGGTAATCGACGGGGTTACGCCGAACATGATCGAC TATTTCGGACGGCCGTATGAAGGCATCGCCGTGTTCGACGGCAAAAAGATCACTGTAAC AGGGACCCTGTGGAACGGCAACAAAATTATCGACGAGCGCCTGATCAACCCCGACGGC TCCCTGCTGTTCCGAGTAACCATCAACGGAGTGACCGGCTGGCGGCTGTGCGAACGCAT TCTGGCGGCTGCTGCTGCGCGCCGGGAGGCAGAGGACCTGCAGGTGGGGCAGGTGG AGCTGGGCGGGGGCCCAGGCGCAGGCAGCCTGCAGCCCTTGGCCCTGGAGGGGTCC CTGCAGAAGCGTGGCATTGTGGAACAATGCTGTACCAGCATCTGCTCCCTCTACCAGCT GGAGAACTACTGCAACTAG (SEQ ID NO: 65) |
| Proinsulin B NLuc Delta B25-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCATGGTCTTCACACTCGAAGATTTCGTTGGGGA CTGGCGACAGACAGCCGGCTACAACCTGGACCAAGTCCTTGAACAGGGAGGTGTGTCC AGTTTGTTTCAGAATCTCGGGGTGTCCGTAACTCCGATCCAAAGGATTGTCCTGAGCGG TGAAAATGGGCTGAAGATCGACATCCATGTCATCATCCCGTATGAAGGTCTGAGCGGCG ACCAAATGGGCCAGATCGAAAAAATTTTTAAGGTGGTGTACCCTGTGGATGATCATCAC TTTAAGGTGATCCTGCACTATGGCACACTGGTAATCGACGGGGTTACGCCGAACATGATC GACTATTTCGGACGGCCGTATGAAGGCATCGCCGTGTTCGACGGCAAAAAGATCACTGT AACAGGGACCCTGTGGAACGGCAACAAAATTATCGACGAGCGCCTGATCAACCCCGAC GGCTCCCTGCTGTTCCGAGTAACCATCAACGGAGTGACCGGCTGGCGGCTGTGCGAAC GCATTCTGGCGGCTGCTGCTGCGCGCCGGGAGGCAGAGGACCTGCAGGTGGGGCAG GTGGAGCTGGGCGGGGGCCCAGGCGCAGGCAGCCTGCAGCCCTTGGCCCTGGAGGG GTCCCTGCAGAAGCGTGGCATTGTGGAACAATGCTGTACCAGCATCTGCTCCCTCTACCA GCTGGAGAACTACTGCAACTAG (SEQ ID NO: 66) |
| Proinsulin B NLuc Delta B26-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCATGGTCTTCACACTCGAAGATTTCGTTGGG GACTGGCGACAGACAGCCGGCTACAACCTGGACCAAGTCCTTGAACAGGGAGGTGTG TCCAGTTTGTTTCAGAATCTCGGGGTGTCCGTAACTCCGATCCAAAGGATTGTCCTGAGC GGTGAAAATGGGCTGAAGATCGACATCCATGTCATCATCCCGTATGAAGGTCTGAGCGG CGACCAAATGGGCCAGATCGAAAAAATTTTTAAGGTGGTGTACCCTGTGGATGATCATC ACTTTAAGGTGATCCTGCACTATGGCACACTGGTAATCGACGGGGTTACGCCGAACATG ATCGACTATTTCGGACGGCCGTATGAAGGCATCGCCGTGTTCGACGGCAAAAAGATCAC TGTAACAGGGACCCTGTGGAACGGCAACAAAATTATCGACGAGCGCCTGATCAACCCC GACGGCTCCCTGCTGTTCCGAGTAACCATCAACGGAGTGACCGGCTGGCGGCTGTGCG AACGCATTCTGGCGGCTGCTGCTGCGCGCCGGGAGGCAGAGGACCTGCAGGTGGGGC AGGTGGAGCTGGGCGGGGGCCCAGGCGCAGGCAGCCTGCAGCCCTTGGCCCTGGAG GGGTCCCTGCAGAAGCGTGGCATTGTGGAACAATGCTGTACCAGCATCTGCTCCCTCTA CCAGCTGGAGAACTACTGCAACTAG (SEQ ID NO: 67) |
| Proinsulin B NLuc Delta B27-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACATGGTCTTCACACTCGAAGATTTCGTT GGGGACTGGCGACAGACAGCCGGCTACAACCTGGACCAAGTCCTTGAACAGGGAGGT GTGTCCAGTTTGTTTCAGAATCTCGGGGTGTCCGTAACTCCGATCCAAAGGATTGTCCTG AGCGGTGAAAATGGGCTGAAGATCGACATCCATGTCATCATCCCGTATGAAGGTCTGAG CGGCGACCAAATGGGCCAGATCGAAAAAATTTTTAAGGTGGTGTACCCTGTGGATGATC ATCACTTTAAGGTGATCCTGCACTATGGCACACTGGTAATCGACGGGGTTACGCCGAAC ATGATCGACTATTTCGGACGGCCGTATGAAGGCATCGCCGTGTTCGACGGCAAAAAGAT CACTGTAACAGGGACCCTGTGGAACGGCAACAAAATTATCGACGAGCGCCTGATCAAC CCCGACGGCTCCCTGCTGTTCCGAGTAACCATCAACGGAGTGACCGGCTGGCGGCTGT GCGAACGCATTCTGGCGGCTGCTGCTGCGCGCCGGGAGGCAGAGGACCTGCAGGTGG GGCAGGTGGAGCTGGGCGGGGGCCCAGGCGCAGGCAGCCTGCAGCCCTTGGCCCTG GAGGGGTCCCTGCAGAAGCGTGGCATTGTGGAACAATGCTGTACCAGCATCTGCTCCCT CTACCAGCTGGAGAACTACTGCAACTAG (SEQ ID NO: 68) |
| Proinsulin B NLuc Delta B28-30 | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACAATGGTCTTCACACTCGAAGATTTC GTTGGGGACTGGCGACAGACAGCCGGCTACAACCTGGACCAAGTCCTTGAACAGGGA GGTGTGTCCAGTTTGTTTCAGAATCTCGGGGTGTCCGTAACTCCGATCCAAAGGATTGT CCTGAGCGGTGAAAATGGGCTGAAGATCGACATCCATGTCATCATCCCGTATGAAGGTC TGAGCGGCGACCAAATGGGCCAGATCGAAAAAATTTTTAAGGTGGTGTACCCTGTGGA |
| | TGATCATCACTTTAAGGTGATCCTGCACTATGGCACACTGGTAATCGACGGGGTTACGCC GAACATGATCGACTATTTCGGACGGCCGTATGAAGGCATCGCCGTGTTCGACGGCAAAA AGATCACTGTAACAGGGACCCTGTGGAACGGCAACAAAATTATCGACGAGCGCCTGATC AACCCCGACGGCTCCCTGCTGTTCCGAGTAACCATCAACGGAGTGACCGGCTGGCGGC TGTGCGAACGCATTCTGGCGGCTGCTGCTGCGCGCCGGGAGGCAGAGGACCTGCAGG TGGGGCAGGTGGAGCTGGGCGGGGGCCCAGGCGCAGGCAGCCTGCAGCCCTTGGCC CTGGAGGGGTCCCTGCAGAAGCGTGGCATTGTGGAACAATGCTGTACCAGCATCTGCTC CCTCTACCAGCTGGAGAACTACTGCAACTAG (SEQ ID NO: 69) |
| Proinsulin B NLuc mut A L13A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGGAGGCAGAGGAC CTGCAGGTGGGGCAGGTGGAGCTGGGCGGGGGCCCAGGCGCAGGCAGCCTGCAGCC CTTGGCCCTGGAGGGGTCCCTGCAGAAGCGTGGCATTGTGGAACAATGCTGTACCAGC ATCTGCTCcGCctaccagctggagaactactgcaactag (SEQ ID NO: 70) |
| Proinsulin B NLuc mut A L13W | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGGAGGCAGAGGAC CTGCAGGTGGGGCAGGTGGAGCTGGGCGGGGGCCCAGGCGCAGGCAGCCTGCAGCC |
| | CTTGGCCCTGGAGGGGTCCCTGCAGAAGCGTGGCATTGTGGAACAATGCTGTACCAGC ATCTGCTCCTGGTACCAGCTGGAGAACTACTGCAACTAG (SEQ ID NO: 71) |
| Proinsulin B NLuc mut A Y14A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGGAGGCAGAGGAC CTGCAGGTGGGGCAGGTGGAGCTGGGCGGGGGCCCAGGCGCAGGCAGCCTGCAGCC CTTGGCCCTGGAGGGGTCCCTGCAGAAGCGTGGCATTGTGGAACAATGCTGTACCAGC ATCTGCTCCCTCGCCCAGCTGGAGAACTACTGCAACTAG (SEQ ID NO: 72) |
| Proinsulin B NLuc mut B F1A | ATGGCCCTGTGGATGCGCCTCCTGCCCCTGCTGGCGCTGCTGGCCCTCTGGGGACCTGA CCCAGCCGCAGCCGCTGTGAACCAACACCTGTGCGGCTCACACCTGGTGGAAGCTCTC TACCTAGTGTGCGGGGAACGAGGCTTCTTCTACACACCCAAGACCATGGTCTTCACACT CGAAGATTTCGTTGGGGACTGGCGACAGACAGCCGGCTACAACCTGGACCAAGTCCTT GAACAGGGAGGTGTGTCCAGTTTGTTTCAGAATCTCGGGGTGTCCGTAACTCCGATCCA AAGGATTGTCCTGAGCGGTGAAAATGGGCTGAAGATCGACATCCATGTCATCATCCCGT ATGAAGGTCTGAGCGGCGACCAAATGGGCCAGATCGAAAAAATTTTTAAGGTGGTGTA CCCTGTGGATGATCATCACTTTAAGGTGATCCTGCACTATGGCACACTGGTAATCGACGG GGTTACGCCGAACATGATCGACTATTTCGGACGGCCGTATGAAGGCATCGCCGTGTTCG ACGGCAAAAAGATCACTGTAACAGGGACCCTGTGGAACGGCAACAAAATTATCGACGA GCGCCTGATCAACCCCGACGGCTCCCTGCTGTTCCGAGTAACCATCAACGGAGTGACCG GCTGGCGGCTGTGCGAACGCATTCTGGCGGCTGCTGCTGCGCGCCGGGAGGCAGAGG ACCTGCAGGTGGGGCAGGTGGAGCTGGGCGGGGGCCCAGGCGCAGGCAGCCTGCAG CCCTTGGCCCTGGAGGGGTCCCTGCAGAAGCGTGGCATTGTGGAACAATGCTGTACCA GCATCTGCTCCCTCTACCAGCTGGAGAACTACTGCAACTAG (SEQ ID NO: 73) |
| Proinsulin B NLuc mut B L17A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACGCAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA |
| | AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGGAGGCAGAGGAC CTGCAGGTGGGGCAGGTGGAGCTGGGCGGGGGCCCAGGCGCAGGCAGCCTGCAGCC CTTGGCCCTGGAGGGGTCCCTGCAGAAGCGTGGCATTGTGGAACAATGCTGTACCAGC ATCTGCTCCCTCTACCAGCTGGAGAACTACTGCAACTAG (SEQ ID NO: 74) |

**Table 7: exemplary amino acid sequences of recombinant proinsulin luciferase backbone antigen molecules.**

| Name | Sequence (SEQ ID NO) |
|---|---|
| Proinsulin A NLuc | ATGGCCCTGTGGATGCGCTTGTTGCCCCTTCTGGCCCTTCTCGCACTGTGGGGACCTGAT CCAGCTGCGGCCTTTGTGAACCAGCACCTGTGTGGGTCCCATCTGGTGGAAGCCCTGTA TCTCGTCTGTGGAGAACGCGGCTTCTTCTACACACCGAAGACCCGCCGGGAGGCAGAG GACCTGCAGGTGGGGCAGGTGGAGCTGGGCGGGGGCCCAGGCGCAGGCAGCCTGCA GCCCTTGGCCCTGGAGGGGTCCCTGCAGAAGCGTGGCATTGTGGAGCAGTGCTGCACC AGCATCTGCTCCCTCTACCAGCTGGAGAACTACTGCAACGAATTCGGTGGAGGCATGGT CTTCACACTCGAAGATTTCGTTGGGGACTGGCGACAGACAGCCGGCTACAACCTGGAC CAAGTCCTTGAACAGGGAGGTGTGTCCAGTTTGTTTCAGAATCTCGGGGTGTCCGTAAC TCCGATCCAAAGGATTGTCCTGAGCGGTGAAAATGGGCTGAAGATCGACATCCATGTCA TCATCCCGTATGAAGGTCTGAGCGGCGACCAAATGGGCCAGATCGAAAAAATTTTTAAG GTGGTGTACCCTGTGGATGATCATCACTTTAAGGTGATCCTGCACTATGGCACACTGGTA ATCGACGGGGTTACGCCGAACATGATCGACTATTTCGGACGGCCGTATGAAGGCATCGC CGTGTTCGACGGCAAAAAGATCACTGTAACAGGGACCCTGTGGAACGGCAACAAAATT ATCGACGAGCGCCTGATCAACCCCGACGGCTCCCTGCTGTTCCGAGTAACCATCAACGG AGTGACCGGCTGGCGGCTGTGCGAACGCATTCTGGCGTAA (SEQ ID NO: 75) |
| Proinsulin B NLuc no PG duplication | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG |
| | TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGGAGGCAGAGGAC CTGCAGGTGGGGCAGGTGGAGCTGGGCGGGGGCCCAGGCGCAGGCAGCCTGCAGCC CTTGGCCCTGGAGGGGTCCCTGCAGAAGCGTGGCATTGTGGAACAATGCTGTACCAGC ATCTGCTCCCTCTACCAGCTGGAGAACTACTGCAACTAG (SEQ ID NO: 76) |
| Proinsulin B NLuc | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGGCTGCTGCTGCGCGCCGGGAGGCAGAGGAC CTGCAGGTGGGGCAGGTGGAGCTGGGCGGGGGCCCAGGCCCTGGTGCAGGCAGCCT GCAGCCCTTGGCCCTGGAGGGGTCCCTGCAGAAGCGTGGCATTGTGGAACAATGCTGT ACCAGCATCTGCTCCCTCTACCAGCTGGAGAACTACTGCAACTAG (SEQ ID NO: 77) |
| Proinsulin C pep NLuc C pep | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGGAGGCAGA GGACCTGCAGGTGGGGCAGGTGGAGCTGGGCGGGggccCaATGGTCTTCACACTCGAA GATTTCGTTGGGGACTGGCGACAGACAGCCGGCTACAACCTGGACCAAGTCCTTGAAC AGGGAGGTGTGTCCAGTTTGTTTCAGAATCTCGGGGTGTCCGTAACTCCGATCCAAAGG ATTGTCCTGAGCGGTGAAAATGGGCTGAAGATCGACATCCATGTCATCATCCCGTATGAA GGTCTGAGCGGCGACCAAATGGGCCAGATCGAAAAAATTTTTAAGGTGGTGTACCCTG TGGATGATCATCACTTTAAGGTGATCCTGCACTATGGCACACTGGTAATCGACGGGGTTA CGCCGAACATGATCGACTATTTCGGACGGCCGTATGAAGGCATCGCCGTGTTCGACGGC AAAAAGATCACTGTAACAGGGACCCTGTGGAACGGCAACAAAATTATCGACGAGCGCC TGATCAACCCCGACGGCTCCCTGCTGTTCCGAGTAACCATCAACGGAGTGACCGGCTGG CGGCTGTGCGAACGCATTCTGGCGgctgctgctgcGggccCTGGTGCAGGCAGCCTGCAGC CCTTGGCCCTGGAGGGGTCCCTGCAGAAGCGTGGCATTGTGGAACAATGCTGTACCAG CATCTGCTCcctctaccagctggagaactactgcaactag (SEQ ID NO: 78) |
| Proinsulin NLuc A c peptide PG duplication | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGGAGGCAGA GGACCTGCAGGTGGGGCAGGTGGAGCTGGGCGGGGGCCCAGGCCCTGGTGCAGGCA GCCTGCAGCCCTTGGCCCTGGAGGGGTCCCTGCAGAAGCGTATGGTCTTCACACTCGA AGATTTCGTTGGGGACTGGCGACAGACAGCCGGCTACAACCTGGACCAAGTCCTTGAA CAGGGAGGTGTGTCCAGTTTGTTTCAGAATCTCGGGGTGTCCGTAACTCCGATCCAAAG GATTGTCCTGAGCGGTGAAAATGGGCTGAAGATCGACATCCATGTCATCATCCCGTATGA AGGTCTGAGCGGCGACCAAATGGGCCAGATCGAAAAAATTTTTAAGGTGGTGTACCCT GTGGATGATCATCACTTTAAGGTGATCCTGCACTATGGCACACTGGTAATCGACGGGGTT ACGCCGAACATGATCGACTATTTCGGACGGCCGTATGAAGGCATCGCCGTGTTCGACGG CAAAAAGATCACTGTAACAGGGACCCTGTGGAACGGCAACAAAATTATCGACGAGCGC CTGATCAACCCCGACGGCTCCCTGCTGTTCCGAGTAACCATCAACGGAGTGACCGGCTG GCGGCTGTGCGAACGCATTCTGGCGGCTGCTGCTGCGGGCATTGTGGAACAATGCTGT ACCAGCATCTGCTCCCTCTACCAGCTGGAGAACTACTGCAACTAG (SEQ ID NO: 79) |
| Proinsulin NLuc A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGGAGGCAGA GGACCTGCAGGTGGGGCAGGTGGAGCTGGGCGGGGGCCCAGGCGCAGGCAGCCTGC AGCCCTTGGCCCTGGAGGGGTCCCTGCAGAAGCGTATGGTCTTCACACTCGAAGATTTC GTTGGGGACTGGCGACAGACAGCCGGCTACAACCTGGACCAAGTCCTTGAACAGGGA GGTGTGTCCAGTTTGTTTCAGAATCTCGGGGTGTCCGTAACTCCGATCCAAAGGATTGT CCTGAGCGGTGAAAATGGGCTGAAGATCGACATCCATGTCATCATCCCGTATGAAGGTC TGAGCGGCGACCAAATGGGCCAGATCGAAAAAATTTTTAAGGTGGTGTACCCTGTGGA TGATCATCACTTTAAGGTGATCCTGCACTATGGCACACTGGTAATCGACGGGGTTACGCC GAACATGATCGACTATTTCGGACGGCCGTATGAAGGCATCGCCGTGTTCGACGGCAAAA AGATCACTGTAACAGGGACCCTGTGGAACGGCAACAAAATTATCGACGAGCGCCTGATC AACCCCGACGGCTCCCTGCTGTTCCGAGTAACCATCAACGGAGTGACCGGCTGGCGGC TGTGCGAACGCATTCTGGCGGCTGCTGCTGCGGGCATTGTGGAACAATGCTGTACCAGC ATCTGCTCCCTCTACCAGCTGGAGAACTACTGCAACTAG (SEQ ID NO: 80) |
| secNLuc Proinsulin | ATGAACTCCTTCTCCACAAGCGCCTTCGGTCCAGTTGCCTTCTCCCTGGGCCTGCTCCTG GTGTTGCCTGCTGCCTTCCCTGCCCCAGTCTTCACACTCGAAGATTTCGTTGGGGACTG GCGACAGACAGCCGGCTACAACCTGGACCAAGTCCTTGAACAGGGAGGTGTGTCCAGT TTGTTTCAGAATCTCGGGGTGTCCGTAACTCCGATCCAAAGGATTGTCCTGAGCGGTGA AAATGGGCTGAAGATCGACATCCATGTCATCATCCCGTATGAAGGTCTGAGCGGCGACC AAATGGGCCAGATCGAAAAAATTTTTAAGGTGGTGTACCCTGTGGATGATCATCACTTTA AGGTGATCCTGCACTATGGCACACTGGTAATCGACGGGGTTACGCCGAACATGATCGAC |
| | TATTTCGGACGGCCGTATGAAGGCATCGCCGTGTTCGACGGCAAAAAGATCACTGTAAC AGGGACCCTGTGGAACGGCAACAAAATTATCGACGAGCGCCTGATCAACCCCGACGGC TCCCTGCTGTTCCGAGTAACCATCAACGGAGTGACCGGCTGGCGGCTGTGCGAACGCAT TCTGGCGGGTGGAGGCTTTGTGAACCAACACCTGTGCGGCTCACACCTGGTGGAAGCT CTCTACCTAGTGTGCGGGGAACGAGGCTTCTTCTACACACCCAAGACCCGCCGGgAGG CaGAGGACCTGCAGGTGGGGCAGGTGGAGCTGGGCGGgggccCaggccCTGGTGCAGG CAGCCTGCAGCCCTTGGCCCTGGAGGGGTCCcTgcAGAAGCGTGGCATTGTGGAACAAT GCTGTACCAGCATCTGCTCcctctaccagctggagaactactgcaactag (SEQ ID NO: 81) |

**Table 8: exemplary amino acid sequences of mutated proinsulin NLuc A antigen molecules.**

| Name | Sequence (SEQ ID NO) |
|---|---|
| Proinsulin NLuc A mut A L13A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGGAGGCAGA GGACCTGCAGGTGGGGCAGGTGGAGCTGGGCGGGGGCCCAGGCGCAGGCAGCCTGC AGCCCTTGGCCCTGGAGGGGTCCCTGCAGAAGCGTATGGTCTTCACACTCGAAGATTTC GTTGGGGACTGGCGACAGACAGCCGGCTACAACCTGGACCAAGTCCTTGAACAGGGA GGTGTGTCCAGTTTGTTTCAGAATCTCGGGGTGTCCGTAACTCCGATCCAAAGGATTGT CCTGAGCGGTGAAAATGGGCTGAAGATCGACATCCATGTCATCATCCCGTATGAAGGTC TGAGCGGCGACCAAATGGGCCAGATCGAAAAAATTTTTAAGGTGGTGTACCCTGTGGA TGATCATCACTTTAAGGTGATCCTGCACTATGGCACACTGGTAATCGACGGGGTTACGCC GAACATGATCGACTATTTCGGACGGCCGTATGAAGGCATCGCCGTGTTCGACGGCAAAA AGATCACTGTAACAGGGACCCTGTGGAACGGCAACAAAATTATCGACGAGCGCCTGATC AACCCCGACGGCTCCCTGCTGTTCCGAGTAACCATCAACGGAGTGACCGGCTGGCGGC TGTGCGAACGCATTCTGGCGGCTGCTGCTGCGGGCATTGTGGAACAATGCTGTACCAGC ATCTGCTCCGCCTACCAGCTGGAGAACTACTGCAACTAG (SEQ ID NO: 82) |
| Proinsulin NLuc A mut A L13W | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGGAGGCAGA GGACCTGCAGGTGGGGCAGGTGGAGCTGGGCGGGGGCCCAGGCGCAGGCAGCCTGC AGCCCTTGGCCCTGGAGGGGTCCCTGCAGAAGCGTATGGTCTTCACACTCGAAGATTTC GTTGGGGACTGGCGACAGACAGCCGGCTACAACCTGGACCAAGTCCTTGAACAGGGA GGTGTGTCCAGTTTGTTTCAGAATCTCGGGGTGTCCGTAACTCCGATCCAAAGGATTGT CCTGAGCGGTGAAAATGGGCTGAAGATCGACATCCATGTCATCATCCCGTATGAAGGTC TGAGCGGCGACCAAATGGGCCAGATCGAAAAAATTTTTAAGGTGGTGTACCCTGTGGA TGATCATCACTTTAAGGTGATCCTGCACTATGGCACACTGGTAATCGACGGGGTTACGCC GAACATGATCGACTATTTCGGACGGCCGTATGAAGGCATCGCCGTGTTCGACGGCAAAA |
| | AGATCACTGTAACAGGGACCCTGTGGAACGGCAACAAAATTATCGACGAGCGCCTGATC AACCCCGACGGCTCCCTGCTGTTCCGAGTAACCATCAACGGAGTGACCGGCTGGCGGC TGTGCGAACGCATTCTGGCGGCTGCTGCTGCGGGCATTGTGGAACAATGCTGTACCAGC ATCTGCTCCTGGTACCAGCTGGAGAACTACTGCAACTAG (SEQ ID NO: 83) |
| Proinsulin NLuc A mut A Y14A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCCGCCGGGAGGCAGA GGACCTGCAGGTGGGGCAGGTGGAGCTGGGCGGGGGCCCAGGCGCAGGCAGCCTGC AGCCCTTGGCCCTGGAGGGGTCCCTGCAGAAGCGTATGGTCTTCACACTCGAAGATTTC GTTGGGGACTGGCGACAGACAGCCGGCTACAACCTGGACCAAGTCCTTGAACAGGGA GGTGTGTCCAGTTTGTTTCAGAATCTCGGGGTGTCCGTAACTCCGATCCAAAGGATTGT CCTGAGCGGTGAAAATGGGCTGAAGATCGACATCCATGTCATCATCCCGTATGAAGGTC TGAGCGGCGACCAAATGGGCCAGATCGAAAAAATTTTTAAGGTGGTGTACCCTGTGGA TGATCATCACTTTAAGGTGATCCTGCACTATGGCACACTGGTAATCGACGGGGTTACGCC GAACATGATCGACTATTTCGGACGGCCGTATGAAGGCATCGCCGTGTTCGACGGCAAAA AGATCACTGTAACAGGGACCCTGTGGAACGGCAACAAAATTATCGACGAGCGCCTGATC AACCCCGACGGCTCCCTGCTGTTCCGAGTAACCATCAACGGAGTGACCGGCTGGCGGC TGTGCGAACGCATTCTGGCGGCTGCTGCTGCGGGCATTGTGGAACAATGCTGTACCAGC ATCTGCTCCCTCGCCCAGCTGGAGAACTACTGCAACTAG (SEQ ID NO: 84) |
| Proinsulin NLuc A mut B F1A | ATGGCCCTGTGGATGCGCCTCCTGCCCCTGCTGGCGCTGCTGGCCCTCTGGGGACCTGA CCCAGCCGCAGCCGCTGTGAACCAACACCTGTGCGGCTCACACCTGGTGGAAGCTCTC TACCTAGTGTGCGGGGAACGAGGCTTCTTCTACACACCCAAGACCCGCCGGGAGGCAG AGGACCTGCAGGTGGGGCAGGTGGAGCTGGGCGGGGGCCCAGGCGCAGGCAGCCTG CAGCCCTTGGCCCTGGAGGGGTCCCTGCAGAAGCGTATGGTCTTCACACTCGAAGATTT CGTTGGGGACTGGCGACAGACAGCCGGCTACAACCTGGACCAAGTCCTTGAACAGGG AGGTGTGTCCAGTTTGTTTCAGAATCTCGGGGTGTCCGTAACTCCGATCCAAAGGATTG TCCTGAGCGGTGAAAATGGGCTGAAGATCGACATCCATGTCATCATCCCGTATGAAGGT CTGAGCGGCGACCAAATGGGCCAGATCGAAAAAATTTTTAAGGTGGTGTACCCTGTGG ATGATCATCACTTTAAGGTGATCCTGCACTATGGCACACTGGTAATCGACGGGGTTACGC CGAACATGATCGACTATTTCGGACGGCCGTATGAAGGCATCGCCGTGTTCGACGGCAAA AAGATCACTGTAACAGGGACCCTGTGGAACGGCAACAAAATTATCGACGAGCGCCTGA TCAACCCCGACGGCTCCCTGCTGTTCCGAGTAACCATCAACGGAGTGACCGGCTGGCG GCTGTGCGAACGCATTCTGGCGGCTGCTGCTGCGGGCATTGTGGAACAATGCTGTACCA GCATCTGCTCCCTCTACCAGCTGGAGAACTACTGCAACTAG (SEQ ID NO: 85) |
| Proinsulin NLuc A mut B L17A V18A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACGCAGCGTGCGGGGAACGAGGCTTCTTCTACACACCCAAGACCCGCCGGGAGGCAG |
| | AGGACCTGCAGGTGGGGCAGGTGGAGCTGGGCGGGGGCCCAGGCGCAGGCAGCCTG CAGCCCTTGGCCCTGGAGGGGTCCCTGCAGAAGCGTATGGTCTTCACACTCGAAGATTT CGTTGGGGACTGGCGACAGACAGCCGGCTACAACCTGGACCAAGTCCTTGAACAGGG AGGTGTGTCCAGTTTGTTTCAGAATCTCGGGGTGTCCGTAACTCCGATCCAAAGGATTG TCCTGAGCGGTGAAAATGGGCTGAAGATCGACATCCATGTCATCATCCCGTATGAAGGT CTGAGCGGCGACCAAATGGGCCAGATCGAAAAAATTTTTAAGGTGGTGTACCCTGTGG ATGATCATCACTTTAAGGTGATCCTGCACTATGGCACACTGGTAATCGACGGGGTTACGC CGAACATGATCGACTATTTCGGACGGCCGTATGAAGGCATCGCCGTGTTCGACGGCAAA AAGATCACTGTAACAGGGACCCTGTGGAACGGCAACAAAATTATCGACGAGCGCCTGA TCAACCCCGACGGCTCCCTGCTGTTCCGAGTAACCATCAACGGAGTGACCGGCTGGCG GCTGTGCGAACGCATTCTGGCGGCTGCTGCTGCGGGCATTGTGGAACAATGCTGTACCA GCATCTGCTCCCTCTACCAGCTGGAGAACTACTGCAACTAG (SEQ ID NO: 86) |
| Proinsulin NLuc A mut B L17A | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACGCAGTGTGCGGGGAACGAGGCTTCTTCTACACACCCAAGACCCGCCGGGAGGCAG AGGACCTGCAGGTGGGGCAGGTGGAGCTGGGCGGGGGCCCAGGCGCAGGCAGCCTG CAGCCCTTGGCCCTGGAGGGGTCCCTGCAGAAGCGTATGGTCTTCACACTCGAAGATTT CGTTGGGGACTGGCGACAGACAGCCGGCTACAACCTGGACCAAGTCCTTGAACAGGG AGGTGTGTCCAGTTTGTTTCAGAATCTCGGGGTGTCCGTAACTCCGATCCAAAGGATTG TCCTGAGCGGTGAAAATGGGCTGAAGATCGACATCCATGTCATCATCCCGTATGAAGGT CTGAGCGGCGACCAAATGGGCCAGATCGAAAAAATTTTTAAGGTGGTGTACCCTGTGG ATGATCATCACTTTAAGGTGATCCTGCACTATGGCACACTGGTAATCGACGGGGTTACGC CGAACATGATCGACTATTTCGGACGGCCGTATGAAGGCATCGCCGTGTTCGACGGCAAA AAGATCACTGTAACAGGGACCCTGTGGAACGGCAACAAAATTATCGACGAGCGCCTGA TCAACCCCGACGGCTCCCTGCTGTTCCGAGTAACCATCAACGGAGTGACCGGCTGGCG GCTGTGCGAACGCATTCTGGCGGCTGCTGCTGCGGGCATTGTGGAACAATGCTGTACCA GCATCTGCTCCCTCTACCAGCTGGAGAACTACTGCAACTAG (SEQ ID NO: 87) |

**Table 9: exemplary amino acid sequences of mutated proinsulin antigen molecules in a pCMVTnT vector.**

| Name | Sequence (SEQ ID NO) |
|---|---|
| pCMVTnT ProIns B Nluc A chain L13A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG |
| | CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGgAGGCaGAGGACCTGCAGGTGGGGCAGGTGGAGCTGGGCGGg ggccCaggcGCAGGCAGCCTGCAGCCCTTGGCCCTGGAGGGGTCCcTgcAGAAGCGTGGC ATTGTGGAACAATGCTGTACCAGCATCTGCTCcGCctaccagctggagaactactgcaactagGaa ttCACGCGTGGTACCTCTAGAGTCGACCCGGGCGGCCGCTTCGAGCAGACATGATAAGA TACATTGATGAGTTTGGACAAACCACAACTAGAATGCAGTGAAAAAAATGCTTTATTTGT GAAATTTGTGATGCTATTGCTTTATTTGTAACCATTATAAGCTGCAATAAACAAGTTAACA ACAACAATTGCATTCATTTTATGTTTCAGGTTCAGGGGGAGATGTGGGAGGTTTTTTAA AGCAAGTAAAACCTCTACAAATGTGGTAAAATCGATAAGGATCCGGGCTGGCGTAATAG CGAAGAGGCCCGCACCGATCGCCCTTCCCAACAGTTGCGCAGCCTGAATGGCGAATGG ACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCC ACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGG |
| | GCCATCGCCCTGATAGACGGTTTTTCGCCCTTTGACGTTGGAGTCCACGTTCTTTAATAG TGGACTCTTGTTCCAAACTGGAACAACACTCAACCCTATCTCGGTCTATTCTTTTGATTTA TAAGGGATTTTGCCGATTTCGGCCTATTGGTTAAAAAATGAGCTGATTTAACAAAAATTT AACGCGAATTTTAACAAAATATTAACGCTTACAATTTCCTGATGCGGTATTTTCTCCTTAC GCATCTGTGCGGTATTTCACACCGCATATGGTGCACTCTCAGTACAATCTGCTCTGATGCC GCATAGTTAAGCCAGCCCCGACACCCGCCAACACCCGCTGACGCGCCCTGACGGGCTT GTCTGCTCCCGGCATCCGCTTACAGACAAGCTGTGACCGTCTCCGGGAGCTGCATGTGT CAGAGGTTTTCACCGTCATCACCGAAACGCGCGAGACGAAAGGGCCTCGTGATACGCC TATTTTTATAGGTTAATGTCATGATAATAATGGTTTCTTAGACGTCAGGTGGCACTTTTCG GGGAAATGTGCGCGGAACCCCTATTTGTTTATTTTTCTAAATACATTCAAATATGTATCCG CTCATGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTTGC TCACCCAGAAACGCTGGTGAAAGTAAAAGATGCTGAAGATCAGTTGGGTGCACGAGTG GGTTACATCGAACTGGATCTCAACAGCGGTAAGATCCTTGAGAGTTTTCGCCCCGAAGA ACGTTTTCCAATGATGAGCACTTTTAAAGTTCTGCTATGTGGCGCGGTATTATCCCGTATT GACGCCGGGCAAGAGCAACTCGGTCGCCGCATACACTATTCTCAGAATGACTTGGTTGA GTACTCACCAGTCACAGAAAAGCATCTTACGGATGGCATGACAGTAAGAGAATTATGCA GTGCTGCCATAACCATGAGTGATAACACTGCGGCCAACTTACTTCTGACAACGATCGGA GGACCGAAGGAGCTAACCGCTTTTTTGCACAACATGGGGGATCATGTAACTCGCCTTGA TCGTTGGGAACCGGAGCTGAATGAAGCCATACCAAACGACGAGCGTGACACCACGATG CCTGTAGCAATGGCAACAACGTTGCGCAAACTATTAACTGGCGAACTACTTACTCTAGCT TCCCGGCAACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAGCGTGGGT CTCGCGGTATCATTGCAGCACTGGGGCCAGATGGTAAGCCCTCCCGTATCGTAGTTATCT ACACGACGGGGAGTCAGGCAACTATGGATGAACGAAATAGACAGATCGCTGAGATAGG TGCCTCACTGATTAAGCATTGGTAACTGTCAGACCAAGTTTACTCATATATACTTTAGATT GATTTAAAACTTCATTTTTAATTTAAAAGGATCTAGGTGAAGATCCTTTTTGATAATCTCAT GACCAAAATCCCTTAACGTGAGTTTTCGTTCCACTGAGCGTCAGACCCCGTAGAAAAGA TCAAAGGATCTTCTTGAGATCCTTTTTTTCTGCGCGTAATCTGCTGCTTGCAAACAAAAA AACCACCGCTACCAGCGGTGGTTTGTTTGCCGGATCAAGAGCTACCAACTCTTTTTCCG AAGGTAACTGGCTTCAGCAGAGCGCAGATACCAAATACTGTTCTTCTAGTGTAGCCGTA GTTAGGCCACCACTTCAAGAACTCTGTAGCACCGCCTACATACCTCGCTCTGCTAATCCT GTTACCAGTGGCTGCTGCCAGTGGCGATAAGTCGTGTCTTACCGGGTTGGACTCAAGAC GATAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGAGA AAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAG |
| | TCCTGTCGGGTTTCGCCACCTCTGACTTGAGCGTCGATTTTTGTGATGCTCGTCAGGGG GGCGGAGCCTATGGAAAAACGCCAGCAACGCGGCCTTTTTACGGTTCCTGGCCTTTTG CTGGCCTTTTGCTCACATGGCTCGACAGATCT (SEQ ID NO: 88) |
| pCMVTnT ProIns B Nluc A chain L13W | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGgAGGCaGAGGACCTGCAGGTGGGGCAGGTGGAGCTGGGCGGGg gccCaggcGCAGGCAGCCTGCAGCCCTTGGCCCTGGAGGGGTCCcTgcAGAAGCGTGGC ATTGTGGAACAATGCTGTACCAGCATCTGCTCcTGGtaccagctggagaactactgcaactagGaa ttCACGCGTGGTACCTCTAGAGTCGACCCGGGCGGCCGCTTCGAGCAGACATGATAAGA |
| | TACATTGATGAGTTTGGACAAACCACAACTAGAATGCAGTGAAAAAAATGCTTTATTTGT GAAATTTGTGATGCTATTGCTTTATTTGTAACCATTATAAGCTGCAATAAACAAGTTAACA ACAACAATTGCATTCATTTTATGTTTCAGGTTCAGGGGGAGATGTGGGAGGTTTTTTAA AGCAAGTAAAACCTCTACAAATGTGGTAAAATCGATAAGGATCCGGGCTGGCGTAATAG CGAAGAGGCCCGCACCGATCGCCCTTCCCAACAGTTGCGCAGCCTGAATGGCGAATGG ACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCC ACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGG GCCATCGCCCTGATAGACGGTTTTTCGCCCTTTGACGTTGGAGTCCACGTTCTTTAATAG TGGACTCTTGTTCCAAACTGGAACAACACTCAACCCTATCTCGGTCTATTCTTTTGATTTA TAAGGGATTTTGCCGATTTCGGCCTATTGGTTAAAAAATGAGCTGATTTAACAAAAATTT AACGCGAATTTTAACAAAATATTAACGCTTACAATTTCCTGATGCGGTATTTTCTCCTTAC GCATCTGTGCGGTATTTCACACCGCATATGGTGCACTCTCAGTACAATCTGCTCTGATGCC GCATAGTTAAGCCAGCCCCGACACCCGCCAACACCCGCTGACGCGCCCTGACGGGCTT GTCTGCTCCCGGCATCCGCTTACAGACAAGCTGTGACCGTCTCCGGGAGCTGCATGTGT CAGAGGTTTTCACCGTCATCACCGAAACGCGCGAGACGAAAGGGCCTCGTGATACGCC TATTTTTATAGGTTAATGTCATGATAATAATGGTTTCTTAGACGTCAGGTGGCACTTTTCG GGGAAATGTGCGCGGAACCCCTATTTGTTTATTTTTCTAAATACATTCAAATATGTATCCG CTCATGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTTGC TCACCCAGAAACGCTGGTGAAAGTAAAAGATGCTGAAGATCAGTTGGGTGCACGAGTG GGTTACATCGAACTGGATCTCAACAGCGGTAAGATCCTTGAGAGTTTTCGCCCCGAAGA ACGTTTTCCAATGATGAGCACTTTTAAAGTTCTGCTATGTGGCGCGGTATTATCCCGTATT GACGCCGGGCAAGAGCAACTCGGTCGCCGCATACACTATTCTCAGAATGACTTGGTTGA GTACTCACCAGTCACAGAAAAGCATCTTACGGATGGCATGACAGTAAGAGAATTATGCA GTGCTGCCATAACCATGAGTGATAACACTGCGGCCAACTTACTTCTGACAACGATCGGA GGACCGAAGGAGCTAACCGCTTTTTTGCACAACATGGGGGATCATGTAACTCGCCTTGA TCGTTGGGAACCGGAGCTGAATGAAGCCATACCAAACGACGAGCGTGACACCACGATG CCTGTAGCAATGGCAACAACGTTGCGCAAACTATTAACTGGCGAACTACTTACTCTAGCT TCCCGGCAACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAGCGTGGGT CTCGCGGTATCATTGCAGCACTGGGGCCAGATGGTAAGCCCTCCCGTATCGTAGTTATCT ACACGACGGGGAGTCAGGCAACTATGGATGAACGAAATAGACAGATCGCTGAGATAGG TGCCTCACTGATTAAGCATTGGTAACTGTCAGACCAAGTTTACTCATATATACTTTAGATT GATTTAAAACTTCATTTTTAATTTAAAAGGATCTAGGTGAAGATCCTTTTTGATAATCTCAT GACCAAAATCCCTTAACGTGAGTTTTCGTTCCACTGAGCGTCAGACCCCGTAGAAAAGA |
| | TCAAAGGATCTTCTTGAGATCCTTTTTTTCTGCGCGTAATCTGCTGCTTGCAAACAAAAA AACCACCGCTACCAGCGGTGGTTTGTTTGCCGGATCAAGAGCTACCAACTCTTTTTCCG AAGGTAACTGGCTTCAGCAGAGCGCAGATACCAAATACTGTTCTTCTAGTGTAGCCGTA GTTAGGCCACCACTTCAAGAACTCTGTAGCACCGCCTACATACCTCGCTCTGCTAATCCT GTTACCAGTGGCTGCTGCCAGTGGCGATAAGTCGTGTCTTACCGGGTTGGACTCAAGAC GATAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGAGA AAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAG TCCTGTCGGGTTTCGCCACCTCTGACTTGAGCGTCGATTTTTGTGATGCTCGTCAGGGG GGCGGAGCCTATGGAAAAACGCCAGCAACGCGGCCTTTTTACGGTTCCTGGCCTTTTG CTGGCCTTTTGCTCACATGGCTCGACAGATCT (SEQ ID NO: 89) |
| pCMVTnT Proinsulin B Nluc A chain E17A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA |
| | GATCGAAAAAATTTTTAAGGTGGTGTACCCTGTGGATGATCATCACTTTAAGGTGATCCT GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGgAGGCaGAGGACCTGCAGGTGGGGCAGGTGGAGCTGGGCGGGg gccCaggcGCAGGCAGCCTGCAGCCCTTGGCCCTGGAGGGGTCCcTgcAGAAGCGTGGC ATTGTGGAACAATGCTGTACCAGCATCTGCTCcctctaccagctggCgaactactgcaactagGaat tCACGCGTGGTACCTCTAGAGTCGACCCGGGCGGCCGCTTCGAGCAGACATGATAAGAT ACATTGATGAGTTTGGACAAACCACAACTAGAATGCAGTGAAAAAAATGCTTTATTTGT GAAATTTGTGATGCTATTGCTTTATTTGTAACCATTATAAGCTGCAATAAACAAGTTAACA ACAACAATTGCATTCATTTTATGTTTCAGGTTCAGGGGGAGATGTGGGAGGTTTTTTAA AGCAAGTAAAACCTCTACAAATGTGGTAAAATCGATAAGGATCCGGGCTGGCGTAATAG CGAAGAGGCCCGCACCGATCGCCCTTCCCAACAGTTGCGCAGCCTGAATGGCGAATGG ACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCC ACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGG GCCATCGCCCTGATAGACGGTTTTTCGCCCTTTGACGTTGGAGTCCACGTTCTTTAATAG TGGACTCTTGTTCCAAACTGGAACAACACTCAACCCTATCTCGGTCTATTCTTTTGATTTA TAAGGGATTTTGCCGATTTCGGCCTATTGGTTAAAAAATGAGCTGATTTAACAAAAATTT AACGCGAATTTTAACAAAATATTAACGCTTACAATTTCCTGATGCGGTATTTTCTCCTTAC GCATCTGTGCGGTATTTCACACCGCATATGGTGCACTCTCAGTACAATCTGCTCTGATGCC GCATAGTTAAGCCAGCCCCGACACCCGCCAACACCCGCTGACGCGCCCTGACGGGCTT GTCTGCTCCCGGCATCCGCTTACAGACAAGCTGTGACCGTCTCCGGGAGCTGCATGTGT CAGAGGTTTTCACCGTCATCACCGAAACGCGCGAGACGAAAGGGCCTCGTGATACGCC TATTTTTATAGGTTAATGTCATGATAATAATGGTTTCTTAGACGTCAGGTGGCACTTTTCG GGGAAATGTGCGCGGAACCCCTATTTGTTTATTTTTCTAAATACATTCAAATATGTATCCG CTCATGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTTGC TCACCCAGAAACGCTGGTGAAAGTAAAAGATGCTGAAGATCAGTTGGGTGCACGAGTG GGTTACATCGAACTGGATCTCAACAGCGGTAAGATCCTTGAGAGTTTTCGCCCCGAAGA ACGTTTTCCAATGATGAGCACTTTTAAAGTTCTGCTATGTGGCGCGGTATTATCCCGTATT GACGCCGGGCAAGAGCAACTCGGTCGCCGCATACACTATTCTCAGAATGACTTGGTTGA GTACTCACCAGTCACAGAAAAGCATCTTACGGATGGCATGACAGTAAGAGAATTATGCA GTGCTGCCATAACCATGAGTGATAACACTGCGGCCAACTTACTTCTGACAACGATCGGA GGACCGAAGGAGCTAACCGCTTTTTTGCACAACATGGGGGATCATGTAACTCGCCTTGA |
| | TCGTTGGGAACCGGAGCTGAATGAAGCCATACCAAACGACGAGCGTGACACCACGATG CCTGTAGCAATGGCAACAACGTTGCGCAAACTATTAACTGGCGAACTACTTACTCTAGCT TCCCGGCAACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAGCGTGGGT CTCGCGGTATCATTGCAGCACTGGGGCCAGATGGTAAGCCCTCCCGTATCGTAGTTATCT ACACGACGGGGAGTCAGGCAACTATGGATGAACGAAATAGACAGATCGCTGAGATAGG TGCCTCACTGATTAAGCATTGGTAACTGTCAGACCAAGTTTACTCATATATACTTTAGATT GATTTAAAACTTCATTTTTAATTTAAAAGGATCTAGGTGAAGATCCTTTTTGATAATCTCAT GACCAAAATCCCTTAACGTGAGTTTTCGTTCCACTGAGCGTCAGACCCCGTAGAAAAGA TCAAAGGATCTTCTTGAGATCCTTTTTTTCTGCGCGTAATCTGCTGCTTGCAAACAAAAA AACCACCGCTACCAGCGGTGGTTTGTTTGCCGGATCAAGAGCTACCAACTCTTTTTCCG AAGGTAACTGGCTTCAGCAGAGCGCAGATACCAAATACTGTTCTTCTAGTGTAGCCGTA GTTAGGCCACCACTTCAAGAACTCTGTAGCACCGCCTACATACCTCGCTCTGCTAATCCT GTTACCAGTGGCTGCTGCCAGTGGCGATAAGTCGTGTCTTACCGGGTTGGACTCAAGAC GATAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGAGA AAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAG TCCTGTCGGGTTTCGCCACCTCTGACTTGAGCGTCGATTTTTGTGATGCTCGTCAGGGG GGCGGAGCCTATGGAAAAACGCCAGCAACGCGGCCTTTTTACGGTTCCTGGCCTTTTG CTGGCCTTTTGCTCACATGGCTCGACAGATCT (SEQ ID NO: 90) |
| pCMVTnT Proinsulin B Nluc A chain Y14A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT |
| | GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGgAGGCaGAGGACCTGCAGGTGGGGCAGGTGGAGCTGGGCGGGg gccCaggcGCAGGCAGCCTGCAGCCCTTGGCCCTGGAGGGGTCCcTgcAGAAGCGTGGC ATTGTGGAACAATGCTGTACCAGCATCTGCTCcctcGCccagctggagaactactgcaactagGaa ttCACGCGTGGTACCTCTAGAGTCGACCCGGGCGGCCGCTTCGAGCAGACATGATAAGA TACATTGATGAGTTTGGACAAACCACAACTAGAATGCAGTGAAAAAAATGCTTTATTTGT GAAATTTGTGATGCTATTGCTTTATTTGTAACCATTATAAGCTGCAATAAACAAGTTAACA ACAACAATTGCATTCATTTTATGTTTCAGGTTCAGGGGGAGATGTGGGAGGTTTTTTAA AGCAAGTAAAACCTCTACAAATGTGGTAAAATCGATAAGGATCCGGGCTGGCGTAATAG CGAAGAGGCCCGCACCGATCGCCCTTCCCAACAGTTGCGCAGCCTGAATGGCGAATGG ACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCC ACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGG GCCATCGCCCTGATAGACGGTTTTTCGCCCTTTGACGTTGGAGTCCACGTTCTTTAATAG TGGACTCTTGTTCCAAACTGGAACAACACTCAACCCTATCTCGGTCTATTCTTTTGATTTA TAAGGGATTTTGCCGATTTCGGCCTATTGGTTAAAAAATGAGCTGATTTAACAAAAATTT AACGCGAATTTTAACAAAATATTAACGCTTACAATTTCCTGATGCGGTATTTTCTCCTTAC GCATCTGTGCGGTATTTCACACCGCATATGGTGCACTCTCAGTACAATCTGCTCTGATGCC GCATAGTTAAGCCAGCCCCGACACCCGCCAACACCCGCTGACGCGCCCTGACGGGCTT GTCTGCTCCCGGCATCCGCTTACAGACAAGCTGTGACCGTCTCCGGGAGCTGCATGTGT CAGAGGTTTTCACCGTCATCACCGAAACGCGCGAGACGAAAGGGCCTCGTGATACGCC TATTTTTATAGGTTAATGTCATGATAATAATGGTTTCTTAGACGTCAGGTGGCACTTTTCG GGGAAATGTGCGCGGAACCCCTATTTGTTTATTTTTCTAAATACATTCAAATATGTATCCG |
| | CTCATGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTTGC TCACCCAGAAACGCTGGTGAAAGTAAAAGATGCTGAAGATCAGTTGGGTGCACGAGTG GGTTACATCGAACTGGATCTCAACAGCGGTAAGATCCTTGAGAGTTTTCGCCCCGAAGA ACGTTTTCCAATGATGAGCACTTTTAAAGTTCTGCTATGTGGCGCGGTATTATCCCGTATT GACGCCGGGCAAGAGCAACTCGGTCGCCGCATACACTATTCTCAGAATGACTTGGTTGA GTACTCACCAGTCACAGAAAAGCATCTTACGGATGGCATGACAGTAAGAGAATTATGCA GTGCTGCCATAACCATGAGTGATAACACTGCGGCCAACTTACTTCTGACAACGATCGGA GGACCGAAGGAGCTAACCGCTTTTTTGCACAACATGGGGGATCATGTAACTCGCCTTGA TCGTTGGGAACCGGAGCTGAATGAAGCCATACCAAACGACGAGCGTGACACCACGATG CCTGTAGCAATGGCAACAACGTTGCGCAAACTATTAACTGGCGAACTACTTACTCTAGCT TCCCGGCAACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAGCGTGGGT CTCGCGGTATCATTGCAGCACTGGGGCCAGATGGTAAGCCCTCCCGTATCGTAGTTATCT ACACGACGGGGAGTCAGGCAACTATGGATGAACGAAATAGACAGATCGCTGAGATAGG TGCCTCACTGATTAAGCATTGGTAACTGTCAGACCAAGTTTACTCATATATACTTTAGATT GATTTAAAACTTCATTTTTAATTTAAAAGGATCTAGGTGAAGATCCTTTTTGATAATCTCAT GACCAAAATCCCTTAACGTGAGTTTTCGTTCCACTGAGCGTCAGACCCCGTAGAAAAGA TCAAAGGATCTTCTTGAGATCCTTTTTTTCTGCGCGTAATCTGCTGCTTGCAAACAAAAA AACCACCGCTACCAGCGGTGGTTTGTTTGCCGGATCAAGAGCTACCAACTCTTTTTCCG AAGGTAACTGGCTTCAGCAGAGCGCAGATACCAAATACTGTTCTTCTAGTGTAGCCGTA GTTAGGCCACCACTTCAAGAACTCTGTAGCACCGCCTACATACCTCGCTCTGCTAATCCT GTTACCAGTGGCTGCTGCCAGTGGCGATAAGTCGTGTCTTACCGGGTTGGACTCAAGAC GATAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGAGA AAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAG TCCTGTCGGGTTTCGCCACCTCTGACTTGAGCGTCGATTTTTGTGATGCTCGTCAGGGG GGCGGAGCCTATGGAAAAACGCCAGCAACGCGGCCTTTTTACGGTTCCTGGCCTTTTG CTGGCCTTTTGCTCACATGGCTCGACAGATCT (SEQ ID NO: 91) |
| pCMVTnT Proinsulin B Nluc B chain F1A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG |
| | GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCGCTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGgAGGCaGAGGACCTGCAGGTGGGGCAGGTGGAGCTGGGCGGGg gccCaggcGCAGGCAGCCTGCAGCCCTTGGCCCTGGAGGGGTCCcTgcAGAAGCGTGGC ATTGTGGAACAATGCTGTACCAGCATCTGCTCcctctaccagctggagaactactgcaactagGaat tCACGCGTGGTACCTCTAGAGTCGACCCGGGCGGCCGCTTCGAGCAGACATGATAAGAT ACATTGATGAGTTTGGACAAACCACAACTAGAATGCAGTGAAAAAAATGCTTTATTTGT GAAATTTGTGATGCTATTGCTTTATTTGTAACCATTATAAGCTGCAATAAACAAGTTAACA ACAACAATTGCATTCATTTTATGTTTCAGGTTCAGGGGGAGATGTGGGAGGTTTTTTAA AGCAAGTAAAACCTCTACAAATGTGGTAAAATCGATAAGGATCCGGGCTGGCGTAATAG CGAAGAGGCCCGCACCGATCGCCCTTCCCAACAGTTGCGCAGCCTGAATGGCGAATGG ACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCC ACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGG GCCATCGCCCTGATAGACGGTTTTTCGCCCTTTGACGTTGGAGTCCACGTTCTTTAATAG |
| | TGGACTCTTGTTCCAAACTGGAACAACACTCAACCCTATCTCGGTCTATTCTTTTGATTTA TAAGGGATTTTGCCGATTTCGGCCTATTGGTTAAAAAATGAGCTGATTTAACAAAAATTT AACGCGAATTTTAACAAAATATTAACGCTTACAATTTCCTGATGCGGTATTTTCTCCTTAC GCATCTGTGCGGTATTTCACACCGCATATGGTGCACTCTCAGTACAATCTGCTCTGATGCC GCATAGTTAAGCCAGCCCCGACACCCGCCAACACCCGCTGACGCGCCCTGACGGGCTT GTCTGCTCCCGGCATCCGCTTACAGACAAGCTGTGACCGTCTCCGGGAGCTGCATGTGT CAGAGGTTTTCACCGTCATCACCGAAACGCGCGAGACGAAAGGGCCTCGTGATACGCC TATTTTTATAGGTTAATGTCATGATAATAATGGTTTCTTAGACGTCAGGTGGCACTTTTCG GGGAAATGTGCGCGGAACCCCTATTTGTTTATTTTTCTAAATACATTCAAATATGTATCCG CTCATGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTTGC TCACCCAGAAACGCTGGTGAAAGTAAAAGATGCTGAAGATCAGTTGGGTGCACGAGTG GGTTACATCGAACTGGATCTCAACAGCGGTAAGATCCTTGAGAGTTTTCGCCCCGAAGA ACGTTTTCCAATGATGAGCACTTTTAAAGTTCTGCTATGTGGCGCGGTATTATCCCGTATT GACGCCGGGCAAGAGCAACTCGGTCGCCGCATACACTATTCTCAGAATGACTTGGTTGA GTACTCACCAGTCACAGAAAAGCATCTTACGGATGGCATGACAGTAAGAGAATTATGCA GTGCTGCCATAACCATGAGTGATAACACTGCGGCCAACTTACTTCTGACAACGATCGGA GGACCGAAGGAGCTAACCGCTTTTTTGCACAACATGGGGGATCATGTAACTCGCCTTGA TCGTTGGGAACCGGAGCTGAATGAAGCCATACCAAACGACGAGCGTGACACCACGATG CCTGTAGCAATGGCAACAACGTTGCGCAAACTATTAACTGGCGAACTACTTACTCTAGCT TCCCGGCAACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAGCGTGGGT CTCGCGGTATCATTGCAGCACTGGGGCCAGATGGTAAGCCCTCCCGTATCGTAGTTATCT ACACGACGGGGAGTCAGGCAACTATGGATGAACGAAATAGACAGATCGCTGAGATAGG TGCCTCACTGATTAAGCATTGGTAACTGTCAGACCAAGTTTACTCATATATACTTTAGATT GATTTAAAACTTCATTTTTAATTTAAAAGGATCTAGGTGAAGATCCTTTTTGATAATCTCAT GACCAAAATCCCTTAACGTGAGTTTTCGTTCCACTGAGCGTCAGACCCCGTAGAAAAGA TCAAAGGATCTTCTTGAGATCCTTTTTTTCTGCGCGTAATCTGCTGCTTGCAAACAAAAA AACCACCGCTACCAGCGGTGGTTTGTTTGCCGGATCAAGAGCTACCAACTCTTTTTCCG AAGGTAACTGGCTTCAGCAGAGCGCAGATACCAAATACTGTTCTTCTAGTGTAGCCGTA GTTAGGCCACCACTTCAAGAACTCTGTAGCACCGCCTACATACCTCGCTCTGCTAATCCT GTTACCAGTGGCTGCTGCCAGTGGCGATAAGTCGTGTCTTACCGGGTTGGACTCAAGAC GATAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGAGA AAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAG TCCTGTCGGGTTTCGCCACCTCTGACTTGAGCGTCGATTTTTGTGATGCTCGTCAGGGG |
| | GGCGGAGCCTATGGAAAAACGCCAGCAACGCGGCCTTTTTACGGTTCCTGGCCTTTTG CTGGCCTTTTGCTCACATGGCTCGACAGATCT (SEQ ID NO: 92) |
| pCMVTnT Proinsulin B Nluc B chain L17A | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACGCAGTGTGCGGGGAACGAGGCTTCTTCT ACACACCCAAGACCATGGTCTTCACACTCGAAGATTTCGTTGGGGACTGGCGACAGAC AGCCGGCTACAACCTGGACCAAGTCCTTGAACAGGGAGGTGTGTCCAGTTTGTTTCAG AATCTCGGGGTGTCCGTAACTCCGATCCAAAGGATTGTCCTGAGCGGTGAAAATGGGCT GAAGATCGACATCCATGTCATCATCCCGTATGAAGGTCTGAGCGGCGACCAAATGGGCC AGATCGAAAAAATTTTTAAGGTGGTGTACCCTGTGGATGATCATCACTTTAAGGTGATCC TGCACTATGGCACACTGGTAATCGACGGGGTTACGCCGAACATGATCGACTATTTCGGA CGGCCGTATGAAGGCATCGCCGTGTTCGACGGCAAAAAGATCACTGTAACAGGGACCC TGTGGAACGGCAACAAAATTATCGACGAGCGCCTGATCAACCCCGACGGCTCCCTGCTG TTCCGAGTAACCATCAACGGAGTGACCGGCTGGCGGCTGTGCGAACGCATTCTGGCGgc tgctgctgcGCGCCGGgAGGCaGAGGACCTGCAGGTGGGGCAGGTGGAGCTGGGCGGG ggccCaggcGCAGGCAGCCTGCAGCCCTTGGCCCTGGAGGGGTCCcTgcAGAAGCGTGG CATTGTGGAACAATGCTGTACCAGCATCTGCTCcctctaccagctggagaactactgcaactagGaa ttCACGCGTGGTACCTCTAGAGTCGACCCGGGCGGCCGCTTCGAGCAGACATGATAAGA TACATTGATGAGTTTGGACAAACCACAACTAGAATGCAGTGAAAAAAATGCTTTATTTGT |
| | GAAATTTGTGATGCTATTGCTTTATTTGTAACCATTATAAGCTGCAATAAACAAGTTAACA ACAACAATTGCATTCATTTTATGTTTCAGGTTCAGGGGGAGATGTGGGAGGTTTTTTAA AGCAAGTAAAACCTCTACAAATGTGGTAAAATCGATAAGGATCCGGGCTGGCGTAATAG CGAAGAGGCCCGCACCGATCGCCCTTCCCAACAGTTGCGCAGCCTGAATGGCGAATGG ACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCC ACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGG GCCATCGCCCTGATAGACGGTTTTTCGCCCTTTGACGTTGGAGTCCACGTTCTTTAATAG TGGACTCTTGTTCCAAACTGGAACAACACTCAACCCTATCTCGGTCTATTCTTTTGATTTA TAAGGGATTTTGCCGATTTCGGCCTATTGGTTAAAAAATGAGCTGATTTAACAAAAATTT AACGCGAATTTTAACAAAATATTAACGCTTACAATTTCCTGATGCGGTATTTTCTCCTTAC GCATCTGTGCGGTATTTCACACCGCATATGGTGCACTCTCAGTACAATCTGCTCTGATGCC GCATAGTTAAGCCAGCCCCGACACCCGCCAACACCCGCTGACGCGCCCTGACGGGCTT GTCTGCTCCCGGCATCCGCTTACAGACAAGCTGTGACCGTCTCCGGGAGCTGCATGTGT CAGAGGTTTTCACCGTCATCACCGAAACGCGCGAGACGAAAGGGCCTCGTGATACGCC TATTTTTATAGGTTAATGTCATGATAATAATGGTTTCTTAGACGTCAGGTGGCACTTTTCG GGGAAATGTGCGCGGAACCCCTATTTGTTTATTTTTCTAAATACATTCAAATATGTATCCG CTCATGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTTGC TCACCCAGAAACGCTGGTGAAAGTAAAAGATGCTGAAGATCAGTTGGGTGCACGAGTG GGTTACATCGAACTGGATCTCAACAGCGGTAAGATCCTTGAGAGTTTTCGCCCCGAAGA ACGTTTTCCAATGATGAGCACTTTTAAAGTTCTGCTATGTGGCGCGGTATTATCCCGTATT GACGCCGGGCAAGAGCAACTCGGTCGCCGCATACACTATTCTCAGAATGACTTGGTTGA GTACTCACCAGTCACAGAAAAGCATCTTACGGATGGCATGACAGTAAGAGAATTATGCA GTGCTGCCATAACCATGAGTGATAACACTGCGGCCAACTTACTTCTGACAACGATCGGA GGACCGAAGGAGCTAACCGCTTTTTTGCACAACATGGGGGATCATGTAACTCGCCTTGA TCGTTGGGAACCGGAGCTGAATGAAGCCATACCAAACGACGAGCGTGACACCACGATG CCTGTAGCAATGGCAACAACGTTGCGCAAACTATTAACTGGCGAACTACTTACTCTAGCT TCCCGGCAACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAGCGTGGGT CTCGCGGTATCATTGCAGCACTGGGGCCAGATGGTAAGCCCTCCCGTATCGTAGTTATCT ACACGACGGGGAGTCAGGCAACTATGGATGAACGAAATAGACAGATCGCTGAGATAGG TGCCTCACTGATTAAGCATTGGTAACTGTCAGACCAAGTTTACTCATATATACTTTAGATT GATTTAAAACTTCATTTTTAATTTAAAAGGATCTAGGTGAAGATCCTTTTTGATAATCTCAT GACCAAAATCCCTTAACGTGAGTTTTCGTTCCACTGAGCGTCAGACCCCGTAGAAAAGA TCAAAGGATCTTCTTGAGATCCTTTTTTTCTGCGCGTAATCTGCTGCTTGCAAACAAAAA |
| | AACCACCGCTACCAGCGGTGGTTTGTTTGCCGGATCAAGAGCTACCAACTCTTTTTCCG AAGGTAACTGGCTTCAGCAGAGCGCAGATACCAAATACTGTTCTTCTAGTGTAGCCGTA GTTAGGCCACCACTTCAAGAACTCTGTAGCACCGCCTACATACCTCGCTCTGCTAATCCT GTTACCAGTGGCTGCTGCCAGTGGCGATAAGTCGTGTCTTACCGGGTTGGACTCAAGAC GATAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGAGA AAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAG TCCTGTCGGGTTTCGCCACCTCTGACTTGAGCGTCGATTTTTGTGATGCTCGTCAGGGG GGCGGAGCCTATGGAAAAACGCCAGCAACGCGGCCTTTTTACGGTTCCTGGCCTTTTG CTGGCCTTTTGCTCACATGGCTCGACAGATCT (SEQ ID NO: 93) |
| pCMVTnT Proinsulin B Nluc no cpeptide PG duplication | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT |
| | GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGgAGGCaGAGGACCTGCAGGTGGGGCAGGTGGAGCTGGGCGGGg gccCaggcGCAGGCAGCCTGCAGCCCTTGGCCCTGGAGGGGTCCcTgcAGAAGCGTGGC ATTGTGGAACAATGCTGTACCAGCATCTGCTCcctctaccagctggagaactactgcaactagGaat tCACGCGTGGTACCTCTAGAGTCGACCCGGGCGGCCGCTTCGAGCAGACATGATAAGAT ACATTGATGAGTTTGGACAAACCACAACTAGAATGCAGTGAAAAAAATGCTTTATTTGT GAAATTTGTGATGCTATTGCTTTATTTGTAACCATTATAAGCTGCAATAAACAAGTTAACA ACAACAATTGCATTCATTTTATGTTTCAGGTTCAGGGGGAGATGTGGGAGGTTTTTTAA AGCAAGTAAAACCTCTACAAATGTGGTAAAATCGATAAGGATCCGGGCTGGCGTAATAG CGAAGAGGCCCGCACCGATCGCCCTTCCCAACAGTTGCGCAGCCTGAATGGCGAATGG ACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCC ACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGG GCCATCGCCCTGATAGACGGTTTTTCGCCCTTTGACGTTGGAGTCCACGTTCTTTAATAG TGGACTCTTGTTCCAAACTGGAACAACACTCAACCCTATCTCGGTCTATTCTTTTGATTTA TAAGGGATTTTGCCGATTTCGGCCTATTGGTTAAAAAATGAGCTGATTTAACAAAAATTT AACGCGAATTTTAACAAAATATTAACGCTTACAATTTCCTGATGCGGTATTTTCTCCTTAC GCATCTGTGCGGTATTTCACACCGCATATGGTGCACTCTCAGTACAATCTGCTCTGATGCC GCATAGTTAAGCCAGCCCCGACACCCGCCAACACCCGCTGACGCGCCCTGACGGGCTT GTCTGCTCCCGGCATCCGCTTACAGACAAGCTGTGACCGTCTCCGGGAGCTGCATGTGT CAGAGGTTTTCACCGTCATCACCGAAACGCGCGAGACGAAAGGGCCTCGTGATACGCC TATTTTTATAGGTTAATGTCATGATAATAATGGTTTCTTAGACGTCAGGTGGCACTTTTCG GGGAAATGTGCGCGGAACCCCTATTTGTTTATTTTTCTAAATACATTCAAATATGTATCCG CTCATGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTTGC TCACCCAGAAACGCTGGTGAAAGTAAAAGATGCTGAAGATCAGTTGGGTGCACGAGTG GGTTACATCGAACTGGATCTCAACAGCGGTAAGATCCTTGAGAGTTTTCGCCCCGAAGA ACGTTTTCCAATGATGAGCACTTTTAAAGTTCTGCTATGTGGCGCGGTATTATCCCGTATT GACGCCGGGCAAGAGCAACTCGGTCGCCGCATACACTATTCTCAGAATGACTTGGTTGA GTACTCACCAGTCACAGAAAAGCATCTTACGGATGGCATGACAGTAAGAGAATTATGCA GTGCTGCCATAACCATGAGTGATAACACTGCGGCCAACTTACTTCTGACAACGATCGGA GGACCGAAGGAGCTAACCGCTTTTTTGCACAACATGGGGGATCATGTAACTCGCCTTGA TCGTTGGGAACCGGAGCTGAATGAAGCCATACCAAACGACGAGCGTGACACCACGATG |
| | CCTGTAGCAATGGCAACAACGTTGCGCAAACTATTAACTGGCGAACTACTTACTCTAGCT TCCCGGCAACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAGCGTGGGT CTCGCGGTATCATTGCAGCACTGGGGCCAGATGGTAAGCCCTCCCGTATCGTAGTTATCT ACACGACGGGGAGTCAGGCAACTATGGATGAACGAAATAGACAGATCGCTGAGATAGG TGCCTCACTGATTAAGCATTGGTAACTGTCAGACCAAGTTTACTCATATATACTTTAGATT GATTTAAAACTTCATTTTTAATTTAAAAGGATCTAGGTGAAGATCCTTTTTGATAATCTCAT GACCAAAATCCCTTAACGTGAGTTTTCGTTCCACTGAGCGTCAGACCCCGTAGAAAAGA TCAAAGGATCTTCTTGAGATCCTTTTTTTCTGCGCGTAATCTGCTGCTTGCAAACAAAAA AACCACCGCTACCAGCGGTGGTTTGTTTGCCGGATCAAGAGCTACCAACTCTTTTTCCG AAGGTAACTGGCTTCAGCAGAGCGCAGATACCAAATACTGTTCTTCTAGTGTAGCCGTA GTTAGGCCACCACTTCAAGAACTCTGTAGCACCGCCTACATACCTCGCTCTGCTAATCCT GTTACCAGTGGCTGCTGCCAGTGGCGATAAGTCGTGTCTTACCGGGTTGGACTCAAGAC GATAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGAGA AAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAG TCCTGTCGGGTTTCGCCACCTCTGACTTGAGCGTCGATTTTTGTGATGCTCGTCAGGGG GGCGGAGCCTATGGAAAAACGCCAGCAACGCGGCCTTTTTACGGTTCCTGGCCTTTTG CTGGCCTTTTGCTCACATGGCTCGACAGATCT (SEQ ID NO: 94) |
| pCMVTnT_hP reProlns B chain Nluc cpeptide | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG |
| | GCTAGAGTACTTAATACGACTCACTATAGGCTAGCATTTAGGTGACACTATAGAATACAAG CTACTTGTTCTTTTTGCACTCGAGaattCATGGCCCTGTGGATGCGCCTCCTGCCCCTGCT GGCGCTGCTGGCCCTCTGGGGACCTGACCCAGCCGCAGCCTTTGTGAACCAACACCTG TGCGGCTCACACCTGGTGGAAGCTCTCTACCTAGTGTGCGGGGAACGAGGCTTCTTCTA CACACCCAAGACCATGGTCTTCACACTCGAAGATTTCGTTGGGGACTGGCGACAGACA GCCGGCTACAACCTGGACCAAGTCCTTGAACAGGGAGGTGTGTCCAGTTTGTTTCAGA ATCTCGGGGTGTCCGTAACTCCGATCCAAAGGATTGTCCTGAGCGGTGAAAATGGGCTG AAGATCGACATCCATGTCATCATCCCGTATGAAGGTCTGAGCGGCGACCAAATGGGCCA GATCGAAAAAATTTTTAAGGTGGTGTACCCTGTGGATGATCATCACTTTAAGGTGATCCT GCACTATGGCACACTGGTAATCGACGGGGTTACGCCGAACATGATCGACTATTTCGGAC GGCCGTATGAAGGCATCGCCGTGTTCGACGGCAAAAAGATCACTGTAACAGGGACCCT GTGGAACGGCAACAAAATTATCGACGAGCGCCTGATCAACCCCGACGGCTCCCTGCTGT TCCGAGTAACCATCAACGGAGTGACCGGCTGGCGGCTGTGCGAACGCATTCTGGCGgct gctgctgcGCGCCGGgAGGCaGAGGACCTGCAGGTGGGGCAGGTGGAGCTGGGCGGGg gccCaggccCTGGTGCAGGCAGCCTGCAGCCCTTGGCCCTGGAGGGGTCCcTgcAGAAGC GTGGCATTGTGGAACAATGCTGTACCAGCATCTGCTCcctctaccagctggagaactactgcaact agGaattCACGCGTGGTACCTCTAGAGTCGACCCGGGCGGCCGCTTCGAGCAGACATGAT AAGATACATTGATGAGTTTGGACAAACCACAACTAGAATGCAGTGAAAAAAATGCTTTA TTTGTGAAATTTGTGATGCTATTGCTTTATTTGTAACCATTATAAGCTGCAATAAACAAGTT AACAACAACAATTGCATTCATTTTATGTTTCAGGTTCAGGGGGAGATGTGGGAGGTTTT TTAAAGCAAGTAAAACCTCTACAAATGTGGTAAAATCGATAAGGATCCGGGCTGGCGTA ATAGCGAAGAGGCCCGCACCGATCGCCCTTCCCAACAGTTGCGCAGCCTGAATGGCGA ATGGACGCGCCCTGTAGCGGCGCATTAAGCGCGGCGGGTGTGGTGGTTACGCGCAGCG TGACCGCTACACTTGCCAGCGCCCTAGCGCCCGCTCCTTTCGCTTTCTTCCCTTCCTTTCT CGCCACGTTCGCCGGCTTTCCCCGTCAAGCTCTAAATCGGGGGCTCCCTTTAGGGTTCC GATTTAGTGCTTTACGGCACCTCGACCCCAAAAAACTTGATTAGGGTGATGGTTCACGTA GTGGGCCATCGCCCTGATAGACGGTTTTTCGCCCTTTGACGTTGGAGTCCACGTTCTTTA ATAGTGGACTCTTGTTCCAAACTGGAACAACACTCAACCCTATCTCGGTCTATTCTTTTGA TTTATAAGGGATTTTGCCGATTTCGGCCTATTGGTTAAAAAATGAGCTGATTTAACAAAA ATTTAACGCGAATTTTAACAAAATATTAACGCTTACAATTTCCTGATGCGGTATTTTCTCCT TACGCATCTGTGCGGTATTTCACACCGCATATGGTGCACTCTCAGTACAATCTGCTCTGAT GCCGCATAGTTAAGCCAGCCCCGACACCCGCCAACACCCGCTGACGCGCCCTGACGGG CTTGTCTGCTCCCGGCATCCGCTTACAGACAAGCTGTGACCGTCTCCGGGAGCTGCATG TGTCAGAGGTTTTCACCGTCATCACCGAAACGCGCGAGACGAAAGGGCCTCGTGATAC GCCTATTTTTATAGGTTAATGTCATGATAATAATGGTTTCTTAGACGTCAGGTGGCACTTT TCGGGGAAATGTGCGCGGAACCCCTATTTGTTTATTTTTCTAAATACATTCAAATATGTAT CCGCTCATGAGACAATAACCCTGATAAATGCTTCAATAATATTGAAAAAGGAAGAGTATG |
| | AGTATTCAACATTTCCGTGTCGCCCTTATTCCCTTTTTTGCGGCATTTTGCCTTCCTGTTTT TGCTCACCCAGAAACGCTGGTGAAAGTAAAAGATGCTGAAGATCAGTTGGGTGCACGA GTGGGTTACATCGAACTGGATCTCAACAGCGGTAAGATCCTTGAGAGTTTTCGCCCCGA AGAACGTTTTCCAATGATGAGCACTTTTAAAGTTCTGCTATGTGGCGCGGTATTATCCCG TATTGACGCCGGGCAAGAGCAACTCGGTCGCCGCATACACTATTCTCAGAATGACTTGG TTGAGTACTCACCAGTCACAGAAAAGCATCTTACGGATGGCATGACAGTAAGAGAATTA TGCAGTGCTGCCATAACCATGAGTGATAACACTGCGGCCAACTTACTTCTGACAACGATC GGAGGACCGAAGGAGCTAACCGCTTTTTTGCACAACATGGGGGATCATGTAACTCGCC TTGATCGTTGGGAACCGGAGCTGAATGAAGCCATACCAAACGACGAGCGTGACACCAC GATGCCTGTAGCAATGGCAACAACGTTGCGCAAACTATTAACTGGCGAACTACTTACTCT AGCTTCCCGGCAACAATTAATAGACTGGATGGAGGCGGATAAAGTTGCAGGACCACTTC TGCGCTCGGCCCTTCCGGCTGGCTGGTTTATTGCTGATAAATCTGGAGCCGGTGAGCGT GGGTCTCGCGGTATCATTGCAGCACTGGGGCCAGATGGTAAGCCCTCCCGTATCGTAGT TATCTACACGACGGGGAGTCAGGCAACTATGGATGAACGAAATAGACAGATCGCTGAG ATAGGTGCCTCACTGATTAAGCATTGGTAACTGTCAGACCAAGTTTACTCATATATACTTT AGATTGATTTAAAACTTCATTTTTAATTTAAAAGGATCTAGGTGAAGATCCTTTTTGATAA TCTCATGACCAAAATCCCTTAACGTGAGTTTTCGTTCCACTGAGCGTCAGACCCCGTAGA AAAGATCAAAGGATCTTCTTGAGATCCTTTTTTTCTGCGCGTAATCTGCTGCTTGCAAAC AAAAAAACCACCGCTACCAGCGGTGGTTTGTTTGCCGGATCAAGAGCTACCAACTCTTT TTCCGAAGGTAACTGGCTTCAGCAGAGCGCAGATACCAAATACTGTTCTTCTAGTGTAGC CGTAGTTAGGCCACCACTTCAAGAACTCTGTAGCACCGCCTACATACCTCGCTCTGCTAA TCCTGTTACCAGTGGCTGCTGCCAGTGGCGATAAGTCGTGTCTTACCGGGTTGGACTCA AGACGATAGTTACCGGATAAGGCGCAGCGGTCGGGCTGAACGGGGGGTTCGTGCACA CAGCCCAGCTTGGAGCGAACGACCTACACCGAACTGAGATACCTACAGCGTGAGCTATG AGAAAGCGCCACGCTTCCCGAAGGGAGAAAGGCGGACAGGTATCCGGTAAGCGGCAG GGTCGGAACAGGAGAGCGCACGAGGGAGCTTCCAGGGGGAAACGCCTGGTATCTTTA TAGTCCTGTCGGGTTTCGCCACCTCTGACTTGAGCGTCGATTTTTGTGATGCTCGTCAGG GGGGCGGAGCCTATGGAAAAACGCCAGCAACGCGGCCTTTTTACGGTTCCTGGCCTTT TGCTGGCCTTTTGCTCACATGGCTCGACAGATCT (SEQ ID NO: 95) |
| PreProins B chain Nluc c peptide PG duplication | ATGGCCCTGTGGATGCGCCTCCTGCCCCTGCTGGCGCTGCTGGCCCTCTGGGGACCTGA CCCAGCCGCAGCCTTTGTGAACCAACACCTGTGCGGCTCACACCTGGTGGAAGCTCTCT ACCTAGTGTGCGGGGAACGAGGCTTCTTCTACACACCCAAGACCATGGTCTTCACACTC GAAGATTTCGTTGGGGACTGGCGACAGACAGCCGGCTACAACCTGGACCAAGTCCTTG AACAGGGAGGTGTGTCCAGTTTGTTTCAGAATCTCGGGGTGTCCGTAACTCCGATCCAA AGGATTGTCCTGAGCGGTGAAAATGGGCTGAAGATCGACATCCATGTCATCATCCCGTAT GAAGGTCTGAGCGGCGACCAAATGGGCCAGATCGAAAAAATTTTTAAGGTGGTGTACC CTGTGGATGATCATCACTTTAAGGTGATCCTGCACTATGGCACACTGGTAATCGACGGGG |
| | TTACGCCGAACATGATCGACTATTTCGGACGGCCGTATGAAGGCATCGCCGTGTTCGAC GGCAAAAAGATCACTGTAACAGGGACCCTGTGGAACGGCAACAAAATTATCGACGAGC GCCTGATCAACCCCGACGGCTCCCTGCTGTTCCGAGTAACCATCAACGGAGTGACCGGC TGGCGGCTGTGCGAACGCATTCTGGCGgctgctgctgcGCGCCGGgAGGCaGAGGACCTG CAGGTGGGGCAGGTGGAGCTGGGCGGGggccCaggccCTGGTGCAGGCAGCCTGCAGC CCTTGGCCCTGGAGGGGTCCcTgcAGAAGCGTGGCATTGTGGAACAATGCTGTACCAGC ATCTGCTCcctctaccagctggagaactactgcaactag (SEQ ID NO: 96) |
| pCMVTnT_sNI uc_hPPI_c-peptide | TCAATATTGGCCATTAGCCATATTATTCATTGGTTATATAGCATAAATCAATATTGGCTATTG GCCATTGCATACGTTGTATCTATATCATAATATGTACATTTATATTGGCTCATGTCCAATATGA CCGCCATGTTGGCATTGATTATTGACTAGTTATTAATAGTAATCAATTACGGGGTCATTAGT TCATAGCCCATATATGGAGTTCCGCGTTACATAACTTACGGTAAATGGCCCGCCTGGCTG ACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGG CAGTACATCAAGTGTATCATATGCCAAGTCCGCCCCCTATTGACGTCAATGACGGTAAATG GCCCGCCTGGCATTATGCCCAGTACATGACCTTACGGGACTTTCCTACTTGGCAGTACAT CTACGTATTAGTCATCGCTATTACCATGGTGATGCGGTTTTGGCAGTACACCAATGGGCG TGGATAGCGGTTTGACTCACGGGGATTTCCAAGTCTCCACCCCATTGACGTCAATGGGA GTTTGTTTTGGCACCAAAATCAACGGGACTTTCCAAAATGTCGTAATAACCCCGCCCCGT TGACGCAAATGGGCGGTAGGCGTGTACGGTGGGAGGTCTATATAAGCAGAGCTCGTTT AGTGAACCGTCAGATCACTAGAAGCTTTATTGCGGTAGTTTATCACAGTTAAATTGCTAA CGCAGTCAGTGCTTCTGACACAACAGTCTCGAACTTAAGCTGCAGAAGTTGGTCGTGA GGCACTGGGCAGGTAAGTATCAAGGTTACAAGACAGGTTTAAGGAGACCAATAGAAAC TGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAG GCTAGAGTACTTAATACGACTCACTATAGGCTAGCATTTAGGTGACACTATAGAATACAAG CTACTTGTTCTTTTTGCACtcgaGgaattcGCCGCCACCATGAACTCCTTCTCCACAAGCGCC TTCGGTCCAGTTGCCTTCTCCCTGGGCCTGCTCCTGGTGTTGCCTGCTGCCTTCCCTGCC CCAGTCTTCACACTCGAAGATTTCGTTGGGGACTGGCGACAGACAGCCGGCTACAACC TGGACCAAGTCCTTGAACAGGGAGGTGTGTCCAGTTTGTTTCAGAATCTCGGGGTGTC CGTAACTCCGATCCAAAGGATTGTCCTGAGCGGTGAAAATGGGCTGAAGATCGACATCC ATGTCATCATCCCGTATGAAGGTCTGAGCGGCGACCAAATGGGCCAGATCGAAAAAATT TTTAAGGTGGTGTACCCTGTGGATGATCATCACTTTAAGGTGATCCTGCACTATGGCACA CTGGTAATCGACGGGGTTACGCCGAACATGATCGACTATTTCGGACGGCCGTATGAAGG CATCGCCGTGTTCGACGGCAAAAAGATCACTGTAACAGGGACCCTGTGGAACGGCAAC AAAATTATCGACGAGCGCCTGATCAACCCCGACGGCTCCCTGCTGTTCCGAGTAACCATC AACGGAGTGACCGGCTGGCGGCTGTGCGAACGCATTCTGGCGGGTGGAGGCTTTGTG AACCAACACCTGTGCGGCTCACACCTGGTGGAAGCTCTCTACCTAGTGTGCGGGGAAC |
| | GAGGCTTCTTCTACACACCCAAGACCCGCCGGgAGGCaGAGGACCTGCAGGTGGGGC AGGTGGAGCTGGGCGGGggccCaggccCTGGTGCAGGCAGCCTGCAGCCCTTGGCCCTG GAGGGGTCCcTgcAGAAGCGTGGCATTGTGGAACAATGCTGTACCAGCATCTGCTCcctct accagctggagaactactgcaactagGAATTCcggtacCTCTAGAGTCGACCCGGGCGGCCGCTT CGAGCAGACATGATAAGATACATTGATGAGTTTGGACAAACCACAACTAGAATGCAGTG AAAAAAATGCTTTATTTGTGAAATTTGTGATGCTATTGCTTTATTTGTAACCATTATAAGCT GCAATAAACAAGTTAACAACAACAATTGCATTCATTTTATGTTTCAGGTTCAGGGGGAG ATGTGGGAGGTTTTTTAAAGCAAGTAAAACCTCTACAAATGTGGTAAAATCGATAAGGA TCCGGGCTGGCGTAATAGCGAAGAGGCCCGCACCGATCGCCCTTCCCAACAGTTGCGC AGCCTGAATGGCGAATGGACGCGCCCTGTAGCGGCGCATTAAGCGCGGCGGGTGTGGT GGTTACGCGCAGCGTGACCGCTACACTTGCCAGCGCCCTAGCGCCCGCTCCTTTCGCTT TCTTCCCTTCCTTTCTCGCCACGTTCGCCGGCTTTCCCCGTCAAGCTCTAAATCGGGGGC TCCCTTTAGGGTTCCGATTTAGTGCTTTACGGCACCTCGACCCCAAAAAACTTGATTAGG GTGATGGTTCACGTAGTGGGCCATCGCCCTGATAGACGGTTTTTCGCCCTTTGACGTTG GAGTCCACGTTCTTTAATAGTGGACTCTTGTTCCAAACTGGAACAACACTCAACCCTATC TCGGTCTATTCTTTTGATTTATAAGGGATTTTGCCGATTTCGGCCTATTGGTTAAAAAATG AGCTGATTTAACAAAAATTTAACGCGAATTTTAACAAAATATTAACGCTTACAATTTCCTG ATGCGGTATTTTCTCCTTACGCATCTGTGCGGTATTTCACACCGCATATGGTGCACTCTCA GTACAATCTGCTCTGATGCCGCATAGTTAAGCCAGCCCCGACACCCGCCAACACCCGCT GACGCGCCCTGACGGGCTTGTCTGCTCCCGGCATCCGCTTACAGACAAGCTGTGACCGT CTCCGGGAGCTGCATGTGTCAGAGGTTTTCACCGTCATCACCGAAACGCGCGAGACGA AAGGGCCTCGTGATACGCCTATTTTTATAGGTTAATGTCATGATAATAATGGTTTCTTAGA CGTCAGGTGGCACTTTTCGGGGAAATGTGCGCGGAACCCCTATTTGTTTATTTTTCTAAA TACATTCAAATATGTATCCGCTCATGAGACAATAACCCTGATAAATGCTTCAATAATATTGA AAAAGGAAGAGTATGAGTATTCAACATTTCCGTGTCGCCCTTATTCCCTTTTTTGCGGCA TTTTGCCTTCCTGTTTTTGCTCACCCAGAAACGCTGGTGAAAGTAAAAGATGCTGAAGA TCAGTTGGGTGCACGAGTGGGTTACATCGAACTGGATCTCAACAGCGGTAAGATCCTTG AGAGTTTTCGCCCCGAAGAACGTTTTCCAATGATGAGCACTTTTAAAGTTCTGCTATGTG GCGCGGTATTATCCCGTATTGACGCCGGGCAAGAGCAACTCGGTCGCCGCATACACTATT CTCAGAATGACTTGGTTGAGTACTCACCAGTCACAGAAAAGCATCTTACGGATGGCATG ACAGTAAGAGAATTATGCAGTGCTGCCATAACCATGAGTGATAACACTGCGGCCAACTTA CTTCTGACAACGATCGGAGGACCGAAGGAGCTAACCGCTTTTTTGCACAACATGGGGG ATCATGTAACTCGCCTTGATCGTTGGGAACCGGAGCTGAATGAAGCCATACCAAACGAC GAGCGTGACACCACGATGCCTGTAGCAATGGCAACAACGTTGCGCAAACTATTAACTGG CGAACTACTTACTCTAGCTTCCCGGCAACAATTAATAGACTGGATGGAGGCGGATAAAG TTGCAGGACCACTTCTGCGCTCGGCCCTTCCGGCTGGCTGGTTTATTGCTGATAAATCTG GAGCCGGTGAGCGTGGGTCTCGCGGTATCATTGCAGCACTGGGGCCAGATGGTAAGCC |
| | CTCCCGTATCGTAGTTATCTACACGACGGGGAGTCAGGCAACTATGGATGAACGAAATA GACAGATCGCTGAGATAGGTGCCTCACTGATTAAGCATTGGTAACTGTCAGACCAAGTT TACTCATATATACTTTAGATTGATTTAAAACTTCATTTTTAATTTAAAAGGATCTAGGTGAA GATCCTTTTTGATAATCTCATGACCAAAATCCCTTAACGTGAGTTTTCGTTCCACTGAGCG TCAGACCCCGTAGAAAAGATCAAAGGATCTTCTTGAGATCCTTTTTTTCTGCGCGTAATC TGCTGCTTGCAAACAAAAAAACCACCGCTACCAGCGGTGGTTTGTTTGCCGGATCAAG AGCTACCAACTCTTTTTCCGAAGGTAACTGGCTTCAGCAGAGCGCAGATACCAAATACT GTTCTTCTAGTGTAGCCGTAGTTAGGCCACCACTTCAAGAACTCTGTAGCACCGCCTACA TACCTCGCTCTGCTAATCCTGTTACCAGTGGCTGCTGCCAGTGGCGATAAGTCGTGTCTT ACCGGGTTGGACTCAAGACGATAGTTACCGGATAAGGCGCAGCGGTCGGGCTGAACG GGGGGTTCGTGCACACAGCCCAGCTTGGAGCGAACGACCTACACCGAACTGAGATACC TACAGCGTGAGCTATGAGAAAGCGCCACGCTTCCCGAAGGGAGAAAGGCGGACAGGT ATCCGGTAAGCGGCAGGGTCGGAACAGGAGAGCGCACGAGGGAGCTTCCAGGGGGA AACGCCTGGTATCTTTATAGTCCTGTCGGGTTTCGCCACCTCTGACTTGAGCGTCGATTTT TGTGATGCTCGTCAGGGGGGCGGAGCCTATGGAAAAACGCCAGCAACGCGGCCTTTTT ACGGTTCCTGGCCTTTTGCTGGCCTTTTGCTCACATGGCTCGACAGATCT (SEQ ID NO: 97) |

The present invention also provides the following embodiments which may be combined with any aspect or embodiment described throughout the entirety of the present invention.

### The recombinant antigen molecule of the present invention

In one aspect of the invention, the test kit for performing an assay according to the invention comprises a set of at least three recombinant antigen molecules in a single composition. The recombinant antigen molecules according to the invention are not particularly limited other than by their ability to be bound by antibodies, more specifically by antibodies against self-proteins (i.e., autoantibodies), preferably by islets autoantibodies, more preferably by autoantibodies against any of human insulin, proinsulin, human Glutamate decarboxylase 65, human islet antigen 2, and human Zinc transporter 8.

In one embodiment, the set of at least three recombinant antigen molecules is selected from the group comprising recombinant human insulin human, recombinant human proinsulin, recombinant human Glutamate decarboxylase 65, recombinant human islet antigen 2, and recombinant human Zinc transporter 8. In another embodiment, the set of at least three recombinant antigen molecules is selected from the group consisting of human insulin human, recombinant human proinsulin, recombinant human Glutamate decarboxylase 65, recombinant human islet antigen 2, and recombinant human Zinc transporter 8. In a preferred embodiment, the recombinant antigen molecules are fused to a bioluminescent reporter protein.

In one embodiment, the bioluminescent reporter protein is a nanoluciferase reporter protein. In a preferred embodiment, the bioluminescent reporter protein is a Nanoluc luciferase (NLuc) reporter protein. In a more preferred embodiment, the Nanoluc luciferase (NLuc) reporter protein comprises a nucleotide sequence at least 85% identical to the nucleotide sequence encoded by SEQ ID NO: 10, at least 90% identical to the nucleotide sequence encoded by SEQ ID NO: 10, at least 95% identical to the nucleotide sequence encoded by SEQ ID NO: 10, at least 98% identical to the nucleotide sequence encoded by SEQ ID NO: 10, at least 99% identical to the nucleotide sequence encoded by SEQ ID NO: 10, identical to the nucleotide sequence encoded by SEQ ID NO:10.

It will be understood that the recombinant antigen molecules according to the invention are meant to optionally include a secretion signal peptide sequence. It is also understood that any reference to amino acid sequences referred to herein is meant to encompass not only the unmodified amino acid sequence but also typical posttranslational modifications of these amino acid sequences (e.g., glycosylation or deamidation of amino acids, the clipping of particular amino acids or other posttranslational modifications) occurring in cellular expression systems known in the art, including mammalian cells, such as Expi293F^{™} cells, or a sequence with one or more amino acid difference with the herein define amino acid sequence, such as 1, 2, 3, 4, 5, 6 or more amino acid difference.

### The test kit for performing the assay according to the invention

In one aspect of the invention, the assay comprises a luciferase immune precipitation system **(LIPS)** assay or a solid phase capture LIPS (**scLIPS**) assay.

The LIPS assay is well-known in the field of immune precipitation assays. The LIPS assay can be performed according to standard protocols described in the literature. LIPS is a type of liquid phase immunoassay that uses the emission of light from an enzymatic reaction to detect and quantify specific analytes in biological samples. A substrate of the reporter protein is added to the assay and the reporter protein catalyses a reaction that produces light. The amount of light produced can be detected and provides a quantitative measure of the level of the reporter protein in the sample; the level of the reporter protein in a sample may be used to determine the presence and/or level of an antibody molecule in a sample.

In one embodiment, the assay comprises a scLIPS assay is based on the LIPS assay and further comprises a protein selected from the group comprising protein A, protein L, anti IgA, anti IgM, and anti IgG. The protein is immobilized on a surface. As long as a surface can be coated with the protein that can bind an immune complex, any surface can be used. In one embodiment, the protein is immobilized on a surface selected from the group comprising a microplate, a particle, and resin. Preferably, the protein is immobilized on a surface selected from the group consisting of a microplate, a particle, and resin. When the surface is a particle, the particle is preferably a Sepharose bead, an agarose bead, or a paramagnetic particle. The size of the microplate is not particularly limited, and the type of surface can be different as long as it has the ability to be coated with a protein selected from the group comprising protein A, protein L, anti IgA, anti IgM, and anti IgG. For example, polystyrene plates with high binding capacity can be used, as well other types of plates, such as plates with e.g., nylon membranes with a high binding capacity. In another embodiment, the protein is immobilized on the surface of a coated microfluidic device.

Advantageously, the LIPS assay and scLIPS assay offer high sensitivity, a wide dynamic range, and faster execution times compared to other immunoassay formats.

In one embodiment, the recombinant human Glutamate decarboxylase 65, recombinant human islet antigen 2, and recombinant human Zinc transporter 8 are diluted to a concentration of 2.0×10⁶ LU/25 µl (±200,000 LU) and combined to a final concentration of 6.0×10⁶ LU/25 µl (±200,000 LU). In another embodiment, the human insulin and recombinant human proinsulin are diluted to a concentration of 10.0×10⁶ LU/5 µl (±200,000 LU).

In a preferred embodiment, the recombinant human Glutamate decarboxylase 65, the recombinant human islet antigen 2, and the recombinant human Zinc transporter 8 molecules are mixed in a ratio of 1:1:1, preferably in a molar ratio of 1:1:1 in a single composition.

In one embodiment, the recombinant human insulin molecules, recombinant human proinsulin molecules, the recombinant human Glutamate decarboxylase 65 antigen molecules, the recombinant human islet antigen 2 antigen molecules, and the recombinant human Zinc transporter 8 antigen molecules are diluted in a buffer, including but not limited to TBST buffer or PBS. TBST buffer generally comprises 20 mM tris buffer, 150mM NaCl, and 0.5% Tween-20, at pH 7.4.

In one embodiment, the test kit further comprises at least **three single compositions,** wherein each single composition comprises a recombinant antigen molecule selected from the group comprising of recombinant human insulin, recombinant human proinsulin, recombinant human Glutamate decarboxylase 65, recombinant human islet antigen 2, and recombinant human Zinc transporter 8. In another embodiment, the test kit further comprises at least three single compositions, wherein each single composition comprises a recombinant antigen molecule selected from the group consisting of recombinant human insulin, recombinant human proinsulin, recombinant human Glutamate decarboxylase 65, recombinant human islet antigen 2, and recombinant human Zinc transporter 8. In a preferred embodiment, the recombinant antigen molecule is fused to a bioluminescent reporter protein. In a preferred embodiment, the bioluminescent reporter protein is a Nanoluc luciferase (NLuc) reporter protein. In a more preferred embodiment, the Nanoluc luciferase (NLuc) reporter protein comprises a nucleotide sequence at least 85% identical to the nucleotide sequence encoded by SEQ ID NO: 10, at least 90% identical to the nucleotide sequence encoded by SEQ ID NO: 10, at least 95% identical to the nucleotide sequence encoded by SEQ ID NO: 10, at least 98% identical to the nucleotide sequence encoded by SEQ ID NO: 10, at least 99% identical to the nucleotide sequence encoded by SEQ ID NO: 10, identical to the nucleotide sequence encoded by SEQ ID NO:10.

In one embodiment, the recombinant human Glutamate decarboxylase 65, recombinant human islet antigen 2, and recombinant human Zinc transporter 8 are each diluted to a final concentration of about 4.0×10⁶ LU/25µl (±200,000 LU) when present in three single compositions. In another embodiment, the recombinant human insulin and recombinant human proinsulin are each diluted to a final concentration of about 10.0×10⁶ LU/5 µl (±200,000 LU) when present in a single composition. In a preferred embodiment, the recombinant human Glutamate decarboxylase 65, recombinant human islet antigen 2, and recombinant human Zinc transporter 8 are each diluted to a final concentration of about 4.0×10⁶ LU/25µl (±200,000 LU) and/or the recombinant human insulin and recombinant human proinsulin are each diluted to a final concentration of about 10.0×10⁶ LU/5 µl (±200,000 LU) when present in at least **three single compositions.**

In an additional aspect of the invention, the test kit comprises the recombinant human insulin antigen molecule comprises a nucleotide sequence at least 85% identical to a nucleotide sequence selected from a group consisting of SEQ ID NOs: 1, and 11 to 46, at least 90% identical to a nucleotide sequence selected from a group consisting of SEQ ID NOs: 1, and 11 to 46, at least 95% identical to a nucleotide sequence selected from a group consisting of SEQ ID NOs: 1, and 11 to 46, at least 98% identical to a nucleotide sequence selected from a group consisting of SEQ ID NOs: 1, and 11 to 46, at least 99% identical to a nucleotide sequence selected from a group consisting of SEQ ID NOs: 1, and 11 to 46, identical to a nucleotide sequence selected from a group consisting of SEQ ID NOs: 1, and 11 to 46. In a preferred embodiment, the recombinant human insulin antigen molecule comprises a nucleotide sequence at least 85% identical to the nucleotide sequence encoded by SEQ ID NO: 1, at least 90% identical to the nucleotide sequence encoded by SEQ ID NO: 1, at least 95% identical to the nucleotide sequence encoded by SEQ ID NO: 1, at least 98% identical to the nucleotide sequence encoded by SEQ ID NO: 1, at least 99% identical to the nucleotide sequence encoded by SEQ ID NO: 1, identical to the nucleotide sequence encoded by SEQ ID NO: 1.

In an additional aspect of the invention, the test kit comprises the recombinant human proinsulin antigen molecule comprises a nucleotide sequence at least 85% identical to a nucleotide sequence selected from a group consisting of SEQ ID NOs: 65 to 87 and 96, at least 90% identical to a nucleotide sequence selected from a group consisting of SEQ ID NOs: 65 to 87 and 96, at least 95% identical to a nucleotide sequence selected from a group consisting of SEQ ID NOs: 65 to 87 and 96, at least 98% identical to a nucleotide sequence selected from a group consisting of SEQ ID NOs: 65 to 87 and 96, at least 99% identical to a nucleotide sequence selected from a group consisting of SEQ ID NOs: 65 to 87 and 96, identical to a nucleotide sequence selected from a group consisting of SEQ ID NOs: 65 to 87 and 96.

In an additional aspect of the invention, the test kit comprises the recombinant human islet antigen 2 antigen molecule comprises a nucleotide sequence at least 85% identical to a nucleotide sequence selected from a group consisting of SEQ ID NOs: 2 to 4, at least 90% identical to a nucleotide sequence selected from a group consisting of SEQ ID NOs: 2 to 4, at least 95% identical to a nucleotide sequence selected from a group consisting of SEQ ID NOs: 2 to 4, at least 98% identical to a nucleotide sequence selected from a group consisting of SEQ ID NOs: 2 to 4, at least 99% identical to a nucleotide sequence selected from a group consisting of SEQ ID NOs: 2 to 4, identical to a nucleotide sequence selected from a group consisting of SEQ ID NOs: 2 to 4.

In an additional aspect of the invention, the test kit comprises the recombinant human Zinc transporter 8 antigen molecule comprises a nucleotide sequence at least 85% identical to the nucleotide sequence encoded by SEQ ID NO: 5, at least 90% identical to the nucleotide sequence encoded by SEQ ID NO: 5, at least 95% identical to the nucleotide sequence encoded by SEQ ID NO: 5, at least 98% identical to the nucleotide sequence encoded by SEQ ID NO: 5, at least 99% identical to the nucleotide sequence encoded by SEQ ID NO: 5, identical to the nucleotide sequence encoded by SEQ ID NO: 5; and/or

In an additional aspect of the invention, the test kit comprises the recombinant human Glutamate decarboxylase 65 antigen molecule comprises a nucleotide sequence at least 85% identical to the nucleotide sequence encoded by SEQ ID NO: 6, at least 90% identical to the nucleotide sequence encoded by SEQ ID NO: 6, at least 95% identical to the nucleotide sequence encoded by SEQ ID NO: 6, at least 98% identical to the nucleotide sequence encoded by SEQ ID NO: 6, at least 99% identical to the nucleotide sequence encoded by SEQ ID NO: 6, identical to the nucleotide sequence encoded by SEQ ID NO: 6.

In one aspect of the invention, the recombinant antigen molecules of the kit are suitable for the detection of human autoantibody molecules, preferably islets autoantibodies in a sample obtained from a human, e.g., a blood sample, a serum sample, a plasma from a human.

The kit of the present invention is suitable for the identification of at least one autoantibody molecule with reactivity to a self-antigen, such as an islet autoantibody molecule, in a patient developing type 1 diabetes. Preferably, the autoantibody molecule is at least one autoantibody molecule with specificity to human insulin, human proinsulin, human Glutamate decarboxylase 65, human islet antigen 2, or human Zinc transporter 8. More preferably, the antibody is at least one islet autoantibody selected from the group comprising islet autoantibodies IAA (Insulin Auto Antibodies), GADA (Glutamic Acid Decarboxylase Antibodies), IA-2A (Islet Antigen 2 Antibodies), and ZnT8A (Zinc Transporter 8 Antibodies). Most preferably, the autoantibody is at least one islet autoantibody selected from the group consisting of islet autoantibodies IAA (Insulin Auto Antibodies), GADA (Glutamic Acid Decarboxylase Antibodies), IA-2A (Islet Antigen 2 Antibodies), and ZnT8A (Zinc Transporter 8 Antibodies).

Hence, the present invention provides a kit for the *in vitro* diagnosis of type 1 diabetes.

The present invention allows for a rapid, sensible and efficient identification of the presence of autoantibodies, such as islet autoantibodies in a sample, which may be indicative of the presence of an autoimmune disorder, such a type 1 diabetes.

In certain embodiments, the kit may also comprise a sterile container such as boxes, ampules, bottles, vials, tubes, bags, pouches, blister-packs, dishes or other suitable container forms known in the art. Such containers can be made of plastic, glass or other materials suitable for holding cells. The kit may also comprise suitable cell culture medium, shipping medium, buffers and any other reactive that may become necessary for detecting autoantibody molecules.

In one aspect of the invention, the recombinant antigen molecules, or the test kit according to the present invention are meant to be used in a test at entry into a screening program. The assay comprising the recombinant antigen molecules or the test kit according to the invention can be followed by repeat confirmatory tests in which samples positive in the assay of the invention are reanalyzed to confirm the presence and type of an antibody in the sample. In one embodiment, the tests to confirm the presence and type of an antibody present in the sample comprise a recombinant antigen molecule selected from the group consisting of recombinant human insulin, recombinant human proinsulin, recombinant human Glutamate decarboxylase 65, recombinant human islet antigen 2, and recombinant human Zinc transporter 8 according to the invention. In one embodiment, the tests to confirm the presence and type of an antibody present in the sample comprise an immunoassay, such as a LIPS assay.

### The nucleic acid, vector, and cell of the present invention

In a further aspect, the invention provides a nucleic acid encoding the recombinant antigen molecule of the test kit. In a preferred embodiment, each of the recombinant antigen molecule is encoded by a separate nucleic acid. In another preferred embodiment, each of the recombinant antigen molecule is encoded by separate vector. In one embodiment, the vector is a lentiviral vector. In another embodiment, the vector is a pCMV plasmid. In a further embodiment, the vector is a pCMVTnT plasmid.

The invention also provides a vector encoding for:
a. a recombinant human insulin antigen molecule comprising a nucleotide sequence at least 85% identical to a nucleotide sequence selected from the group consisting of SEQ ID NOs: 1, 11 to 46, and 47 to 60, at least 90% identical to a nucleotide sequence selected from the group consisting of SEQ ID NOs: 1, 11 to 46, and 47 to 60, at least 95% identical to a nucleotide sequence selected from the group consisting of SEQ ID NOs: 1, 11 to 46, and 47 to 60, at least 98% identical to a nucleotide sequence selected from the group consisting of SEQ ID NOs: 1, 11 to 46, and 47 to 60, at least 99% identical to a nucleotide sequence selected from the group consisting of SEQ ID NOs: 1, 11 to 46, and 47 to 60, identical to a nucleotide sequence selected from the group consisting of SEQ ID NOs: 1, 11 to 46, and 47 to 60; or
b. a recombinant human proinsulin antigen molecule comprising a nucleotide sequence at least 85% identical to a nucleotide sequence selected from the group consisting of SEQ ID NOs: 62, 63, 65 to 87, and 88 to 96, at least 90% identical to a nucleotide sequence selected from the group consisting of SEQ ID NOs: 62, 63, 65 to 87, and 88 to 96, at least 95% identical to a nucleotide sequence selected from the group consisting of SEQ ID NOs: 62, 63, 65 to 87, and 88 to 96, at least 98% identical to a nucleotide sequence selected from the group consisting of SEQ ID NOs: 62, 63, 65 to 87, and 88 to 96, at least 99% identical to a nucleotide sequence selected from the group consisting of SEQ ID NOs: 62, 63, 65 to 87, and 88 to 96, identical to a nucleotide sequence selected from the group consisting of SEQ ID NOs: 62, 63, 65 to 87, and 88 to 96; or
c. a recombinant human islet antigen 2 antigen molecule comprising a nucleotide sequence at least 85% identical to a nucleotide sequence selected from the group consisting of SEQ ID NOs: 2 to 4, at least 90% identical to a nucleotide sequence selected from the group consisting of SEQ ID NOs: 2 to 4, at least 95% identical to a nucleotide sequence selected from the group consisting of SEQ ID NOs: 2 to 4, at least 98% identical to a nucleotide sequence selected from the group consisting of SEQ ID NOs: 2 to 4, at least 99% identical to a nucleotide sequence selected from the group consisting of SEQ ID NOs: 2 to 4, identical to a nucleotide sequence selected from the group consisting of SEQ ID NOs: 2 to 4; or
d. a recombinant human Zinc transporter 8 antigen molecule comprising a nucleotide sequence at least 85% identical to the nucleotide sequence encoded by SEQ ID NO: 5, at least 90% identical to the nucleotide sequence encoded by SEQ ID NO: 5, at least 95% identical to the nucleotide sequence encoded by SEQ ID NO: 5, at least 98% identical to the nucleotide sequence encoded by SEQ ID NO: 5, at least 99% identical to the nucleotide sequence encoded by SEQ ID NO: 5, identical to the nucleotide sequence encoded by SEQ ID NO: 5; or
e. a recombinant human Glutamate decarboxylase 65 antigen molecule comprising a nucleotide sequence at least 85% identical to the nucleotide sequence encoded by SEQ ID NO: 6, at least 90% identical to the nucleotide sequence encoded by SEQ ID NO: 6, at least 95% identical to the nucleotide sequence encoded by SEQ ID NO: 6, at least 98% identical to the nucleotide sequence encoded by SEQ ID NO: 6, at least 99% identical to the nucleotide sequence encoded by SEQ ID NO: 6, identical to the nucleotide sequence encoded by SEQ ID NO: 6.

In a preferred embodiment, the recombinant human insulin antigen molecule comprises a nucleotide sequence at least 85% identical to the nucleotide sequence encoded by SEQ ID NO: 1, at least 90% identical to the nucleotide sequence encoded by SEQ ID NO: 1, at least 95% identical to the nucleotide sequence encoded by SEQ ID NO: 1, at least 98% identical to the nucleotide sequence encoded by SEQ ID NO: 1, at least 99% identical to the nucleotide sequence encoded by SEQ ID NO: 1, identical to the nucleotide sequence encoded by SEQ ID NO: 1.

In one embodiment, the invention provides a cell comprising the set of nucleic acids or a nucleic acid, or a vector and expressing the recombinant antigen molecules. In another preferred embodiment, the cell is a stably expressing transfectant cell line. In one preferred embodiment, the cell is a mammalian cell, such as an Expi293F^{™} cell. In one embodiment, the cell expresses a nucleic acid or a vector encoding for one recombinant antigen molecule selected from the group comprising recombinant human Glutamate decarboxylase 65, recombinant human islet antigen 2, recombinant human Zinc transporter 8, recombinant human insulin, and recombinant human proinsulin. In a more preferred embodiment, the cell expresses a nucleic acid or a vector encoding for one recombinant antigen molecule selected from the group consisting of recombinant human Glutamate decarboxylase 65, recombinant human islet antigen 2, recombinant human Zinc transporter 8, recombinant human insulin, and recombinant human proinsulin. In another embodiment, the transfectant cell line is an Expi-293F NLuc GAD65 aa96-585 cell line, an Expi-293F Dual NLuc IA-2ic cell line, an Expi-293F Dual NLuc ZnT8 dimer cells line, or an Expi-293F Insulin B NLuc cell line. Advantageously, the stably expressing transfectant cell line facilitates cheaper and larger scale production of the recombinant antigen molecules according to the invention.

### The method of the present invention

In a further aspect, the invention provides a method for producing the recombinant antigen molecule of the test kit, the method comprising expressing the set of nucleic acids or the nucleic acid or the vector in a cell, and harvesting the recombinant antigen molecule.

The recombinant antigen molecules according to the invention can be obtained by methods known in the art. Such methods include methods for the production of recombinant antigen molecules, e.g., of recombinant proteins.

In another aspect, the invention provides a method for determining the presence and/or level of an antibody molecule in a sample.

According to the invention, the present invention provides an *in vitro* method for determining the presence and/or level of an antibody molecule in a sample. In one aspect, the sample is a blood sample, a serum sample, or a plasma sample obtained from a human subject.

The *in vitro* method comprises the steps of:
a) contacting the sample with the set of at least three recombinant antigen molecules, wherein a complex between the recombinant antigen molecule and the autoantibody molecule (referred to as "antigen molecule-autoantibody complex") is formed;
b) contacting the recombinant antigen molecule-autoantibody complex with a protein;
c) removing the unbound recombinant antigen molecules; and
d) contacting the bioluminescence reporter protein covalently linked to the recombinant antigen molecule with the substrate;
e) determining the presence, absence, and/or amount of one or more autoantibody molecules in the sample that binds to one or more recombinant antigen molecules.

In one embodiment of step a), the sample is contacted with a single composition comprising recombinant human Glutamate decarboxylase 65 antigen molecule, recombinant human islet antigen 2 antigen molecule, recombinant human Zinc transporter 8 antigen molecule, recombinant human insulin antigen molecule, and recombinant human proinsulin antigen molecule. In a preferred embodiment of step a), the sample is contacted with a single composition comprising recombinant human Glutamate decarboxylase 65 antigen molecule, recombinant human islet antigen 2 antigen molecule, and recombinant human Zinc transporter 8 antigen molecule. The skilled person is able to determine the conditions and the length of the contacting period which are sufficient for the formation of a complex between the recombinant antigen molecule and the autoantibody molecule (referred to as "antigen molecule-autoantibody complex"). Suitable buffers, salts, solutions, or in culture medium are comprised in the single composition comprising the at least three recombinant antigen molecules.

In one embodiment of step b), the recombinant antigen molecule-autoantibody complex is contacted with a protein selected from the group comprising protein A, protein L, anti IgA, anti IgM, and anti IgG. In another embodiment, the recombinant antigen molecule-autoantibody complex is contacted with a protein selected from the group consisting of protein A, protein L, anti IgA, anti IgM, and anti IgG. In a preferred embodiment, the protein is immobilized on a surface selected from the group comprising a microplate, a particle, and resin. In another preferred embodiment, the protein is immobilized on a surface selected from the group consisting of a microplate, a particle, and resin. Preferably, the particle is a Sepharose bead, an agarose bead, or a paramagnetic particle.

In one embodiment of step c), the removal of unbound recombinant antigen molecules may be achieved by washing to eliminate any unbound recombinant antigen molecules. Preferably, this step is performed to remove unbound recombinant antigen molecules and unbound autoantibody molecules. Most preferably, the recombinant antigen molecule-autoantibody complex is retained and subject to the subsequent step.

In one embodiment of step d), the bioluminescence reporter protein covalently linked to the recombinant antigen molecule is contacted with a substrate for the bioluminescence reporter protein. Suitable substrates for the bioluminescence reporter protein according to the invention are well-known in the field of immunoassays. The substrate might comprise further component and buffers required for the enzymatic activity of the bioluminescent reporter protein.

In one embodiment of step e), the presence, absence, or amount of one or more autoantibody molecules in the sample is determined by measuring the bioluminescence activity of the bioluminescent reporter molecule.

In one embodiment, the sample is further contacted with at least three recombinant antigen molecules in at least three single compositions. Preferably, the sample is contacted with at least three recombinant antigen molecules in at least three single compositions when the sample is found positive for at least one autoantibody molecules as determined in step e).

Accordingly, and subsequently to the method steps a) to e) as described above, the sample is contacted with at least three recombinant antigen molecules in at least **three single compositions,** wherein the method comprises the steps of:
f) contacting the sample with the set of at least three recombinant antigen molecules in at least **three single compositions,** wherein a recombinant antigen molecule-autoantibody complex is formed;
g) contacting the recombinant antigen molecule-autoantibody complex with a protein;
h) removing the unbound recombinant antigen molecules;
i) contacting the bioluminescence reporter protein covalently linked to the recombinant antigen molecule with the substrate; and
j) determining the presence, absence, and/or amount of one or more autoantibody molecules in the sample that binds to any of the at least three recombinant antigen molecule,
wherein the presence, absence, or amount of one or more autoantibody molecules in the sample is determined by measuring the bioluminescence activity of the bioluminescent reporter molecule.

In one embodiment, the at least one autoantibody molecule is against a self-antigen. Preferably, the at least one autoantibody molecule is an islet autoantibody molecule, optionally wherein the islet autoantibody molecule has specificity to human insulin, human proinsulin, human Glutamate decarboxylase 65, human islet antigen 2, or human Zinc transporter 8. More preferably, the islet autoantibody molecule is selected from the group comprising or consisting of islet autoantibodies IAA (Insulin Auto Antibodies), GADA (Glutamic Acid Decarboxylase Antibodies), IA-2A (Islet Antigen 2 Antibodies), and ZnT8A (Zinc Transporter 8 Antibodies).

In one aspect, the assay according to the invention comprises a luciferase immune precipitation system (LIPS) assay or a modified LIPS assay, referred to as solid-phase capture LIPS (scLIPS) assay. The LIPS assay is well-known in the field of immune precipitation assays. The LIPS assay can be performed according to standard protocols described in the literature. LIPS is a type of liquid phase immunoassay that uses the emission of light from an enzymatic reaction to detect and quantify specific analytes in biological samples. A substrate of the reporter protein is added to the assay and the reporter protein catalyses a reaction that produces light. The amount of light produced can be detected and provides a quantitative measure of the level of the reporter protein in the sample; the level of the reporter protein in a sample may be used to determine the presence and/or level of an antibody molecule in a sample. In one embodiment, the assay comprises a scLIPS assay is based on the LIPS assay and further comprises a protein selected from the group comprising protein A, protein L, anti IgA, anti IgM, and anti IgG.

Advantageously, the LIPS assay and scLIPS assay offer high sensitivity, a wide dynamic range, and faster execution times compared to other immunoassay formats.

In another aspect, the *in vitro* method comprises a luciferase immune precipitation system **(LIPS)** assay. In a preferred embodiment, the method comprises the steps of:
a) contacting the sample with the set of at least three recombinant antigen molecules;
b) contacting the recombinant antigen molecule-autoantibody complex with a protein immobilized on a Sepharose bead, , an agarose bead, a paramagnetic particle, or resin;
c) removing the unbound recombinant antigen molecules;
d) contacting the bioluminescence reporter protein covalently linked to the recombinant antigen molecule with the substrate; and
e) determining the presence, absence, and/or amount of one or more autoantibody molecules in the sample that binds to one or more recombinant antigen molecules,
wherein the presence, absence, or amount of one or more autoantibody molecules in the sample is determined by measuring the bioluminescence activity of the bioluminescent reporter molecule.

According to the invention, the LIPS assay can be performed according to standard protocols described in the literature.

In one embodiment of step a), a single-use antigen aliquots of the recombinant antigens are thawed at room temperature and diluted in Tris-buffered saline pH 7.4 with 0.5% Tween 20 (TBST-0.5%) to achieve a concentration for each antigen of 2.0×10⁶ LU/25 µl (±200,000 LU). Antigens are then combined to a yield a cumulative luciferase activity of 6.0×10⁶ LU/25 µl (±200,000 LU) and filtered through a PVDF 0.45µM Millex-SV syringe filter. The total volume of diluted antigen to be prepared is determined by the number of samples to be tested. In one embodiment, 1µl of the sample, preferably serum, is pipetted into a well of a 96-deep well polystyrene.

In one embodiment of step b), the recombinant antigen molecule-autoantibody complex is contacted with 5µL of blocked rProtein A Sepharose 4Fast Flow resuspended in 50µL, for 1h at 4 °C with shaking. In one embodiment, a PAS 50% stock slurry in EtOH 20 is prepared by blocking with glycine. Before use in the LIPS assay, 5 µl/well of PAS is washed four times in TBST and diluted in TBST to a final volume of 50 µl/well and then added to each well of the incubation plates. Plates are then incubated for 1hr with orbital shaking (^{~}700 rpm) at 4°C.

In one embodiment of step c), the unbound recombinant antigen is removed by serial washes. The washes can comprise at least 1, 2, 3, 4, 5 serial washes, preferably, 5 serial washes. The washes can be performed with a suitable buffer, such as for example TBST. In one example, each wash consists in the addition of 750 µl/well of TBST, followed by the plate centrifugation (e.g., 500xg for 3 min at 4°C) and buffer removal e.g., by aspiration using a micro-plate plate washer.

In one embodiment of step d) and step e), the resin pellets are transferred to an OptiPlate^{™} and the bioluminescence activity, e.g., the luciferase activity, is measured upon addition of 20µL to 40µL of Nano-Glo^{®} substrate and 20 of TBST to each well followed by immediate readout in a luminometer. In one embodiment, 0.5X Nano-Glo^{®} Luciferase Assay Reagent is prepared in advance by bringing at room temperature NanoGlo^{®} Luciferase Assay Buffer (stored frozen at -20° C). To measure the luciferase activity, the required reagents are prepared by combining 0.4 ml of Nano-Glo^{®} Luciferase Assay Substrate (which is a liquid stored at -20°C), 20 ml of Nano-Glo^{®} Luciferase Assay Buffer, and 20 ml of TBST previously equilibrated to RT per well. Using a multichannel dispenser, 40 µl of 0.5X Nano-Glo^{®} substrate is added to each well of the OptiPlates^{™} followed by immediate readout in Centro X960 luminometer using a protocol consisting of shaking for 1 seconds followed by light acquisition for 2 seconds/well. Acquired LU are then converted to arbitrary units (AU) using a standard curve constructed from doubling dilutions in normal human serum of a multiple GADA, IA-2A and ZnT8A Ab positive serum. AU are calculated based on the measured LU, using a mixed log10 (for light units) and log2 (for standard concentrations) logarithmic curve fitting algorithm in Excel (Microsoft Corporation).

In one embodiment, the presence of antibody molecule with specificity to human insulin, human proinsulin, human Glutamate decarboxylase 65, human islet antigen 2, and/or human Zinc transporter 8 in the sample is indicative of a risk to develop type 1 diabetes.

Advantageously, the LIPS assay requires an extremely limited amount of serum or plasma for each test. In one embodiment of the present invention, only 1 microliter of serum per replicate is required for the LIPS assay according to the invention, whereas 2-5 microliters are required in the RBA and 25 microliters in the RSR bridge-ELISA. For example, for an IAA LIPS assay, performed with and without cold insulin competition in 4 replicate wells, the total serum consumption is 4 microliters.

A further advantage of the present invention relates to the relatively short assay duration. For example, the incubation time for the GADA, IA-2A, ZnT8A LIPS assay is 2 hours, compared to an overnight incubation for RBA or RSR bridge-ELISA. Similarly, the incubation time for the IAA LIPS assay is of 18 hours compared to 72 hours for the RBA.

In one embodiment, the sample is further contacted with at least three recombinant antigen molecules in at least three single compositions. Preferably, the sample is contacted with at least three recombinant antigen molecules in at least three single compositions when the sample is found positive for at least one autoantibody molecules as determined in step e).

Accordingly, and subsequently to the method steps a) to e) as described above, the sample is contacted with at least three recombinant antigen molecules in at least **three single compositions**, wherein the method comprises the steps of:
f) contacting the sample with the set of at least three recombinant antigen molecules in at least **three single compositions,** wherein a recombinant antigen molecule-autoantibody complex is formed;
g) contacting the recombinant antigen molecule-autoantibody complex with a protein;
h) removing the unbound recombinant antigen molecules;
i) contacting the bioluminescence reporter protein covalently linked to the recombinant antigen molecule with the substrate; and
j) determining the presence, absence, and/or amount of one or more autoantibody molecules in the sample that binds to any of the at least three recombinant antigen molecule,
wherein the presence, absence, or amount of one or more autoantibody molecules in the sample is determined by measuring the bioluminescence activity of the bioluminescent reporter molecule.

In another aspect of the invention, the presence, absence, or amount of one or more antibody molecules in the sample is determined with a solid phase capture LIPS (scLIPS) assay. Accordingly, in one embodiment, the *in vitro* method comprises a solid phase capture luciferase immune precipitation system (**scLIPS**) assay. The scLIPS assay can be performed by performing the LIPS assay, wherein the recombinant antigen molecule-antibody complex is bound by a protein selected from the group comprising protein A, protein L (e.g., this recognizes the light chain of an antibody and can thus recognize several antibody types), anti IgA, anti IgM, and anti IgG prior to measuring the bioluminescence activity.

In a preferred embodiment, the method comprises the steps of:
a) contacting the sample with the set of at least three recombinant antigen molecules, wherein a recombinant antigen molecule-autoantibody complex is formed;
b) contacting the recombinant antigen molecule-autoantibody complex with a protein immobilized on a surface;
c) removing the unbound recombinant antigen molecules;
d) contacting the bioluminescence reporter protein covalently linked to the recombinant antigen molecule with the substrate; and
e) determining the presence, absence, and/or amount of one or more autoantibody molecules in the sample that binds to one or more recombinant antigen molecules,
wherein the presence, absence, or amount of one or more autoantibody molecules in the sample is determined by measuring the bioluminescence activity of the bioluminescent reporter molecule.

In one embodiment of step a), 1µl of the sample, preferably the serum, is pipetted to a polypropylene 96-well plate. 25µl of the composition comprising the at least three recombinant antigen molecules are added, briefly mixed on a plate mixer, and incubated for 2 hr at room temperature protected from light.

In one embodiment of step b), the protein is selected from the group comprising or consisting of protein A, protein L, anti IgA, anti IgM, and anti IgG. In one embodiment, the protein binds to any of the component of the complex. In a preferred embodiment, the protein binds to the constant portion (e.g., the Fc portion) of the autoantibody molecule. In a preferred embodiment, the recombinant antigen molecule-antibody complex is bound by Protein A and captured on an immunoglobulin G-binding protein-A-coated plate prior to measuring the bioluminescence activity, e.g., preferably the Nanoluc luciferase (NLuc) activity. In one embodiment, the reaction mixture of step a) is transferred to 96-well plates pre-coated with 30 mg/well of recombinant Protein A in carbonate buffer pH9 for 4 hours at room temperature and then blocked with freshly prepared 1,5% Fish Gelatin in PBS-HS 1X plus 0,1% Tween-20 pH9 for 18 hours at 4°C. Alternatively, commercial plates can be used coated with recombinant protein A/G and blocked with BSA. Capture plates are incubated for 1hr with orbital shaking (^{~}700 rpm) at 4°C.

In one embodiment of step c), the unbound recombinant antigen molecules are washed e.g., with TBT using the BioTek Elx405 performing 10 washes of 300 ml each.

In one embodiment of step d) and step e), 0.5X Nano-Glo^{®} Luciferase Assay Reagent is prepared in advance by bringing at room temperature NanoGlo^{®} Luciferase Assay Buffer (stored frozen at -20° C). To measure the luciferase activity, the required reagents are prepared by combining 0.4 ml of Nano-Glo^{®} Luciferase Assay Substrate (which is a liquid stored at -20°C), 20 ml of Nano-Glo^{®} Luciferase Assay Buffer, and 20 ml of TBST previously equilibrated to RT per well. Using a multichannel dispenser, 40 µl of 0.5X Nano-Glo^{®} substrate is added to each well of the OptiPlates^{™} followed by immediate readout in Centro X960 luminometer using a protocol consisting of shaking for 1 seconds followed by light acquisition for 2 seconds/well. Acquired LU are then converted to arbitrary units (AU) using a standard curve constructed from doubling dilutions in normal human serum of a multiple GADA, IA-2A and ZnT8A Ab positive serum. AU are calculated based on the measured LU, using a mixed log10 (for light units) and log2 (for standard concentrations) logarithmic curve fitting algorithm in Excel (Microsoft Corporation).

In one embodiment, the sample is contacted with at least three recombinant antigen molecules in at least three single compositions. Preferably, the sample is contacted with at least three recombinant antigen molecules in at least three single compositions when the sample is found positive for at least one autoantibody molecule as determined in step e).

Accordingly, and subsequently to the method steps a) to e) as described above, the sample is contacted with at least three recombinant antigen molecules in at least **three single compositions**, wherein the method comprises the steps of:
f) contacting the sample with the set of at least three recombinant antigen molecules in at least **three single compositions,** wherein a recombinant antigen molecule-autoantibody complex is formed;
g) contacting the recombinant antigen molecule-autoantibody complex with a protein;
h) removing the unbound recombinant antigen molecules;
i) contacting the bioluminescence reporter protein covalently linked to the recombinant antigen molecule with the substrate; and
j) determining the presence, absence, and/or amount of one or more autoantibody molecules in the sample that binds to any of the at least three recombinant antigen molecule,
wherein the presence, absence, or amount of one or more autoantibody molecules in the sample is determined by measuring the bioluminescence activity of the bioluminescent reporter molecule.

In one embodiment, the sample is contacted with the set of at least three recombinant antigen molecules in a single composition. In a preferred embodiment, the single composition comprises recombinant human Glutamate decarboxylase 65, recombinant human islet antigen 2, and recombinant human Zinc transporter 8. In a more preferred embodiment, the single composition does not comprise recombinant human insulin and/or recombinant human proinsulin. In a most preferred embodiment, the sample is further contacted with recombinant human insulin and/or recombinant human proinsulin in a single composition.

In another embodiment, the sample is contacted with at least three recombinant antigen molecules in at least three single compositions. Preferably, the sample is contacted with at least three recombinant antigen molecules in at least three single compositions when the sample is found positive for at least one autoantibody molecules when contacted with the set of at least three recombinant antigen molecules in a single composition and/or when contacted with recombinant human insulin in a single composition and/or recombinant human proinsulin in a single composition.

In one embodiment, the presence of antibody molecule with specificity to human insulin, human proinsulin, human Glutamate decarboxylase 65, human islet antigen 2, and/or human Zinc transporter 8 in the sample is indicative of a risk to develop type 1 diabetes.

Advantageously, the scLIPS assay requires an extremely limited amount of serum or plasma for each test. In one embodiment of the present invention, only 1 microliter of serum per replicate is required for the scLIPS assay according to the invention, whereas 2-5 microliters are required in the RBA and 25 microliters in the RSR bridge-ELISA. For example, for an IAA scLIPS assay, performed with and without cold insulin competition in 4 replicate wells, the total serum consumption is 4 microliters.

A further advantage of the present invention relates to the relatively short assay duration. For example, the incubation time for the GADA, IA-2A, ZnT8A scLIPS assay is 2 hours, compared to an overnight incubation for RBA or RSR bridge-ELISA. Similarly, the incubation time for the IAA scLIPS assay is of 18 hours compared to 72 hours for the RBA.

In another aspect, the autoantibody molecule is specific to an antigen expressed by a human beta cell. In one embodiment, the autoantibody molecule is at least one autoantibody molecule against a self-antigen. In another embodiment, the autoantibody molecule is at least one islet autoantibody molecule. In further embodiment, the islet autoantibody is selected from the group comprising the islet autoantibodies IAA (Insulin Auto Antibodies), GADA (Glutamic Acid Decarboxylase Antibodies), IA-2A (Islet Antigen 2 Antibodies), and ZnT8A (Zinc Transporter 8 Antibodies). In a preferred embodiment, the islet autoantibody is at least one autoantibody molecule with specificity to human insulin, human proinsulin, human Glutamate decarboxylase 65, human islet antigen 2, or human Zinc transporter 8.

In one aspect, the method comprises screening a human subject for the development of type 1 diabetes based on the presence, absence, or amount of one or more antibody molecules in the sample, wherein the presence, absence, or amount of one or more antibody molecules in the sample is determined by comparing the amount of detected antibody molecule in the sample with a standard curve for known amounts of said antibody molecules. In one embodiment, the presence of one or more antibody molecules in the sample is indicative of a risk to develop type 1 diabetes.

In a further aspect, contacting of the autoantibodies present in the sample is performed by seeding 1-µL replicates of each sample into 96-deep-well plates and adding from 6µL to 25µL of a composition comprising the recombinant antigen molecules according to the invention. In one embodiment, the plates are incubated for 2h at room temperature after mixing and centrifugating.

In one embodiment, the recombinant antigen molecule-autoantibody complex are captured by incubation with 5µL of blocked rProtein A Sepharose 4Fast Flow resuspended in 50µL, for 1h at 4 °C with shaking. In one embodiment, plates are washed 5 times by sequential dispensing of 750µL of TBST per well, centrifugation at 500g for 3 minutes at 4 °C, and removal of supernatant using a micro-plate plate washer. In one embodiment, the resin pellets are transferred to an OptiPlate^{™} and the luciferase activity is measured upon addition of 20µL to 40µL of Nano-Glo^{®} substrate and 20 of TBST to each well followed by immediate readout in a luminometer. In one embodiment, the readout is performed using a protocol consisting of shaking for 1 second followed by light acquisition for 2 seconds/well in a luminometer.

In one embodiment, acquired LU are converted to arbitrary units (AU) using a standard curve constructed from doubling dilutions in healthy human sample of an IAA-, GADA-, IA-2A- or ZnT8A-positive sample. In one embodiment, AU are calculated based on the measured LU, using a mixed log10 (for light units) and log2 (for standard concentrations) a logarithmic curve fitting algorithm.

In one embodiment, the presence of at least one autoantibody molecule with specificity to human insulin, human proinsulin, human Glutamate decarboxylase 65, human islet antigen 2, and/or human Zinc transporter 8 in the sample is indicative of a risk to develop type 1 diabetes.

The LIPS and scLIPS assays according to the invention require an extremely limited amount of serum or plasma for each test. In one embodiment of the present invention, only 1 microliter of serum per replicate is required for the LIPS assay according to the invention, whereas 2-5 microliters are required in the RBA and 25 microliters in the RSR bridge-ELISA. For example, for an IAA LIPS assay, performed with and without cold insulin competition in 4 replicate wells, the total serum consumption is 4 microliters.

A further advantage of the present invention relates to the relatively short assay duration. For example, the incubation time for the GADA, IA-2A, ZnT8A LIPS assay is 2 hours, compared to an overnight incubation for RBA or RSR bridge-ELISA. Similarly, the incubation time for the IAA LIPS assay is of 18 hours compared to 72 hours for the RBA.

The presence and/or level of an autoantibody molecule in a sample may be assessed by comparing the level of autoantibody molecules in a sample from a subject at risk of developing T1D, to the level of autoantibody molecules in a control sample, i.e., a sample from a subject that is not at risk of developing T1D, wherein an increase level of autoantibody molecules indicates an increased probability of developing T1D. Preferably, the level is statistically significant as assessed by appropriate statistical tests which are known in the art.

The present invention also provides a method of treating an autoimmune disorder, preferably an autoimmune disorder associated with the presence of islets autoantibodies, more preferably type 1 diabetes, comprising:
administering a treatment to effectively treat the autoimmune disorder in a subject who has been diagnosed as having an autoimmune disorder based on the presence of islets autoantibodies present in a sample obtained from the subject, wherein the islets autoantibodies bind to the recombinant antigen molecules of to the present invention.

The present invention is further illustrated by the following non-limiting examples:

### MATERIALS AND METHODS

### Cloning of antigens in plasmid vectors

The coding sequences of human T1D autoantigens were obtained starting from UniProt (www.uniprot.org) reference sequences. Modified coding sequences were amplified by RT-PCRfrom human islets cDNA and then mutagenized for GAD65 (UniProt entry Q05329), IA-2 (Uniprot entry Q16849) and ZnT8 (UniProt entry Q8IWU4). The antigen coding sequence of GADA65 was truncated to remove the first 95 amino acids that contains an epitope recognized by autoantibody reactivities poorly predictive of T1D development in pre-diabetes subjects²⁸²⁹. The IA-2 intracellular (IA-2ic) coding sequence was truncated to remove the first 604 amino acids, corresponding to extracellular and transmembrane domains and 1) do not contain main IA-2A epitopes and have 2) previously shown to be associated with higher unspecific background in immunoassays³⁰. The ZnT8 sequence was truncated to remove the first 267 amino acids which do not contain major epitopes of ZnT8A and to remove the transmembrane domains previously shown to be associated with higher unspecific background in immunoassays. Two distinct polymorphic variants were cloned containing a different polymorphic residue in amino acid 365 and encoding for a tryptophan or arginine residue, respectively. The two polymorphic ZnT8 COOH domains were then cloned in frame to create a dimeric ZnT8 recombinant antigen. The full-length human pre-proinsulin cDNA sequence was obtained as a synthetic chimeric DNA sequence in which a nanoluciferase reporter was inserted at the end of the insulin B chain before the C peptide and in which the proconvertase 2 recognition sequences in the C peptide were mutagenized to transform them into furin convertase recognition sequences. Several alternative variants of NanoLuc tagged proinsulin and insulin recombinant antigens were produced. In particular, the impact on antigen expression and autoantibody binding of the luciferase reporter tag placement relative to the autoantigen coding sequence was initially investigated. Subsequently, the impact on recombinant protein expression and autoantibody binding of introduction of several deletion or point mutations on a selection of these human recombinant insulin antigens and proinsulin antigens used as backbones was also investigated (see Figures 1 to 6 and Tables 2 to 4, and 6 to 10 containing the sequences of such recombinant antigens).

Site-directed mutagenesis of coding sequences to change specific amino acid residues or to introduce restriction sites at desired location in the coding sequence or to produce truncated forms was performed using the GeneArt^{®} Site-Directed Mutagenesis PLUS Kit protocol (Thermo Fisher Scientific, Carlsbad, CA, USA).

All modified coding sequences were sub-cloned into modified pCMVTNT^{™} plasmid (Promega, Madison WI, USA) expression vectors in which a NanoLuc^{™} (NLuc) luciferase reporter (Promega) was previously inserted, except for the insulin antigen construct. In the case of the truncated GAD65 antigen, the NanoLuc reporter was placed in frame upstream of the antigen coding sequence and joined to it by a glycine spacer. In the case of the IA-2ic cDNA sequence, this was cloned in frame both upstream and downstream of the NanoLuc reporter to generate a "dual" construct in which a dimeric intracellular IA-2 was joined by the interspersed reporter and separated by it by linker and spacer sequences. For NanoLuc tagged ZnT8 antigen a similar approach was used to generate a recombinant antigen in which two copies of the fused polymorphic domains were joined in frame both upstream and downstream of the luciferase reporter, respectively. This construct yielded a "dual" ZnT8 dimer luciferase tagged antigen.

### Expression and preparation of recombinant luciferase tagged antigens

Each recombinant Nanoluc^{®} luciferase tagged antigens was initially expressed and validated using the Expi293^{™} Expression System (Thermo Fisher Scientific Life Technologies). Expression of each cDNA construct subcloned in the pCMVTnT^{™} plasmid (Promega, USA) was driven by the plasmid CMV promoter after transient transfection of Expi293F^{™} cells using Expifectamine (see Tables 5 and 10 which display the sequences of mutated insulin and proinsulin subcloned into the pCMVTnT^{™} plasmid). Briefly, for a typical small-scale transfection, Expi293F^{™} cells were grown until an early logarithmic growth phase was reached and counted in a Bürker counting chamber. Then, 4.5 × 10⁶ Expi293F^{™}cells were resuspended in 2.125 ml of Expi293F Expression Medium and seeded into the well of a 6-well cell culture plate. A transfection mix was then prepared in three steps: 1) the combination of 4 µg of plasmid in 125 µl of Opti-MEM I followed by incubation at room temperature for 5 minutes; 2) the combination of 6.8 ml of ExpiFectamine 293TM Reagent in 118 ml of Opti-MEM I Reduced Serum Medium followed by incubation at room temperature for 5 minutes; 3) the combination of the previous two mixes followed by incubation at room temperature for 20 minutes to allow the formation of plasmid and transfection agents complexes. Seeded Expi293F^{™} cells in the 6-well plate were then added with the transfection mix and incubated for up to 24 hours on an orbital shaker (set at 125 rpm) placed inside a CO₂ cell culture incubator. Approximately 20 hours post-transfection, the transfected Expi293F^{™} cells were added first with 12.5 ml of Expifectamine 293 Transfection Enhancer 1 and then with 125 ml of Expifectamine 293 Transfection Enhancer 2 reagents followed by incubation of the 6-well plate for 24 hours on an orbital shaker (set at 125 rpm) placed inside a CO2 cell culture incubator. Approximately 48 hours post-transfection, transfected cells were transferred into a 2ml Eppendorf^{®} LoBind microcentrifuge tube and centrifuged for 5 minutes at 100xg at room temperature. Depending on the antigen being secreted or not the antigen harvest follows a different procedure.

For cytoplasmic antigens like GAD65, IA-2, and ZnT8 the supernatant is removed, and the cell pellet washed with 2 ml of phosphate buffered saline (PBS) pH 7.4 at room temperature, followed by centrifugation for 5 minutes at 100xg at room temperature. The PBS is then discarded, and the pellet is resuspended by pipetting in 800 ml of Passive Lysis Buffer 1X (PLB) (Promega) to lyse cells and release proteins. The cell lysate is then transferred into a 2 ml low protein binding 2 ml tube (LoBind^{™} Eppendorf) and kept on ice for 10 minutes, this step include vortex thoroughly every 2-3 minutes. Finally, the tube is centrifuged at 13,000 rpm in a refrigerated minicentrifuge at 4°C for 10 minutes. The supernatant is collected taking care to avoid dislodging and transferring insoluble debris and dispensed into PCR strip tubes as single use 5 ml aliquots or alternatively into low protein binding Eppendorf tubes for larger aliquots. All processed aliquots are then stored immediately at -80°C. For secreted antigens, the expressed antigens are present in the transfected cells culture supernatant. The supernatant is collected and transferred to a new Eppendorf tube and centrifuged at 13,000 rpm in a refrigerated minicentrifuge at 4°Cfor 10 minutes to pellet insoluble debris. The supernatant is then dispensed into PCR strip tubes as single use 5 ml aliquots, or alternatively into low protein binding Eppendorf tubes for larger aliquots.

### Estimate of Luciferase activity

After the harvest of expressed antigen and prior to -80°C storage, an estimate of the antigen content is obtained by determining the recovered luciferase activity in the processed cell lysate or the cleared supernatant, for cytoplasmic antigens or secreted antigens respectively. The procedure requires the thaw of NanoGlo^{®} Luciferase Assay Buffer (stored frozen at -20° C) that is then brought at room temperature. For each individual measurement of luciferase activity, the reconstituted Nano-Glo^{®} Luciferase Assay Reagent is prepared by combining 0.8 ml of Nano-Glo^{®} Luciferase Assay Substrate (which is a liquid stored at -20°C) with 40 ml of Nano-Glo^{®} Luciferase Assay Buffer (previously equilibrated to RT). Harvested antigen (2 ml) is serially diluted 1:250, 1:2500, 1:25000. Of each dilution, 25 ml are taken and mixed with 40 ml of Nano-Glo^{®} Luciferase Assay Reagent and read into a luminometer (Centro X960, Berthold Germany) for 2 seconds. The luciferase activity is measured as light units (LU), i.e., single photon counting, and the actual emission due to the antigen-luciferase present in the preparation is converted to light units/ml by multiplying for the appropriate corrected dilution factor measurements within the linear range of the luminometer readout.

### Generation of stable cell lines expressing luciferase tagged T1D autoantigens

### Subcloning of antigen-luciferase coding sequences into lentiviral shuttle plasmid vectors

The coding sequences of NLuc-tagged T1D autoantigens according to the invention were sub-cloned into a lentiviral shuttle plasmid upstream an IRES-GFP sequence. The lentiviral vectors were produced by calcium phosphate transient co-transfection in HEK293T cells of the plasmid containing the gene of interest sequence, the packaging plasmids (pGag-Pol and pRSV-REV), and the envelope plasmid encoding for the VSV.G protein. Medium was changed 14-16 hours after transfection and the supernatant containing the lentiviral vectors was collected 30 hours after medium change. The supernatants were filtered through a 0.22 µm filter (Millipore), transferred into sterile polyallomer tubes (Beckman Coulter) and concentrated by centrifugation at 20,000 g for 120 min at 20° C in a Beckman Optima XL-100K Ultracentrifuge. Obtained transducing units (TU) per ml titre was quantified by flow cytometry looking for GFP fluorescence after infecting HEK293T cells with serial dilutions of the collected lentiviral vectors³¹.

Subsequently, 200,000 Expi293F^{™} cells were then infected in 48-well plates using two different multiplicities of infection (MOI = 5 and MOI = 10) and monitored for 15 days for the appearance of the GFP fluorescence. The brightest cells were sorted using the BD FACSAria^{™} Fusion Flow Cytometer (BD Biosciences, San Jose, CA, USA) and grown in bulk for at least 2 weeks up to a concentration of 5×10⁶ cells/ml. The cells were seeded in a 96-well plate to select single clones by limiting dilution and the GFP-positive clones appeared after a week, cells were then resuspended and expanded. The luciferase activity of the recombinant proteins, harvested from the cell medium or the lysate of different clones, was measured to select the stable cell line expressing with the highest efficiency the recombinant antigen molecules.

A schematic overview of the recombinant antigen molecules expressed in the stable cell lines according to the invention is displayed in Figure 7.

### GADA or IA-2A or ZnT8A LIPS and scLIPS assay

LIPS and scLIPS assays of cytoplasmic antigens adopt the same fundamental protocol. Each assay is characterised using the corresponding recombinant nanoluciferase-tagged antigen. The overall procedure consists in the following steps: 1) dilution of nanoluciferase antigen in assay buffer and assessment and adjustment of the luciferase activity; 2) dispensing of serum and then of diluted recombinant nanoluciferase antigen in incubation plates; 3) capture of immune complexes by addition of protein-A sepharose resin (LIPS) or transfer to a plate coated with protein A (scLIPS); 4) wash of the immune-complexes to remove unbound antigens; 5) addition of the luciferase substrate followed by read-out of recovered luciferase activity in the luminometer (see Figures 8 and 9).

Briefly, each step consists in the following:
Step 1): a single-use recombinant antigen aliquot is thawed at room temperature and diluted in Tris-buffered saline pH 7.4 with 0.5% Tween 20 (TBST-0.5%) to achieve a concentration of 4.0×10⁶ LU/25µl (±200,000 LU) and filtered through a PVDF 0.45µM Millex-SV syringe filter (Merck, Darmstadt, Germany), the total volume of diluted recombinant antigen to be prepared is determined by the number of samples to be tested.

Step 2): for each sample to be tested and for standards included in the assay run, 1µl of serum is pipetted into a well of a 96-deep well polystyrene plate (Sarstedt, Nümbrecht, Germany) for LIPS or to a polypropylene 96-well plate for scLIPS (Sarstedt, Nümbrecht, Germany). Each well is then added with 25µl of diluted nanoluciferase-tagged recombinant antigen, briefly mixed on a plate mixer, and incubated for 2 hr at room temperature protected from light.

Step 3): for LIPS rProtein A Sepharose 4 fast flow (PAS) (GE Healthcare Life Sciences, Amersham, UK) is used to capture immune complexes. A PAS 50% stock slurry in EtOH 20 is prepared by blocking with glycine³², as previously described. Before use in LIPS assays, 5 µl/well of PAS is washed four times in TBST and diluted in TBST to a final volume of 50 µl/well and then added to each well of the incubation plates. Plates are then incubated for 1hr with orbital shaking (^{~}700 rpm) at 4°C. For the scLIPS assay the reaction mixture is transferred to 96-well plates (NUNC^{™} MaxiSORP, Thermo-Fisher, USA) pre-coated with 30 mg/well of recombinant Protein A (Sino Biological, China) in carbonate buffer pH9 for 4 hours at room temperature and then blocked with freshly prepared 1,5% Fish Gelatin in PBS-HS 1X plus 0,1% Tween-20 pH9 for 18 hours at 4°C (can be prepared in advance and stored emptied frozen at -20°C). Alternatively, commercial plates can be used coated with recombinant protein A/G and blocked with BSA (BioMat s.r.l., Italia). Capture plates are then incubated for 1hr with orbital shaking (^{~}700 rpm) at 4°C.

Step 4): For LIPS, unbound nanoluciferase-tagged recombinant antigen is removed by 5 serial washes each consisting in the addition of 750 µl/well of TBST, followed by the plate centrifugation (500xg for 3 min at 4°C) and buffer removal by aspiration. Dispensing and aspiration is performed using a BioTek Elx405 automated plate washer (Agilent, USA). PAS pellets are then transferred into the corresponding well of a 96-well OptiPlates^{™} (Perkin-Elmer, USA) using a multichannel liquid handler. OptiPlates^{™} are then centrifuged (500xg for 3 min at 4°C) and excess buffer aspirated using the BioTek Elx405 to leave a final volume of 30µl inside each well. For scLIPS, capture plates are washed with TBST using the BioTek Elx405 performing 10 washes of 300 ml each.

Step 5): 0.5X Nano-Glo^{®} Luciferase Assay Reagent is prepared in advance by bringing at room temperature NanoGlo^{®} Luciferase Assay Buffer (stored frozen at -20° C). To measure the luciferase activity, the required reagents are prepared by combining 0.4 ml of Nano-Glo^{®} Luciferase Assay Substrate (which is a liquid stored at -20°C), 20 ml of Nano-Glo^{®} Luciferase Assay Buffer, and 20 ml of TBST previously equilibrated to RT per well. Using a multichannel dispenser, 40µl of 0.5X Nano-Glo^{®} substrate are added to each well of the OptiPlates^{™} followed by immediate readout in Centro X960 luminometer using a protocol consisting of shaking for 1 seconds followed by light acquisition for 2 seconds/well. Acquired LU are then converted to arbitrary units (AU) using a standard curve constructed from doubling dilutions in normal human serum of a GADA-, IA-2A- or ZnT8A-positive serum. AU are calculated based on the measured LU, using a mixed log10 (for light units) and log2 (for standard concentrations) logarithmic curve fitting algorithm in Excel (Microsoft Corporation).

### IAA LIPS and scLIPS assays

LIPS and scLIPS assays measuring insulin autoantibodies (IAA) use secreted nanoluciferase-tagged chimeric insulin antigens and adopt a similar but distinct protocol to that of cytoplasmic antigens' assays. The overall procedure consists in the following steps: 1) dilution of nanoluciferase recombinant antigen in assay buffer (Tris Cl buffered pH 8 Tween-20 1% instead of TBST) and assessment and adjustment of the luciferase activity; 2) dispensing in 96-deep well plates incubation plates of serum (3 replicates: one for testing without and two with antibody binding competition using unlabeled insulin at two different concentrations of 10.5 ng/ well and 0.0032 ng/well) followed by the addition of diluted recombinant nanoluciferase-tagged insulin antigen in incubation plates; 3) addition of a mix of protein-A and Protein-G sepharose resins to allow the capture of immune complexes; 4) wash of the immune-complexes to remove unbound antigens; 5) addition of the luciferase substrate followed by read-out of recovered luciferase activity in the luminometer. Briefly, each step consists in the following:
Step 1): a single-use recombinant antigen aliquot is thawed at room temperature and diluted in Tris-buffered pH 8 with 1% Tween 20 (TBT-1%) to achieve a concentration of 10.0×10⁶ LU/5 µl (±200,000 LU) and filtered through a PVDF 0.45µM Millex-SV syringe filter (Merck, Darmstadt, Germany), the total volume of diluted antigen to be prepared is determined by the number of samples to be tested. One third of the diluted nanoluciferase antigen is used as is for dispensing into the wells without competition. For competition, the recombinant antigens are split in two tubes and added with Actrapid^{™} (NovoNOrdisk) at a final concentration of 10.5 ng/5 µl and 0.0032 ng/5 µl.

Step 2): for each sample to be tested and for standards included in the assay run, 1µl of serum is pipetted into each of triplicate wells of a 96-deep well polystyrene plate for LIPS (Sarstedt, Nümbrecht, Germany) or to a polypropylene 96-well plate for scLIPS (Sarstedt, Nümbrecht, Germany). For each serum to be tested, the well without competition ("uncompeted" well) is then added with 5µl of diluted nanoluciferase-tagged recombinant antigen, the second replicate well is added with nanoluciferase-tagged recombinant antigen containing Actrapid^{™} at 10.5 ng/5 µl and the third replicate with nanoluciferase-tagged recombinant antigen containing Actrapid^{™} at 0.0032 ng/5 µl. Incubation plates are then briefly mixed on a plate mixer, and incubated for 18 hr at 4°C protected from light.

Step 3): For LIPS, rProtein A Sepharose 4 fast flow (PAS) and protein-G sepharose (PGS)(GE Healthcare Life Sciences, Amersham, UK) are used to capture immune complexes. To reduce unspecific binding, both 50% slurries PAS and PGS were previously blocked respectively with glycine or ethanolamine, as previously described³³. Before use in LIPS assays, 5 µl/well of PAS and 5 µl/well of PGS are washed four times in TBT-1% and diluted in TBT-1% to a final volume of 50 µl/well and then added to each well of the incubation plates. Plates are then incubated for 1hr with orbital shaking (^{~}700 rpm) at 4°C. For the scLIPS assay the reaction mixture is transferred to 96-well plates (NUNC^{™} MaxiSORP, Thermo-Fisher, USA) pre-coated with 30 mg/well of recombinant Protein A (Sino Biological, China) in carbonate buffer pH9 for 4 hours at room temperature and then blocked with freshly prepared 1,5% Fish Gelatin in PBS-HS 1X plus 0,1% Tween-20 pH9 for 18 hours at 4°C (can be prepared in advance and stored emptied frozen at -20°C). Alternatively, commercial plates can be used coated with recombinant protein A/G and blocked with BSA (BioMat s.r.l., Italia). Capture plates are then incubated for 1hr with orbital shaking (^{~}700 rpm) at 4°C.

Step 4): For LIPS, unbound nanoluciferase antigen is removed by 5 serial washes each consisting in the addition of 750 µl/well of TBT-1%, followed by the plate centrifugation (500xg for 3 min at 4°C) and buffer removal by aspiration. Dispensing and aspiration is performed using a BioTek Elx405 automated plate washer (Agilent, USA). PAS-PGS pellets are then transferred into the corresponding well of a 96-well OptiPlates^{™} (Perkin-Elmer, USA) using a multichannel liquid handler. OptiPlates^{™} are then centrifuged (500xg for 3 min at 4°C) and excess buffer aspirated using the BioTek Elx405 to leave a final volume of 30µl inside each well. For scLIPS, capture plates are washed with TBT using the BioTek Elx405 performing 10 washes of 300 ml each.

Step 5): 0.5X Nano-Glo^{®} Luciferase Assay Reagent is prepared in advance by bringing at room temperature NanoGlo^{®} Luciferase Assay Buffer (stored frozen at -20° C). To measure the luciferase activity, the required reagents are prepared by combining 0.4 ml of Nano-Glo^{®} Luciferase Assay Substrate (which is a liquid stored at -20°C), 20 ml of Nano-Glo^{®} Luciferase Assay Buffer, and 20 ml of TBT-1% previously equilibrated to RT per well. Using a multichannel dispenser, 40 µl of 0.5X Nano-Glo^{®} substrate is added to each well of the OptiPlates^{™} followed by immediate readout in Centro X960 luminometer using a protocol consisting of shaking for 1 seconds followed by light acquisition for 2 seconds/well. Acquired LU are then converted to arbitrary units (AU) using a standard curve constructed from doubling dilutions in normal human serum of an anti-insulin mAb. AU are calculated based on the difference between LU measured in the "uncompleted" well and the LU measured in the well competed with Actrapid^{™} at 10.5 ng/5 µl (Δ LU). Δ LU are converted to AU using a mixed log10 (for light units) and log2 (for standard concentrations) logarithmic curve fitting algorithm in Excel (Microsoft Corporation).

### Triplex GADA, IA-2A and ZnT8A LIPS and scLIPS assays

The procedure of the triplex GADA, IA-2A and ZnT8A LIPS and scLIPS (3-screen LIPS and 3-screen scLIPS, respectively) assays is based on the protocol described for cytoplasmic antigens. The major difference consists in the use of a mixture of the three nanoluciferase-tagged recombinant antigens which was optimised in terms of luciferase activity content.

Briefly, the 3-screen assay consists of the following steps:
Step 1): a single-use antigen aliquots of recombinant GAD, IA-2, and ZnT8 nanoluciferase antigens are thawed at room temperature and diluted in Tris-buffered saline pH 7.4 with 0.5% Tween 20 (TBST-0.5%) to achieve a concentration for each antigen of 2.0×10⁶ LU/25 µl (±200,000 LU). Antigens are then combined to a yield a cumulative luciferase activity of 6.0×10⁶ LU/25 µl (±200,000 LU) and filtered through a PVDF 0.45µM Millex-SV syringe filter (Merck, Darmstadt, Germany). The total volume of diluted antigen to be prepared is determined by the number of samples to be tested.

Step 2): for each sample to be tested and for standards included in the assay run, 1µl of serum is pipetted into a well of a 96-deep well polystyrene for LIPS (Sarstedt, Nümbrecht, Germany) or to a polypropylene 96-well plate for scLIPS (Sarstedt, Nümbrecht, Germany). Each well is then added with 25µl of diluted nanoluciferase-tagged recombinant antigen, briefly mixed on a plate mixer, and incubated for 2 hr at room temperature protected from light.

Step 3): For LIPS, rProtein A Sepharose 4 fast flow (PAS) (GE Healthcare Life Sciences, Amersham, UK) is used to capture immune complexes. A PAS 50% stock slurry in EtOH 20 is prepared by blocking with glycine³⁴ as previously described. Before use in LIPS assays, 5 µl/well of PAS is washed four times in TBST and diluted in TBST to a final volume of 50 µl/well and then added to each well of the incubation plates. Plates are then incubated for 1hr with orbital shaking (^{~}700 rpm) at 4°C. For the scLIPS assay the reaction mixture is transferred to 96-well plates (NUNC^{™} MaxiSORP, Thermo-Fisher, USA) pre-coated with 30 mg/well of recombinant Protein A (Sino Biological, China) in carbonate buffer pH9 for 4 hours at room temperature and then blocked with freshly prepared 1,5% Fish Gelatin in PBS-HS 1X plus 0,1% Tween-20 pH9 for 18 hours at 4°C (can be prepared in advance and stored emptied frozen at -20°C). Alternatively, commercial plates can be used coated with recombinant protein A/G and blocked with BSA (BioMat s.r.l., Italia). Capture plates are then incubated for 1hr with orbital shaking (^{~}700 rpm) at 4°C.

Step 4): For LIPS, unbound nanoluciferase antigen is removed by 5 serial washes each consisting in the addition of 750 µl/well of TBST, followed by the plate centrifugation (500xg for 3 min at 4°C) and buffer removal by aspiration. Dispensing and aspiration is performed using a BioTek Elx405 automated plate washer (Agilent, USA). PAS pellets are then transferred into the corresponding well of a 96-well OptiPlates^{™} (Perkin-Elmer, USA) using a multichannel liquid handler. OptiPlates^{™} are then centrifuged (500xg for 3 min at 4°C) and excess buffer aspirated using the BioTek Elx405 to leave a final volume of 30 µl inside each well. For scLIPS, capture plates are washed with TBT using the BioTek Elx405 performing 10 washes of 300 ml each.

Step 5): 0.5X Nano-Glo^{®} Luciferase Assay Reagent is prepared in advance by bringing at room temperature NanoGlo^{®} Luciferase Assay Buffer (stored frozen at -20° C). To measure the luciferase activity, the required reagents are prepared by combining 0.4 ml of Nano-Glo^{®} Luciferase Assay Substrate (which is a liquid stored at -20°C), 20 ml of Nano-Glo^{®} Luciferase Assay Buffer, and 20 ml of TBST previously equilibrated to RT per well. Using a multichannel dispenser, 40 µl of 0.5X Nano-Glo^{®} substrate is added to each well of the OptiPlates^{™} followed by immediate readout in Centro X960 luminometer using a protocol consisting of shaking for 1 seconds followed by light acquisition for 2 seconds/well. Acquired LU are then converted to arbitrary units (AU) using a standard curve constructed from doubling dilutions in normal human serum of a multiple GADA, IA-2A and ZnT8A Ab positive serum. AU are calculated based on the measured LU, using a mixed log10 (for light units) and log2 (for standard concentrations) logarithmic curve fitting algorithm in Excel (Microsoft Corporation).

### RESULTS

### Example 1: Stable cell lines expressing nanoluciferase tagged T1D autoantigens.

First, the recombinant antigens according to the invention were used to generate stably expressing transfectant cell lines that facilitate cheaper and larger scale production recombinant antigens. Production of recombinant antigens by transient transfection requires dedicated and costly reagents and is inherently prone to variability. While the production by transient transfection is relatively large for the need of a single laboratory, its scale-up would be intrinsically expensive and the required expertise less easily transferrable to interested parties. A schematic overview of the recombinant antigen molecules expressed in the stable cell lines according to the invention is displayed in Figure 7. The sequences of the recombinant antigen molecules expressed in stable cell lines are displayed in Table 1.

### Example 2: Preliminary validation of the performance of antigens and assays in IASP interlaboratory comparison studies

The antigens produced by the stable cell lines according to the invention have been validated by participating in the Islet Autoantibody Standardization Program workshop in 2023. The IASP workshop is an international interlaboratory assay performance comparison study in which a set of T1D new onset patients, blood donor controls and selected standards are distributed in blind to participants. Each laboratory is free to test the sera in blind using antigens and assay format of choice but must report qualitative and quantitative results to the IASP committee before obtaining the decode. The IASP committee proceeds to a centralized, unbiased assay performance evaluation that is then shared with participants. Among the metrics evaluated for each T1D autoantibody in IASP are sensitivity (the proportion of T1D new onset sera identified as positive) and specificity (the proportion of blood donor sera identified as negative) from scores assigned to the sera by each laboratory based on locally established thresholds; receiver operator curve area under the curve (ROC-AUC) a threshold independent analysis that determines the assay probability of score correctly each tested sample as a case or a control; concordance of positive and negative scores between assays.

### Preliminary validation of recombinant luciferase tagged GAD65 antigen molecule.

The recombinant luciferase tagged GAD65 antigen molecule according to the invention was used in a LIPS assay or scLIPS assay and the results for sensitivity and specificity compared to several other assays measuring GADA (Figure 10). The median assays sensitivity of these GADA LIPS assays was 82%, slightly below the overall median of all assays submitted to IASP2023 (84%) while their median assay specificity was 98% against a median of 99%. The GADA LIPS assay performed with the recombinant luciferase tagged GAD65 antigen molecule according to the invention showed a sensitivity of 86% and a specificity of 97% compared to an overall median sensitivity of 84% and a median assay specificity of 99% amongst all assays submitted to IASP2023 (Figure 10 B and G). The recombinant luciferase tagged GAD65 antigen molecule according to the invention was also used in a scLIPS assay and the results for sensitivity and specificity compared to several other assays measuring GADA (Figure 10 D-F and G-I).

The pAUC95 analysis of GADA assays performed with a recombinant luciferase tagged GAD65 antigen molecule according to the invention had median pAUC95 of 0.042 and above the median of all assays (0.041). Similar to some other non-radioactive assays, LIPS and scLIPS assays proved to be better than the previous gold standard RBA assays (Figure 10 C and H).

The analysis of GADA positive or negative scores assigned to serum samples by the laboratories shows a substantial concordance with those of laboratories with high overall sensitivity, specificity and pAUC95 (Figure 10 I). Discrepancies were few and limited to samples with particularly low levels of GADA.

### Preliminary validation of recombinant luciferase tagged IA-2 antigen molecule.

The recombinant luciferase tagged IA-2 antigen molecule according to the invention was used in a LIPS or scLIPS assay and the results for sensitivity and specificity compared to several other assays measuring IA-2A (Figure 11). Based on laboratory assigned scores, LIPS assays showed high median sensitivity (83% vs an overall median of 74%) and variable specificity (97% vs and overall median of a 100%). The LIPS assay performed with a recombinant luciferase tagged IA-2 antigen molecule according to the invention showed a sensitivity of 78% and a specificity of 100% (Figure 11 B and G).

The recombinant luciferase tagged IA-2 antigen molecule according to the invention was used in a scLIPS assay and the results for sensitivity and specificity compared to several other assays measuring IA-2A (Figure 11 D-F and G-I).

The high performance of the LIPS and scLIPS assays performed with a recombinant luciferase tagged GAD65 antigen molecule according to the invention was confirmed by the threshold independent analysis of the pAUC95 which also was among the highest of all assays participating in IASP2023 (Figure 11 C and H).

The analysis of IA-2A positive or negative scores assigned to serum samples by the laboratories show a substantial concordance with those of laboratories with high overall sensitivity, specificity and pAUC95 (Figure 11 I). Discrepancies were few and limited to samples with particularly low levels of IA-2A.

### Preliminary validation of recombinant luciferase tagged ZnT8 antigen molecule.

The recombinant luciferase tagged ZnT8antigen molecule according to the invention was used in a LIPS or scLIPS assay and the results for sensitivity and specificity compared to several other assays measuring ZnT8A (Figure 12). Based on laboratory assigned scores LIPS assays showed high median sensitivity (83% vs an overall median of 74%) and variable specificity (97% vs and overall median of a 100%). LIPS assays performed with a recombinant luciferase tagged ZnT8 antigen molecule according to the invention showed a sensitivity of 78% and a specificity of 100% (Figure 12 B and G).

The recombinant luciferase tagged ZnT8antigen molecule according to the invention was used in a scLIPS assay and the results for sensitivity and specificity compared to several other assays measuring ZnT8A (Figure 15 A-C and Figure 12 D-F and G-I).

The high performance of the LIPS and scLIPS assays according to the invention was confirmed by the threshold independent analysis of the pAUC95 which also was among the highest of all assays participating in IASP2023 (Figure 12 C and H).

The analysis of ZnT8A positive or negative scores assigned to serum samples by the laboratories show a substantial concordance with those of laboratories with high overall sensitivity, specificity and pAUC95 (Figure 12 I). Discrepancies were few and limited to samples with particularly low levels of ZnT8A.

### Preliminary validation of recombinant luciferase tagged Insulin antigen molecule

The recombinant luciferase tagged insulin antigen molecule according to the invention was used in a LIPS or scLIPS assay and the results for sensitivity and specificity compared to several other assays measuring IAA (Figure 13). Based on laboratory assigned scores LIPS assays showed high median sensitivity (60% vs an overall median of 56%) and good specificity (100% vs and overall median of a 100%). LIPS assays performed with a recombinant luciferase tagged IAA antigen molecule according to the invention showed a sensitivity of 54% and a specificity of 100% (Figure 13 B and G).

The recombinant luciferase tagged insulin antigen molecule according to the invention was used in a LIPS assay and the results for sensitivity and specificity compared to several other assays measuring IAA (Figure 13 D-F and G-I).

The high performance of the LIPS and scLIPS assays according to the invention was confirmed by the threshold independent analysis of the pAUC95 which also was among the highest of all assays participating in IASP2023 (Figure 13 C and H).

The analysis of IAA positive or negative scores assigned to serum samples by the laboratories show a substantial concordance with those of laboratories with high overall sensitivity, specificity and pAUC95 (Figure 13 I). Discrepancies were few and limited to samples with particularly low levels of IAA.

### Example 3: 3-screen LIPS performance according to the invention

Population screening programs for T1D autoantibodies require the testing of a very large number of individuals. Since the evaluation of T1D risk requires the gathering of information for more than one autoantibody, strategies and assays have been developed that aim at minimizing the number of tests to be conducted. Currently, the use of so called 3-screen assays³⁵ is the approach implemented in ongoing autoantibody screenings. The strategy consists in using an initial 3-screen test in which several antigens are mixed and yield a "generic" information on the presence of autoantibodies. According to the invention, the inventors developed a 3-screen LIPS assay for the combined measurement of autoantibodies to GAD65, IA-2, and ZnT8.

The combined detection of three autoantibodies in the 3-screen LIPS and scLIPS assays according to the invention achieved high sensitivity and specificity that is comparable to standard assay formats (Figures 14 to 17). The multiplexed LIPS performances were optimized by varying the antigen input assay. Different assays were performed where different amounts of each GAD, IA-2 and ZnT8 antigens were multiplexed: 4 million light units equivalents of each antigen i.e., like in single LIPS assays, and 2 million light units equivalents of each antigen, respectively (Figures 14 and 15). The optimized antigen input in multiplexed LIPS and scLIPS assay lead to increased performances in discriminating case from control samples tested in blind (Figures 16 and 17).

The advantages of the present invention are manyfold: luciferase-tagged antigens are safe and can be produced in-house with potentially long half-lives, giving greater control over label variability and eliminating reliance on radiolabels. The protocol can be completed within one working day and has the lowest serum requirement of all the widely available tests. This is critical for high-throughput testing of low-volume samples, for instance capillary blood collection for general population screening. The LIPS and scLIPS assays for the combined measurements of autoantibodies according to the invention is particularly suited for large-scale population screening purposes in order to detect up to three key beta-cell specific autoantibodies simultaneously. Further analyses using individual antibody assay formats can subsequently be performed to validate and retest solely the samples found to be positives. Single autoantibody tests can then provide confirmation of the initial result and information on the number and type of positive autoantibodies.

### INDUSTRIAL APPLICABILITY

The recombinant antigen molecules, nucleic acids, vectors, cells, kits, and methods according to the invention are industrially applicable.

### REFERENCES

1 Ingeborg Waernbaum et al., "The Incidence of Childhood-Onset Type 1 Diabetes, Time Trends and Association with the Population Composition in Sweden: A 40 Year Follow-Up," Diabetologia, October 20, 2022, https://doi.org/10.1007/s00125-022-05816-0.
2 William E Winter and David Pittman, "The Clinical Application of Islet Autoantibody Testing for the Diagnosis of Autoimmune Diabetes.," MLO: Medical Laboratory Observer 45, no. 10 (October 2013): 16, 20, 22 passim, http://www.ncbi.nlm.nih.gov/pubmed/24294692.
3 Ezio Bonifacio, "Predicting Type 1 Diabetes Using Biomarkers.," Diabetes Care 38, no. 6 (June 2015): 989-96, https://doi.org/10.2337/dc15-0101.
4 Muhammad Ahmad Mujtaba et al., "Re-Exposure to Beta Cell Autoantigens in Pancreatic Allograft Recipients with Preexisting Beta Cell Autoantibodies.," Clinical Transplantation 29, no. 11 (November 2015): 991-96, https://doi.org/10.1111/ctr.12619.
5 Lorenzo Piemonti et al., "Alloantibody and Autoantibody Monitoring Predicts Islet Transplantation Outcome in Human Type 1 Diabetes.," Diabetes 62, no. 5 (May 28, 2013): 1656-64, https://doi.org/10.2337/db12-1258.
6 Mikael Knip et al., "Hydrolyzed Infant Formula and Early β-Cell Autoimmunity: A Randomized Clinical Trial.," JAMA : The Journal of the American Medical Association 311, no. Stenbäckinkatu 11 (2014): 2279-87, https://doi.org/10.1001/jama.2014.5610.
7 Emily K. Sims et al., "Screening for Type 1 Diabetes in the General Population: A Status Report and Perspective," Diabetes 71, no. 4 (April 1, 2022): 610-23, https://doi.org/10.2337/dbi20-0054.
8 Sandra Hummel et al., "Children Diagnosed with Presymptomatic Type 1 Diabetes through Public Health Screening Have Milder Diabetes at Clinical Manifestation," Diabetologia 66, no. 9 (September 2023): 1633-42, https://doi.org/10.1007/s00125-023-05953-0.
9 Seth A. Sharp et al., "Development and Standardization of an Improved Type 1 Diabetes Genetic Risk Score for Use in Newborn Screening and Incident Diagnosis," Diabetes Care 42, no. 2 (February 1, 2019): 200-207, https://doi.org/10.2337/dc18-1785.
10 Christiane Winkler et al., "Identification of Infants with Increased Type 1 Diabetes Genetic Risk for Enrollment into Primary Prevention Trials-GPPAD-02 Study Design and First Results," Pediatric Diabetes, June 13, 2019, pedi.12870, https://doi.org/10.1111/pedi.12870.
11 He L, Jia X, Rasmussen CG, Waugh K, Miao D, Dong F, et al. High-throughput multiplex electrochemiluminescence assay applicable to general population screening for type 1 diabetes and celiac disease. Diabetes Technol Ther 2022, 24, 502-9. doi:10.1089/dia.2021.0517
12 Marie Amoroso et al., "3 Screen Islet Cell Autoantibody ELISA: A Sensitive and Specific ELISA for the Combined Measurement of Autoantibodies to GAD 65 , to IA-2 and to ZnT8," Clinica Chimica Acta 462 (November 2016): 60-64, https://doi.org/10.1016/j.cca.2016.08.013.
13 Seftel D, et al. Multiplex agglutination-PCR (ADAP) autoantibody assays compared to radiobinding autoantibodies in type 1 diabetes and celiac disease. J Immunol Methods 2022, 506, 113265. doi:10.1016/j.jim.2022.113265
14 Edwin Liu and George S. Eisenbarth, "Accepting Clocks That Tell Time Poorly: Fluid-Phase versus Standard ELISA Autoantibody Assays.," Clinical Immunology (Orlando, Fla.) 125, no. 2 (November 2007): 120-26, https://doi.org/10.1016/j.clim.2007.08.005.
15 Gareth Dunseath et al., "Bridging-Type Enzyme-Linked Immunoassay for Zinc Transporter 8 Autoantibody Measurements in Adult Patients with Diabetes Mellitus.," Clinica Chimica Acta; International Journal of Clinical Chemistry 447 (July 20, 2015): 90-95, https://doi.org/10.1016/j.cca.2015.05.010.
16 Helen Brooking et al., "A Sensitive Non-Isotopic Assay for GAD65 Autoantibodies," Clinica Chimica Acta 331, no. 1-2 (May 2003): 55-59, https://doi.org/10.1016/S0009-8981(03)00088-3; Shu Chen et al., "Sensitive Non-Isotopic Assays for Autoantibodies to IA-2 and to a Combination of Both IA-2 and GAD65," Clinica Chimica Acta 357, no. 1 (July 2005): 74-83, https://doi.org/10.1016/j.cccn.2005.02.006.
17 C J Greenbaum et al., "Insulin Autoantibodies Measured by Radioimmunoassay Methodology Are More Related to Insulin-Dependent Diabetes Mellitus than Those Measured by Enzyme-Linked Immunosorbent Assay: Results of the Fourth International Workshop on the Standardization of Insuli," The Journal of Clinical Endocrinology and Metabolism 74, no. 5 (May 1992): 1040-44, https://doi.org/10.1210/jcem.74.5.1569152.
18 Zhiyuan Zhao et al., "A Multiplex Assay Combining Insulin, GAD, IA-2 and Transglutaminase Autoantibodies to Facilitate Screening for Pre-Type 1 Diabetes and Celiac Disease.," Journal of Immunological Methods 430 (March 2016): 28-32, https://doi.org/10.1016/j.j im.2016.01.011.
19 Dongmei Miao et al., "GAD65 Autoantibodies Detected by Electrochemiluminescence Assay Identify High Risk for Type 1 Diabetes.," Diabetes 62, no. 12 (December 2013): 4174-78, https://doi.org/10.2337/db13-0534.
20 Liping Yu et al., "Distinguishing Persistent Insulin Autoantibodies with Differential Risk: Nonradioactive Bivalent Proinsulin/Insulin Autoantibody Assay.," Diabetes 61, no. 1 (January 2012): 179-86, https://doi.org/10.2337/db11-0670.
21 Felipe de Jesus Cortez et al., "Sensitive Detection of Multiple Islet Autoantibodies in Type 1 Diabetes Using Small Sample Volumes by Agglutination-PCR," PLOS ONE 15, no. 11 (11-13 2020): e0242049, https://doi.org/10.1371/journal.pone.0242049.
22 Peter D Burbelo et al., "Comparison of Radioimmunoprecipitation with Luciferase Immunoprecipitation for Autoantibodies to GAD65 and IA-2beta.," Diabetes Care 33, no. 4 (April 2010): 754-56, https://doi.org/10.2337/dc09-1938.
23 Peter D Burbelo et al., "High Definition Profiling of Autoantibodies to Glutamic Acid Decarboxylases GAD65/GAD67 in Stiff-Person Syndrome," Biochemical and Biophysical Research Communications 366, no. 1 (February 2008): 1-7, https://doi.org/S0006-291X(07)02397-2.
24 Mary P. Hall et al., "Engineered Luciferase Reporter from a Deep Sea Shrimp Utilizing a Novel Imidazopyrazinone Substrate," ACS Chemical Biology 7, no. 11 (2012): 1848-57, https://doi.org/10.1021/cb3002478.
25 Williams, C. L., Marzinotto, I., Brigatti, C., Gillespie, K. M., Lampasona, V., Williams, A. J., & Long, A. E. (2024). A novel, high-performance, low-volume, rapid luciferase immunoprecipitation system (LIPS) assay to detect autoantibodies to zinc transporter 8. Clinical and Experimental Immunology, 215(3), 215-224.
26 Wyatt, R. C., Grace, S. L., Brigatti, C., Marzinotto, I., Gillard, B. T., Shoemark, D. K., ... & Williams, A. J. (2024). Improved Specificity of Glutamate Decarboxylase 65 Autoantibody Measurement Using Luciferase-Based Immunoprecipitation System Assays. Diabetes, 73(4), 565-571.
27 Liberati, D., Wyatt, R. C., Brigatti, C., Marzinotto, I., Ferrari, M., Bazzigaluppi, E., ... & Lampasona, V. (2018). A novel LIPS assay for insulin autoantibodies. Acta diabetologica, 55, 263-270.
28 Alistair J K Williams et al., "Detection of Antibodies Directed to the N-Terminal Region of GAD Is Dependent on Assay Format and Contributes to Differences in the Specificity of GAD Autoantibody Assays for Type 1 Diabetes.," Diabetes 64, no. 9 (September 2015): 3239-46, https://doi.org/10.2337/db14-1693
29 Alistair J K Williams et al., "Reactivity to N-Terminally Truncated GAD65(96-585) Identifies GAD Autoantibodies That Are More Closely Associated With Diabetes Progression in Relatives of Patients With Type 1 Diabetes.," Diabetes 64, no. 9 (September 2015): 3247-52, https://doi.org/10.2337/db14-1694.
30 Bonifacio, Ezio, Vito Lampasona, and Polly J. Bingley. "IA-2 (Islet Cell Antigen 512) Is the Primary Target of Humoral Autoimmunity Against Type 1 Diabetes-Associated Tyrosine Phosphatase Autoantigens." The Journal of Immunology 161, no. 5 (September 1, 1998): 2648-54. https://doi.org/10.4049/jimmunol.161.5.2648.
31 Michela Milani et al., "Phagocytosis-Shielded Lentiviral Vectors Improve Liver Gene Therapy in Nonhuman Primates," Science Translational Medicine 11, no. 493 (May 22, 2019): eaav7325, https://doi.org/10.1126/scitransImed.aav7325.
32 Williams, Alistair J K, Alastair J. Norcross, Kyla A. Chandler, and Polly J. Bingley. "NonSpecific Binding to Protein A Sepharose and Protein G Sepharose in Insulin Autoantibody Assays May Be Reduced by Pre-Treatment with Glycine or Ethanolamine." Journal of Immunological Methods 314, no. 1-2 (July 31, 2006): 170-73. https://doi.org/10.1016/j.jim.2006.06.003.
33 Williams, Alistair J K, Alastair J. Norcross, Kyla A. Chandler, and Polly J. Bingley. "NonSpecific Binding to Protein A Sepharose and Protein G Sepharose in Insulin Autoantibody Assays May Be Reduced by Pre-Treatment with Glycine or Ethanolamine." Journal of Immunological Methods 314, no. 1-2 (July 31, 2006): 170-73. https://doi.org/10.1016/j.jim.2006.06.003.
34 Williams, Alistair J K, Alastair J. Norcross, Kyla A. Chandler, and Polly J. Bingley. "NonSpecific Binding to Protein A Sepharose and Protein G Sepharose in Insulin Autoantibody Assays May Be Reduced by Pre-Treatment with Glycine or Ethanolamine." Journal of Immunological Methods 314, no. 1-2 (July 31, 2006): 170-73. https://doi.org/10.1016/j.jim.2006.06.003.

## Claims

1. A test kit for performing an assay, the test kit comprising:
a) a set of at least three recombinant antigen molecules in a single composition, wherein the recombinant antigen molecules are selected from the group comprising or consisting of recombinant human Glutamate decarboxylase 65, recombinant human islet antigen 2, recombinant human Zinc transporter 8, recombinant human insulin, and recombinant human proinsulin, wherein each of the recombinant antigen molecules is fused to a bioluminescent reporter protein; and
b) a substrate for the bioluminescent reporter protein.

2. The test kit according to claim 1, wherein the bioluminescent reporter protein is a nanoluciferase reporter protein, optionally a Nanoluc luciferase (NLuc) reporter protein, further optionally wherein the Nanoluc luciferase (NLuc) reporter protein comprises a nucleotide sequence at least 85% identical to the nucleotide sequence encoded by SEQ ID NO: 10, at least 90% identical to the nucleotide sequence encoded by SEQ ID NO: 10, at least 95% identical to the nucleotide sequence encoded by SEQ ID NO: 10, at least 98% identical to the nucleotide sequence encoded by SEQ ID NO: 10, at least 99% identical to the nucleotide sequence encoded by SEQ ID NO: 10, identical to the nucleotide sequence encoded by SEQ ID NO:10.

3. The test kit according to claim 1 or 2, wherein the assay comprises a luciferase immune precipitation system (LIPS) assay or a solid phase capture LIPS (scLIPS) assay, wherein the test kit for performing the scLIPS assay further comprises a protein selected from the group comprising or consisting of protein A, protein L, anti IgA, anti IgM, and anti IgG and wherein the protein is immobilized on a surface selected from the group comprising or consisting of a microplate, a particle, and resin, optionally wherein the particle is preferably a Sepharose bead, an agarose bead, or a paramagnetic particle.

4. The test kit according to any one of claims 1 to 3, wherein recombinant human Glutamate decarboxylase 65, recombinant human islet antigen 2, and recombinant human Zinc transporter 8 are each diluted to a concentration of about 2.0×10⁶ LU/25 µl (±200,000 LU) and combined to a final concentration of about 6.0×10⁶ LU/25 µl (±200,000 LU), and/or human insulin and recombinant human proinsulin are each diluted to a concentration of about 10.0×10⁶ LU/5 µl (±200,000 LU).

5. The test kit according to any one of claims 1 to 4, wherein the test kit further comprises at least three single compositions, wherein each single composition comprises a recombinant antigen molecule selected from the group comprising or consisting of recombinant human insulin, recombinant human proinsulin, recombinant human Glutamate decarboxylase 65, recombinant human islet antigen 2, and recombinant human Zinc transporter 8, wherein the recombinant antigen molecule is fused to a bioluminescent reporter protein, optionally the bioluminescent reporter protein according to claim 2,
optionally wherein the recombinant human Glutamate decarboxylase 65, recombinant human islet antigen 2, and recombinant human Zinc transporter 8 are each diluted to a final concentration of about 4.0×10⁶ LU/25µl (±200,000 LU) and the recombinant human insulin and recombinant human proinsulin are each diluted to a final concentration of about 10.0×10⁶ LU/5 µl (±200,000 LU) when present in at least three single compositions.

6. The test kit according to any one of claims 1 to 5, wherein
a) the recombinant human insulin antigen molecule comprises a nucleotide sequence at least 85% identical to a nucleotide sequence selected from a group consisting of SEQ ID NOs: 1, and 11 to 46, at least 90% identical to a nucleotide sequence selected from a group consisting of SEQ ID NOs: 1, and 11 to 46, at least 95% identical to a nucleotide sequence selected from a group consisting of SEQ ID NOs: 1, and 11 to 46, at least 98% identical to a nucleotide sequence selected from a group consisting of SEQ ID NOs: 1, and 11 to 46, at least 99% identical to a nucleotide sequence selected from a group consisting of SEQ ID NOs: 1, and 11 to 46, identical to a nucleotide sequence selected from a group consisting of SEQ ID NOs: 1, and 11 to 46, optionally wherein the recombinant human insulin antigen molecule comprises a nucleotide sequence at least 85% identical to the nucleotide sequence encoded by SEQ ID NO: 1, at least 90% identical to the nucleotide sequence encoded by SEQ ID NO: 1, at least 95% identical to the nucleotide sequence encoded by SEQ ID NO: 1, at least 98% identical to the nucleotide sequence encoded by SEQ ID NO: 1, at least 99% identical to the nucleotide sequence encoded by SEQ ID NO: 1, identical to the nucleotide sequence encoded by SEQ ID NO: 1;
b) the recombinant human proinsulin antigen molecule comprises a nucleotide sequence at least 85% identical to a nucleotide sequence selected from a group consisting of SEQ ID NOs: 65 to 87 and 96, at least 90% identical to a nucleotide sequence selected from a group consisting of SEQ ID NOs: 65 to 87 and 96, at least 95% identical to a nucleotide sequence selected from a group consisting of SEQ ID NOs: 65 to 87 and 96, at least 98% identical to a nucleotide sequence selected from a group consisting of SEQ ID NOs: 65 to 87 and 96, at least 99% identical to a nucleotide sequence selected from a group consisting of SEQ ID NOs: 65 to 87 and 96, identical to a nucleotide sequence selected from a group consisting of SEQ ID NOs: 65 to 87 and 96;
c) the recombinant human islet antigen 2 antigen molecule comprises a nucleotide sequence at least 85% identical to a nucleotide sequence selected from a group consisting of SEQ ID NOs: 2 to 4, at least 90% identical to a nucleotide sequence selected from a group consisting of SEQ ID NOs: 2 to 4, at least 95% identical to a nucleotide sequence selected from a group consisting of SEQ ID NOs: 2 to 4, at least 98% identical to a nucleotide sequence selected from a group consisting of SEQ ID NOs: 2 to 4, at least 99% identical to a nucleotide sequence selected from a group consisting of SEQ ID NOs: 2 to 4, identical to a nucleotide sequence selected from a group consisting of SEQ ID NOs: 2 to 4;
d) the recombinant human Zinc transporter 8 antigen molecule comprises a nucleotide sequence at least 85% identical to the nucleotide sequence encoded by SEQ ID NO: 5, at least 90% identical to the nucleotide sequence encoded by SEQ ID NO: 5, at least 95% identical to the nucleotide sequence encoded by SEQ ID NO: 5, at least 98% identical to the nucleotide sequence encoded by SEQ ID NO: 5, at least 99% identical to the nucleotide sequence encoded by SEQ ID NO: 5, identical to the nucleotide sequence encoded by SEQ ID NO: 5; and/or
e) the recombinant human Glutamate decarboxylase 65 antigen molecule comprises a nucleotide sequence at least 85% identical to the nucleotide sequence encoded by SEQ ID NO: 6, at least 90% identical to the nucleotide sequence encoded by SEQ ID NO: 6, at least 95% identical to the nucleotide sequence encoded by SEQ ID NO: 6, at least 98% identical to the nucleotide sequence encoded by SEQ ID NO: 6, at least 99% identical to the nucleotide sequence encoded by SEQ ID NO: 6, identical to the nucleotide sequence encoded by SEQ ID NO: 6.

7. A set of nucleic acids encoding the recombinant antigen molecules of the test kit as defined in any one of claims 1 to 6, optionally wherein each recombinant antigen molecule is encoded by a separate nucleic acid.

8. A vector encoding for:
a. a recombinant human insulin antigen molecule comprising a nucleotide sequence at least 85% identical to a nucleotide sequence selected from the group consisting of SEQ ID NOs: 1, 11 to 46, and 47 to 60, at least 90% identical to a nucleotide sequence selected from the group consisting of SEQ ID NOs: 1, 11 to 46, and 47 to 60, at least 95% identical to a nucleotide sequence selected from the group consisting of SEQ ID NOs: 1, 11 to 46, and 47 to 60, at least 98% identical to a nucleotide sequence selected from the group consisting of SEQ ID NOs: 1, 11 to 46, and 47 to 60, at least 99% identical to a nucleotide sequence selected from the group consisting of SEQ ID NOs: 1, 11 to 46, and 47 to 60, identical to a nucleotide sequence selected from the group consisting of SEQ ID NOs: 1, 11 to 46, and 47 to 60, optionally wherein the recombinant human insulin antigen molecule comprises a nucleotide sequence at least 85% identical to the nucleotide sequence encoded by SEQ ID NO: 1, at least 90% identical to the nucleotide sequence encoded by SEQ ID NO: 1, at least 95% identical to the nucleotide sequence encoded by SEQ ID NO: 1, at least 98% identical to the nucleotide sequence encoded by SEQ ID NO: 1, at least 99% identical to the nucleotide sequence encoded by SEQ ID NO: 1, identical to the nucleotide sequence encoded by SEQ ID NO: 1; or
b. a recombinant human proinsulin antigen molecule comprising a nucleotide sequence at least 85% identical to a nucleotide sequence selected from the group consisting of SEQ ID NOs: 62, 63, 65 to 87, and 88 to 96, at least 90% identical to a nucleotide sequence selected from the group consisting of SEQ ID NOs: 62, 63, 65 to 87, and 88 to 96, at least 95% identical to a nucleotide sequence selected from the group consisting of SEQ ID NOs: 62, 63, 65 to 87, and 88 to 96, at least 98% identical to a nucleotide sequence selected from the group consisting of SEQ ID NOs: 62, 63, 65 to 87, and 88 to 96, at least 99% identical to a nucleotide sequence selected from the group consisting of SEQ ID NOs: 62, 63, 65 to 87, and 88 to 96, identical to a nucleotide sequence selected from the group consisting of SEQ ID NOs: 62, 63, 65 to 87, and 88 to 96; or
c. a recombinant human islet antigen 2 antigen molecule comprising a nucleotide sequence at least 85% identical to a nucleotide sequence selected from the group consisting of SEQ ID NOs: 2 to 4, at least 90% identical to a nucleotide sequence selected from the group consisting of SEQ ID NOs: 2 to 4, at least 95% identical to a nucleotide sequence selected from the group consisting of SEQ ID NOs: 2 to 4, at least 98% identical to a nucleotide sequence selected from the group consisting of SEQ ID NOs: 2 to 4, at least 99% identical to a nucleotide sequence selected from the group consisting of SEQ ID NOs: 2 to 4, identical to a nucleotide sequence selected from the group consisting of SEQ ID NOs: 2 to 4; or
d. a recombinant human Zinc transporter 8 antigen molecule comprising a nucleotide sequence at least 85% identical to the nucleotide sequence encoded by SEQ ID NO: 5, at least 90% identical to the nucleotide sequence encoded by SEQ ID NO: 5, at least 95% identical to the nucleotide sequence encoded by SEQ ID NO: 5, at least 98% identical to the nucleotide sequence encoded by SEQ ID NO: 5, at least 99% identical to the nucleotide sequence encoded by SEQ ID NO: 5, identical to the nucleotide sequence encoded by SEQ ID NO: 5; or
e. a recombinant human Glutamate decarboxylase 65 antigen molecule comprising a nucleotide sequence at least 85% identical to the nucleotide sequence encoded by SEQ ID NO: 6, at least 90% identical to the nucleotide sequence encoded by SEQ ID NO: 6, at least 95% identical to the nucleotide sequence encoded by SEQ ID NO: 6, at least 98% identical to the nucleotide sequence encoded by SEQ ID NO: 6, at least 99% identical to the nucleotide sequence encoded by SEQ ID NO: 6, identical to the nucleotide sequence encoded by SEQ ID NO: 6.

9. A cell comprising the set of nucleic acids, or a nucleic acid as defined in claim 7, or a vector as defined in claim 8 and expressing the recombinant antigen molecules as defined in the test kit of any one of claims 1 to 6, optionally wherein the cell is a stably expressing transfectant cell line, further optionally wherein the transfectant cell line is an Expi-293F cell line.

10. A method for producing the recombinant antigen molecules of the test kit as defined in any one of claims 1 to 6, the method comprising expressing the set of nucleic acids or the nucleic acid or the vector as defined in claims 7 to 8 in a cell as defined in claim 9, and harvesting the recombinant antigen molecules.

11. An *in vitro* method for determining the presence and/or level of at least one autoantibody molecule in a sample from a human subject, wherein the sample is a blood sample, a serum sample, or a plasma sample obtained from a human subject, the method comprising:
a) contacting the sample with the set of at least three recombinant antigen molecules as defined in any one of claims 1 to 4, and 6 to 7, wherein a complex between the recombinant antigen molecule and the autoantibody molecule (referred to as "recombinant antigen molecule-autoantibody complex") is formed;
b) contacting the recombinant antigen molecule-autoantibody complex with a protein as defined in claim 3;
c) removing the unbound recombinant antigen molecules;
d) contacting the bioluminescence reporter protein covalently linked to the recombinant antigen molecule with the substrate; and
e) determining the presence, absence, and/or amount of one or more autoantibody molecules in the sample that binds to one or more recombinant antigen molecules,
wherein the presence, absence, or amount of one or more autoantibody molecules in the sample is determined by measuring the bioluminescence activity of the bioluminescent reporter molecule,
and wherein the at least one autoantibody molecule has specificity to a self-antigen, optionally wherein the at least one autoantibody molecule is an islet autoantibody molecule,
optionally wherein the islet autoantibody molecule has specificity to human insulin, human proinsulin, human Glutamate decarboxylase 65, human islet antigen 2, or human Zinc transporter 8, further optionally wherein the islet autoantibody molecule is selected from the group comprising or consisting of islet autoantibodies IAA (Insulin Auto Antibodies), GADA (Glutamic Acid Decarboxylase Antibodies), IA-2A (Islet Antigen 2 Antibodies), and ZnT8A (Zinc Transporter 8 Antibodies).

12. The *in vitro* method according to claim 11, wherein the method comprises a luciferase immune precipitation system (LIPS) assay and comprises the steps of:
(a) contacting the sample with the set of at least three recombinant antigen molecules as defined in any one of claims 1 to 4, and 6 to 7;
(b) contacting the recombinant antigen molecule-autoantibody complex with a protein as defined in claim 3 immobilized on a Sepharose bead,, an agarose bead, a paramagnetic particle, or resin;
(c) removing the unbound recombinant antigen molecules;
(d) contacting the bioluminescence reporter protein covalently linked to the recombinant antigen molecule with the substrate; and
(e) determining the presence, absence, and/or amount of one or more autoantibody molecules in the sample that binds to one or more recombinant antigen molecules,
wherein the presence, absence, or amount of one or more autoantibody molecules in the sample is determined by measuring the bioluminescence activity of the bioluminescent reporter molecule.

13. The *in vitro* method according to claim 11, wherein the method comprises a solid phase capture luciferase immune precipitation system (scLIPS) assay and comprises the steps of:
(a) contacting the sample with the set of at least three recombinant antigen molecules as defined in any one of claims 1 to 4, and 6 to 7, wherein a recombinant antigen molecule-autoantibody complex is formed;
(b) contacting the recombinant antigen molecule-autoantibody complex with a protein as defined in claim 3;
(c) removing the unbound recombinant antigen molecules;
(d) contacting the bioluminescence reporter protein covalently linked to the recombinant antigen molecule with the substrate; and
(e) determining the presence, absence, and/or amount of one or more autoantibody molecules in the sample that binds to one or more recombinant antigen molecules,
wherein the presence, absence, or amount of one or more autoantibody molecules in the sample is determined by measuring the bioluminescence activity of the bioluminescent reporter molecule.

14. The method according to any one of claims 11 to 13, wherein
a) the sample is contacted with
i. the set of at least three recombinant antigen molecules in the single composition, wherein the single composition comprises recombinant human Glutamate decarboxylase 65, recombinant human islet antigen 2, and recombinant human Zinc transporter 8 as defined in any one of claims 1 to 4, and 6 to 7, and wherein the single composition comprising at least three recombinant antigen molecules does not comprise insulin; and
ii. recombinant human insulin and/or recombinant human proinsulin each in a single composition, as defined in claim 5; and/or
b) subsequent to step e) of any of claims 11 to 13, the sample is contacted with at least three recombinant antigen molecules in at least three single compositions as defined in claim 5.

15. The *in vitro* method according to any one of claims 11 to 14, the method comprising screening a human subject for the development of type 1 diabetes based on the presence, absence, or amount of one or more autoantibody molecules in the sample, wherein the presence, absence, or amount of one or more autoantibody molecules in the sample is determined by comparing the amount of detected autoantibody molecule in the sample with a standard curve for known amounts of said autoantibody molecules, optionally wherein the presence of one or more autoantibody molecules in the sample is indicative of a risk to develop type 1 diabetes.
